(19) **Europäisches Patentamt / European Patent Office / Office européen des brevets**

(11) **EP 4 059 964 A1**

(12) **EUROPEAN PATENT APPLICATION**

(43) Date of publication:
**21.09.2022 Bulletin 2022/38**

(21) Application number: **22156910.6**

(22) Date of filing: **03.04.2008**

(51) International Patent Classification (IPC):
**C07K 16/28** (2006.01)  **C07K 16/30** (2006.01)
**C07K 16/32** (2006.01)  **C07K 16/42** (2006.01)
**C07K 16/46** (2006.01)  **C07K 14/725** (2006.01)
**A61K 39/395** (2006.01)  **A61P 35/00** (2006.01)

(52) Cooperative Patent Classification (CPC):
**C07K 16/2809; A61P 31/00; A61P 31/12;
A61P 31/14; A61P 35/00; A61P 35/02;
A61P 37/00; A61P 37/02; A61P 43/00;
C07K 16/082; C07K 16/109; C07K 16/2803;
C07K 16/2863; C07K 16/2878; C07K 16/2884;**

(Cont.)

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **03.04.2007 EP 07006990
03.04.2007 EP 07006988
13.03.2008 EP 08004741
24.04.2007 US 913668 P**

(62) Document number(s) of the earlier application(s) in accordance with Art. 76 EPC:
**18184899.5 / 3 461 842
12163656.7 / 2 520 590
08735001.3 / 2 155 783**

(71) Applicant: **Amgen Research (Munich) GmbH
81477 München (DE)**

(72) Inventors:
• **KUFER, Peter
81477 Munich (DE)**
• **RAUM, Tobias
81477 Munich (DE)**

• **KLINGER, Matthias
81477 Munich (DE)**
• **RAU, Doris
81477 Munich (DE)**
• **MANGOLD, Susanne
6330 Cham (CH)**
• **KISCHEL, Roman
81477 Munich (DE)**
• **LUTTERBÜSE, Ralf
81477 Munich (DE)**
• **HOFFMANN, Patrick
81477 Munich (DE)**

(74) Representative: **Schiweck Weinzierl Koch
Patentanwälte Partnerschaft mbB
Ganghoferstraße 68 B
80339 München (DE)**

Remarks:
•The complete document including Reference Table(s) and the Sequence Listing(s) can be downloaded from the EPO website
•This application was filed on 16.02.2022 as a divisional application to the application mentioned under INID code 62.

(54) **CROSS-SPECIES-SPECIFIC BINDING DOMAIN**

(57) The present invention relates to a polypeptide comprising a human binding domain capable of binding to an epitope of human and non-chimpanzee primate CD3 (epsilon) chain as well as to a process for the production of the mentioned polypeptide. The invention further relates to nucleic acids encoding for the polypeptide, to vectors comprising the same and to host cells comprising the vector. In another aspect, the invention provides for a pharmaceutical composition comprising the mentioned polypeptide and medical uses of the polypeptide. In a further aspect the invention provides a method for the identification of polypeptides comprising a cross-species specific binding domain capable of binding to an epitope of human and non-chimpanzee primate CD3ε (CD3 epsilon).

EP 4 059 964 A1

**(Cont. next page)**

(52) Cooperative Patent Classification (CPC): (Cont.)
**C07K 16/30; C07K 16/3053; C07K 16/3092;**
**C07K 16/32; C07K 16/4291; G01N 33/566;**
**G01N 33/57492;** A61K 2039/505; C07K 2317/24;
C07K 2317/31; C07K 2317/33; C07K 2317/34;
C07K 2317/56; C07K 2317/565; C07K 2317/622;
C07K 2317/73; C07K 2317/90; C07K 2317/92;
C07K 2317/94; C07K 2319/40; G01N 2500/00

**Description**

The present invention relates to a polypeptide comprising a first human binding domain capable of binding to an epitope of human and non-chimpanzee primate CD3 (epsilon) as well as to a process for the production of the mentioned polypeptide. The invention further relates to nucleic acids encoding for the polypeptide, to vectors comprising the same and to host cells comprising the vector. In another aspect, the invention provides for a pharmaceutical composition comprising the mentioned polypeptide and medical uses of the polypeptide.

T cell recognition is mediated by clonotypically distributed alpha beta and gamma delta T cell receptors (TcR) that interact with the peptide-loaded molecules of the peptide MHC (pMHC) (Davis & Bjorkman, Nature 334 (1988), 395-402). The antigen-specific chains of the TcR do not possess signalling domains but instead are coupled to the conserved multisubunit signaling apparatus CD3 (Call, Cell 111 (2002), 967-979, Alarcon, Immunol. Rev. 191 (2003), 38-46, Malissen Immunol. Rev. 191 (2003), 7-27). The mechanism by which TcR ligation is directly communicated to the signalling apparatus remains a fundamental question in T cell biology (Alarcon, loc. cit.; Davis, Cell 110 (2002), 285-287). It seems clear that sustained T cell responses involve coreceptor engagement, TcR oligomerization, and a higher order arrangement of TcR-pMHC complexes in the immunological synapse (Davis & van der Merwe, Curr. Biol. 11 (2001), R289-R291, Davis, Nat. Immunol. 4 (2003), 217-224). However very early TcR signalling occurs in the absence of these events and may involve a ligand-induced conformational change in CD3 epsilon (Alarcon, loc. cit., Davis (2002), loc. cit., Gil, J. Biol. Chem. 276 (2001), 11174-11179, Gil, Cell 109 (2002), 901-912). The epsilon, gamma, delta and zeta subunits of the signaling complex associate with each other to form a CD3 epsilon-gamma heterodimer, a CD3 epsilon-delta. heterodimer, and a CD3 zeta-zeta homodimer (Call, loc. cit.). Various studies have revealed that the CD3 molecules are important for the proper cell surface expression of the alpha beta TcR and normal T cell development (Berkhout, J. Biol. Chem. 263 (1988), 8528-8536, Wang, J. Exp. Med. 188 (1998), 1375-1380, Kappes, Curr. Opin. Immunol. 7 (1995), 441-447). The solution structure of the ectodomain fragments of the mouse CD3 epsilon gamma heterodimer showed that the epsilon gamma subunits are both C2-set Ig domains that interact with each other to form an unusual side-to-side dimer configuration (Sun, Cell 105 (2001), 913-923). Although the cysteine-rich stalk appears to play an important role in driving CD3 dimerization (Su, loc. cit., Borroto, J. Biol. Chem. 273 (1998), 12807-12816), interaction by means of the extracellular domains of CD3 epsilon and CD3 gamma is sufficient for assembly of these proteins with TcR beta (Manolios, Eur. J. Immunol. 24 (1994), 84-92, Manolios & Li, Immunol. Cell Biol. 73 (1995), 532-536). Although still controversial, the dominant stoichiometry of the TcR most likely comprises one alpha beta TcR, one CD3 epsilon gamma heterodimer, one CD3 epsilon delta heterodimer and one CD3 zeta zeta homodimer (Call, loc. cit.). Given the central role of the human CD3 epsilon gamma heterodimer in the immune response, the crystal structure of this complex bound to the therapeutic antibody OKT3 has recently been elucidated (Kjer-Nielsen, PNAS 101, (2004), 7675-7680).

A number of therapeutic strategies modulate T cell immunity by targeting TcR signaling, particularly the anti-human CD3 monoclonal antibodies (mAbs) that are widely used clinically in immunosuppressive regimes. The CD3-specific mouse mAb OKT3 was the first mAb licensed for use in humans (Sgro, Toxicology 105 (1995), 23-29) and is widely used clinically as an immunosuppressive agent in transplantation (Chatenoud, Clin. Transplant 7 (1993), 422-430, Chatenoud, Nat. Rev. Immunol. 3 (2003), 123-132, Kumar, Transplant. Proc. 30 (1998), 1351-1352), type 1 diabetes (Chatenoud (2003), loc. cit.), and psoriasis (Utset, J. Rheumatol. 29 (2002), 1907-1913). Moreover, anti-CD3 mAbs can induce partial T cell signalling and clonal anergy (Smith, J. Exp. Med. 185 (1997), 1413-1422). OKT3 has been described in the literature as a potent T cell mitogen (Van Wauve, J. Immunol. 124 (1980), 2708-18) as well as a potent T cell killer (Wong, Transplantation 50 (1990), 683-9). OKT3 exhibits both of these activities in a time-dependent fashion; following early activation of T cells leading to cytokine release, upon further administration OKT3 later blocks all known T cell functions. It is due to this later blocking of T cell function that OKT3 has found such wide application as an immunosuppressant in therapy regimens for reduction or even abolition of allograft tissue rejection.

OKT3 reverses allograft tissue rejection most probably by blocking the function of all T cells, which play a major role in acute rejection. OKT3 reacts with and blocks the function of the CD3 complex in the membrane of human T cells, which is associated with the antigen recognition structure of T cells (TCR) and is essential for signal transduction. Which subunit of the TCR/CD3 is bound by OKT3 has been the subject of multiple studies. Though some evidence has pointed to a specificity of OKT3 for the epsilon-subunit of the TCR/CD3 complex (Tunnacliffe, Int. Immunol. 1 (1989), 546-50; Kjer-Nielsen, PNAS 101, (2004), 7675-7680). Further evidence has shown that OKT3 binding of the TCR/CD3 complex requires other subunits of this complex to be present (Salmeron, J. Immunol. 147 (1991), 3047-52).

Other well known antibodies specific for the CD3 molecule are listed in Tunnacliffe, Int. Immunol. 1 (1989), 546-50. As indicated above, such CD3 specific antibodies are able to induce various T cell responses such as lymphokine production (Von Wussow, J. Immunol. 127 (1981), 1197; Palacious, J. Immunol. 128 (1982), 337), proliferation (Van Wauve, J. Immunol. 124 (1980), 2708-18) and suppressor-T cell induction (Kunicka, in "Lymphocyte Typing II" 1 (1986), 223). That is, depending on the experimental conditions, CD3 specific monoclonal antibody can either inhibit or induce cytotoxicity (Leewenberg, J. Immunol. 134 (1985), 3770; Phillips, J. Immunol. 136 (1986) 1579; Platsoucas, Proc. Natl.

Acad. Sci. USA 78 (1981), 4500; Itoh, Cell. Immunol. 108 (1987), 283-96; Mentzer, J. Immunol. 135 (1985), 34; Landegren, J. Exp. Med. 155 (1982), 1579; Choi (2001), Eur. J. Immunol. 31, 94-106; Xu (2000), Cell Immunol. 200, 16-26; Kimball (1995), Transpl. Immunol. 3, 212-221).

Although many of the CD3 antibodies described in the art have been reported to recognize the CD3 epsilon subunit of the CD3 complex, most of them bind in fact to conformational epitopes and, thus, only recognize CD3 epsilon in the native context of the TCR. Conformational epitopes are characterized by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The conformational epitopes bound by CD3 epsilon antibodies described in the art may be separated in two groups. In the major group, said epitopes are being formed by two CD3 subunits, e.g. of the CD3 epsilon chain and the CD3 gamma or CD3 delta chain. For example, it has been found in several studies that the most widely used CD3 epsilon monoclonal antibodies OKT3, WT31, UCHT1, 7D6 and Leu-4 did not bind to cells singly transfected with the CD3-epsilon chain. However, these antibodies stained cells doubly transfected with a combination of CD3 epsilon plus either CD3 gamma or CD3 delta (Tunnacliffe, loc. cit.; Law, Int. Immunol. 14 (2002), 389-400; Salmeron, J. Immunol. 147 (1991), 3047-52; Coulie, Eur. J. Immunol. 21 (1991), 1703-9). In a second smaller group, the conformational epitope is being formed within the CD3 epsilon subunit itself. A member of this group is for instance mAb APA 1/1 which has been raised against denatured CD3 epsilon (Risueno, Blood 106 (2005), 601-8). Taken together, most of the CD3 epsilon antibodies described in the art recognize conformational epitopes located on two or more subunits of CD3. The discrete amino acid residues forming the three-dimensional structure of these epitopes may hereby be located either on the CD3 epsilon subunit itself or on the CD3 epsilon subunit and other CD3 subunits such as CD3 gamma or CD3 delta.

Another problem with respect to CD3 antibodies is that many CD3 antibodies have been found to be species-specific. Anti-CD3 monoclonal antibodies - as holds generally true for any other monoclonal antibodies - function by way of highly specific recognition of their target molecules. They recognize only a single site, or epitope, on their target CD3 molecule. For example, one of the most widely used and best characterized monoclonal antibodies specific for the CD3 complex is OKT-3. This antibody reacts with chimpanzee CD3 but not with the CD3 homolog of other primates, such as macaques, or with dog CD3 (Sandusky et al., J. Med. Primatol. 15 (1986), 441-451). The anti-CD3 monoclonal antibody UCHT-1 is also reactive with CD3 from chimpanzee but not with CD3 from macaques (own data). On the other hand, there are also examples of monoclonal antibodies, which recognize macaque antigens, but not their human counterparts. One example of this group is monoclonal antibody FN-18 directed to CD3 from macaques (Uda et al., J. Med. Primatol. 30 (2001), 141-147). Interestingly, it has been found that peripheral lymphocytes from about 12% of cynomolgus monkeys lacked reactivity with anti-rhesus monkey CD3 monoclonal antibody (FN-18) due to a polymorphism of the CD3 antigen in macaques. Uda et al. described a substitution of two amino acids in the CD3 sequence of cynomolgus monkeys, which are not reactive with FN-18 antibodies, as compared to CD3 derived from animals, which are reactive with FN-18 antibodies (Uda et al., J Med Primatol. 32 (2003), 105-10; Uda et al., J Med Primatol. 33 (2004), 34-7).

This discriminatory ability, i.e. the species specificity, inherent to CD3 monoclonal antibodies and fragments thereof is a significant impediment to their development as therapeutic agents for the treatment of human diseases. In order to obtain market approval any new candidate medication must pass through rigorous testing. This testing can be subdivided into preclinical and clinical phases: Whereas the latter - further subdivided into the generally known clinical phases I, II and III - is performed in human patients, the former is performed in animals. The aim of pre-clinical testing is to prove that the drug candidate has the desired activity and most importantly is safe. Only when the safety in animals and possible effectiveness of the drug candidate has been established in preclinical testing this drug candidate will be approved for clinical testing in humans by the respective regulatory authority. Drug candidates can be tested for safety in animals in the following three ways, (i) in a relevant species, i.e. a species where the drug candidates can recognize the ortholog antigens, (ii) in a transgenic animal containing the human antigens and (iii) by use of a surrogate for the drug candidate that can bind the ortholog antigens present in the animal. Limitations of transgenic animals are that this technology is typically limited to rodents. Between rodents and man there are significant differences in the physiology and the safety results cannot be easily extrapolated to humans. The limitations of a surrogate for the drug candidate are the different composition of matter compared to the actual drug candidate and often the animals used are rodents with the limitation as discussed above. Therefore, preclinical data generated in rodents are of limited predictive power with respect to the drug candidate. The approach of choice for safety testing is the use of a relevant species, preferably a lower primate. The limitation now of the CD3 binding molecules suitable for therapeutic intervention in man described in the art is that the relevant species are higher primates, in particular chimpanzees. Chimpanzees are considered as endangered species and due to their human-like nature, the use of such animals for drug safety testing has been banned in Europe and is highly restricted elsewhere.

The present invention relates to a polypeptide comprising a, preferably human, binding domain capable of binding to an epitope of human and non-chimpanzee primate CD3ε (epsilon) chain, wherein the epitope is part of an amino acid sequence comprised in the group consisting of SEQ ID NOs. 2, 4, 6, or 8. Sequences as shown in SEQ ID NOs. 2, 4, 6 and 8 and functional fragments thereof are context independent CD3 epitopes.

The advantage of the present invention is the provision of a polypeptide comprising a binding domain exhibiting cross-species specificity to human and non-chimpanzee primate CD3ε (epsilon) chain, which can be used both for preclinical evaluation of safety, activity and/or pharmacokinetic profile of these binding domains in primates and - in the identical form - as drugs in humans. The same molecule can be used in preclinical animal studies as well as in clinical studies in humans. This leads to highly comparable results and a much-increased predictive power of the animal studies compared to species-specific surrogate molecules. In the present invention, an N-terminal 1-27 amino acid residue polypeptide fragment of the extracellular domain of CD3 epsilon was surprisingly identified which - in contrast to all other known epitopes of CD3 epsilon described in the art - maintains its three-dimensional structural integrity when taken out of its native environment in the CD3 complex (and fused to a heterologous amino acid sequence such as EpCAM or an immunoglobulin Fc part).

The context-independence of the CD3 epitope provided in this invention corresponds to the first 27 N-terminal amino acids of CD3 epsilon or functional fragments of this 27 amino acid stretch. The phrase "context-independent," as used herein in relation to the CD3 epitope means that binding of the herein described inventive binding molecules/antibody molecules does not lead to a change or modification of the conformation, sequence, or structure surrounding the antigenic determinant or epitope. In contrast, the CD3 epitope recognized by a conventional CD3 binding molecule (e.g. as disclosed in WO 99/54440 or WO 04/106380) is localized on the CD3 epsilon chain C-terminal to the N-terminal 1-27 amino acids of the context-independent epitope, where it only takes the correct conformation if it is embedded within the rest of the epsilon chain and held in the right position by heterodimerization of the epsilon chain with either the CD3 gamma or delta chain.

Anti-CD3 binding molecules as part of a bispecific binding molecule as provided herein and generated (and directed) against a context-independent CD3 epitope provide for a surprising clinical improvement with regard to T cell redistribution and, thus, a more favourable safety profile. Without being bound by theory, since the CD3 epitope is context-independent, forming an autonomous selfsufficient subdomain without much influence on the rest of the CD3 complex, the CD3 binding molecules provided herein induce less allosteric changes in CD3 conformation than the conventional CD3 binding molecules (like molecules provided in WO 99/54440), which recognize context-dependent CD3 epitopes.

The context-independence of the CD3 epitope of CD3 binding molecules of the invention as part of a bispecific binding molecule is associated with less T cell redistribution during the starting phase of treatment with CD3 binding molecules of the invention resulting in a better safety profile of CD3 binding molecules of the invention compared to conventional CD3 binding molecules known in the art, which recognize context-dependent CD3 epitopes. Particularly, because T cell redistribution during the starting phase of treatment with CD3 binding molecules is a major risk factor for CNS adverse events, the CD3 binding molecules of the invention by recognizing a context-independent rather than a context-dependent CD3 epitope have a substantial safety advantage over the CD3 binding molecules known in the art. Patients with such CNS adverse events related to T cell redistribution during the starting phase of treatment with conventional CD3 binding molecules usually suffer from confusion and disorientation, in some cases also from urinary incontinence. Confusion is a change in mental status in which the patient is not able to think with his or her usual level of clarity. The patient usually has difficulties to concentrate and thinking is not only blurred and unclear but often significantly slowed down. Patients with CNS adverse events related to T cell redistribution during the starting phase of treatment with conventional CD3 binding molecules may also suffer from loss of memory. Frequently, the confusion leads to the loss of ability to recognize people and/or places, or tell time and the date. Feelings of disorientation are common in confusion, and the decision-making ability is impaired. CNS adverse events related to T cell redistribution during the starting phase of treatment with conventional CD3 binding molecules may further comprise blurred speech and/or word finding difficulties. This disorder may impair both, the expression and understanding of language as well as reading and writing. Besides urinary incontinence, also vertigo and dizziness may accompany CNS adverse events related to T cell redistribution during the starting phase of treatment with conventional CD3 binding molecules in some patients.

The maintenance of the three-dimensional structure within the mentioned 27 amino acid N-terminal polypeptide fragment of CD3 epsilon can be used for the generation of, preferably human, binding domains which are capable of binding to the N-terminal CD3 epsilon polypeptide fragment in vitro and to the native (CD3 epsilon subunit of the) CD3 complex on T cells in vivo with the same binding affinity. These data strongly indicate that the N-terminal fragment as described herein forms a tertiary conformation, which is similar to its structure normally existing in vivo. A very sensitive test for the importance of the structural integrity of the amino acid 1-27 of the N-terminal polypeptide fragment of CD3 epsilon was performed. Individual amino acids of amino acids 1-27 of the N-terminal polypeptide fragment of CD3 epsilon were changed to alanine (alanine scanning) to test the sensitivity of the amino acids 1-27 of the N-terminal polypeptide fragment of CD3 epsilon for minor disruptions. CD3 specific antibody molecules as part of a bispecific binding molecule of the invention were used to test for binding to the alanine-mutants of amino acids 1-27 of the N-terminal polypeptide fragment of CD3 epsilon (see appended Example 5). Individual exchanges of the first five amino acid residues at the very N-terminal end of the fragment and two of the amino acids at positions 23 and 25 of the amino acids 1-27 of the N-terminal polypeptide fragment of CD3 epsilon were critical for binding of the antibody molecules. The substitution of amino acid residues in the region of position 1-5 comprising the residues Q (Glutamine at position 1), D (Aspartic acid at position 2), G (Glycine at position 3), N (Asparagine at position 4), and E (Glutamic acid at position 5) to Alanine abolished binding of the, preferably human, binding molecules of the invention to said fragment. While, for at least some of the, preferably human, binding molecules of the invention, two amino acid residues at the C-terminus of the mentioned fragment T (Threonine at position 23) and I (Isoleucine at position 25) reduced the binding energy to the, preferably human, binding molecules of the invention.

Unexpectedly, it has been found that the thus isolated, preferably human, binding molecules not only recognize the human N-terminal fragment of CD3 epsilon, but also the corresponding homologous fragments of CD3 epsilon of various primates, including New-World Monkeys (Marmoset, Callithrix jacchus; Saguinus oedipus; Saimiri sciureus) and Old-World Monkeys (Macaca fascicularis, also known as Cynomolgus Monkey; or Macaca mulatta, also known as Rhesus Monkey). Thus, multi-primate specificity of the CD3-binding molecules of the invention was detected. The following sequence analyses confirmed that human and primates share a highly homologous sequence stretch at the N-terminus of the extracellular domain of CD3 epsilon.

It has been found in the present invention that it is possible to generate, preferably human, binding molecules specific for CD3 epsilon wherein the identical molecule can be used in preclinical animal testing, as well as clinical studies and even in therapy in human. This is due to the unexpected identification of, preferably human, binding molecules, which, in addition to binding to human CD3 epsilon (and due to genetic similarity likely to the chimpanzee counterpart), also bind to the homologs of said antigens of non-chimpanzee primates, including New-World Monkeys and Old-World Monkeys. As shown in the following Examples, said CD3 epsilon specific, preferably human, binding molecules can be integrated into bispecific single chain antibodies in order to generate therapeutics against various diseases, including but not limited to cancer or immunological disorders. Thus, the need to construct a surrogate CD3 epsilon binding domain or a bispecific single chain antibody including the same for testing in a phylogenetic distant (from humans) species disappears. As a result, the very same molecule can be used in animal preclinical testing as is intended to be administered to humans in clinical testing as well as following market approval and therapeutic drug administration. The ability to use the same molecule for preclinical animal testing as in later administration to humans virtually eliminates, or at least greatly reduces, the danger that the data obtained in preclinical animal testing have limited applicability to the human case. In short, obtaining preclinical safety data in animals using the same molecule as will actually be administered to humans does much to ensure the applicability of the data to a human-relevant scenario. In contrast, in conventional approaches using surrogate molecules, said surrogate molecules have to be molecularly adapted to the animal test system used for preclinical safety assessment. Thus, the molecule to be used in human therapy in fact differs in sequence and also likely in structure from the surrogate molecule used in preclinical testing in pharmacokinetic parameters and/or biological activity, with the consequence that data obtained in preclinical animal testing have limited applicability / transferability to the human case. The use of surrogate molecules requires the construction, production, purification and characterization of a completely new construct. This leads to additional development costs and time necessary to obtain that molecule. In sum, surrogates have to be developed separately in addition to the actual drug to be used in human therapy, so that two lines of development for two molecules have to be carried out. Therefore, a major advantage of the human binding molecule or a antibody-based constructs exhibiting cross-species specificity described herein is that the identical molecule can be used for therapeutics in humans and in preclinical animal testing.

It is preferred that binding molecules of the invention capable of binding to an epitope of the human and non-chimpanzee primate CD3 epsilon chain are of human origin. In addition, due to the human origin of the human binding molecules of the invention the generation of an immune reaction against said binding molecules is excluded to the maximum possible extent upon administration of the binding molecules to human patients.

Another major advantage of the, preferably human, CD3 epsilon specific binding molecules as part of a bispecific binding molecule of the invention is their applicability for preclinical testing in various primates. The behavior of a drug candidate in animals should ideally be indicative of the expected behavior of this drug candidate upon administration to humans. As a result, the data obtained from such preclinical testing should therefore generally have a high predictive power for the human case. However, as learned from the tragic outcome of the recent Phase I clinical trial on TGN1412 (a CD28 monoclonal antibody), a drug candidate may act differently in a primate species than in humans: Whereas in preclinical testing of said antibody no or only limited adverse effects have been observed in animal studies performed with cynomolgus monkeys, six human patients developed multiple organ failure upon administration of said antibody (Lancet 368 (2006), 2206-7). The results of these not-desired negative events suggest that it may not be sufficient to limit preclinical testing to only one (primate) species. The fact that the CD3 epsilon specific human binding molecules of the invention bind to a series of New-World and Old-World Monkeys may help to overcome the problems faced in the case mentioned above. Accordingly, the present invention provides means and methods for minimizing species differences in effects when drugs for human therapy are being developed and tested.

With the, preferably human, cross-species specific CD3 epsilon binding domain as part of a bispecific binding molecule of the invention it is also no longer necessary to adapt the test animal to the drug candidate intended for administration to humans, such as e.g. the creation of transgenic animals. The CD3 epsilon specific, preferably human, binding molecules (or bispecific single chain antibodies containing the same), exhibiting cross-species specificity according to the uses and the methods of invention can be directly used for preclinical testing in non-chimpanzee primates, without any genetic manipulation of the animals. As well known to those skilled in the art, approaches in which the test animal is adapted to the drug candidate always bear the risk that the results obtained in the preclinical safety testing are less representative and predictive for humans due to the modification of the animal. For example, in transgenic animals, the proteins encoded by the transgenes are often highly over-expressed. Thus, data obtained for the biological activity of an antibody against this protein antigen may be limited in their predictive value for humans in which the protein is expressed at much lower, more physiological levels.

A further advantage of the uses of the CD3 epsilon specific, preferably human, binding molecules (or bispecific single chain antibodies containing the same) exhibiting cross-species specificity is the fact that chimpanzees as an endangered species are avoided for animal testing. Chimpanzees are the closest relatives to humans and were recently grouped into the family of hominids based on the genome sequencing data (Wildman et al., PNAS 100 (2003), 7181). Therefore, data obtained with chimpanzee is generally considered to be highly predictive for humans. However, due to their status as endangered species, the number of chimpanzees, which can be used for medical experiments, is highly restricted. As stated above, maintenance of chimpanzees for animal testing is therefore both costly and ethically problematic. The uses of CD3 epsilon specific, preferably human, binding molecules of the invention (or bispecific single chain antibodies containing the same) avoids both ethical objections and financial burden during preclinical testing without prejudicing the quality, i.e. applicability, of the animal testing data obtained. In light of this, the uses of CD3 epsilon specific, preferably human, binding molecules (or bispecific single chain antibodies containing the same) provides for a reasonable alternative for studies in chimpanzees.

A further advantage of the CD3 epsilon specific, preferably human, binding molecules of the invention (or bispecific single chain antibodies containing the same) is the ability of extracting multiple blood samples when using it as part of animal preclinical testing, for example in the course of pharmacokinetic animal studies. Multiple blood extractions can be much more readily obtained with a non-chimpanzee primate than with lower animals, e.g. a mouse. The extraction of multiple blood samples allows continuous testing of blood parameters for the determination of the biological effects induced by the, preferably human, binding molecule (or bispecific single chain antibody) of the invention. Furthermore, the extraction of multiple blood samples enables the researcher to evaluate the pharmacokinetic profile of the, preferably human, binding molecule (or bispecific single chain antibody containing the same) as defined herein. In addition, potential side effects, which may be induced by said, preferably human, binding molecule (or bispecific single chain antibody containing the same) reflected in blood parameters can be measured in different blood samples extracted during the course of the administration of said antibody. This allows the determination of the potential toxicity profile of the, preferably human, binding molecule (or bispecific single chain antibody containing the same) as defined herein.

The advantages of the, preferably human, binding molecules (or bispecific single chain antibodies) as defined herein exhibiting cross-species specificity may be briefly summarized as follows:

First, the, preferably human, binding molecules (or bispecific single chain antibodies) as defined herein used in preclinical testing is the same as the one used in human therapy. Thus, it is no longer necessary to develop two independent molecules, which may differ in their pharmacokinetic properties and biological activity. This is highly advantageous in that e.g. the pharmacokinetic results are more directly transferable and applicable to the human setting than e.g. in conventional surrogate approaches.

Second, the uses of the, preferably human, binding molecules (or bispecific single chain antibodies) as defined herein for the preparation of therapeutics in human is less cost- and labor-intensive than surrogate approaches.

Third, the, preferably human, binding molecules (or bispecific single chain antibodies) as defined herein can be used for preclinical testing not only in one primate species, but in a series of different primate species, thereby limiting the risk of potential species differences between primates and human.

Fourth, chimpanzee as an endangered species for animal testing is avoided.

Fifth, multiple blood samples can be extracted for extensive pharmacokinetic studies. Sixth, due to the human origin of the, preferably human, binding molecules according to a preferred embodiment of the invention the generation of an immune reaction against said binding molecules is minimalized when administered to human patients. Induction of an immune response with antibodies specific for a drug candidate derived from a non-human species as e.g. a mouse leading to the development of human-anti-mouse antibodies (HAMAs) against therapeutic molecules of murine origin is excluded.

The term "protein" is well known in the art and describes biological compounds. Proteins comprise one or more amino acid chains (polypeptides), whereby the amino acids are bound among one another via a peptide bond. The term "polypeptide" as used herein describes a group of molecules, which consist of more than 30 amino acids. In accordance with the invention, the group of polypeptides comprises "proteins" as long as the proteins consist of a single polypeptide.

Also in line with the definition the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

As used herein, "human" and "man" refers to the species *Homo sapiens.* As far as the medical uses of the constructs described herein are concerned, human patients are to be treated with the same molecule.

The term "human origin" as used in the context with the molecules of the invention describes molecules derivable from human libraries or having a structure/sequence corresponding to the human equivalent. Accordingly, proteins having an amino acid sequence corresponding to the analog human sequence, e.g. an antibody fragment having an amino acid sequences in the framework corresponding to the human germline sequences, are understood as molecules of human origin.

As used herein, a "non-chimpanzee primate" or "non-chimp primate" or grammatical variants thereof refers to any primate other than chimpanzee, i.e. other than an animal of belonging to the genus *Pan,* and including the species *Pan paniscus* and *Pan troglodytes,* also known as *Anthropopithecus troglodytes* or *Simia satyrus.* A "primate", "primate species", "primates" or grammatical variants thereof denote/s an order of eutherian mammals divided into the two suborders of prosimians and anthropoids and comprising man, apes, monkeys and lemurs. Specifically, "primates" as used herein comprises the suborder *Strepsirrhini* (non-tarsier prosimians), including the infraorder *Lemuriformes* (itself including the superfamilies *Cheirogaleoidea* and *Lemuroidea*), the infraorder *Chiromyiformes* (itself including the family *Daubentoniidae*) and the infraorder *Lorisiformes* (itself including the families *Lorisidae* and *Galagidae*). "Primates" as used herein also comprises the suborder *Haplorrhini,* including the infraorder *Tarsiiformes* (itself including the family *Tarsiidae*), the infraorder *Simiiformes* (itself including the *Platyrrhini,* or New-World monkeys, and the *Catarrhini,* including the *Cercopithecidea,* or Old-World Monkeys).

The non-chimpanzee primate species may be understood within the meaning of the invention to be a lemur, a tarsier, a gibbon, a marmoset (belonging to New-World Monkeys of the family *Cebidae*) or an Old-World Monkey (belonging to the superfamily *Cercopithecoidea*).

As used herein, an "Old-World Monkey" comprises any monkey falling in the superfamily *Cercopithecoidea,* itself subdivided into the families: the *Cercopithecinae,* which are mainly African but include the diverse genus of macaques which are Asian and North African; and the *Colobinae,* which include most of the Asian genera but also the African colobus monkeys.

Specifically, within the subfamily *Cercopithecinae,* an advantageous non-chimpanzee primate may be from the Tribe *Cercopithecini,* within the genus *Allenopithecus* (Allen's Swamp Monkey, *Allenopithecus nigroviridis*); within the genus *Miopithecus* (Angolan Talapoin, *Miopithecus talapoin;* Gabon Talapoin, *Miopithecus ogouensis*); within the genus *Erythrocebus* (Patas Monkey, *Erythrocebus patas*); within the genus *Chlorocebus* (Green Monkey, *Chlorocebus sabaeus;* Grivet, *Chlorocebus aethiops;* Bale Mountains Vervet, *Chlorocebus djamdjamensis;* Tantalus Monkey, *Chlorocebus tantalus;* Vervet Monkey, *Chlorocebus pygerythrus;* Malbrouck, *Chlorocebus cynosuros*); or within the genus *Cercopithecus* (Dryas Monkey or Salongo Monkey, *Cercopithecus dryas;* Diana Monkey, *Cercopithecus diana;* Roloway Monkey, *Cercopithecus roloway,* Greater Spot-nosed Monkey, *Cercopithecus nictitans;* Blue Monkey, *Cercopithecus mitis;* Silver Monkey, *Cercopithecus doggetti;* Golden Monkey, *Cercopithecus kandti;* Sykes's Monkey, *Cercopithecus albogularis;* Mona Monkey, *Cercopithecus mona;* Campbell's Mona Monkey, *Cercopithecus campbelli;* Lowe's Mona Monkey, *Cercopithecus lowei;* Crested Mona Monkey, *Cercopithecus pogonias;* Wolfs Mona Monkey, *Cercopithecus wolfi;* Dent's Mona Monkey, *Cercopithecus denti;* Lesser Spot-nosed Monkey, *Cercopithecus petaurista;* White-throated Guenon, *Cercopithecus erythrogaster,* Sclater's Guenon, *Cercopithecus sclateri;* Red-eared Guenon, *Cercopithecus erythrotis;* Moustached Guenon, *Cercopithecus cephus;* Red-tailed Monkey, *Cercopithecus ascanius;* L'Hoest's Monkey, *Cercopithecus lhoesti;* Preuss's Monkey, *Cercopithecus preussi;* Sun-tailed Monkey, *Cercopithecus solatus;* Hamlyn's Monkey or Owl-faced Monkey, *Cercopithecus hamlyni;* De Brazza's Monkey, *Cercopithecus neglectus*). Alternatively, an advantageous non-chimpanzee primate, also within the subfamily *Cercopithecinae* but within the Tribe *Papionini,* may be from within the genus *Macaca* (Barbary Macaque, *Macaca sylvanus;* Lion-tailed Macaque, *Macaca silenus;* Southern Pig-tailed Macaque or Beruk, *Macaca nemestrina;* Northern Pig-tailed Macaque, *Macaca leonina;* Pagai Island Macaque or Bokkoi, *Macaca pagensis;* Siberut Macaque, *Macaca siberu;* Moor Macaque, *Macaca maura;* Booted Macaque, *Macaca ochreata;* Tonkean Macaque, *Macaca tonkeana;* Heck's Macaque, Macaca *hecki;* Gorontalo Macaque, *Macaca nigriscens;* Celebes Crested Macaque or Black "Ape", *Macaca nigra;* Cynomolgus monkey or Crab-eating Macaque or Long-tailed Macaque or Kera, *Macaca fascicularis;* Stump-tailed Macaque or Bear Macaque, *Macaca arctoides;* Rhesus Macaque, *Macaca mulatta;* Formosan Rock Macaque, *Macaca cyclopis;* Japanese Macaque, *Macaca*

*fuscata*; Toque Macaque, *Macaca sinica*; Bonnet Macaque, *Macaca radiata*; Barbary Macaque, *Macaca sylvanmus*; Assam Macaque, *Macaca assamensis*; Tibetan Macaque or Milne-Edwards' Macaque, *Macaca thibetana*; Arunachal Macaque or Munzala, *Macaca munzala*); within the genus *Lophocebus* (Gray-cheeked Mangabey, *Lophocebus albigena*; *Lophocebus albigena albigena*; *Lophocebus albigena osmani*; *Lophocebus albigena johnstoni*; Black Crested Mangabey, *Lophocebus aterrimus*; Opdenbosch's Mangabey, *Lophocebus opdenboschi*; Highland Mangabey, *Lophocebus kipunji*); within the genus *Papio* (Hamadryas Baboon, *Papio hamadryas*; Guinea Baboon, *Papio papio*; Olive Baboon, *Papio anubis*; Yellow Baboon, *Papio cynocephalus*; Chacma Baboon, *Papio ursinus*); within the genus *Theropithecus* (Gelada, *Theropithecus gelada*); within the genus *Cercocebus* (Sooty Mangabey, *Cercocebus atys*; *Cercocebus atys atys*; *Cercocebus atys lunulatus*; Collared Mangabey, *Cercocebus torquatus;* Agile Mangabey, *Cercocebus agilis;* Golden-bellied Mangabey, *Cercocebus chrysogaster*, Tana River Mangabey, *Cercocebus galeritus*; Sanje Mangabey, *Cercocebus sanjei*); or within the genus *Mandrillus* (Mandrill, *Mandrillus sphinx*; Drill, *Mandrillus leucophaeus*).

Most preferred is *Macaca fascicularis* (also known as Cynomolgus monkey and, therefore, in the Examples named "Cynomolgus") and *Macaca mulatta* (rhesus monkey, named "rhesus").

Within the subfamily *Colobinae,* an advantageous non-chimpanzee primate may be from the African group, within the genus *Colobus* (Black Colobus, *Colobus satanas*; Angola Colobus, *Colobus angolensis*; King Colobus, *Colobus polykomos*; Ursine Colobus, *Colobus vellerosus*; Mantled Guereza, *Colobus guereza*); within the genus *Piliocolobus* (Western Red Colobus, *Piliocolobus badius*; *Piliocolobus badius badius*; *Piliocolobus badius temminckii*; *Piliocolobus badius waldronae*; Pennant's Colobus, *Piliocolobus pennantii*; *Piliocolobus pennantii pennantii*; *Piliocolobus pennantii epieni*; *Piliocolobus pennantii bouvieri*; Preuss's Red Colobus, *Piliocolobus preussi*; Thollon's Red Colobus, *Piliocolobus tholloni*; Central African Red Colobus, *Piliocolobus foai*; *Piliocolobus foai foai*; *Piliocolobus foai ellioti*; *Piliocolobus foai oustaleti*; *Piliocolobus foai semlikiensis*; *Piliocolobus foai parmentierorum*; Ugandan Red Colobus, *Piliocolobus tephrosceles*; Uzyngwa Red Colobus, *Piliocolobus gordonorum*; Zanzibar Red Colobus, *Piliocolobus kirkii*; Tana River Red Colobus, *Piliocolobus rufomitratus*); or within the genus *Procolobus* (Olive Colobus, *Procolobus verus). Within* the subfamily *Colobinae,* an advantageous non-chimpanzee primate may alternatively be from the Langur (leaf monkey) group, within the genus *Semnopithecus* (Nepal Gray Langur, *Semnopithecus schistaceus*; Kashmir Gray Langur, *Semnopithecus ajax*; Tarai Gray Langur, *Semnopithecus hector*, Northern Plains Gray Langur, *Semnopithecus entellus*; Black-footed Gray Langur, *Semnopithecus hypoleucos*; Southern Plains Gray Langur, *Semnopithecus dussumieri*; Tufted Gray Langur, *Semnopithecus priam*); within the *T. vetulus* group or the genus *Trachypithecus* (Purple-faced Langur, *Trachypithecus vetulus*; Nilgiri Langur, *Trachypithecus johnii*); within the *T. cristatus* group of the genus *Trachypithecus* (Javan Lutung, *Trachypithecus auratus*; Silvery Leaf Monkey or Silvery Lutung, *Trachypithecus cristatus*; Indochinese Lutung, *Trachypithecus germaini*; Tenasserim Lutung, *Trachypithecus barbei*); within the *T. obscurus* group of the genus *Trachypithecus* (Dusky Leaf Monkey or Spectacled Leaf Monkey, *Trachypithecus obscurus*; Phayre's Leaf Monkey, *Trachypithecus phayrei*); within the *T. pileatus* group of the genus *Trachypithecus* (Capped Langur, *Trachypithecus pileatus*; Shortridge's Langur, *Trachypithecus shortridgei*; Gee's Golden Langur, *Trachypithecus geei*); within the *T. francoisi* group of the genus *Trachypithecus* (Francois' Langur, *Trachypithecus francoisi*; Hatinh Langur, *Trachypithecus hatinhensis;* White-headed Langur, *Trachypithecus poliocephalus;* Laotian Langur, *Trachypithecus laotum*; Delacour's Langur, *Trachypithecus delacouri*; Indochinese Black Langur, *Trachypithecus ebenus*); or within the genus *Presbytis* (Sumatran Surili, *Presbytis melalophos*; Banded Surili, *Presbytis femoralis*; Sarawak Surili, *Presbytis chrysomelas*; White-thighed Surili, *Presbytis siamensis*; White-fronted Surili, *Presbytis frontata*; Javan Surili, *Presbytis comata*; Thomas's Langur, *Presbytis thomasi*; Hose's Langur, *Presbytis hosei*; Maroon Leaf Monkey, *Presbytis rubicunda*; Mentawai Langur or Joja, *Presbytis potenziani*; Natuna Island Surili, *Presbytis natunae*).

Within the subfamily *Colobinae,* an advantageous non-chimpanzee primate may alternatively be from the Odd-Nosed group, within the genus *Pygathrix* (Red-shanked Douc, *Pygathrix nemaeus*; Black-shanked Douc, *Pygathrix nigripes*; Gray-shanked Douc, *Pygathrix cinerea*); within the genus *Rhinopithecus* (Golden Snub-nosed Monkey, *Rhinopithecus roxellana*; Black Snub-nosed Monkey, *Rhinopithecus bieti*; Gray Snub-nosed Monkey, *Rhinopithecus brelichi*; Tonkin Snub-nosed Langur, *Rhinopithecus avunculus*); within the genus *Nasalis* (Proboscis Monkey, *Nasalis larvatus*); or within the genus *Simias* (Pig-tailed Langur, *Simias concolor*).

As used herein, the term "marmoset" denotes any New-World Monkeys of the genus *Callithrix*, for example belonging to the Atlantic marmosets of subgenus *Callithrix* (sic!) (Common Marmoset, *Callithrix (Callithrix) jacchus*; Black-tufted Marmoset, *Callithrix (Callithrix) penicillata*; Wied's Marmoset, *Callithrix (Callithrix) kuhlii*; White-headed Marmoset, *Callithrix (Callithrix) geoffroyi;* Buffy-headed Marmoset, *Callithrix (Callithrix) flaviceps*; Buffy-tufted Marmoset, *Caliithrix (Callithrix) aurita*); belonging to the Amazonian marmosets of subgenus *Mico* (Rio Acari Marmoset, *Callithrix (Mico) acariensis;* Manicore Marmoset, *Callithrix (Mico) manicorensis;* Silvery Marmoset, *Callithrix (Mico) argentata*; White Marmoset, *Callithrix (Mico) leucippe*; Emilia's Marmoset, *Callithrix (Mico) emiliae*; Black-headed Marmoset, *Callithrix (Mico) nigriceps*; Marca's Marmoset, *Callithrix (Mico)marcai*; Black-tailed Marmoset, *Callithrix (Mico) melanura*; Santarem Marmoset, *Callithrix (Mico) humeralifera*; Maués Marmoset, *Callithrix (Mico) mauesi*; Gold-and-white Marmoset, *Callithrix (Mico) chrysoleuca*; Hershkovitz's Marmoset, *Callithrix (Mico) intermedia*; Satéré Marmoset, *Callithrix (Mico) saterei*); Roosmalens' Dwarf Marmoset belonging to the subgenus Callibella *(Callithrix (Callibella) humilis*); or the Pygmy Marmoset belonging to the subgenus Cebuella (*Callithrix (Cebuella) pygmaea*).

Other genera of the New-World Monkeys comprise tamarins of the genus *Saguinus* (comprising the *S. oedipus*-group, the *S. midas* group, the *S. nigricollis* group, the *S. mystax* group, the *S. bicolor* group and the *S. inustus* group) and squirrel monkeys of the genus *Samiri* (e.g. *Saimiri sciureus, Saimiri oerstedii, Saimiri ustus, Saimiri boliviensis, Saimiri vanzolini*)

The term "binding domain" characterizes in connection with the present invention a domain of a polypeptide which specifically binds/interacts with a given target structure/antigen/epitope. Thus, the binding domain is an "antigen-interaction-site". The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". The term "specifically recognizing" means in accordance with this invention that the antibody molecule is capable of specifically interacting with and/or binding to at least two, preferably at least three, more preferably at least four amino acids of an antigen, e.g. the human CD3 antigen as defined herein. Such binding may be exemplified by the specificity of a "lock-and-key-principle". Thus, specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the antigen-interaction-site with its specific antigen may alternatively result in the initiation of a signal, e.g. due to the induction of a change of the conformation of the antigen, an oligomerization of the antigen, etc. A preferred example of a binding domain in line with the present invention is an antibody. The binding domain may be a monoclonal or polyclonal antibody or derived from a monoclonal or polyclonal antibody.

The term "antibody" comprises derivatives or functional fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also comprises immunoglobulins (Ig's) of different classes (i.e. IgA, IgG, IgM, IgD and IgE) and subclasses (such as IgG1, IgG2 etc.). These antibodies can be used, for example, for the immunoprecipitation, affinity purification and immunolocalization of the polypeptides or fusion proteins of the invention as well as for the monitoring of the presence and amount of such polypeptides, for example, in cultures of recombinant prokaryotes or eukaryotic cells or organisms.

The definition of the term "antibody" also includes embodiments such as chimeric, single chain and humanized antibodies, as well as antibody fragments, like, inter alia, Fab fragments. Antibody fragments or derivatives further comprise F(ab')$_2$, Fv, scFv fragments or single domain antibodies, single variable domain antibodies or immunoglobulin single variable domain comprising merely one variable domain, which might be VH or VL, that specifically bind an antigen or epitope independently of other V regions or domains; see, for example, Harlow and Lane (1988) and (1999), loc. cit. Such immunoglobulin single variable domain encompasses not only an isolated antibody single variable domain polypeptide, but also larger polypeptides that comprise one or more monomers of an antibody single variable domain polypeptide sequence.

Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used.

Examples for such techniques include the hybridoma technique (Kohler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

The term "specific interaction" as used in accordance with the present invention means that the binding (domain) molecule does not or does not significantly cross-react with polypeptides which have similar structure as those bound by the binding molecule, and which might be expressed by the same cells as the polypeptide of interest. Cross-reactivity of a panel of binding molecules under investigation may be tested, for example, by assessing binding of said panel of binding molecules under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999). Examples for the specific interaction of a binding domain with a specific antigen comprise the specificity of a ligand for its receptor. Said definition particularly comprises the interaction of ligands, which induce a signal upon binding to its specific receptor. Examples for said interaction, which is also particularly comprised by said definition, is the interaction of an antigenic determinant (epitope) with the binding domain (antigenic binding site) of an antibody.

The term "cross-species specificity" or "interspecies specificity" as used herein means binding of a binding domain described herein to the same target molecule in humans and non-chimpanzee primates. Thus, "cross-species specificity" or "interspecies specificity" is to be understood as an interspecies reactivity to the same molecule X expressed in different species, but not to a molecule other than X. Cross-species specificity of a monoclonal antibody recognizing e.g. human CD3 epsilon, to a non-chimpanzee primate CD3 epsilon, e.g. macaque CD3 epsilon, can be determined, for instance, by FACS analysis. The FACS analysis is carried out in a way that the respective monoclonal antibody is tested for binding to human and non-chimpanzee primate cells, e.g. macaque cells, expressing said human and non-chimpanzee primate CD3 epsilon antigens, respectively. An appropriate assay is shown in the following examples.

As used herein, CD3 epsilon denotes a molecule expressed as part of the T cell receptor and has the meaning as typically ascribed to it in the prior art. In human, it encompasses in individual or independently combined form all known CD3 subunits, for example CD3 epsilon, CD3 delta, CD3 gamma, CD3 zeta, CD3 alpha and CD3 beta. The non-chimpanzee primate CD3 antigens as referred to herein are, for example, *Macaca fascicularis* CD3 and *Macaca mulatta* CD3. In *Macaca fascicularis,* it encompasses CD3 epsilon FN-18 negative and CD3 epsilon FN-18 positive, CD3 gamma and CD3 delta. In *Macaca mulatta,* it encompasses CD3 epsilon, CD3 gamma and CD3 delta. Preferably, said CD3 as used herein is CD3 epsilon.

The human CD3 epsilon is indicated in GenBank Accession No.NM_000733 and comprises SEQ ID NO.1. The human CD3 gamma is indicated in GenBank Accession NO. NM_000073. The human CD3 delta is indicated in GenBank Accession No. NM_000732.

The CD3 epsilon "FN-18 negative" of *Macaca fascicularis* (i.e. CD3 epsilon not recognized by monoclonal antibody FN-18 due to a polymorphism as set forth above) is indicated in GenBank Accession No. AB073994.

The CD3 epsilon "FN-18 positive" of *Macaca fascicularis* (i.e. CD3 epsilon recognized by monoclonal antibody FN-18) is indicated in GenBank Accession No. AB073993. The CD3 gamma of *Macaca fascicularis* is indicated in GenBank Accession No. AB073992. The CD3 delta of *Macaca fascicularis* is indicated in GenBank Accession No. AB073991.

The nucleic acid sequences and amino acid sequences of the respective CD3 epsilon, gamma and delta homologs of *Macaca mulatta* can be identified and isolated by recombinant techniques described in the art (Sambrook et al. Molecular Cloning: A Laboratory Manual; Cold Spring Harbor Laboratory Press, 3rd edition 2001). This applies mutatis mutandis to the CD3 epsilon, gamma and delta homologs of other non-chimpanzee primates as defined herein. The identification of the amino acid sequence of Callithrix jacchus, Saimiri sciureus und Saguinus oedipus is described in the appended examples. The amino acid sequence of the extracellular domain of the CD3 epsilon of *Callithrix jacchus* is depicted in SEQ ID NO: 3, the one of *Saguinus oedipus* is depicted in SEQ ID NO: 5 and the one of *Saimiri sciureus* is depicted in SEQ ID NO: 7.

In line with the above the term "epitope" defines an antigenic determinant, which is specifically bound/identified by a binding molecule as defined above. The binding domain or molecules may specifically bind to/interact with conformational or continuous epitopes, which are unique for the target structure, e.g. the human and non-chimpanzee primate CD3 epsilon chain. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. Within the present invention, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast, a context-independent CD3 epitope as provided herein refers to an N-terminal 1-27 amino acid residue polypeptide or a functional fragment thereof of CD3 epsilon. This N-terminal 1-27 amino acid residue polypeptide or a functional fragment thereof maintains its three-dimensional structural integrity and correct conformation when taken out of its native environment in the CD3 complex. The context-independency of the N-terminal 1-27 amino acid residue polypeptide or a functional fragment thereof, which is part of the extracellular domain of CD3 epsilon, represents, thus, an epitope which is completely different to the epitopes of CD3 epsilon described in connection with a method for the preparation of human binding molecules in WO 2004/106380. Said method used solely expressed recombinant CD3 epsilon. The conformation of this solely expressed recombinant CD3 epsilon differed from that adopted in its natural form, that is, the form in which the CD3 epsilon subunit of the TCR/CD3 complex exists as part of a noncovalent complex with either the CD3 delta or the CD3-gamma subunit of the TCR/CD3 complex. When such solely expressed recombinant CD3 epsilon protein is used as an antigen for selection of antibodies from an antibody library, antibodies specific for this antigen are identified from the library although such a library does not contain antibodies with specificity for self-antigens/autoantigens. This is due to the fact that solely expressed recombinant CD3 epsilon protein does not exist in vivo; it is not an autoantigen. Consequently, subpopulations of B cells expressing antibodies specific for this protein have not been depleted in vivo; an antibody library constructed from such B cells would contain genetic material for antibodies specific for solely expressed recombinant CD3 epsilon protein.

However, since the context-independent N-terminal 1-27 amino acid residue polypeptide or a functional fragment thereof is an epitope, which folds in its native form, binding domains in line with the present invention cannot be identified by methods based on the approach described in WO 04/106380. Therefore, it could be verified in tests that binding molecules as disclosed in WO 2004/106380 are not capable of binding to the N-terminal 1-27 amino acid residues of the CD3 epsilon chain. Hence, conventional anti-CD3 binding molecules or anti-CD3 antibody molecules (e.g. as disclosed in WO 99/54440) bind CD3 epsilon chain at a position which is more C-terminally located than the context-independent N-terminal 1-27 amino acid residue polypeptide or a functional fragment provided herein. Prior art antibody molecules OKT3 and UCHT-1 have also a specificity for the epsilon-subunit of the TCR/CD3 complex between amino acid residues 35 to 85 and, accordingly, the epitope of these antibodies is also more C-terminally located. In addition, UCHT-1 binds to the CD3 epsilon chain in a region between amino acid residues 43 to 77 (Tunnacliffe, Int. Immunol. 1 (1989), 546-50; Kjer-Nielsen, PNAS 101, (2004), 7675-7680; Salmeron, J. Immunol. 147 (1991), 3047-52). Therefore, prior art anti-CD3 molecules do not bind to and are not directed against the herein defined context-independent N-terminal 1-27 amino acid residue epitope (or a functional fragment thereof).

For the generation of a, preferably human, binding domain comprised in a polypeptide of the invention, e.g. in a bispecific single chain antibody as defined herein, e.g. monoclonal antibodies binding to both the human and non-chimpanzee primate CD3 epsilon (e.g. macaque CD3 epsilon) can be used.

In a preferred embodiment of the polypeptide of the invention, the non-chimpanzee primate is an old world monkey. In a more preferred embodiment of the polypeptide, the old world monkey is a monkey of the Papio genus Macaque genus. Most preferably, the monkey of the Macaque genus is Assamese macaque (Macaca *assamensis*), Barbary macaque (*Macaca sylvanus*), Bonnet macaque (*Macaca radiata*), Booted or Sulawesi-Booted macaque (*Macaca ochreata*), Sulawesi-crested macaque (*Macaca nigra*), Formosan rock macaque (*Macaca cyclopsis*), Japanese snow macaque or Japanese macaque (*Macaca fuscata*), Cynomologus monkey or crab-eating macaque or long-tailed macaque or Java macaque (*Macaca fascicularis*), Lion-tailed macaque (*Macaca silenus*), Pigtailed macaque (*Macaca nemestrina*), Rhesus macaque (*Macaca mulatta*), Tibetan macaque (*Macaca thibetana*), Tonkean macaque (*Macaca tonkeana*), Toque macaque (*Macaca sinica*), Stump-tailed macaque or Red-faced macaque or Bear monkey (*Macaca arctoides*), or Moor macaque (*Macaca maurus*). Most preferably, the monkey of the Papio genus is *Hamadryas Baboon, Papio hamadryas; Guinea Baboon, Papio papio; Olive Baboon, Papio anubis; Yellow Baboon, Papio cynocephalus; Chacma Baboon, Papio ursinus*

In an alternatively preferred embodiment of the polypeptide of the invention, the non-chimpanzee primate is a new world monkey. In a more preferred embodiment of the polypeptide, the new world monkey is a monkey of the Callithrix genus (marmoset), the Saguinus genus or the Samiri genus. Most preferably, the monkey of the Callithrix genus is *Callithrix jacchus,* the monkey of the Saguinus genus is *Saguinus oedipus* and the monkey of the Samiri genus is *Saimiri sciureus.*

As described herein above the polypeptide of the invention binds with the first binding domain to an epitope of human and non-chimpanzee primate CD3ε (epsilon) chain, wherein the epitope is part of an amino acid sequence comprised in the group consisting of 27 amino acid residues as depicted in SEQ ID NOs. 2, 4, 6, or 8 or a functional fragment thereof.

In line with the present invention it is preferred for the polypeptide of the invention that said epitope is part of an amino acid sequence comprising 26, 25, 24, 23, 22, 21, 20, 19, 18, 17, 16, 15, 14, 13, 12, 11, 10, 9, 8, 7, 6 or 5 amino acids.

More preferably, wherein said epitope comprises at least the amino acid sequence Gln-Asp-Gly-Asn-Glu (Q-D-G-N-E-D).

Within the present invention, a functional fragment of the N-terminal 1-27 amino acid residues means that said functional fragment is still a context-independent epitope maintaining its three-dimensional structural integrity when taken out of its native environment in the CD3 complex (and fused to a heterologous amino acid sequence such as EpCAM or an immunoglobulin Fc part, e.g. as shown in Example 3.1). The maintenance of the three-dimensional structure within the 27 amino acid N-terminal polypeptide or functional fragment thereof of CD3 epsilon can be used for the generation of binding domains which bind to the N-terminal CD3 epsilon polypeptide fragment *in vitro* and to the native (CD3 epsilon subunit of the) CD3 complex on T cells *in vivo* with the same binding affinity. Within the present invention, a functional fragment of the N-terminal 1-27 amino acid residues means that CD3 binding molecules provided herein can still bind to such functional fragments in a context-independent manner. The person skilled in the art is aware of methods for epitope mapping to determine which amino acid residues of an epitope are recognized by such anti-CD3 binding molecules (e.g. alanine scanning).

In a preferred embodiment of the invention, the polypeptide of the invention comprises a (first) binding domain capable of binding to an epitope of human and non-chimpanzee primate CD3ε chain and a second binding domain capable of binding to a cell surface antigen.

The term "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located in the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface molecules which are located on the plasma membrane are proteins which have been modified at a cysteine residue to bear a palmitoyl group, proteins modified at a C-terminal cysteine residue to bear a farnesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies. Examples of cell surface antigens include EGFR, EGFRvIII, MCSP, Carbonic anhydrase IX (CAIX), CD30, CD33, Her2/neu, IgE, CD44v6 and Muc-1. Additionally, examples for corresponding cell surface antibodies comprise antigens which are characteristic for a specific disease or ailment, i.e. cancer, autoimmune diseases or infections diseases including viral infections. Accordingly, the term "cell surface antigens" explicitly includes viral proteins such as native, unprocessed viral proteins exposed on the surface of infected cells (described inter alia for envelope proteins of Hepatitis virus B, C and HIV-1).

One defense function of cytotoxic T cells is the destruction of virus-infected cells, therefore, the unique property of the bispecific binding molecules of the invention to activate and redirect cytotoxic T cells irrespecitve of their autochthonous specificity has a great impact on the broad field of chronic virus infections. For the majority of these infections elimination of persistently infected cells is the only chance for cure. Adoptive T cell therapies are currently being developed against chronic CMV and EBV infections (Rooney, C.M., et al., Use of gene-modified virus-specific T lymphocytes to control Epstein-Barr-virus-related lymphoproliferation. Lancet, 1995. 345 (8941): p. 9-13; Walter, E.A., et al., Reconstitution of cellular immunity against cytomegalovirus in recipients of allogeneic bone marrow by transfer of T-cell clones from the donor. N Engl J Med, 1995. 333 (16): p. 1038-44).

Chronic hepatitis B infection is clearly one of the most interesting and rewarding indications. Worldwide between 350 and 400 million people are infected with HBV. Current treatment of chronic HBV hepatitis rests on interferon gamma and nucleosid or nucleotide analogues, a long term therapy with considerable side-effects such as induction of hepatitis flares, fever, myalgias, thrombocytopenia and depression. Although there are now more than 4 approved therapeutic regimens, elimination of the virus is rarely achieved. A persistent inflammation in chronic hepatitis B leads to liver cirrhosis and hepatocellular carcinoma in more than 25% of patients. Moreover, up to 40 % of patients with chronic hepatitis B will will die from serious complications, accounting for 0.6 to 1.0 million deaths per year worldwide

HBV, the prototype of the Hepadnaviruses is an enveloped virus whose relaxed circular (rc) genome is reverse transcribed into an RNA pregenome. After infection the rc DNA is imported into the hepatocyte nucleus where it is completed to a covalently closed circular DNA (cccDNA) containing four overlapping reading frames. It serves as transcription template for the pregenomic RNA and three subgenomic RNAs. The RNA pregenome functions as mRNA for translation of the viral core and polymerase protein. Infected cells produce continuously HBV surface protein (HBsAg) from the cccDNA even when HBV replication is stopped. HBsAg consists of the small surface (S) proteins with very few portions of middle and large (L) surface proteins. Both the HBV S and L are targeted to the Endoplasmatic Reticulum (ER) membrane from where they are transported in membrane vesicles via the trans golgi organelle to the plasma membrane (Gorelick, F.S. and-C. Shugrue, Exiting the endoplasmic reticulum. Mol Cell Endocrinol, 2001. 177 (1-2): p. 13-8). S and L proteins are permanently expressed on the surface of HBV replicating hepatocytes as shown recently (Chu, C.M. and Y.F. Liaw, Membrane staining for hepatitis B surface antigen on hepatocytes: a sensitive and specific marker of active viral replication in hepatitis B. J Clin Pathol, 1995. 48(5): p. 470-3).

Prototype viruses that expose envelope proteins at the cell surface are Hepatitis virus B (HBV), Hepatitis virus C (HCV) and HIV-1 both of which represent an enormous burden of disease globally. For the HIV-1 virus indication, it has recently been shown that T cells modified by a chimeric TCR with an Fv antibody construct directed at the gp120 envelope protein can kill HIV-1 infected target cells (Masiero, S., et al., T-cell engineering by a chimeric T-cell receptor with antibody-type specificity for the HIV-1 gp120. Gene Ther, 2005. 12 (4): p. 299-310). Of the hepadna viruses, hepatitis virus B (HBV) expresses the envelope protein complex HBsAg which is continuously produced from episomal cccDNA even when HBV replication subsides.

The expression as intact S and L HBV proteins on the cell surface makes them accessible for antibodies which are the hallmark of seroconversion when patients recover from the acute phase of infections and change from circulating HBsAg to antiHBs. If seroconversion does not occur, up to 30 % of hepatocytes continue to express HBV S protein also after highly active antiviral therapy of long duration. Thus beyond T lymphocytes recognizing specifically intracellularly processed HBV peptides and presented by MHC molecules at the cell surface other forms of T cell engagement are feasible aimed at intact surface protein such as S and L antigens accessible in the outer cell membrane. Using single chain antibody fragments recognizing hepatitis B virus small (S) and large (L) envelope proteins, artificial T-cell receptors have been generated which allow directing grafted T-cells to infected hepatocytes and upon antigen contact activation of these T-cells to secrete cytokines and kill infected hepatocytes.

The limitation of this approach is, (i) that T-cells need to be manipulated in vitro, (ii) retroviruses used to transfer the T-cell receptors may cause insertional mutagenesis in the T-cells, and (iii) that once T-cells have been transferred the cytotoxic response cannot be limited.

To overcome these limitations, bispecific single chain antibody molecules comprising a first domain with a binding specificity for the human and the non-chimpanzee primate CD3 epsilon antigen (as provided herein in context of this invention) as well as a second domain with a binding specificity for HBV or HCV envelope proteins of infected hepatocytes may be generated and are within the scope of this invention.

Within the present invention it is further preferred that the second binding domain binds to a cell surface antigen of a human and/or a non-chimpanzee primate.

For the generation of the second binding domain of the polypeptide of the invention, e.g. bispecific single chain antibodies as defined herein, monoclonal antibodies binding to both of the respective human and/or non-chimpanzee primate cell surface antigens can be utilized. Appropriate binding domains for the bispecific polypeptide as defined herein e.g. can be derived from cross-species specific monoclonal antibodies by recombinant methods described in the art. A monoclonal antibody binding to a human cell surface antigen and to the homolog of said cell surface antigen in a non-chimpanzee primate can be tested by FACS assays as set forth above. It is evident to those skilled in the art that cross-species specific antibodies can also be generated by hybridoma techniques described in the literature (Milstein and Köhler, Nature 256 (1975), 495-7). For example, mice may be alternately immunized with human and non-chimpanzee primate CD33. From these mice, cross-species specific antibody-producing hybridoma cells are isolated via hybridoma technology and analysed by FACS as set forth above. The generation and analysis of bispecific polypeptides such as bispecific single chain antibodies exhibiting cross-species specificity as described herein is shown in the following examples. The advantages of the bispecific single chain antibodies exhibiting cross-species specificity include the points enumerated below.

It is particularly preferred for the polypeptide of the invention that the first binding domain capable of binding to an epitope of human and non-chimpanzee primate CD3ε chain comprises a VL region comprising CDR-L1, CDR-L2 and CDR-L3 selected from:

(a) CDR-L1 as depicted in SEQ ID NO. 27, CDR-L2 as depicted in SEQ ID NO. 28 and CDR-L3 as depicted in SEQ ID NO. 29;
(b) CDR-L1 as depicted in SEQ ID NO. 117, CDR-L2 as depicted in SEQ ID NO. 118 and CDR-L3 as depicted in SEQ ID NO. 119; and
(c) CDR-L1 as depicted in SEQ ID NO. 153, CDR-L2 as depicted in SEQ ID NO. 154 and CDR-L3 as depicted in SEQ ID NO. 155.

The variable regions, i.e. the variable light chain ("L" or "VL") and the variable heavy chain ("H" or "VH") are understood in the art to provide the binding domain of an antibody. This variable regions harbor the complementary determining regions.

The term "complementary determining region" (CDR) is well known in the art to dictate the antigen specificity of an antibody. The term "CDR-L" or "L CDR" refers to CDRs in the VL, whereas the term "CDR-H" or "H CDR" refers to the CDRs in the VH.

In an alternatively preferred embodiment of the polypeptide of the invention the first binding domain capable of binding to an epitope of human and non-chimpanzee primate CD3ε chain comprises a VH region comprising CDR-H 1, CDR-H2 and CDR-H3 selected from:

(a) CDR-H1 as depicted in SEQ ID NO. 12, CDR-H2 as depicted in SEQ ID NO. 13 and CDR-H3 as depicted in SEQ ID NO. 14;
(b) CDR-H1 as depicted in SEQ ID NO. 30, CDR-H2 as depicted in SEQ ID NO. 31 and CDR-H3 as depicted in SEQ ID NO. 32;
(c) CDR-H1 as depicted in SEQ ID NO. 48, CDR-H2 as depicted in SEQ ID NO. 49 and CDR-H3 as depicted in SEQ ID NO. 50;
(d) CDR-H1 as depicted in SEQ ID NO. 66, CDR-H2 as depicted in SEQ ID NO. 67 and CDR-H3 as depicted in SEQ ID NO. 68;

(e) CDR-H1 as depicted in SEQ ID NO. 84, CDR-H2 as depicted in SEQ ID NO. 85 and CDR-H3 as depicted in SEQ ID NO. 86;

(f) CDR-H1 as depicted in SEQ ID NO. 102, CDR-H2 as depicted in SEQ ID NO. 103 and CDR-H3 as depicted in SEQ ID NO. 104;

(g) CDR-H1 as depicted in SEQ ID NO. 120, CDR-H2 as depicted in SEQ ID NO. 121 and CDR-H3 as depicted in SEQ ID NO. 122;

(h) CDR-H1 as depicted in SEQ ID NO. 138, CDR-H2 as depicted in SEQ ID NO. 139 and CDR-H3 as depicted in SEQ ID NO. 140;

(i) CDR-H1 as depicted in SEQ ID NO. 156, CDR-H2 as depicted in SEQ ID NO. 157 and CDR-H3 as depicted in SEQ ID NO. 158; and

(j) CDR-H1 as depicted in SEQ ID NO. 174, CDR-H2 as depicted in SEQ ID NO. 175 and CDR-H3 as depicted in SEQ ID NO. 176.

It is further preferred that the binding domain capable of binding to an epitope of human and non-chimpanzee primate CD3ε chain comprises a VL region selected from the group consisting of a VL region as depicted in SEQ ID NO. 35, 39, 125, 129, 161 or 165.

It is alternatively preferred that the first binding domain capable of binding to an epitope of human and non-chimpanzee primate CD3ε chain comprises a VH region selected from the group consisting of a VH region as depicted in SEQ ID NO. 15, 19, 33, 37, 51, 55, 69, 73, 87, 91, 105, 109, 123, 127, 141, 145, 159, 163, 177 or 181.

More preferably, the polypeptide of the invention is characterized by the first binding domain capable of binding to an epitope of human and non-chimpanzee primate CD3ε chain, which comprises a VL region and a VH region selected from the group consisting of:

(a) a VL region as depicted in SEQ ID NO. 17 or 21 and a VH region as depicted in SEQ ID NO. 15 or 19;

(b) a VL region as depicted in SEQ ID NO. 35 or 39 and a VH region as depicted in SEQ ID NO. 33 or 37;

(c) a VL region as depicted in SEQ ID NO. 53 or 57 and a VH region as depicted in SEQ ID NO. 51 or 55;

(d) a VL region as depicted in SEQ ID NO. 71 or 75 and a VH region as depicted in SEQ ID NO. 69 or 73;

(e) a VL region as depicted in SEQ ID NO. 89 or 93 and a VH region as depicted in SEQ ID NO. 87 or 91;

(f) a VL region as depicted in SEQ ID NO. 107 or 111 and a VH region as depicted in SEQ ID NO. 105 or 109;

(g) a VL region as depicted in SEQ ID NO. 125 or 129 and a VH region as depicted in SEQ ID NO. 123 or 127;

(h) a VL region as depicted in SEQ ID NO. 143 or 147 and a VH region as depicted in SEQ ID NO. 141 or 145;

(i) a VL region as depicted in SEQ ID NO. 161 or 165 and a VH region as depicted in SEQ ID NO. 159 or 163; and

(j) a VL region as depicted in SEQ ID NO. 179 or 183 and a VH region as depicted in SEQ ID NO. 177 or 181.

According to a preferred embodiment of the polypeptide of the invention the pairs of VH-regions and VL-regions are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence.

A preferred embodiment of the above described polypeptide of the invention is characterized by the first binding domain capable of binding to an epitope of human and non-chimpanzee primate CD3ε chain comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187.

The invention further relates to an above described polypeptide, wherein the second binding domain binds to a cell surface antigen, which is preferably a tumor antigen.

The term "tumor antigen" as used herein may be understood as those antigens that are presented on tumor cells. These antigens can be presented on the cell surface with an extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue. Non-limiting examples of tumor antigens as used herein are EGFR (Liu, Br. J. Cancer 82/12 (2000), 1991-1999; Bonner, Semin. Radiat. Oncol. 12 (2002), 11-20; Kiyota, Oncology 63/1 (2002), 92-98; Kuan, Brain Tumor Pathol. 17/2 (2000), 71-78), EGFRvIII (Kuan, Brain Tumor Pathol. 17/2 (2000), 71-78), Carboanhydrase IX (MN/CA IX) (Uemura, Br. J. Cancer 81/4 (1999), 741-746; Longcaster, Cancer Res. 61/17 (2001), 6394-6399; Chia, J. Clin. Oncol. 19/16 (2001), 3660-3668; Beasley, Cancer Res. 61/13 (2001), 5262-5267), CD33 (Abutalib, Curr Pharm Biotechnol. 7 (2006), 343-69), MCSP (Campoli, Crit Rev Immunol. 24 (2004), 267-96), or IgE (Infuhr, Allergy 60 (2005), 977-85).

It is preferred that the tumor antigen is selected from EGFR, EGFRvIII, MCSP, EpCAM, Carbonic anhydrase IX (CAIX), CD30, CD33, Her2/neu, CD44v6 and Muc-1.

EGFR (also known as c-erb1 or HER1) belongs to the erbB receptor tyrosine kinase family. When activated by binding of a ligand from the EGF family of growth factors, EGFR homodimerizes or heterodimerizes with a second EGFR or another member of the erbB receptor family, respectively, initiating a signaling cascade through mitogen-activated protein kinases and other transcription factors leading to proliferation, differentiation and repair (Olayioye, EMBO J. 19 (2000), 3159-67). EGFR is overexpressed in many epithelial cancers, including colorectal, breast, lung, and head and neck cancers (Mendelsohn, J. Clin. Oncol. 21 (2003), 2787-99; Mendelsohn, J. Clin. Oncol. 20 (18, Suppl.) (2002), 1S-13S; Prewett, Clin. Cancer Res. 8 (2002), 994-1003). Overexpression and/or mutation of EGFR in malignant cells leads to constitutive activation of kinase activity resulting in proliferation, angiogenesis, invasion, metastasis, and inhibition of apoptosis (Mendelsohn (2003, loc. cit.; Ciardiello, Clin. Cancer Res. 7 (2001), 2958-70; Perez-Soler, Oncologist 9 (2004), 58-67). Monoclonal antibodies that target the extracellular ligand binding domain or the intracellular tyrosine kinase signaling cascade of EGFR have been shown efficacy as antitumor target (Laskin, Cancer Treat. Review 30 (2004), 1-17). For example, cetuximab (Erbitux) a humanized monoclonal antibody to EGFR, which competitively inhibits the extracellular domain of EGFR to inhibit ligand activation of the receptor, was approved by the Food and Drug Administration (FDA) in 2004 for the treatment of metastatic colon cancer in combination with the topoisomerase inhibitor irinotecan.

Melanoma-associated cell surface chondroitin sulphate proteoglycane (MCSP), also known as high-molecular weight melanoma-associated antigen (HMW-MAA) or melanoma-associated proteoglycan is a large cell surface proteoglycan of more than 450 kDa comprising a 250 kDa glycoprotein core and glycosaminoglycan chains attached to it (Pluschke, PNAS 93 (1996), 9710; Nishiyama, J. Cell Biol. 114 (1991), 359). The exact function of MCSP is still unknown. It may act as adhesion receptor that mediate cellular interactions with the extracellular matrix (ECM) and are probably implicated in angiogenesis, tissue invasion and cell spreading (Burg, Cancer Res. 59 (1999), 2869; Iida, J. Biol. Chem. 276 (2001), 18786; Eisenmann, Nat. Cell Biol. 1 (1999), 507; Iida, Cancer Res. 55 (1995), 2177; Campoli (2004), loc. cit). MCSP can enhance ligand binding by a4b1 integrin, and MCSP activation can enhance integrin-mediated cell spreading by cdc42- and p130cas-dependent mechanisms. MCSP associates through chondroitin sulphate with MT3-MMP, which can degrade various ECM-proteins. It may specifically localize MT3-MMP to cellular ECM-adhesion sites. Thus, both molecules seem to be required for invasion of type I collagen and degradation of type I gelatine (Iida (2001), loc. cit.). MSCP is a major marker for melanoma, where it is highly expressed on the surface of the tumor cells. It is expressed by at least 90% of melanoma lesions (Natali, J. Natl. Cancer Inst. 72 (1984), 13). Among all melanoma associated antigens, MCSP displays the lowest degree of heterogeneity (Natali, Cancer Res. 45 (1985), 2883). In contrast to the primary tumors of superficial spreading melanoma (frequency: 60%), nodular melanoma (frequency: 20%) and lentigo maligna melanoma (frequency: 10%), which are usually MCSP-positive (Reza Ziai 1987 Cancer Res. 47: 2474), primary tumors of acral lentiginous melanoma (frequency: 5%) are frequently MCSP-negative (Kageshita, Cancer Res. 51 (1991), 1726). Currently, there are 53.600 newly diagnosed cases of melanoma each year in the U.S. (2002). Overall the incidence of melanoma is increasing.

In a preferred embodiment of the invention the polypeptide is a bispecific single chain antibody molecule.

The herein above described problems with regard to the development of surrogate molecules for preclinical studies is further aggravated, if the drug candidate is a bispecific antibody, e.g. a bispecific single chain antibody. Such a bispecific antibody requires that both antigens recognized are cross-species specific with a given animal species to allow for safety testing in such animal.

As also noted herein above, the present invention provides polypeptides comprising a first binding domain capable of binding to an epitope of human and non-chimpanzee primate CD3ε chain and a second binding domain capable of binding to a cell surface antigen, wherein the second binding domain preferably also binds to a cell surface antigen of a human and a non-chimpanzee primate. The advantage of bispecific single chain antibody molecules as drug candidates fulfilling the requirements of the preferred polypeptide of the invention is the use of such molecules in preclinical animal testing as well as in clinical studies and even for therapy in human. In a preferred embodiment of the cross-species specific bispecific single chain antibodies of the invention the second binding domain binding to a cell surface antigen is of human origin. In a cross-species specific bispecific molecule according to the invention the binding domain binding to an epitope of human and non-chimpanzee primate CD3 epsilon chain is located in the order VH-VL or VL-VH at the N-terminus or the C-terminus of the bispecific molecule. Examples for cross-species specific bispecific molecules according to the invention in different arrangements of the VH- and the VL-chain in the first and the second binding domain are described in the appended examples.

As used herein, a "bispecific single chain antibody" denotes a single polypeptide chain comprising two binding domains. Each binding domain comprises one variable region from an antibody heavy chain ("VH region"), wherein the VH region of the first binding domain specifically binds to the CD3ε molecule, and the VH region of the second binding domain specifically binds to a cell surface antigen, as defined in more detail below. The two binding domains are optionally linked to one another by a short polypeptide spacer. A non-limiting example for a polypeptide spacer is Gly-Gly-Gly-Gly-Ser (G-G-G-G-S) and repeats thereof. Each binding domain may additionally comprise one variable region from an antibody light chain ("VL region"), the VH region and VL region within each of the first and second binding domains being linked to one another via a polypeptide linker, for example of the type disclosed and claimed in EP 623679 B1, but in any case long enough to allow the VH region and VL region of the first binding domain and the VH region and VL region of the second binding domain to pair with one another such that, together, they are able to specifically bind to the respective first and second molecules.

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

SEQ ID NOs: 189-194, SEQ ID NOs: 201-206, SEQ ID NOs: 219-224, SEQ ID NOs: 237-242, SEQ ID NOs:255-260, SEQ ID NOs:273-279, SEQ ID NOs: 382-384 and 387-389, SEQ ID NOs: 400-402 and 405-407, SEQ ID NOs: 418-420 and 423-425, SEQ ID NOs: 436-438 and 441-443, SEQ ID NOs: 454-456 and 459-461, SEQ ID NOs: 472-474 and 477-479, SEQ ID NOs: 490-492 and 495-497, SEQ ID NOs: and 508-510 and 513-515,
SEQ ID NOs: 530-532 and 1568 to 1570, SEQ ID NOs: 545-547 and 550-552, SEQ ID NOs: 559-561 and 564-566, SEQ ID NOs: 577-579 and 582-584,
SEQ ID NOs: 615-617 and 620-622, SEQ ID NOs: 633-635, 638, 639 and 1571,
SEQ ID NOs: 650-652 and 655-657, SEQ ID NOs: 668-670 and 673-675, SEQ ID NOs: 682-684 and 687-689, SEQ ID NOs: 696-698 and 701-703, SEQ ID NOs: 710-712 and 715-717, SEQ ID NOs: 724-726 and 729-731, SEQ ID NOs: 738-740 and 743-745, SEQ ID NOs: 752-754 and 757-759, SEQ ID NOs: 766-768 and 771-773,
SEQ ID NOs: 780-782 and 785-787, SEQ ID NOs: 794-796 and 799-801, SEQ ID NOs: 808-810 and 813-815, SEQ ID NOs: 822-824 and 827-829, SEQ ID NOs: 836-838 and 841-843, SEQ ID NOs: 850-852 and 855-857,
SEQ ID NOs: 866-868 and 871-873, SEQ ID NOs: 884-886 and 889-891, SEQ ID NOs: 902-904 and 907-909, SEQ ID NOs: 920-922 and 925-927, SEQ ID NOs: 938-940 and 943-945, SEQ ID NOs: 956-958 and 961-963, SEQ ID NOs: 974-976 and 979-981, SEQ ID NOs: 992-994 and 997-999, SEQ ID NOs: 1006-1008 and 1011-1013, SEQ ID NOs: 1020-1022 and 1025-1027, SEQ ID NOs: 1034-1036 and 1039-1041, SEQ ID NOs: 1048-1050 and 1053-1055, SEQ ID NOs: 1062-1064 and 1067-1069, SEQ ID NOs: 1076-1078 and 1081-1083, SEQ ID NOs: 1090-1092 and 1095-1097,
SEQ ID NOs: 1114-1116 and 1119-1121, SEQ ID NOs: 1128-1130 and 1133-1135,
SEQ ID NOs: 1141-1143 and 1146-1148, SEQ ID NOs: 1155-1157 and 1160-1162,
SEQ ID NOs: 1169-1171 and 1174-1176, SEQ ID NOs: 1183-1185 and 1188-1190,
SEQ ID NOs: 1197-1199 and 1202-1204, SEQ ID NOs: 1211-1213 and 1216-1218,
SEQ ID NOs: 1125-1227 and 1230-1232, SEQ ID NOs: 1239-1241 and 1244-1246,
SEQ ID NOs:, 1253-1255 and 1258-1260, SEQ ID NOs: 1267-1269 and 1272-1274,
SEQ ID NOs: 1281-1283 and 1286-1288, SEQ ID NOs: 1295-1297 and 1300-1302,
SEQ ID NOs: 1309-1311 and 1314-1316, SEQ ID NOs: 1323-1325 and 1328-1330,
SEQ ID NOs:1343-1345 and 1348-1350, SEQ ID NOs: 1361-1363 and 1366-1368,
SEQ ID NOs:1375-1377 and 1380-1382, SEQ ID NOs: 1389- 1391 and 1394-1396,
SEQ ID NOs:1403-1405 and 1408-1410, SEQ ID NOs: 1417-1419 and 1422-1424,
SEQ ID NOs: 1431-1433 and 1436-1438, SEQ ID NOs:1445-1447 and 1450-1452,
SEQ ID NOs:1459-1461 and 1464-1466, SEQ ID NOs: 1473-1475 and 1478-1480,
SEQ ID NOs: 1486-1491, SEQ ID NOs: 1492-1497,
SEQ ID NOs: 1505-1507 and 1510-1512, SEQ ID NOs: 1531-1533 and 1536-1538,
SEQ ID NOs: 1573-1575 and 1578-1580, SEQ ID NOs: 1591-1593 and 1596-1598,
SEQ ID NOs: 1605-1607 and 1610-1612, and SEQ ID NOs: 1619-1621, 1624-1626,
SEQ ID NOs: 1634-1636 and 1639-1641 and SEQ ID NOs: 1652-1654, SEQ ID NOs: 1657-1659 and SEQ ID NOs: 1669-1674.

A particularly preferred embodiment of the invention concerns an above characterized polypeptide, wherein the bispecific single chain antibody molecule comprises a sequence selected from:

(a) an amino acid sequence as depicted in any of SEQ ID NOs. 291, 293, 295, 297, 299, 301, 303, 305, 307, 309, 311, 313, 315, 317, 319, 321, 323, 325, 327, 329, 331, 333, 335, 337, 339, 341, 343, 345 or 347, 393, 395, 397, 411, 413, 415, 429, 431, 433, 447, 449, 451, 465, 467, 469, 483, 485, 487, 501, 503, 505, 519, 521, 523, 1336, 1338, 1340, 538, 540, 542, 556, 570, 572, 574, 588, 590, 592, 626, 628, 630, 643, 645, 647, 661, 663, 665, 679, 693, 707, 721, 735, 749, 763, 777, 791, 805, 819, 833, 847, 861, 863, 877, 879, 881, 895, 897, 899, 913, 915, 917, 931, 933, 935, 949, 951, 953, 967, 969, 971, 985, 987, 989, 1003, 1017, 1031, 1045, 1059, 1073, 1087, 1101, 1125, 1138, 1152, 1166, 1180, 1194, 1208, 1222, 1236, 1250, 1264, 1278, 1292, 1306, 1320, 1334, 1354, 1356, 1358, 1372, 1386, 1400, 1414, 1428, 1442, 1456, 1470, 1484, 1500, 1502, 1518, 1520, 1522, 1524, 1526, 1528, 1544, 1546, 1548, 1550, 1552, 1554, 1556, 1558, 1560, 1562, 1564, 1566, 1586, 1588, 1602, 1616, 1630, 1647, 1649 or 1663; and

(b) an amino acid sequence encoded by a nucleic acid sequence as depicted in any of SEQ ID NOs. 292, 294, 296, 298, 300, 302, 304, 306, 308, 310, 312, 314, 316, 318, 320, 322, 324, 326, 328, 330, 332, 334, 336, 338, 340, 342, 344, 346 or 348, 394, 396, 398, 412, 414, 416, 430, 432, 434, 448, 450, 452, 466, 468, 470, 484, 486, 488, 502, 504, 506, 520, 522, 524, 1337, 1339, 1341, 539, 541, 543, 557, 571, 573, 575, 589, 591, 593, 627, 629, 631, 644, 646, 648, 662, 664, 666, 680, 694, 708, 722, 736, 750, 764, 778, 792, 806, 820, 834, 848, 862, 864, 878, 880, 882, 896, 898, 900, 914, 916, 918, 932, 934, 936, 950, 952, 954, 968, 970, 972, 986, 988, 990, 1004, 1018, 1032, 1046, 1060, 1074, 1088, 1102, 1126, 1139, 1153, 1167, 1181, 1195, 1209, 1223, 1237, 1251, 1265, 1279, 1293, 1307, 1321, 1335, 1355, 1357, 1359, 1373, 1387, 1401, 1415, 1429, 1443, 1457, 1471, 1485, 1501, 1503, 1519, 1521, 1523, 1525, 1527, 1529, 1545, 1547, 1549, 1551, 1553, 1555, 1557, 1559, 1561, 1563, 1565, 1567, 1587, 1589, 1603, 1617, 1631, 1648, 1650 or 1664.

In a preferred embodiment of the invention, the bispecific single chain antibodies are cross-species specific for CD3 epsilon and for the cell surface antigen recognized by their second binding domain. In a more preferred embodiment these bispecific single chain antibodies are cross-species specific for human and non-chimpanzee primate CD3 epsilon and for human and non-chimpanzee primate MCSP, EGFR, EGFRvIII, Carbonic anhydrase IX, CD30, CD33, IgE, CD44v6, Muc-1, HBV and HCV. In an alternative embodiment the present invention provides a nucleic acid sequence encoding an above described polypeptide of the invention.

The present invention also relates to a vector comprising the nucleic acid molecule of the present invention.

Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook et al. (loc cit.) and Ausubel, Current Protocols in Molecular Biology, Green Publishing Associates and Wiley Interscience, N.Y. (1989), (1994). Alternatively, the polynucleotides and vectors of the invention can be reconstituted into liposomes for delivery to target cells. As discussed in further details below, a cloning vector was used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

Preferably said vector comprises a nucleic acid sequence which is a regulatory sequence operably linked to said nucleic acid sequence defined herein.

The term "regulatory sequence" refers to DNA sequences, which are necessary to effect the expression of coding sequences to which they are ligated. The nature of such control sequences differs depending upon the host organism. In prokaryotes, control sequences generally include promoter, ribosomal binding site, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the recited vector is preferably an expression vector. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, e.g., the $P_L$, *lac*, *trp* or *tac* promoter in E. *coli*, and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells.

Beside elements, which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art; see also the appended Examples. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an. N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (In-vitrogene), pEF-DHFR, pEF-ADA or pEF-neo (Mack et al. PNAS (1995) 92, 7021-7025 and Raum et al. Cancer Immunol Immunother (2001) 50(3), 141-150) or pSPORT1 (GIBCO BRL).

Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the invention may follow; see, e.g., the appended examples.

An alternative expression system, which can be used to express a cell cycle interacting protein is an insect system. In one such system, *Autographa californica* nuclear polyhedrosis virus (AcNPV) is used as a vector to express foreign genes in *Spodoptera frugiperda* cells or in *Trichoplusia* larvae. The coding sequence of a recited nucleic acid molecule may be cloned into a nonessential region of the virus, such as the polyhedrin gene, and placed under control of the polyhedrin promoter. Successful insertion of said coding sequence will render the polyhedrin gene inactive and produce recombinant virus lacking coat protein coat. The recombinant viruses are then used to infect *S. frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227).

Additional regulatory elements may include transcriptional as well as translational enhancers. Advantageously, the above-described vectors of the invention comprise a selectable and/or scorable marker.

Selectable marker genes useful for the selection of transformed cells and, e.g., plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, PI. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used alone or as part of a vector to express the polypeptide of the invention in cells, for, e.g., purification but also for gene therapy purposes. The nucleic acid molecules or vectors containing the DNA sequence(s) encoding any one of the above described polypeptide of the invention is introduced into the cells which in turn produce the polypeptide of interest. Gene therapy, which is based on introducing therapeutic genes into cells by ex-vivo or in-vivo techniques is one of the most important applications of gene transfer. Suitable vectors, methods or gene-delivery systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Verma, Nature 389 (1994), 239; Isner, Lancet 348 (1996), 370-374; Muhlhauser, Circ. Res. 77 (1995), 1077-1086; Onodera, Blood 91 (1998), 30-36; Verma, Gene Ther. 5 (1998), 692-699; Nabel, Ann. N.Y. Acad. Sci. 811 (1997), 289-292; Verzeletti, Hum. Gene Ther. 9 (1998), 2243-51; Wang, Nature Medicine 2 (1996), 714-716; WO 94/29469; WO 97/00957, US 5,580,859; US 5,589,466; or Schaper, Current Opinion in Biotechnology 7 (1996), 635-640. The recited nucleic acid molecules and vectors may be designed for direct introduction or for introduction via liposomes, or viral vectors (e.g., adenoviral, retroviral) into the cell. Preferably, said cell is a germ line cell, embryonic cell, or egg cell or derived there from, most preferably said cell is a stem cell. An example for an embryonic stem cell can be, inter alia, a stem cell as described in Nagy, Proc. Natl. Acad. Sci. USA 90 (1993), 8424-8428.

The invention also provides for a host transformed or transfected with a vector of the invention. Said host may be produced by introducing the above described vector of the invention or the above described nucleic acid molecule of the invention into the host. The presence of at least one vector or at least one nucleic acid molecule in the host may mediate the expression of a gene encoding the above described single chain antibody constructs.

The described nucleic acid molecule or vector of the invention, which is introduced in the host may either integrate into the genome of the host or it may be maintained extrachromosomally.

The host can be any prokaryote or eukaryotic cell.

The term "prokaryote" is meant to include all bacteria, which can be transformed or transfected with DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. Especially preferred is the use of a plasmid or a virus containing the coding sequence of the polypeptide of the invention and genetically fused thereto an N-terminal FLAG-tag and/or C-terminal His-tag. Preferably, the length of said FLAG-tag is about 4 to 8 amino acids, most preferably 8 amino acids. An above described polynucleotide can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, loc cit.).

Preferably, said the host is a bacterium or an insect, fungal, plant or animal cell.

It is particularly envisaged that the recited host may be a mammalian cell. Particularly preferred host cells comprise CHO cells, COS cells, myeloma cell lines like SP2/0 or NS/0. As illustrated in the appended examples, particularly preferred are CHO-cells as hosts.

More preferably said host cell is a human cell or human cell line, e.g. per.c6 (Kroos, Biotechnol. Prog., 2003, 19:163-168).

In a further embodiment, the present invention thus relates to a process for the production of a polypeptide of the invention, said process comprising culturing a host of the invention under conditions allowing the expression of the polypeptide of the invention and recovering the produced polypeptide from the culture.

The transformed hosts can be grown in fermentors and cultured according to techniques known in the art to achieve optimal cell growth. The polypeptide of the invention can then be isolated from the growth medium, cellular lysates, or cellular membrane fractions. The isolation and purification of the, e.g., microbially expressed polypeptides of the invetion may be by any conventional means such as, for example, preparative chromatographic separations and immunological separations such as those involving the use of monoclonal or polyclonal antibodies directed, e.g., against a tag of the polypeptide of the invention or as described in the appended examples.

The conditions for the culturing of a host, which allow the expression are known in the art to depend on the host system and the expression system/vector used in such process. The parameters to be modified in order to achieve conditions allowing the expression of a recombinant polypeptide are known in the art. Thus, suitable conditions can be determined by the person skilled in the art in the absence of further inventive input.

Once expressed, the polypeptide of the invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure polypeptides of at least about 90 to 95% homogeneity are preferred, and 98 to 99% or more homogeneity are most preferred, for pharmaceutical uses. Once purified, partially or to homogeneity as desired, the polypeptide of the invention may then be used therapeutically (including extracorporeally) or in developing and performing assay procedures. Furthermore, examples for methods for the recovery of the polypeptide of the invention from a culture are described in detail in the appended examples.

Furthermore, the invention provides for a composition comprising a polypeptide of the invention or a polypeptide as produced by the process disclosed above. Preferably, said composition is a pharmaceutical composition.

In accordance with the invention, the term "pharmaceutical composition" relates to a composition for administration to a patient, preferably a human patient. The particular preferred pharmaceutical composition of this invention comprises binding molecules directed against and generated against context-independent CD3 epitopes. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient. The pharmaceutical composition comprising the binding molecules directed against and generated against context-independent CD3 epitopes of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of these binding molecules directed against and generated against context-independent CD3 epitopes of this invention may be intravenuous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The composition of the present invention, comprising in particular binding molecules directed against and generated against context-independent CD3 epitopes may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate. The compositions of the present invention can be administered to the subject at a suitable dose which can be determined e.g. by dose escalating studies by administration of increasing doses of the polypeptide of the invention exhibiting cross-species specificity described herein to non-chimpanzee primates, for instance macaques. As set forth above, the polypeptide of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the polypeptide of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the polypeptide of the invention defined herein, further biologically active agents, depending on the intended use of the composition. Such agents might be drugs acting on the gastro-intestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art.

The biological activity of the pharmaceutical composition defined herein can be determined for instance by cytotoxicity assays, as described in the following examples, in WO 99/54440 or by Schlereth et al. (Cancer Immunol.

Immunother. 20 (2005), 1 - 12). "Efficacy" or "in vivo efficacy" as used herein refers to the response to therapy by the pharmaceutical composition of the invention, using e.g. standardized NCI response criteria. The success or *in vivo* efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that effect the ability of a particular drug to treat a given condition, is established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

"Half-life" means the time where 50% of an administered drug are eliminated through biological processes, e.g. metabolism, excretion, etc.

By "hepatic first-pass metabolism" is meant the propensity of a drug to be metabolized upon first contact with the liver, i.e. during its first pass through the liver. "Volume of distribution" means the degree of retention of a drug throughout the various compartments of the body, like e.g. intracellular and extracellular spaces, tissues and organs, etc. and the distribution of the drug within these compartments.

"Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment.

"Lag time" means the time delay between the administration of the drug and its detection and measurability in blood or plasma.

"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetik parameters of bispecific single chain antibodies, a preferred embodiment of the polypeptide of the invention, exhibiting cross-species specificity, which may be determined in preclinical animal testing in non-chimpanzee primates as outlined above are also set forth e.g. in the publication by Schlereth et al. (Cancer Immunol. Immunother. 20 (2005), 1 - 12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", "in vivo safety" or "tolerability" as used herein defines the administration of a drug without inducing severe adverse events directly after administration (local tolerance) and during a longer period of application of the drug. "Safety", "in vivo safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviating to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance haematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective and non-toxic dose" as used herein refers to a tolerable dose of the bispecific single chain antibody as defined herein which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

Moreover, the invention relates to a pharmaceutical composition comprising a polypeptide of this invention (i.e. a polypeptide comprising at least one binding domain capable of binding to an epitope of human and non-chimpanzee primate CD3 epsilon chain, wherein the epitope is part of an amino acid sequence comprised in the group consisting of SEQ ID NOs. 2, 4, 6, or 8 in accordance with this invention or produced according to the process according to the invention) for the prevention, treatment or amelioration of a disease selected from a proliferative disease, a tumorous disease, or an immunological disorder. Preferably, said pharmaceutical composition further comprises suitable formulations of carriers, stabilizers and/or excipients.

A further aspect of the invention relates to a use of a polypeptide as defined herein above or produced according to a process defined herein above, for the preparation of a pharmaceutical composition for the prevention, treatment or amelioration of a disease. Preferably, said disease is a proliferative disease, a tumorous disease, or an immunological disorder. It is further preferred that said tumorous disease is a malignant disease, preferably cancer.

In another preferred embodiment of use of the polypeptide of the invention said pharmaceutical composition is suitable to be administered in combination with an additional drug, i.e. as part of a co-therapy. In said co-therapy, an active agent may be optionally included in the same pharmaceutical composition as the polypeptide of the invention, or may be included in a separate pharmaceutical composition. In this latter case, said separate pharmaceutical composition is suitable for administration prior to, simultaneously as or following administration of said pharmaceutical composition comprising the polypeptide of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.

Preferably, said proteinaceous compound or non-proteinaceous compound may be administered simultaneously or non-simultaneously with the polypeptide of the invention, a nucleic acid molecule as defined hereinabove, a vector as defined as defined hereinabove, or a host as defined as defined hereinabove.

Another aspect of the invention relates to a method for the prevention, treatment or amelioration of a disease in a subject in the need thereof, said method comprising the step of administration of an effective amount of a pharmaceutical composition of the invention. Preferably, said disease is a proliferative disease, a tumorous disease, or an immunological disorder. Even more preferred, said tumorous disease is a malignant disease, preferably cancer.

In another preferred embodiment of the method of the invention said pharmaceutical composition is suitable to be administered in combination with an additional drug, i.e. as part of a co-therapy. In said co-therapy, an active agent may be optionally included in the same pharmaceutical composition as the polypeptide of the invention, or may be included in a separate pharmaceutical composition. In this latter case, said separate pharmaceutical composition is suitable for administration prior to, simultaneously as or following administration of said pharmaceutical composition comprising the polypeptide of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.

Preferably, said proteinaceous compound or non-proteinaceous compound may be administered simultaneously or non-simultaneously with the polypeptide of the invention, a nucleic acid molecule as defined hereinabove, a vector as defined as defined hereinabove, or a host as defined as defined hereinabove.

It is preferred for the above described method of the invention that said subject is a human.

In a further aspect, the invention relates to a kit comprising a polypeptide of the invention, a nucleic acid molecule of the invention, a vector of the invention, or a host of the invention.

The present invention is further characterized by the following list of items:

Item 1. A method for the identification of (a) polypeptide(s) comprising a cross-species specific binding domain capable of binding to an epitope of human and non-chimpanzee primate CD3 epsilon (CD3ε), the method comprising the steps of:

(a) contacting the polypeptide(s) with an N-terminal fragment of the extracellular domain of CD3ε of maximal 27 amino acids comprising the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382), fixed via its C-terminus to a solid phase;
(b) eluting the bound polypeptide(s) from said fragment; and
(c) isolating the polypeptide(s) from the eluate of (b).

It is preferred that the polypeptide(s) identified by the above method of the invention are of human origin.

The present "method for the identification of (a) polypeptide(s)" is understood as a method for the isolation of one or more different polypeptides with the same specificity for the fragment of the extracellular domain of CD3ε comprising at its N-terminus the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382) from a plurality of polypeptide candidates as well as a method for the purification of a polypeptide from a solution. Non-limiting embodiments of a method of the isolation of one or more different polypeptides with the same specificity for the fragment of the extracellular domain of CD3ε comprise methods for the selection of antigen-specific binding entities, e.g. panning methods as commonly used for hybridoma screening, screening of transiently/stably transfected clones of eukaryotic host cells or in phage display methods. A non-limiting example for the latter method for the purification of a polypeptide from a solution is e.g. the purification of a recombinantly expressed polypeptide from a culture supernatant or a preparation from such culture.

As stated above the fragment used in the method of the invention is a N-terminal fragment of the extracellular domain of the primate CD3ε molecule. The amino acid sequence of the extracellular domain of the CD3ε molecule of different primates is depicted in SEQ ID NOs: 1, 3, 5 and 7. The two forms of the N-terminal octamer are depicted in SEQ ID NOs: 381 and 382. It is preferred that this N-terminus is freely available for binding of the polypeptides to be identified by the method of the invention. The term "freely available" is understood in the context of the invention as free of additional motives such as a His-tag. The interference of such a His-tag with a binding molecule identified by the method of the invention is described in the appended Examples 6 and 34.

According to the method of the invention said fragment is fixed via its C-terminus to a solid phase. The person skilled in the art will easily and without any inventive ado elect a suitable solid phase support dependent from the used embodiment of the method of the invention. Examples for a solid support comprise but are not limited to matrices like beads (e.g. agarose beads, sepharose beads, polystyrol beads, dextran beads), plates (culture plates or MultiWell plates) as well as chips known e.g. from Biacore®. The selection of the means and methods for the fixation/immobilization of the fragment to said solid support depend on the election of the solid support. A commonly used method for the fixation/immobilization is a coupling via an N-hydroxysuccinimide (NHS) ester. The chemistry underlying this coupling as well as alternative methods for the fixation/immobilization are known to the person skilled in the art, e.g. from Hermanson "Bioconjugate Techniques", Academic Press, Inc. (1996). For the fixation to/immobilization on chromatographic supports the following means are commonly used: NHS-activated sepharose (e.g. HiTrap-NHS of GE Life Science - Amersham), CnBr-activated sepharose (e.g. GE Life Science - Amersham), NHS-activated dextran beads (Sigma) or activated polymethacrylate. These reagents may also be used in a batch approach. Moreover, dextran beads comprising iron oxide (e.g. available from Miltenyi) may be used in a batch approach. These beads may be used in combination with a magnet for the separation of the beads from a solution. Polypeptides can be immobilized on a Biacore chip (e.g. CM5 chips) by the use of NHS activated carboxymethyldextran. Further examples for an appropriate solid support are amine reactive MultiWell plates (e.g. Nunc Immobilizer™ plates).

According to the method of the invention said fragment of the extracellular domain of CD3 epsilon can be directly coupled to the solid support or via a stretch of amino acids, which might be a linker or another protein/polypeptide moiety. Alternatively, the extracellular domain of CD3 epsilon can be indirectly coupled via one or more adaptor molecule(s).

Means and methods for the eluation of a peptide bound to an immobilized epitope are well known in the art. The same holds true for methods for the isolation of the identified polypeptide(s) from the eluate.

In line with the invention a method for the isolation of one or more different polypeptides with the same specificity for the fragment of the extracellular domain of CD3ε comprising at its N-terminus the amino acid sequence Gln-Asp-Gly-Asn-Glu-Glu-X-Gly from a plurality of polypeptide candidates may comprise one or more steps of the following methods for the selection of antigen-specific entities:

CD3ε specific binding molecules can be selected from antibody derived repertoires. A phage display library can be constructed based on standard procedures, as for example disclosed in "Phage Display: A Laboratory Manual"; Ed. Barbas, Burton, Scott & Silverman; Cold Spring Harbor Laboratory Press, 2001. The format of the antibody fragments in the antibody library can be scFv, but may generally also be a Fab fragment or even a single domain antibody fragment. For the isolation of antibody fragments naïve antibody fragment libraries may be used. For the selection of potentially low immunogenic binding entities in later therapeutic use, human antibody fragment libraries may be favourable for the direct selection of human antibody fragments. In some cases they may form the basis for synthetic antibody libraries (Knappik et al. J Mol. Biol. 2000, 296:57 ff). The corresponding format may be Fab, scFv (as described below) or domain antibodies (dAbs, as reviewed in Holt et al., Trends Biotechnol. 2003, 21:484 ff).

It is also known in the art that in many cases there is no immune human antibody source available against the target antigen. Therefore animals are immunized with the target antigen and the respective antibody libraries isolated from animal tissue as e.g. spleen or PBMCs. The N-terminal fragment may be biotinylated or covalently linked to proteins like KLH or bovine serum albumin (BSA). According to common approaches rodents are used for immunization. Some immune antibody repertoires of non-human origin may be especially favourable for other reasons, e.g. for the presence of single domain antibodies (VHH) derived from cameloid species (as described in Muyldermans, J Biotechnol. 74:277; De Genst et al. Dev Como Immunol. 2006, 30:187 ff). Therefore a corresponding format of the antibody library may be Fab, scFv (as described below) or single domain antibodies (VHH).

In one possible approach ten weeks old F1 mice from balb/c × C57black crossings can be immunized with whole cells e.g. expressing transmembrane EpCAM N-terminally displaying as translational fusion the N-terminal amino acids 1 to 27 of the mature CD3ε chain. Alternatively, mice can be immunized with 1-27 CD3 epsilon-Fc fusion protein (a corresponding approach is described in the appended Example 2). After booster immunization(s), blood samples can be taken and antibody serum titer against the CD3-positive T cells can be tested e.g. in FACS analysis. Usually, serum titers are significantly higher in immunized than in non-immunized animals.

Immunized animals may form the basis for the construction of immune antibody libraries. Examples of such libraries comprise phage display libraries. Such libraries may be generally constructed based on standard procedures, as for example disclosed in "Phage Display: A Laboratory Manual"; Ed. Barbas, Burton, Scott & Silverman; Cold Spring Harbor Laboratory Press, 2001.

The non-human antibodies can also be humanized via phage display due to the generation of more variable antibody libraries that can be subsequently enriched for binders during selection.

In a phage display approach any one of the pools of phages that displays the antibody libraries forms a basis to select binding entities using the respective antigen as target molecule. The central step in which antigen specific, antigen bound phages are isolated is designated as panning. Due to the display of the antibody fragments on the surface of the phages, this general method is called phage display. One preferred method of selection is the use of small proteins such as the filamentous phage N2 domain translationally fused to the N-terminus of the scFv displayed by the phage. Another display method known in the art, which may be used to isolate binding entities is the ribosome display method (reviewed in Groves & Osbourn, Expert Opin Biol Ther. 2005, 5:125 ff; Lipovsek & Pluckthun, J Immunol Methods 2004, 290:52 ff).

In order to demonstrate binding of scFv phage particles to a 1-27 CD3ε-Fc fusion protein a phage library carrying the cloned scFv-repertoire can be harvested from the respective culture supernatant by PEG (polyethyleneglycole). ScFv phage particles may be incubated with immobilized CD3ε Fc fusion protein. The immobilized CD3ε Fc fusion protein may be coated to a solid phase. Binding entities can be eluted and the eluate can be used for infection of fresh uninfected bacterial hosts. Bacterial hosts successfully transduced with a phagemid copy, encoding a human scFv-fragment, can be selected again for carbenicillin resistance and subsequently infected with e.g. VCMS 13 helper phage to start the second round of antibody display and in vitro selection. A total of 4 to 5 rounds of selections is carried out, normally.

The binding of isolated binding entities can be tested on CD3epsilon positive Jurkat cells, HPBall cells, PBMCs or transfected eukaryotic cells that carry the N-terminal CD3ε sequence fused to surface displayed EpCAM using a flow cytometric assay (see appended Example 4).

Item 2. The method of item 1, wherein the polypeptide(s) comprise(s) the identified binding domain as a first binding domain and a second binding domain capable of binding to a cell surface antigen.

For the generation of the second binding domain of the polypeptide identified by the method of the invention, e.g. bispecific single chain antibodies as defined herein, monoclonal antibodies binding to both of the respective human and non-chimpanzee primate cell surface antigens can be used. Appropriate binding domains for the bispecific polypeptide as defined herein e.g. can be derived from cross-species specific monoclonal antibodies by recombinant methods described in the art. A monoclonal antibody binding to a human cell surface antigen and to the homolog of said cell surface antigen in a non-chimpanzee primate can be tested by FACS assays as set forth above. Hybridoma techniques as described in the literature (Milstein and Köhler, Nature 256 (1975), 495-7) can also be used for the generation of cross-species specific antibodies. For example, mice may be alternately immunized with human and non-chimpanzee primate CD33. From these mice, cross-species specific antibody-producing hybridoma cells can be isolated via hybridoma technology and analysed by FACS as set forth above. The generation and analysis of bispecific polypeptides such as bispecific single chain antibodies exhibiting cross-species specificity as described herein is shown in the following Examples. The advantages of the bispecific single chain antibodies exhibiting cross-species specificity include the points enumerated below.

Item 3. The method of item 2, wherein the second binding domain binds to a cell surface antigen of a human and a non-chimpanzee primate.

Item 4. The method of any of items 1 to 3, wherein the first binding domain is an antibody.

Item 5. The method of item 4, wherein the antibody is a single chain antibody.

Item 6. The method of any of items 2 to 5, wherein the second binding domain is an antibody.

Item 7. The method of any of items 1 to 6, wherein the fragment of the extracellular domain of CD3ε consists of one or more fragments of a polypeptide having an amino acid sequence of any one depicted in SEQ ID NOs.2, 4, 6 or 8.

Item 8. The method of item 7, wherein said fragment is 8, 9, 10, 11, 12, 13, 14, 15, 16, 17, 18, 19, 20, 21, 22, 23, 24, 25, 26, 27 amino acid residues in length.

Item 9.The method of any of items 1 to 8, wherein the method of identification is a method of screening a plurality of polypeptides comprising a cross-species specific binding domain binding to an epitope of human and non-chimpanzee primate CD3ε.

Item 10. The method of any of items 1 to 8, wherein the method of identification is a method of purification/isolation of a polypeptide comprising a cross-species specific binding domain binding to an epitope of human and non-chimpanzee primate CD3ε.

Item 11. Use of an N-terminal fragment of the extracellular domain of CD3ε of maximal 27 amino acids comprising the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382) for the generation of a cross-species specific binding domain.

In line with said use of the invention it is preferred that the generated cross-species specific binding domain is of human origin.

Item 12. Use according to item 11, wherein the cross-species specific binding domain is an antibody.

Item 13. Use according to item 12, wherein the antibody is a single chain antibody.

Item 14. Use according to items 12 to 13, wherein the antibody is a bispecific antibody.

These and other embodiments are disclosed and encompassed by the description and Examples of the present invention. Recombinant techniques and methods in immunology are described e.g. in Sambrook et al. Molecular Cloning: A Laboratory Manual; Cold Spring Harbor Laboratory Press, 3rd edition 2001; Lefkovits; Immunology Methods Manual; The Comprehensive Sourcebook of Techniques; Academic Press, 1997; Golemis; Protein-Protein Interactions: A Molecular Cloning Manual; Cold Spring Laboratory Press, 2002. Further literature concerning any one of the antibodies, methods, uses and compounds to be employed in accordance with the present invention may be retrieved from public libraries and databases, using for example electronic devices. For example, the public database "Medline", available on the Internet, may be utilized, for example under http://www.ncbi.nlm.nih.gov/PubMed/medline.html. Further databases and addresses such as http://www.ncbi.nlm.nih.gov/ or listed at the EMBL-services homepage under http://www.embl.de/services/index.html are known to the person skilled in the art and can also be obtained using, e. g., http://www.google.com.

The figures show:

### Figure 1
Fusion of the N-terminal amino acids 1-27 of primate CD3 epsilon to a heterologous soluble protein.

### Figure 2
The figure shows the average absorption values of quadruplicate samples measured in an ELISA assay detecting the presence of a construct consisting of the N-terminal amino acids 1-27 of the mature human CD3 epsilon chain fused to the hinge and Fc gamma portion of human IgG1 and a C-terminal 6 Histidine tag in a supernatant of transiently transfected 293 cells. The first column labeled "27 aa huCD3E" shows the average absorption value for the construct, the second column labeled "irrel. SN" shows the average value for a supernatant of 293 cells transfected with an irrelevant construct as negative control. The comparison of the values obtained for the construct with the values obtained for the negative control clearly demonstrates the presence of the recombinant construct.

### Figure 3
The figure shows the average absorption values of quadruplicate samples measured in an ELISA assay detecting the binding of the cross species specific anti-CD3 binding molecules in form of crude preparations of periplasmatically expressed single-chain antibodies to a construct comprising the N-terminal 1-27 amino acids of the mature human CD3 epsilon chain fused to the hinge and Fc gamma portion of human IgG1 and a C-terminal His6 tag. The columns show from left to right the average absorption values for the specificities designated as A2J HLP, I2C HLP E2M HLP, F7O HLP, G4H HLP, H2C HLP, E1L HLP, F12Q HLP, F6A HLP and H1E HLP. The rightmost column labelled "neg. contr." shows the average absorption value for the single-chain preparation of a murine anti-human CD3 antibody as negative control. The comparison of the values obtained for the anti-CD3 specificities with the values obtained for the negative control clearly demonstrates the strong binding of the anti-CD3 specificities to the N-terminal 1-27 amino acids of the mature human CD3 epsilon chain.

### Figure 4
Fusion of the N-terminal amino acids 1-27 of primate CD3 epsilon to a heterologous membrane bound protein.

### Figure 5
Histogram overlays of different transfectants tested in a FACS assay detecting the presence of recombinant transmembrane fusion proteins consisting of cynomolgus EpCAM and the N-terminal 1-27 amino acids of the human, marmoset, tamarin, squirrel monkey and domestic swine CD3 epsilon chain respectively. The histogram overlays from left to right and top to bottom show the results for the transfectants expressing the constructs comprising the human 27 mer, marmoset 27 mer, tamarin 27 mer, squirrel monkey 27 mer and swine 27 mer respectively. In the individual overlays the thin line represents a sample incubated with PBS with 2% FCS instead of anti-Flag M2 antibody as negative control and the bold line shows a sample incubated with the anti-Flag M2 antibody. For each construct the overlay of the histograms shows binding of the anti-Flag M2 antibody to the transfectants, which clearly demonstrates the expression of the recombinant constructs on the transfectants.

**Figure 6**

Histogram overlays of different transfectants tested in a FACS assay detecting the binding of the cross-species specific anti-CD3 binding molecules in form of crude preparations of periplasmatically expressed single-chain antibodies to the N-terminal amino acids 1-27 of the human, marmoset, tamarin and squirrel monkey CD3 epsilon chain respectively fused to cynomolgus EpCAM.

**Figure 6A:**

The histogram overlays from left to right and top to bottom show the results for the transfectants expressing the 1-27 CD3-EpCAM comprising the human 27 mer tested with the CD3 specific binding molecules designated H2C HLP, F12Q HLP, E2M HLP and G4H HLP respectively.

**Figure 6B:**

The histogram overlays from left to right and top to bottom show the results for the transfectants expressing the 1-27 CD3-EpCAM comprising the marmoset 27 mer tested with the CD3 specific binding molecules designated H2C HLP, F12Q HLP, E2M HLP and G4H HLP respectively.

**Figure 6C:**

The histogram overlays from left to right and top to bottom show the results for the transfectants expressing the 1-27 CD3-EpCAM comprising the tamarin 27 mer tested with the CD3 specific binding molecules designated H2C HLP, F12Q HLP, E2M HLP and G4H HLP respectively.

**Figure 6D:**

The histogram overlays from left to right and top to bottom show the results for the transfectants expressing the 1-27 CD3-EpCAM comprising the squirrel monkey 27 mer tested with the CD3 specific binding molecules designated H2C HLP, F12Q HLP, E2M HLP and G4H HLP respectively.

**Figure 6E:**

The histogram overlays from left to right and top to bottom show the results for the transfectants expressing the 1-27 CD3-EpCAM comprising the swine 27 mer tested with the CD3 specific binding molecules designated H2C HLP, F12Q HLP, E2M HLP and G4H HLP respectively.

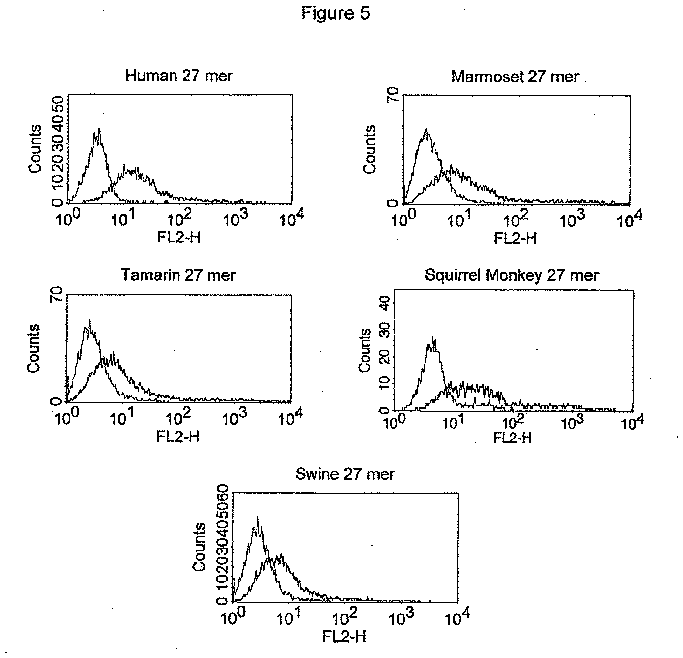

In the individual overlays the thin line represents a sample incubated with a single-chain preparation of a murine anti-human CD3-antibody as negative control and the bold line shows a sample incubated with the respective anti-CD3 binding molecules indicated. Considering the lack of binding to the swine 27 mer transfectants and the expression levels of the constructs shown in Figure 5 the overlays of the histograms show specific and strong binding of the tested anti-CD3 specificities of the fully cross-species specific human bispecific single chain antibodies to cells expressing the recombinant transmembrane fusion proteins comprising the N-terminal amino acids 1-27 of the human, marmoset, tamarin and squirrel monkey CD3 epsilon chain respectively fused to cynomolgus EpCAM and show therefore multi primate cross-species specificity of the anti-CD3 binding molecules.

**Figure 7**

FACS assay for detection of human CD3 epsilon on transfected murine EL4 T cells. Graphical analysis shows an overlay of histograms. The bold line shows transfected cells incubated with the anti-human CD3 antibody UCHT-1. The thin line represents cells incubated with a mouse IgG1 isotype control. Binding of the anti CD3 antibody UCHT1 clearly shows expression of the human CD3 epsilon chain on the cell surface of transfected murine EL4 T cells.

**Figure 8**

Binding of cross-species specific anti CD3 antibodies to alanine-mutants in an alanine scanning experiment. In the individual Figures the columns show from left to right the calculated binding values in arbitrary units in logarithmic scale for the wild-type transfectant (WT) and for all alanine-mutants from the position 1 to 27. The binding values are calculated using the following formula:

$$value\_Sample(x,y) = \cfrac{Sample(x,y) - neg\_Contr.(x)}{(UCHT-1(x)-neg\_Contr.(x)) * \cfrac{WT(y)-neg\_Contr.(wt)}{UCHT-1(wt)-neg\_Contr.(wt)}}$$

In this equation *value_Sample* means the value in arbitrary units of binding depicting the degree of binding of a specific anti-CD3 antibody to a specific alanine-mutant as shown in the Figure, *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT-1* means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, *x* specifies the respective transfectant, *y* specifies the respective anti-CD3 antibody and *wt* specifies that the respective transfectant is the wild-type. Individual alanine-mutant positions are labelled with the single letter code of the wild-type amino acid and the number of the position.

**Figure 8A:**
The figure shows the results for cross-species specific anti CD3 antibody A2J HLP expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8B:**
The figure shows the results for cross-species specific anti CD3 antibody E2M HLP, expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8C:**
The figure shows the results for cross-species specific anti CD3 antibody H2C HLP, expressed as chimeric IgG molecule. Reduced binding activity is observed for mutations to alanine at position 4 (asparagine). Complete loss of binding is observed for mutations to alanine glutamine at position 1 (glutamine), at position 2 (aspartate), at position 3 (glycine) and at position 5 (glutamate).

**Figure 8D:**
shows the results for cross-species specific anti CD3 antibody F12Q HLP, tested as periplasmatically expressed single-chain antibody. Complete loss of binding is observed for mutations to alanine at position 1 (glutamine), at position 2 (aspartate), at position 3 (glycine) and at position 5 (glutamate).

**Figure 9**
FACS assay detecting the binding of the cross-species specific anti-CD3 binding molecule H2C HLP to human CD3 with and without N-terminal His6 tag.
Histogram overlays are performed of the EL4 cell line transfected with wild-type human CD3 epsilon chain (left histogram) or the human CD3 epsilon chain with N-terminal His6 tag (right histogram) tested in a FACS assay detecting the binding of cross-species specific binding molecule H2C HLP. Samples are incubated with an appropriate isotype control as negative control (thin line), anti-human CD3 antibody UCHT-1 as positive control (dotted line) and cross-species specific anti-CD3 antibody H2C HLP in form of a chimeric IgG molecule (bold line).
Histogram overlays show comparable binding of the UCHT-1 antibody to both transfectants as compared to the isotype control demonstrating expression of both recombinant constructs. Histogram overlays also show binding of the anti-CD3 binding molecule H2C HLP only to the wild-type human CD3 epsilon chain but not to the His6-human CD3 epsilon chain. These results demonstrate that a free N-terminus is essential for binding of the cross-species specific anti-CD3 binding molecule H2C HLP.

**Figure 10**
Saturation binding of EGFR-21-63 LH × H2C on human CD3 positive PBMC to determine the KD value of CD3 binding on cells by FACS analysis. The assay is performed as described in Example 7.

**Figure 11**

FACS binding analysis of designated cross-species specific bispecific single chain constructs to CHO cells transfected with human EGFR, human CD3+ T cell line HPB-ALL, CHO cells transfected with cynomolgus EGFR and a macaque T cell line 4119 LnPx. The FACS staining is performed as described in Example 12. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 12**

Cytotoxic activity induced by designated cross-species specific single chain constructs redirected to indicated target cell lines. A) Stimulated CD4-/CD56- human PBMCs are used as effector cells, CHO cells transfected with human EGFR as target cells. B) The macaque T cell line 4119 LnPx are used as effector cells, CHO cells transfected with cynomolgus EGFR as target cells. The assay is performed as described in Example 13.

**Figure 13**

Cytotoxic activity induced by designated cross-species specific single chain constructs redirected to indicated target cell lines. A) Stimulated CD4-/CD56- human PBMCs are used as effector cells, CHO cells transfected with human EGFR as target cells. B) The macaque T cell line 4119 LnPx are used as effector cells, CHO cells transfected with cynomolgus EGFR as target cells. The assay is performed as described in Example 13.

**Figure 14**

FACS binding analysis of designated cross-species specific bispecific single chain constructs to CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 15**

FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 16**

FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified monomeric protein that are subsequently incubated with the anti-his antibody and the PE labeled detection antibody. The thin histogram line reflects the negative control: cells only incubated with the anti-his antibody and the detection antibody.

**Figure 17**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 18**

Cytotoxicity activity induced by designated cross-species specific MCSP specific single chain constructs redirected to indicated target cell lines. A) and B) The macaque T cell line 4119 LnPx are used as effector cells, CHO cells transfected with cynomolgus MCSP D3 as target cells. The assay is performed as described in Example 18.

**Figure 19**

Cytotoxicity activity induced by designated cross-species specific MCSP specific single chain constructs redirected to indicated target cell lines. A) and B) Stimulated CD4-/CD56- human PBMCs are used as effector cells, CHO cells transfected with human MCSP D3 as target cells. The assay is performed as described in Example 18.

**Figure 20**
Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 21**
Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 22**
Plasma stability of MCSP and CD3 cross-species specific bispecific single chain antibodies tested by the measurement of cytotoxicity activity induced by samples of the designated single chain constructs incubated with 50% human plasma at 37°C and 4°C for 24 hours respectively or with addition of 50% human plasma immediately prior to cytotoxicity testing or without addition of plasma. CHO cells transfected with human MCSP are used as target cell line and stimulated CD4-/CD56- human PBMCs are used as effector cells. The assay is performed as described in Example 19.

**Figure 23**
Initial drop and recovery (i.e. redistribution) of absolute T cell counts (open squares), in peripheral blood of B-NHL patients (patent numbers 1, 7, 23, 30, 31, and 33 of Table 4), who had essentially no circulating CD19-positive target B cells (filled triangles), during the starting phase of intravenous infusion with the CD3 binding molecule CD19xCD3 recognizing a conventional context dependent CD3 epitope. Absolute cell counts are given in 1000 cells per microliter blood. The first data point shows baseline counts immediately prior to the start of infusion. The CD19xCD3 dose is given in parentheses beside the patient number.

**Figure 24**

(A) Repeated T cell redistribution (open squares) in B-NHL patient #19 (Table 4) who had no circulating CD19-positive target B cells (filled triangles) and developed CNS symptoms under continuous intravenous infusion with CD19xCD3 at a starting dose of $5\mu g/m^2/24h$ for one day followed by a sudden dose increase to $15\mu g/m^2/24h$. Absolute cell counts are given in 1000 cells per microliter blood. The first data point shows baseline counts immediately prior to the start of infusion. After recovery of circulating T cells from the first episode of redistribution triggered by the treatment start at $5\mu g/m^2/24h$ the stepwise dose increase from 5 to $15\mu g/m^2/24h$ triggered a second episode of T cell redistribution that was associated with the development of CNS symptoms dominated by confusion and disorientation.
(B) Repeated T cell redistribution in a B-NHL patient, who developed CNS symptoms under repeated intravenous bolus infusion with CD19xCD3 at $1.5\mu g/m^2$. Absolute cell counts are given in 1000 cells per microliter blood. The infusion time for each bolus administration was 2 to 4 hours. Vertical arrows indicate the start of bolus infusions. Data points at the beginning of each bolus administration show the T cell counts immediately prior to start of bolus infusion. Each bolus infusion triggered an episode of T cell redistribution followed by recovery of the T cell counts prior to the next bolus infusion. Finally the third episode of T cell redistribution was associated with the development of CNS symptoms in this patient.

**Figure 25**
Complex T cell redistribution pattern (open squares) in B-NHL patient #20 (Table 4) without circulating CD19-positive target B cells (filled triangles), during ramp initiation of the CD19xCD3 infusion i.e. even gradual increase of flow-rate from almost zero to $15\mu g/m^2/24h$ during the first 24 hours of treatment. Absolute cell counts are given in 1000 cells per microliter blood. The first data point shows baseline counts immediately prior to the start of infusion. The CD19xCD3 dose is given in parentheses beside the patient number. T cells reappearing in the circulating blood after the initial redistribution triggered by the first exposure to CD19xCD3 are partially induced to redisappear from circulating blood again by still increasing levels of CD19xCD3 during the ramp phase.

**Figure 26**
T and B cell counts during treatment with CD19xCD3 of B-NHL patient #13 (Table 4) who had a significant number of circulating CD19-positive target B (lymphoma) cells (filled triangles). Absolute cell counts are given in 1000 cells per microliter blood. The first data point shows baseline counts immediately prior to the start of infusion. The CD19xCD3 dose is given in parentheses beside the patient number. T cells (open squares) disappear completely from the circulation upon start of CD19xCD3 infusion and do not reappear until the circulating CD19-positive B (lymphoma) cells (filled triangles) are depleted from the peripheral blood.

**Figure 27**
Repeated T cell redistribution (open squares) in B-NHL patient #24 (Table 4), who had essentially no circulating CD19-positive target B cells (filled triangles) and developed CNS symptoms upon initiation of CD19xCD3 infusion without additional HSA as required for stabilisation of the drug (upper panel). After first recovery of circulating T cells from initial redistribution the uneven drug flow due to the lack of stabilizing HSA triggered a second episode of T cell redistribution that was associated with the development of CNS symptoms dominated by confusion and disorientation. When the same patient was restarted correctly with CD19xCD3 solution containing additional HSA for drug stabilisation, no repeated T cell redistribution was observed (lower panel) and the patient did not again develop any CNS symptoms. Absolute cell counts are given in 1000 cells per microliter blood. The first data point shows baseline counts immediately prior to the start of infusion. The CD19xCD3 dose is given in parentheses beside the patient number.

**Figure 28**
Model of T cell adhesion to endothelial cells induced by monovalent binding to context dependent CD3 epitopes. Monovalent interaction of a conventional CD3 binding molecule to its context dependent epitope on CD3 epsilon can lead to an allosteric change in the conformation of CD3 followed by the recruitment of Nck2 to the cytoplasmic domain of CD3 epsilon (Gil et al. (2002) Cell 109: 901). As Nck2 is directly linked to integrins via PINCH and ILK (Legate et al. (2006) Nat Rev Mol Cell Biol 7: 20), recruitment of Nck2 to the cytoplasmic domain of CD3 epsilon following an allosteric change in the conformation of CD3 through binding of a conventional CD3 binding molecule (like the CD19xCD3 of example 20) to its context dependent epitope on CD3 epsilon, can increase the adhesiveness of T cells to endothelial cells by transiently switching integrins on the T cell surface into their more adhesive isoform via inside-out-signalling.

**Figure 29**
Cytotoxic activity of CD33-AF5 VH-VL × I2C VH-VL test material used for the in vivo study in cynomolgus monkeys as described in Example 21. Specific lysis of CD33-positive target cells was determined in a standard $^{51}$Chromium release assay at increasing concentrations of CD33-AF5 VH-VL × I2C VH-VL. Assay duration was 18 hours. The macaque T cell line 4119 LnPx was used as source of effector cells. CHO cells transfected with cynomolgus CD33 served as target cells. Effector- to target cell ratio (E:T-ratio) was 10:1. The concentration of CD33-AF5 VH-VL × I2C VH-VL required for half-maximal target cell lysis (EC50) was calculated from the dose response curve with a value of 2.7 ng/ml.

**Figure 30**

**(A)** Dose- and time-dependent depletion of CD33-positive monocytes from the peripheral blood of cynomolgus monkeys through intravenous continuous infusion of CD33-AF5 VH-VL × I2C VH-VL as described in Example 21. The percentage relative to baseline (i.e. 100%) of absolute circulating CD33-positive monocyte counts after the duration of treatment as indicated above the columns is shown for each of two cynomolgus monkeys per dose level. The dose level (i.e. infusion flow-rate) is indicated below the columns. No depletion of circulating CD33-positive monocytes was observed in animals 1 and 2 treated for 7 days at a dose of 30 $\mu$g/m$^2$/24h. In animals 3 and 4 treated for 7 days at a dose of 60 $\mu$g/m$^2$/24h circulating CD33-positive monocyte counts were reduced to 68% and 40% of baseline, respectively. At 240 $\mu$g/m$^2$/24h circulating CD33-positive monocytes were almost completely depleted from the peripheral blood after 3 days of treatment (animals 5 and 6). At 1000 $\mu$g/m$^2$/24h depletion of circulating CD33-positive monocytes from the peripheral blood was completed already after 1 day of treatment (animals 7 and 8).
**(B)** Course of T cell and CD33-monocyte counts in peripheral blood of two cynomolgus monkeys during continuous infusion of CD33-AF5 VH-VL × I2C VH-VL for 14 days at 120 $\mu$g/m$^2$/24h. Absolute cell counts are given in 1000 cells per microliter blood. The first data point shows baseline counts immediately prior to the start of infusion. After initial mobilisation of CD33-monocytes during the first 12 hours upon start of infusion CD33-monocytes in peripheral blood (filled triangles) are depleted by two thirds (animal 10) and 50% (animal 9) relative to the respective baseline counts during the further course of infusion. Circulating T cell counts (open squares) show a limited initial drop followed by recovery still during the presence of circulating CD33-positive monocytic target cells.

**Figure 31**

Cytotoxic activity of MCSP-G4 VH-VL × I2C VH-VL test material used for the in vivo study in cynomolgus monkeys as described in Example 22. Specific lysis of MCSP-positive target cells was determined in a standard [51]Chromium release assay at increasing concentrations of MCSP-G4 VH-VL × I2C VH-VL. Assay duration was 18 hours. The macaque T cell line 4119 LnPx was used as source of effector cells. CHO cells transfected with cynomolgus MCSP served as target cells. Effector- to target cell ratio (E:T-ratio) was 10:1. The concentration of MCSP-G4 VH-VL × I2C VH-VL required for half-maximal target cell lysis (EC50) was calculated from the dose response curve with a value of 1.9 ng/ml.

**Figure 32**

Absence of initial episodes of drop and subsequent recovery of absolute T cell counts (i.e. redistribution) in peripheral blood of cynomolgus monkeys during the starting phase of intravenous infusion with the CD3 binding molecule MCSP-G4 VH-VL × I2C VH-VL recognizing an essentially context independent CD3 epitope. Absolute cell counts are given in 1000 cells per microliter blood. The first data point shows baseline counts immediately prior to the start of infusion. The MCSP-G4 VH-VL × I2C VH-VL dose is given in parentheses beside the animal number. In the known absence of MCSP-positive target cells from the circulating blood of cynomolgus monkeys there is no induction of T cell redistribution (i.e. an initial episode of drop and subsequent recovery of absolute T cell counts) through target cell mediated crosslinking of CD3. Moreover, induction of T cell redistribution (i.e. an initial episode of drop and subsequent recovery of absolute T cell counts) through a signal, which the T cells may receive through exclusive interaction with a CD3 binding site only, can be avoided by the use of CD3 binding molecules like MCSP-G4 VH-VL × I2C VH-VL recognizing an essentially context independent CD3 epitope.

**Figure 33**

FACS binding analysis of designated cross-species specific bispecific constructs to CHO cells transfected with human CD33, the human CD3+ T cell line HPB-ALL, CHO cells transfected with macaque CD33 and macaque PBMC respectively. The FACS staining is performed as described in Example 23.4. The bold lines represent cells incubated with 5 μg/ml purified bispecific single chain construct or cell culture supernatant of transfected cells expressing the cross-species specific bispecific antibody constructs. The filled histograms reflect the negative controls. Supernatant of untransfected CHO cells was used as negative control. For each cross-species specific bispecific single chain construct the overlay of the histograms shows specific binding of the construct to human and macaque CD33 and human and macaque CD3.

**Figure 34**

The diagrams show results of chromium release assays measuring cytotoxic activity induced by designated cross-species specific CD33 specific single chain constructs redirected to the indicated target cell lines. Effector cells were also used as indicated. The assays are performed as described in Example 23.5. The diagrams clearly demonstrate for each construct the potent recruitment of cytotoxic activity of human and macaque effector cells against human and macaque CD33 transfected CHO cells, respectively.

**Figure 35**

FACS binding analysis of designated cross-species specific bispecific single chain constructs to CHO cells transfected with human CAIX, the human CD3+ T cell line HPB-ALL, CHO cells transfected with macaque CAIX and the macaque T cell line 4119 LnPx respectively. The FACS staining was performed as described in Example 24.5. The bold lines represent cells incubated with 2 μg/ml purified bispecific single chain construct. The filled histograms reflect the negative controls. Supernatant of untransfected CHO cells was used as negative control for binding to the T cell lines. A single chain construct with irrelevant target specificity was used as negative control for binding to the CAIX transfected CHO cells. For each cross-species specific bispecific single chain construct the overlay of the histograms shows specific binding of the construct to human and macaque CAIX and human and macaque CD3.

**Figure 36**

The diagrams show results of chromium release assays measuring cytotoxic activity induced by designated cross-species specific CAIX specific single chain constructs redirected to the indicated target cell lines. Effector cells were also used as indicated. The assays are performed as described in Example 24.6. The diagrams clearly demonstrate for each construct the potent recruitment of cytotoxic activity of human and macaque effector cells against human and macaque CAIX transfected CHO cells respectively.

**Figure 37**

Cytotoxic activity induced by designated EpCAM specific single chain constructs against human EpCAM transfected CHO cells using human T cells as effector cells. The assay was performed as described in the example section. The diagrams clearly demonstrate potent recruitment of cytotoxic activity by each construct.

Effector cells: stimulated CD4/CD56 depleted human PBMC.
Target cells: CHO transfected with human EpCAM.

**Figure 38**
FACS binding analysis of designated bispecific single chain constructs to CHO cells transfected with human EpCAM (2a), untransfected CHO cells as EpCAM and CD3 negative cell line (2b), human PBMCs (2c) and the macaque T cell line 4119 LnPx (2d) respectively. Cells were incubated with supernatant containing the respective bispecific single chain construct. The FACS staining is performed as described in the example section. For each cross-species specific bispecific single chain construct the overlay of the histograms shows specific binding of the construct to human EpCAM and human and macaque CD3.

a: CHO-EpCAM (human)
b: CHO (untransfected)
c: human PBMCs
d: macaque 4119LnPx (CD3+).

**Figure 39**
FACS binding analysis of designated cross-species specific bispecific single chain constructs to CHO cells transfected with the human EGFRvIII, human CD3+ T cell line HPB-ALL, CHO cells transfected with cynomolgus EGFRvIII and a macaque T cell line 4119 LnPx. The FACS staining is performed as described in Example 26.3. The thick line represents cells incubated with 2 µg/ml purified monomeric protein that are subsequently incubated with the anti-his antibody and the PE labeled detection antibody. The thin histogram line reflects the negative control: cells only incubated with the anti-his antibody and the detection antibody.

**Figure 40**
Cytotoxic activity induced by designated cross-species specific single chain constructs redirected to indicated target cell lines. A) Stimulated CD4-/CD56- human PBMCs are used as effector cells, CHO cells transfected with human EGFRvIII as target cells. B) The macaque T cell line 4119 LnPx are used as effector cells, CHO cells transfected with cynomolgus EGFRvIII as target cells. The assay is performed as described in Example 26.3.

**Figure 41**
FACS binding analysis of designated cross-species specific bispecific single chain constructs to J558L cells transfected with human IgE, human CD3+ T cell line HPB-ALL, J558L cells transfected with macaque IgE and a macaque T cell line 4119 LnPx. The FACS staining is performed as described in Example 27.2. The thick line represents cells incubated with cell culture supernatant of CHO cells transfected with designated cross-species specific bispecific single chain construct that are subsequently incubated with the anti-his antibody and the PE labeled detection antibody. The thin histogram line reflects the negative control: cells only incubated with the anti-his antibody and the detection antibody.

**Figure 42**
Cytotoxic activity induced by designated cross-species specific single chain constructs redirected to indicated target cell lines. A) Stimulated CD4-/CD56- human PBMCs are used as effector cells, J558L cells transfected with human IgE as target cells. B) The macaque T cell line 4119 LnPx are used as effector cells, J558L cells transfected with macaque IgE as target cells. The assay is performed as described in Example **27.2.**

**Figure 43**
FACS binding analysis of designated cross-species specific bispecific single chain constructs to CHO cells transfected with human CD44, the human CD3+ T cell line HPB-ALL, CHO cells transfected with macaque CD44 and macaque PBMC respectively. The FACS staining was performed as described in Example 28.6. The bold lines represent cells incubated with 5 µg/ml purified bispecific single chain construct. The filled histograms reflect the negative controls. PBS with 2 % FCS was used as negative control. For each cross-species specific bispecific single chain construct the overlay of the histograms shows specific binding of the construct to human and macaque CD44 and human and macaque CD3.

**Figure 44**

FACS binding analysis of designated cross-species specific bispecific single chain constructs to CHO cells transfected with human CD44 lacking the v6 exon. The FACS staining is performed as described in Example 28.6. As control for expression of CD44 the CHO cells transfected with human CD44 lacking the v6 exon and the CHO cells transfected with the full length human CD44 were incubated with a anti-CD44 antibody. The bold lines represent cells incubated with 5 μg/ml purified bispecific single chain construct or the anti-CD44 antibody (5 μg/ml) as indicated. The filled histograms reflect the negative controls. PBS with 2 % FCS was used as negative control. For each cross-species specific bispecific single chain construct the overlay of the histograms shows lack of binding of the construct to human CD44 lacking the v6 exon. Presence of CD44 on the CHO cells transfected with human CD44 lacking the v6 exon was demonstrated by comparable binding of the anti-CD44 antibody to these cells and to the cells transfected with full length human CD44. FACS binding analysis showed the specificity of the cross-species specific bispecific single chain constructs for the v6 exon of CD44.

**Figure 45**

The diagram shows results of a chromium release assay measuring cytotoxic activity induced by designated cross-species specific CD44 specific single chain constructs redirected to CHO cells transfected with human CD44 as described in Example 28.1. As effector cells stimulated human PBMC depleted for CD4 and CD56 were used with an E:T ratio of 10:1. The assay is performed as described in Example 28.7. The diagram demonstrated for each construct the potent recruitment of cytotoxic activity of human effector cells against human CD44 transfected CHO cells.

**Figure 46**

FACS binding analysis of designated cross-species specific bispecific single chain constructs to CHO cells transfected with human CD30, the human CD30+ B cell line MEC-1, the human CD3+ T cell line HPB-ALL, CHO cells transfected with macaque CD30 and macaque PBMC respectively. The FACS staining was performed as described in Example 29.5. The bold lines represent cells incubated with 5 μg/ml purified bispecific single chain construct. The filled histograms reflect the negative controls. PBS with 2 % FCS was used as negative control. For each cross-species specific bispecific single chain construct the overlay of the histograms shows specific binding of the construct to human and macaque CD30 and human and macaque CD3.

**Figure 47**

The diagrams show results of chromium release assays measuring cytotoxic activity induced by designated cross-species specific CD30 specific single chain constructs redirected to the indicated target cell lines. Effector cells were also used as indicated. The assays were performed as described in Example 29.6. The diagrams clearly demonstrate for each construct the potent recruitment of cytotoxic activity of human and macaque effector cells against cells positive for human and macaque CD30, respectively.

**Figure 48**

FACS binding analysis of designated cross-species specific bispecific single chain constructs to CHO cells transfected with human HER2, the human CD3+ T cell line HPB-ALL, CHO cells transfected with macaque HER2 and the macaque T cell line 4119LnPx respectively. The FACS staining was performed as described in Example 30.5. The bold lines represent cells incubated with 2 μg/ml purified bispecific single chain construct. The thin lines reflect the negative controls. PBS with 2% FCS was used as negative control. For each cross-species specific bispecific single chain construct the overlay of the histograms shows specific binding of the construct to human and macaque HER2 and human and macaque CD3.

**Figure 49**

The diagrams show results of chromium release assays measuring cytotoxic activity induced by designated cross-species specific HER2 specific single chain constructs redirected to the indicated target cell lines. Effector cells were also used as indicated. The assays were performed as described in Example 30.6. The diagrams clearly demonstrate for each construct shown the potent recruitment of cytotoxic activity of human and macaque effector cells against human and macaque HER2 transfected CHO cells, respectively.

**Figure 50**

SDS PAGE gel and Western blot monitoring the purification of the cross-species specific bispecific single chain molecule designated E292F3 HL × I2C HL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 31.2. The Western blot detecting the histidine$_6$ tag confirms the identity of the protein band in the eluate as the cross-species specific bispecific single chain molecule. The faint signal for the flow through sample in this sensitive detection method further shows the nearly complete capture of bispecific single chain molecules by the purification method.

**Figure 51**

SDS PAGE gel and Western blot monitoring the purification of the cross-species specific bispecific single chain molecule designated V207C12 HL × H2C HL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 31.2. The Western blot detecting the histidine$_6$ tag confirms the identity of the protein band in the eluate as the cross-species specific bispecific single chain molecule. The faint signal for the flow through sample in this sensitive detection method further shows the nearly complete capture of bispecific single chain molecules by the purification method.

**Figure 52**

SDS PAGE gel and Western blot monitoring the purification of the cross-species specific bispecific single chain molecule designated AF5HLxF12QHL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 31.2. The Western blot detecting the histidine$_6$ tag confirms the identity of the protein band in the eluate as the cross-species specific bispecific single chain molecule. The signal in the flow through sample in this sensitive detection method is explained by saturation of the affinity column due to the high concentration of bispecific single chain molecules in the supernatant.

**Figure 53**

Standard curve of AF5HLxI2CHL in 50% macaque monkey serum. The upper diagram shows the standard curve generated for the assay as described in Example 32.2.
The lower diagram shows results for quality control samples of AF5HLxI2CHL in 50% macaque monkey serum. The recovery rates are above 90% for the high and mid QC sample and above 80% for the low QC sample.
Thus the assay allows for detection of AF5HLxI2CHL in serum samples in the range from 10 ng/ml to 200 ng/ml (before dilution).

**Figure 54**

Standard curve of MCSP-G4 HL × I2C HL in 50% macaque monkey serum. The upper diagram shows the standard curve generated for the assay as described in Example 32.2.
The lower diagram shows results for quality control samples of MCSP-G4 HL × I2C HL in 50% macaque monkey serum. The recovery rates are above 98% for the high and mid QC sample and above 85% for the low QC sample. Thus the assay allows for detection of MCSP-G4 HL × I2C HL in serum samples in the range from 10 ng/ml to 200 ng/ml (before dilution).

**Figure 55**

FACS binding analysis of an anti-Flag antibody to CHO cells transfected with the 1-27 N-terminal amino acids of CD3 epsilon of the designated species fused to cynomolgus EpCAM. The FACS staining was performed as described in Example 33.1. The bold lines represent cells incubated with the anti-Flag antibody. The filled histograms reflect the negative controls. PBS with 2 % FCS was used as negative control. The histograms show strong and comparable binding of the anti-Flag antibody to all transfectants indicating strong and equal expression of the transfected constructs.

**Figure 56**

FACS binding analysis of the I2C IgG1 construct to CHO cells expressing the 1-27 N-terminal amino acids of CD3 epsilon of the designated species fused to cynomolgus EpCAM. The FACS staining is performed as described in Example 33.3. The bold lines represent cells incubated with 50 μl cell culture supernatant of cells expressing the I2C IgG1 construct. The filled histograms reflect the negative control. Cells expressing the 1-27 N-terminal amino acids of CD3 epsilon of swine fused to cynomolgus EpCAM were used as negative control. In comparison with the negative control the histograms clearly demonstrate binding of the I2C IgG1 construct to 1-27 N-terminal amino acids of CD3 epsilon of human, marmoset, tamarin and squirrel monkey.

**Figure 57**

FACS binding analysis of the I2C IgG1 construct as described in Example 33.2 to human CD3 with and without N-terminal His6 tag as described in Examples 6.1 and 5.1 respectively. The bold lines represent cells incubated with the anti-human CD3 antibody UCHT-1, the penta-His antibody (Qiagen) and cell culture supernatant of cells expressing the I2C IgG1 construct respectively as indicated. The filled histograms reflect cells incubated with an irrelevant murine IgG1 antibody as negative control.

The upper two histogram overlays show comparable binding of the UCHT-1 antibody to both transfectants as compared to the isotype control demonstrating expression of both recombinant constructs. The centre histogram overlays show binding of the penta his antibody to the cells expressing the His6-human CD3 epsilon chain (His6-CD3) but not to the cells expressing the wild-type CD3 epsilon chain (WT-CD3). The lower Histogram overlays show binding of the I2C IgG1 construct to the wild-type human CD3 epsilon chain but not to the His6-human CD3 epsilon chain. These results demonstrate that a free N-terminus is essential for binding of the cross-species specific anti-CD3 binding molecule I2C to the CD3 epsilon chain.

**Figure 58**

FACS binding analysis of designated cross-species specific bispecific single chain constructs to CHO cells transfected with human MCSP D3, the human CD3+ T cell line HPB-ALL, CHO cells transfected with macaque MCSP D3 and the macaque T cell line 4119 LnPx respectively. The FACS staining was performed as described in Example 17. The bold lines represents cells incubated with 2 μg/ml purified bispecific single chain construct or cell supernatant containing the bispecific single chain construct respectively. The filled histograms reflect the negative controls. Supernatant of untransfected CHO cells was used as negative control for binding to the T cell lines. A single chain construct with irrelevant target specificity was used as negative control for binding to the MCSP D3 transfected CHO cells. For each cross-species specific bispecific single chain construct the overlay of the histograms shows specific binding of the construct to human and macaque MCSP D3 and human and macaque CD3.

**Figure 59**

Cytotoxic activity induced by designated cross-species specific MCSP D3 specific single chain constructs redirected to the indicated target cell lines. Effector cells and effector to target ratio were also used as indicated. The assay is performed as described in Example 18. The diagrams clearly demonstrate potent cross-species specific recruitment of cytotoxic activity by each construct.

**Figure 60**

FACS binding analysis of designated cross-species specific bispecific single chain constructs to CHO cells transfected with human CD33, the human CD3+ T cell line HPB-ALL, CHO cells transfected with macaque CD33 and macaque PBMC respectively. The FACS staining was performed as described in Example 36.2. The bold lines represent cells incubated with cell culture supernatant of transfected cells expressing the cross-species specific bispecific antibody constructs. The filled histograms reflect the negative controls. Supernatant of untransfected CHO cells was used as negative control. For each cross-species specific bispecific single chain construct the overlay of the histograms shows specific binding of the construct to human and macaque CD33 and human and macaque CD3.

**Figure 61**

The diagrams show results of chromium release assays measuring cytotoxic activity induced by designated cross-species specific CD33 specific single chain constructs redirected to the indicated target cell lines. Effector cells were also used as indicated. The assays are performed as described in Example 36.3. The diagrams clearly demonstrate for each construct the potent recruitment of cytotoxic activity of human and macaque effector cells against human and macaque CD33 transfected CHO cells, respectively.

**Figure 62**

T cell redistribution in a chimpanzee under weekly intravenous bolus infusion with PBS/5% HSA and PBS/5% HSA plus single-chain EpCAM/CD3-bispecific antibody construct at doses of 1.6, 2.0, 3.0 and 4.5 μg/kg. The infusion time for each bolus administration was 2 hours. Vertical arrows indicate the start of bolus infusions. Data points at the beginning of each bolus administration show the T cell counts immediately prior to start of bolus infusion. Each bolus infusion of the single-chain EpCAM/CD3-bispecific antibody construct, which recognizes a conventional context dependent CD3 epitope, triggered an episode of T cell redistribution followed by recovery of T cells to baseline values prior to the next bolus infusion.

**Figure 63**

CD3 specific ELISA analysis of periplasmic preparations containing Flag tagged scFv protein fragments from selected clones. Periplasmic preparations of soluble scFv protein fragments were added to wells of an ELISA plate, which had been coated with soluble human CD3 epsilon (aa 1-27)- Fc fusion protein and had been additionally blocked with PBS 3% BSA. Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an ELISA reader. Clone names are presented on the x axis.

**Figure 64**

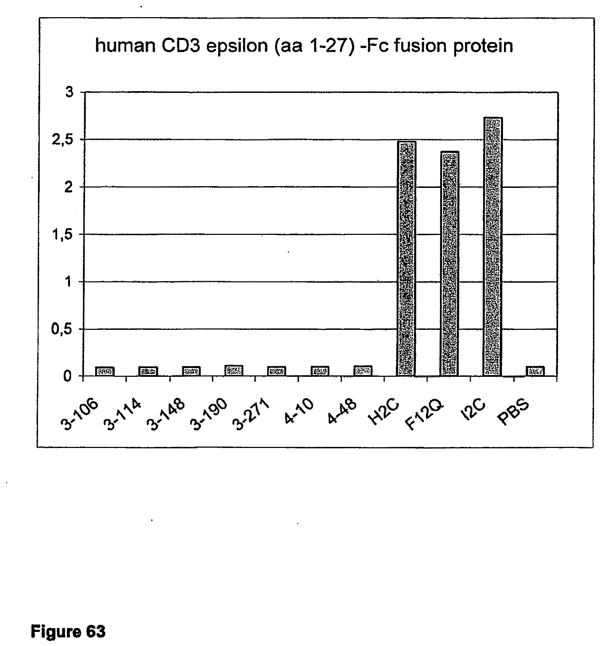

ELISA analysis of periplasmic preparations containing Flag tagged scFv protein fragments from selected clones. The same periplasmic preparations of soluble scFv protein fragments as in Figure 63 were added to wells of an ELISA plate which had not been coated with human CD3 epsilon (aa 1-27)- Fc fusion protein but with huIgG1 (Sigma) and blocked with 3% BSA in PBS.

Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an ELISA reader. Clone names are presented on the x axis.

**Figure 65**

FACS binding analysis of designated cross-species specific bispecific single chain constructs to CHO cells transfected with human CD44, the human CD3+ T cell line HPB-ALL, CHO cells transfected with macaque CD44 and the macaque T cell line 4119 LnPx respectively. CHO cells transfected with human CD44delv6 were used as control to emphasize the specificity of designated cross-species specific bispecific single chain constructs to CD44. The FACS staining is performed as described in Example 39.3. The bold lines represent cells incubated with cell supernatant containing the bispecific single chain construct. The filled histograms reflect the negative controls. Supernatant of untransfected CHO cells was used as negative control for binding to the T cell lines. A single chain construct with irrelevant target specificity was used as negative control for binding to the CD44 and CD44delv6 transfected CHO cells. For each cross-species specific bispecific single chain construct the overlay of the histograms shows specific binding of the construct to human and macaque CD44 and human and macaque CD3. The overlay of the histograms for each construct shows no specific binding to the human CD44delv6 transfected CHO cells.

**Figure 66**

Cytotoxic activity induced by designated cross-species specific CD44 specific single chain constructs redirected to the indicated target cell lines. Effector cells and effector to target ratio were also used as indicated. The assay is performed as described in Example 39.4. The diagrams clearly demonstrate the potency of mediating cytotoxicity by each of the bispecific constructs.

**Figure 67**

FACS binding analysis of designated cross-species specific bispecific single chain constructs as described in example 41.

**Figure 68**

Cytotoxic activity induced by designated cross-species specific binding molecules as described in example 41.
The present invention is additionally described by way of the following illustrative non-limiting examples that provide a better understanding of the present invention and of its many advantages.

**EXAMPLES**

**1. Identification of CD3epsilon sequences from blood samples of non-human primates**

Blood samples of the following non-human primates were used for CD3epsilon-identification: *Callithrix jacchus, Saguinus oedipus* and *Saimiris ciureus.* Fresh heparin-treated whole blood samples were prepared for isolating total cellular RNA according to manufacturer's protocol (QIAamp RNA Blood Mini Kit, Qiagen). The extracted mRNA was transcribed into cDNA according to published protocols. In brief, 10 μl of precipitated RNA was incubated with 1.2 μl of 10x hexanucleotide mix (Roche) at 70 °C for 10 minutes and stored on ice. A reaction mix consisting of 4 μl of 5x superscript II buffer, 0.2 μl of 0.1M dithiothreitole, 0.8 μl of superscript II (Invitrogen), 1.2 μl of desoxyribonucleoside triphosphates (25 μM), 0.8 μl of RNase Inhibitor (Roche) and 1.8 μl of DNase and RNase free water (Roth) was added. The reaction mix was incubated at room temperature for 10 minutes followed by incubation at 42 °C for 50 minutes and at 90 °C for 5 minutes. The reaction was cooled on ice before adding 0.8 μl of RNaseH (1 U/μl, Roche) and incubated

for 20 minutes at 37 °C.

The first-strand cDNAs from each species were subjected to separate 35-cycle polymerase chain reactions using Taq DNA polymerase (Sigma) and the following primer combination designed on database research: forward primer 5'-AGAGTTCTGGGCCTCTGC-3' (SEQ ID NO: 377); reverse primer 5'-CGGATGGGCTCATAGTCTG-3' (SEQ ID NO: 378);. The amplified 550 bp-bands were gel purified (Gel Extraction Kit, Qiagen) and sequenced (Sequiserve, Vaterstetten/Germany, see sequence listing).

CD3epsilon *Callithrix jacchus*

Nucleotides

CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTCGTAAATA
GTCAAAACAAAGAAGGTCATGAGGACCACCTGTTACTGGAGGACTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT

Amino acids

QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD

CD3epsilon *Saguinus oedipus*

Nucleotides

CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTTGTAAATA
GTCAAAACAAAGAAGGTCATGAGGACCACCTGTTACTGGAGGATTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT

Amino acids

QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD

CD3epsilon *Saimiris ciureus*

Nucleotides

CAGGACGGTAATGAAGAGATTGGTGATACTACCCAGAACCCATATAAAGTTTCCATCTCAGG

AACCACAGTAACACTGACATGCCCTCGGTATGATGGACAGGAAATAAAATGGCTCGTAAATG

ATCAAAACAAAGAAGGTCATGAGGACCACCTGTTACTGGAAGATTTTTCAGAAATGGAACAA

AGTGGTTATTATGCCTGCCTCTCCAAAGAGACCCCCACAGAAGAGGCGAGCCATTATCTCTA

CCTGAAGGCAAGAGTGTGTGAGAACTGCGTGGAGGTGGAT

Amino acids

QDGNEEIGDTTQNPYKVSISGTTVTLTCPRYDGQEIKWLVNDQNKEGHEDHLLLEDFSEMEQ

SGYYACLSKETPTEEASHYLYLKARVCENCVEVD

**2. Generation of cross-species specific single chain antibody fragments (scFv) binding to the N-terminal amino acids 1-27 of CD3epsilon of man and different non-chimpanzee primates**

**2.1. Immunization of mice using the N-terminus of CD3epsilon separated from its native CD3-context by fusion to a heterologous soluble protein**

Ten weeks old F1 mice from balb/c × C57black crossings were immunized with the CD3epsilon-Fc fusion protein carrying themost N-terminal amino acids 1-27 of the mature CD3epsilon chain (1-27 CD3-Fc) of man and/or saimiris ciureus. To this end 40 μg of the 1-27 CD3-Fc fusion protein with 10 nmol of a thioate-modified CpG-Oligonucleotide (5'-tccatgacgttcctgatgct-3') in 300 ul PBS were injected per mouse intra-peritoneally. Mice receive booster immunizations after 21, 42 and optionally 63 days in the same way. Ten days after the first booster immunization, blood samples were taken and antibody serum titer against 1-27 CD3-Fc fusion protein iwa tested by ELISA. Additionally, the titer against the CD3-positive human T cell line HPBall was tested in flow cytometry according to standard protocols. Serum titers were significantly higher in immunized than in non-immunized animals.

**2.2. Generation of an immune murine antibody scFv library: Construction of a combinatorial antibody library and phage display**

Three days after the last injection the murine spleen cells were harvested for the preparation of total RNA according to standard protocols.

A library of murine immunoglobuline (Ig) light chain (kappa) variable region (VK) and Ig heavy chain variable region (VH) DNA-fragments was constructed by RT-PCR on murine spleen RNA using VK-and VH specific primer. cDNA was synthesized according to standard protocols.

The primers were designed in a way to give rise to a 5'-Xhol and a 3'-BstEII recognition site for the amplified heavy chain V-fragments and to a 5'-Sacl and a 3'-Spel recognition site for amplified VK DNA fragments.

For the PCR-amplification of the VH DNA-fragments eight different 5'-VH-family specific primers (MVH1(GC)AG GTG CAG CTC GAG GAG TCA GGA CCT; MVH2 GAG GTC CAG CTC GAG CAG TCT GGA CCT; MVH3 CAG GTC CAA CTC GAG CAG CCT GGG GCT; MVH4 GAG GTT CAG CTC GAG CAG TCT GGG GCA; MVH5 GA(AG) GTG AAG CTC GAG GAG TCT GGA GGA; MVH6 GAG GTG AAG CTT CTC GAG TCT GGA GGT; MVH7 GAA GTG AAG CTC GAG GAG TCT .GGG GGA; MVH8 GAG GTT CAG CTC GAG CAG TCT GGA GCT) were each combined with one 3'-VH primer (3'MuVHBstEII tga gga gac ggt gac cgt ggt ccc ttg gcc cca g); for the PCR amplification of the VK-chain fragments seven different 5'-VK-family specific primers (MUVK1 CCA GTT CCG AGC TCG TTG TGA CTC AGG AAT CT; MUVK2 CCA GTT CCG AGC TCG TGT TGA CGC AGC CGC CC; MUVK3 CCA GTT CCG AGC TCG TGC TCA CCC AGT CTC CA; MUVK4 CCA GTT CCG AGC TCC AGA TGA CCC AGT CTC CA; MUVK5 CCA GAT GTG AGC TCG TGA TGA CCC AGA CTC CA; MUVK6 CCA GAT GTG AGC TCG TCA TGA CCC AGT CTC CA; MUVK7 CCA GTT CCG AGC TCG TGA TGA CAC AGT CTC CA) were each combined with one 3'-VK primer (3'MuVkHindlII/BsiW1 tgg tgc act agt cgt acg ttt gat ctc aag ctt ggt ccc).

The following PCR program was used for amplification: denaturation at 94°C for 20 sec; primer annealing at 52°C for 50 sec and primer extension at 72°C for 60 sec and 40 cycles, followed by a 10 min final extension at 72°C.

450 ng of the kappa light chain fragments (Sacl-Spel digested) were ligated with 1400 ng of the phagemid pComb3H5Bhis (Sacl-Spel digested; large fragment). The resulting combinatorial antibody library was then transformed into 300 ul of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells were then harvested by centrifugation and plasmid preparation was carried out using a commercially available plasmid preparation kit (Qiagen).

2800 ng of this plasmid-DNA containing the VK-library (XhoI-BstEII digested; large fragment) were ligated with 900 ng of the heavy chain V-fragments (XhoI-BstEII digested) and again transformed into two 300 ul aliquots of electrocompetent E. coli XL1 Blue cells by electroporation (2.5 kV, 0.2 cm gap cuvette, 25 uFD, 200 Ohm) resulting in a total VH-VK scFv (single chain variable fragment) library size of more than $10^7$ independent clones.

After phenotype expression and slow adaptation to carbenicillin, the E. coli cells containing the antibody library were transferred into SB-Carbenicillin (50 ug/mL) selection medium. The E. coli cells containing the antibody library wass then infected with an infectious dose of $10^{12}$ particles of helper phage VCSM13 resulting in the production and secretion of filamentous M13 phage, wherein phage particle contains single stranded pComb3H5BHis-DNA encoding a murine scFv-fragment and displayed the corresponding scFv-protein as a translational fusion to phage coat protein III. This pool of phages displaying the antibody library was later used for the selection of antigen binding entities.

## 2.3. Phage display based selection of CD3-specific binders

The phage library carrying the cloned scFv-repertoire was harvested from the respective culture supernatant by PEG8000/NaCl precipitation and centrifugation. Approximately $10^{11}$ to $10^{12}$ scFv phage particles were resuspended in 0.4 ml of PBS/0.1% BSA and incubated with $10^5$ to $10^7$ Jurkat cells (a CD3-positive human T-cell line) for 1 hour on ice under slow agitation. These Jurkat cells were grown beforehand in RPMI medium enriched with fetal calf serum (10 %), glutamine and penicillin/streptomycin, harvested by centrifugation, washed in PBS and resuspended in PBS/1 % FCS (containing Na Azide). scFv phage which do not specifically bind to the Jurkat cells were eliminated by up to five washing steps with PBS/1 % FCS (containing Na Azide). After washing, binding entities were eluted from the cells by resuspending the cells in HCl-glycine pH 2.2 (10 min incubation with subsequent vortexing) and after neutralization with 2 M Tris pH 12, the eluate was used for infection of a fresh uninfected E. coli XL1 Blue culture (OD600 > 0.5). The E. coli culture containing E. coli cells successfully transduced with a phagemid copy, encoding a human scFv-fragment, were again selected for carbenicillin resistance and subsequently infected with VCMS 13 helper phage to start the second round of antibody display and in vitro selection. A total of 4 to 5 rounds of selections were carried out, normally.

## 2.4. Screening for CD3-specific binders

Plasmid DNA corresponding to 4 and 5 rounds of panning was isolated from E. coli cultures after selection. For the production of soluble scFv-protein, VH-VL-DNA fragments were excised from the plasmids (XhoI-SpeI). These fragments were cloned via the same restriction sites in the plasmid pComb3H5BFlag/His differing from the original pComb3H5BHis in that the expression construct (e.g. scFv) includes a Flag-tag (TGD YKDDDDK) between the scFv and the His6-tag and the additional phage proteins were deleted. After ligation, each pool (different rounds of panning) of plasmid DNA was transformed into 100 μl heat shock competent E. coli TG1 or XLI blue and plated onto carbenicillin LB-agar. Single colonies were picked into 100 ul of LB carb (50 ug/ml).

E. coli transformed with pComb3H5BHis containing a VL-and VH-segment produce soluble scFv in sufficient amounts after excision of the gene III fragment and induction with 1 mM IPTG. Due to a suitable signal sequence, the scFv-chain was exported into the periplasma where it folds into a functional conformation.

Single E. coli TG1 bacterial colonies from the transformation plates were picked for periplasmic small scale preparations and grown in SB-medium (e.g. 10 ml) supplemented with 20 mM MgCl2 and carbenicillin 50 μg/ml (and re-dissolved in PBS (e.g. 1 ml) after harvesting. By four rounds of freezing at -70°C and thawing at 37°C, the outer membrane of the bacteria was destroyed by temperature shock and the soluble periplasmic proteins including the scFvs were released into the supernatant. After elimination of intact cells and cell-debris by centrifugation, the supernatant containing the human anti-human CD3-scFvs was collected and used for further examination.

## 2.5. Identification of CD3-specific binders

Binding of the isolated scFvs was tested by flow cytometry on eukaryotic cells, which on their surface express a heterologous protein displaying at its N-terminus the first 27 N-terminal amino acids of CD3epsilon.

As described in Example 4, the first amino acids 1-27 of the N-terminal sequence of the mature CD3 epsilon chain of the human T cell receptor complex (amino acid sequence: QDGNEEMGGITQTPYKVSISGTTVILT) were fused to the N-terminus of the transmembrane protein EpCAM so that the N-terminus was located at the outer cell surface. Additionally, a FLAG epitope was inserted between the N-terminal 1-27 CD3epsilon sequence and the EpCAM sequence. This fusion product was expressed in human embryonic kidney (HEK) and chinese hamster ovary (CHO) cells. Eukaryotic cells displaying the 27 most N-terminal amino acids of mature CD3epsilon of other primate species were prepared in the same way for Saimiri ciureus (Squirrel monkey) (CD3epsilon N-terminal amino acid sequence: QDGNEEIGDTTQNPYKVSISGTTVTLT), for Callithrix jacchus (CD3epsilon N-terminal amino acid sequence: QDGNEEMGDTTQNPYKVSISGTTVTLT) and for Saguinus oedipus (CD3epsilon N-terminal amino acid sequence: QDGNEEMGDTTQNPYKVSISGTTVTL T).

For flow cytometry $2{,}5 \times 10^5$ cells are incubated with 50 ul supernatant or with 5 $\mu$g/ml of the purified constructs in 50 $\mu$l PBS with 2% FCS. The binding of the constructs was detected with an anti-His antibody (Penta-His Antibody, BSA free, Qiagen GmbH, Hilden, FRG) at 2 $\mu$g/ml in 50 $\mu$l PBS with 2% FCS. As a second step reagent a R-Phycoerythrin-conjugated affinity purified F(ab')2 fragment, goat anti-mouse IgG (Fc-gamma fragment specific), diluted 1:100 in 50 $\mu$l PBS with 2% FCS (Dianova, Hamburg, FRG) was used. The samples were measured on a FACSscan (BD biosciences, Heidelberg, FRG).

Binding was always confirmed by flowcytometry as described in the foregoing paragraph on primary T cells of man and different primates (e.g. saimiris ciureus, callithrix jacchus, saguinus oedipus).

### 2.6. Generation of human/humanized equivalents of non-human CD3epsilon specific scFvs

The VH region of the murine anti-CD3 scFv was aligned against human antibody germline amino acid sequences. The human antibody germline VH sequence was chosen which has the closest homology to the non-human VH and a direct alignment of the two amino acid sequences was performed. There were a number of framework residues of the non-human VH that differ from the human VH framework regions ("different framework positions"). Some of these residues may contribute to the binding and activity of the antibody to its target.

To construct a library that contain the murine CDRs and at every framework position that differs from the chosen human VH sequence both possibilities (the human and the maternal murine amino acid residue), degenerated oligonucleotides were synthesized. These oligonucleotides incorporate at the differing positions the human residue with a probability of 75 % and the murine residue with a probability of 25 %. For one human VH e.g. six of these oligonucleotides had to be synthesized that overlap in a terminal stretch of approximately 20 nucleotides. To this end every second primer was an antisense primer. Restriction sites needed for later cloning within the oligonucleotides were deleted.

These primers may have a length of 60 to 90 nucleotides, depending on the number of primers that were needed to span over the whole V sequence.

These e.g. six primers were mixed in equal amounts (e.g. 1 $\mu$l of each primer (primer stocks 20 to 100 $\mu$M) to a 20 $\mu$l PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

This PCR product was then used as a template for a standard PCR reaction using primers that incorporate N-terminal and C-terminal suitable cloning restriction sites. The DNA fragment of the correct size (for a VH approximately 350 nucleotides) was isolated by agarose gel electrophoresis according to standard methods. In this way sufficient VH DNA fragment was amplified. This VH fragment was now a pool of VH fragments that have each one a different amount of human and murine residues at the respective differing framework positions (pool of humanized VH). The same procedure was performed for the VL region of the murine anti-CD3 scFv (pool of humanized VL).

The pool of humanized VH was then combined with the pool of humanized VL in the phage display vector pComb3H5Bhis to form a library of functional scFvs from which - after display on filamentous phage - anti-CD3 binders were selected, screened, identified and confirmed as described above for the parental non-human (murine) anti-CD3 scFv. Single clones were then analyzed for favorable properties and amino acid sequence. Those scFvs which were closest in amino acid sequence homology to human germline V-segments are preferred particularly those wherein at least one CDR among CDR I and II of VH and CDR I and II of VLkappa or CDR I and II of VLlambda shows more than 80% amino acid sequence identity to the closest respective CDR of all human germline V-segments. Anti-CD3 scFvs were converted into recombinant bispecific single chain antibodies as described in the following Examples 10 and 16 and further characterized.

### 3. Generation of a recombinant fusion protein of the N-terminal amino acids 1-27 of the human CD3 epsilon chain fused to the Fc-part of an IgG1 (1-27 CD3-Fc).

### 3.1. Cloning and expression of 1-27 CD3-Fc

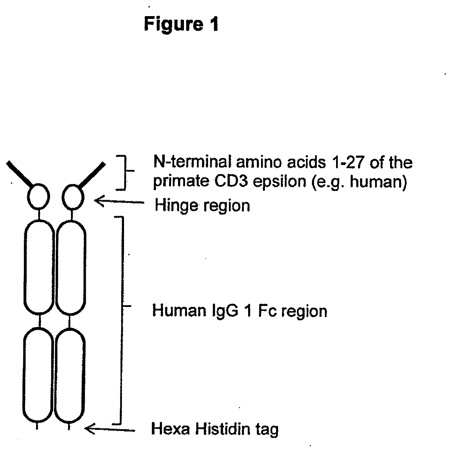

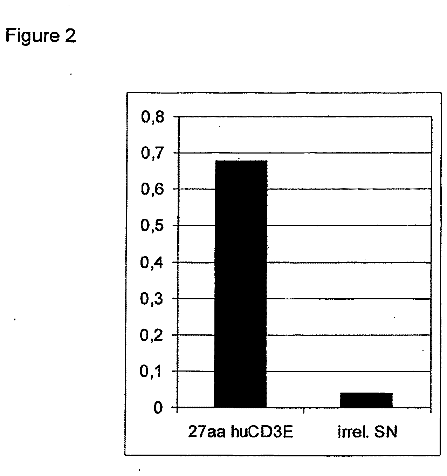

The coding sequence of the 1-27 N-terminal amino acids of the human CD3 epsilon chain fused to the hinge and Fc gamma region of human immunoglobulin IgG1 as well as an 6 Histidine Tag were obtained by gene synthesis according to standard protocols (cDNA sequence and amino acid sequence of the recombinant fusion protein are listed under SEQ ID NOs 350 and 349). The gene synthesis fragment was designed as to contain first a Kozak site for eukaryotic expression of the construct, followed by an 19 amino acid immunoglobulin leader peptide, followed in frame by the coding sequence of the first 27 amino acids of the extracellular portion of the mature human CD3 epsilon chain, followed in frame by the coding sequence of the hinge region and Fc gamma portion of human IgG1, followed in frame by the coding sequence of a 6 Histidine tag and a stop codon (Figure 1). The gene synthesis fragment was also designed as to introduce restriction sites at the beginning and at the end of the cDNA coding for the fusion protein. The introduced restriction sites, EcoRI at the 5' end and Sall at the 3' end, are utilized in the following cloning procedures. The gene synthesis fragment was cloned via EcoRI and Sall into a plasmid designated pEF-DHFR (pEF-DHFR is described in Mack et al. Proc. Natl. Acad. Sci. USA 92 (1995) 7021-7025) following standard protocols. A sequence verified plasmid was used for transfection in the FreeStyle 293 Expression System (Invitrogen GmbH, Karlsruhe, Germany) according to the manufacturers protocol. After 3 days cell culture supernatants of the transfectants were harvested and tested for the presence of the recombinant construct in an ELISA assay. Goat anti-human IgG, Fc-gamma fragment specific antibody (obtained from Jackson ImmunoResearch Europe Ltd., Newmarket, Suffolk, UK) was diluted in PBS to 5 $\mu$g/ml and coated with 100 $\mu$l per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with 3 % BSA in PBS (bovine Albumin, fraction V, Sigma-Aldrich Chemie GmbH, Taufkirchen, Germany) for 60 minutes at room temperature (RT). Subsequently, wells were washed again PBS/Tween and then incubated with cell culture supernatants for 60 minutes at RT. After washing wells were incubated with a peroxidase conjugated anti-His6 antibody (Roche Diagnostics GmbH, Roche Applied Science, Mannheim, Germany) diluted 1:500 in PBS with 1 % BSA for 60 minutes at RT. Subsequently, wells were washed with 200 $\mu$l PBS/Tween and 100 $\mu$l of the SIGMAFAST OPD (SIGMAFAST OPD [o-Phenylenediamine dihydrochloride] substrate solution (Sigma-Aldrich Chemie GmbH, Taufkirchen, Germany) was added according to the manufacturers protocol. The reaction was stopped by adding 100 $\mu$l 1 M $H_2SO_4$. Color reaction was measured on a PowerWaveX microplate spectrophotometer (BioTek Instruments, Inc., Winooski, Vermont, USA) at 490 nm and subtraction of background absorption at 620 nm. As shown in Figure 2 presence of the construct as compared to irrelevant supernatant of mock-transfected HEK 293 cells used as negative control was clearly detectable.

**3.2. Binding assay of cross-species specific single chain antibodies to 1-27 CD3-Fc.**

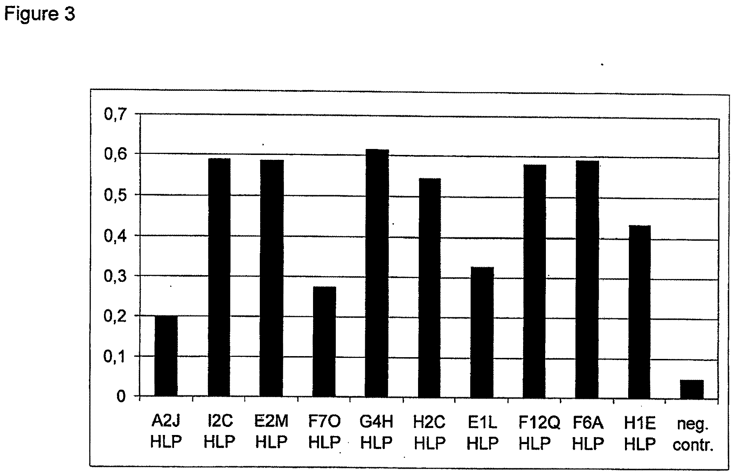

Binding of crude preparations of periplasmatically expressed cross-species specific single chain antibodies specific for CD3 epsilon to 1-27 CD3-Fc was tested in an ELISA assay. Goat anti-human IgG, Fc-gamma fragment specific antibody (Jackson ImmunoResearch Europe Ltd., Newmarket, Suffolk, UK) was diluted in PBS to 5 $\mu$g/ml and coated with 100 $\mu$l per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with PBS with 3 % BSA (bovine Albumin, fraction V, Sigma-Aldrich Chemie GmbH, Taufkirchen, Germany) for 60 minutes at RT. Subsequently, wells were washed with PBS/Tween and incubated with supernatants of cells expressing the 1-27 CD3-Fc construct for 60 minutes at RT. Wells were washed with PBS/Tween and incubated with crude preparations of periplasmatically expressed cross-species specific single-chain antibodies as described above for 60 minutes at room temperature. After washing with PBS/Tween wells were incubated with peroxidase conjugated anti-Flag M2 antibody (Sigma-Aldrich Chemie GmbH, Taufkirchen, Germany) diluted 1:10000 in PBS with 1 % BSA for 60 minutes at RT. Wells were washed with PBS/Tween and incubated with 100 $\mu$l of the SIGMAFAST OPD (OPD [o-Phenylenediamine dihydrochloride] substrate solution (Sigma-Aldrich Chemie GmbH, Taufkirchen, Germany) according to the manufacturers protocol. Color reaction was stopped with 100 $\mu$l 1 M $H_2SO_4$ and measured on a PowerWaveX microplate spectrophotometer (BioTek Instruments, Inc., Winooski, Vermont, USA) at 490 nm and subtraction of background absorption at 620 nm. Strong binding of cross-species specific human single chain antibodies specific for CD3 epsilon to the 1-27 CD3-Fc construct compared to a murine anti CD3 single-chain antibody was observed (Figure 3).

**4. Generation of recombinant transmembrane fusion proteins of the N-terminal amino acids 1-27 of CD3 epsilon from different non-chimpanzee primates fused to EpCAM from cynomolgus monkey(1-27 CD3-EpCAM).**

**4.1. Cloning and expression of 1-27 CD3-EpCAM**

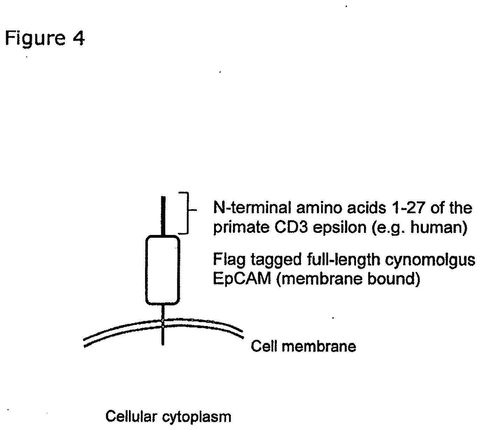

CD3 epsilon was isolated from different non-chimpanzee primates (marmoset, tamarin, squirrel monkey) and swine. The coding sequences of the 1-27 N-terminal amino acids of CD3 epsilon chain of the mature human, common marmoset (*Callithrix jacchus),* cottontop tamarin (*Saguinus oedipus*)*,* common squirrel monkey (*Saimiri sciureus*) and domestic swine (*Sus scrofa*; used as negative control) fused to the N-terminus of Flag tagged cynomolgus EpCAM were obtained by gene synthesis according to standard protocols. cDNA sequence and amino acid sequence of the recombinant fusion proteins are listed under SEQ ID NOs 351 to 360). The gene synthesis fragments were designed as to contain first a BsrGI site to allow fusion in correct reading frame with the coding sequence of a 19 amino acid immunoglobulin leader peptide already present in the target expression vector, which is followed in frame by the coding sequence of the N-terminal 1-27 amino acids of the extracellular portion of the mature CD3 epsilon chains, which is followed in frame by the coding sequence of a Flag tag and followed in frame by the coding sequence of the mature cynomolgus EpCAM transmembrane protein (Figure 4). The gene synthesis fragments were also designed to introduce a restriction site at the end of the cDNA coding for the fusion protein. The introduced restriction sites BsrGI at the 5' end and SalI at the 3' end, were utilized in the following cloning procedures. The gene synthesis fragments were then cloned via BsrGI and SalI into a derivative of the plasmid designated pEF DHFR (pEF-DHFR is described in Mack et al. Proc. Natl. Acad. Sci. USA 92 (1995) 7021-7025), which already contained the coding sequence of the 19 amino acid immunoglobulin leader peptide following standard protocols. Sequence verified plasmids were used to transiently transfect 293-HEK cells using the MATra-A Reagent (IBA GmbH, Göttingen, Germany) and 12 $\mu$g of plasmid DNA for adherent 293-HEK cells in 175 ml cell culture flasks according to the manufacturers protocol. After 3 days of cell culture the transfectants were tested for cell surface expression of the recombinant transmembrane protein via an FACS assay according to standard protocols. For that purpose a number of $2,5x10^5$ cells were incubated with the anti-Flag M2 antibody (Sigma-Aldrich Chemie GmbH, Taufkirchen, Germany) at 5 $\mu$g/ml in PBS with 2% FCS. Bound antibody was detected with an R-Phycoerythrin-conjugated affinity purified F(ab')2 fragment, goat anti-mouse IgG, Fc-gamma fragment specific 1:100 in PBS with 2% FCS (Jackson ImmunoResearch Europe Ltd., Newmarket, Suffolk, UK). The samples were measured on a FACScalibur (BD biosciences, Heidelberg, Germany). Expression of the Flag tagged recombinant transmembrane fusion proteins consisting of cynomolgus EpCAM and the 1-27 N-terminal amino acids of the human, marmoset, tamarin, squirrel monkey and swine CD3 epsilon chain respectively on transfected cells was clearly detectable (Figure 5).

**4.2. Binding of cross-species specific anti-CD3 single chain antibodies to the 1-27 CD3-EpCAM**

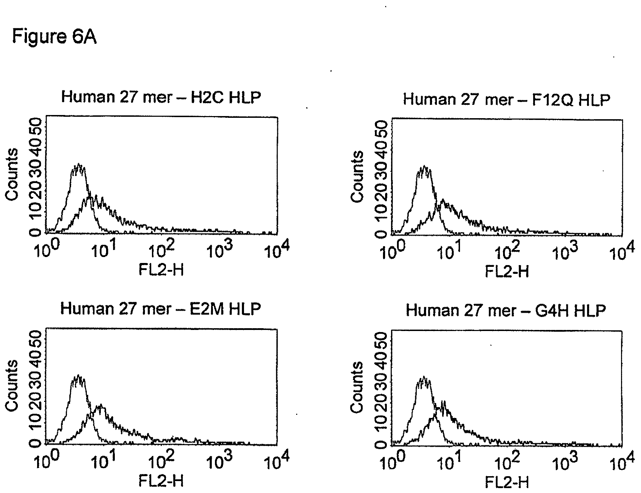

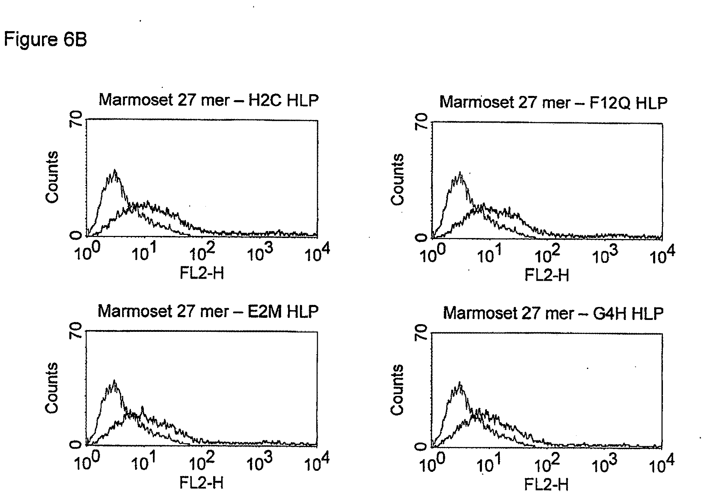

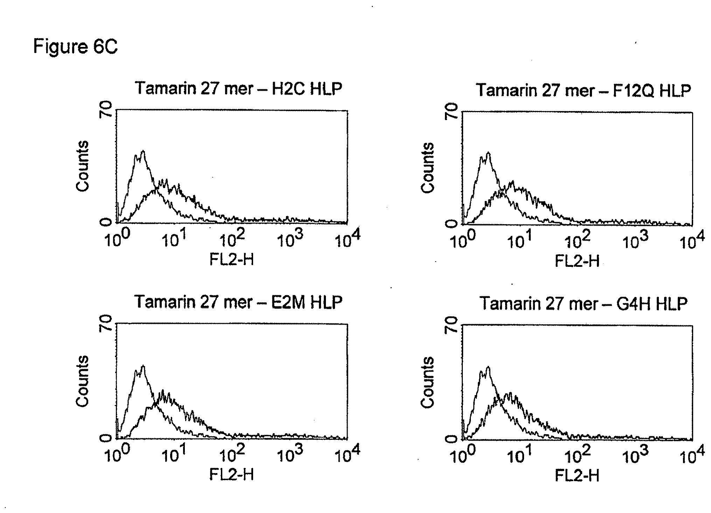

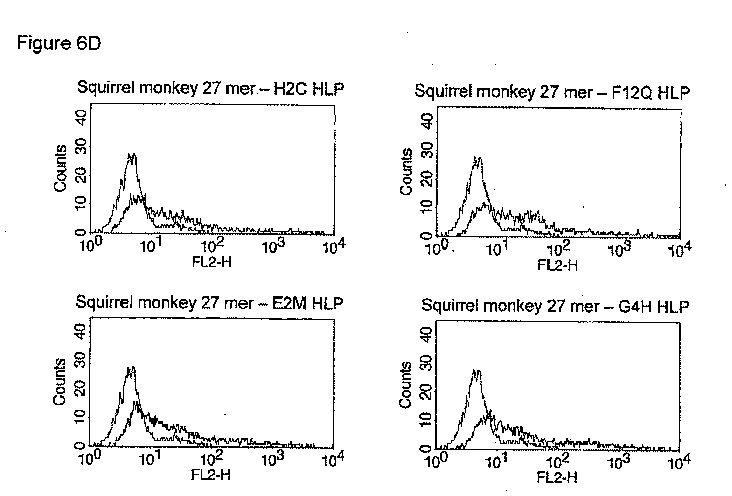

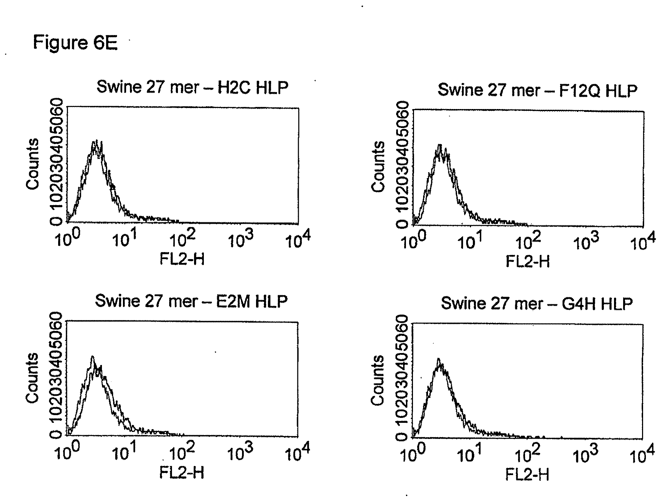

Binding of crude preparations of periplasmatically expressed cross-species specific anti CD3 single-chain antibodies to the 1-27 N-terminal amino acids of the human, marmoset, tamarin and squirrel monkey CD3 epsilon chains respectively fused to cynomolgus Ep-CAM was tested in an FACS assay according to standard protocols. For that purpose a number of $2,5x10^5$ cells were incubated with crude preparations of periplasmatically expressed cross-species specific anti CD3 single-chain antibodies (preparation was performed as described above and according to standard protocols) and a single-chain murine anti-human CD3 antibody as negative control. As secondary antibody the Penta-His antibody (Qiagen GmbH, Hildesheim, Germany) was used at 5 $\mu$g/ml in 50 $\mu$l PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACScalibur (BD biosciences, Heidelberg, Germany). As shown in Figures 6 (A to E) binding of single chain antibodies to the transfectants expressing the recombinant transmembrane fusion proteins consisting of the 1-27 N-terminal amino acids of CD3 epsilon of the human, marmoset, tamarin or squirrel monkey fused to cynomolgus EpCAM was observed. No binding of cross-species specific single chain antibodies was observed to a fusion protein consisting of the 1-27 N-terminal CD3 epsilon of swine fused to cynomolgus EpCAM used as negative control. Multi-primate cross-species specificity of the anti-CD3 single chain antibodies was shown. Signals obtained with the anti Flag M2 antibody and the cross-species specific single chain antibodies were comparable, indicating a strong binding activity of the cross-species specific single chain antibodies to the N-terminal amino acids 1-27 of CD3 epsilon.

**5. Binding analysis of cross-species specific anti-CD3 single chain antibodies by alanine-scanning of mouse cells transfected with the human CD3 epsilon chain and its alanine mutants**

**5.1. Cloning and expression of human wild-type CD3 epsilon**

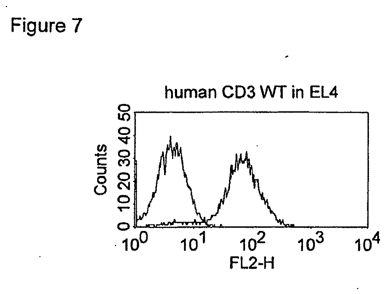

The coding sequence of the human CD3 epsilon chain was obtained by gene synthesis according to standard protocols (cDNA sequence and amino acid sequence of the human CD3 epsilon chain are listed under SEQ ID NOs 362 and 361). The gene synthesis fragment was designed as to contain a Kozak site for eukaryotic expression of the construct and restriction sites at the beginning and the end of the cDNA coding for human CD3 epsilon. The introduced restriction sites EcoRI at the 5' end and SalI at the 3' end, were utilized in the following cloning procedures. The gene synthesis fragment was then cloned via EcoRI and SalI into a plasmid designated pEF NEO following standard protocols. pEF NEO was derived of pEF DHFR (Mack et al. Proc. Natl. Acad. Sci. USA 92 (1995) 7021-7025) by replacing the cDNA of the DHFR with the cDNA of the neomycin resistance by conventional molecular cloning. A sequence verified plasmid was used to transfect the murine T cell line EL4 (ATCC No. TIB-39) cultivated in RPMI with stabilized L-glutamine supplemented with 10 % FCS, 1% penicillin/streptomycin, 1% HEPES, 1% pyruvate, 1% non-essential amino acids (all Biochrom AG Berlin, Germany) at 37 °C, 95 % humidity and 7 % $CO_2$. Transfection was performed with the SuperFect Transfection Reagent (Qiagen GmbH, Hilden, Germany) and 2 μg of plasmid DNA according to the manufacturer's protocol. After 24 hours the cells were washed with PBS and cultivated again in the aforementioned cell culture medium with 600 μg/ml G418 for selection (PAA Laboratories GmbH, Pasching, Austria). 16 to 20 days after transfection the outgrowth of resistant cells was observed. After additional 7 to 14 days cells were tested for expression of human CD3 epsilon by FACS analysis according to standard protocols. $2,5 \times 10^5$ cells were incubated with anti-human CD3 antibody UCHT-1 (BD biosciences, Heidelberg, Germany) at 5 μg/ml in PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACSCalibur (BD biosciences, Heidelberg, Germany). Expression of human wild-type CD3 on transfected EL4 cells is shown in Figure 7.

## 5.2. Cloning and expression of the cross-species specific anti-CD3 single chain antibodies as IgG1 antibodies

In order to provide improved means of detection of binding of the cross-species specific single chain anti-CD3 antibodies H2C HLP, A2J HLP and E2M HLP were converted into IgG1 antibodies with murine IgG1 and human lambda constant regions. cDNA sequences coding for the heavy and light chains of respective IgG antibodies were obtained by gene synthesis according to standard protocols. The gene synthesis fragments for each specificity were designed as to contain first a Kozak site to allow eukaryotic expression of the construct, which is followed by an 19 amino acid immunoglobulin leader peptide (SEQ ID NOs 364 and 363), which is followed in frame by the coding sequence of the respective heavy chain variable region or respective light chain variable region, followed in frame by the coding sequence of the heavy chain constant region of murine IgG1 (SEQ ID NOs 366 and 365) or the coding sequence of the human lambda light chain constant region (SEQ ID NO 368 and 367), respectively. Restriction sites were introduced at the beginning and the end of the cDNA coding for the fusion protein. Restriction sites EcoRI at the 5' end and SalI at the 3' end were used for the following cloning procedures. The gene synthesis fragments were cloned via EcoRI and SalI into a plasmid designated pEF DHFR (Mack et al. Proc. Natl. Acad. Sci. USA 92 (1995) 7021-7025) for the heavy chain constructs and pEF ADA (pEF ADA is described in Raum et al., Cancer Immunol Immunother., 50(3), (2001), 141-50) for the light chain constructs) according to standard protocols. Sequence verified plasmids were used for co-transfection of respective light and heavy chain constructs in the FreeStyle 293 Expression System (Invitrogen GmbH, Karlsruhe, Germany) according to the manufacturers protocol. After 3 days cell culture supernatants of the transfectants were harvested and used for the alanine-scanning experiment.

## 5.3. Cloning and expression of alanine mutants of human CD3 epsilon for alanine-scanning

27 cDNA fragments coding for the human CD3 epsilon chain with an exchange of one codon of the wild-type sequence of human CD3 epsilon into a codon coding for alanine (GCC) for each amino acid of amino acids 1-27 of the extracellular domain of the mature human CD3 epsilon chain respectively were obtained by gene synthesis. Except for the exchanged codon the cDNA fragments were identical to the aforementioned human wild-type CD3 cDNA fragment. Only one codon was replaced in each construct compared to the human wild-type CD3 cDNA fragment described above. Restriction sites EcoRI and SalI were introduced into the cDNA fragments at identical positions compared to the wild-type construct. All alanine-scanning constructs were cloned into pEF NEO and sequence verified plasmids were transfected into EL4 cells. Transfection and selection of transfectants was performed as described above. As result a panel of expressed constructs was obtained wherein the first amino acid of the human CD3 epsilon chain, glutamine (Q, Gln) at position 1 was replaced by alanine. The last amino acid replaced by alanine was the threonine (T, Thr) at position 27 of mature human wild-type CD3 epsilon. For each amino acid between glutamine 1 and threonine 27 respective transfectants with an exchange of the wild-type amino acid into alanine were generated.

## 5.4. Alanine-scanning experiment

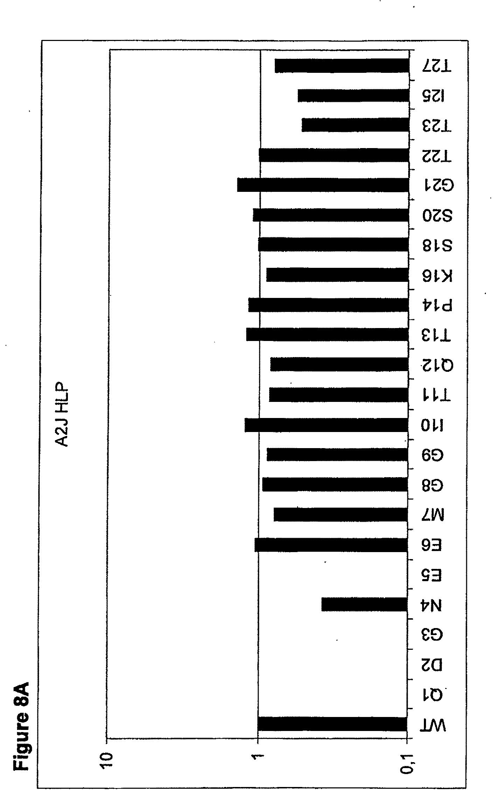

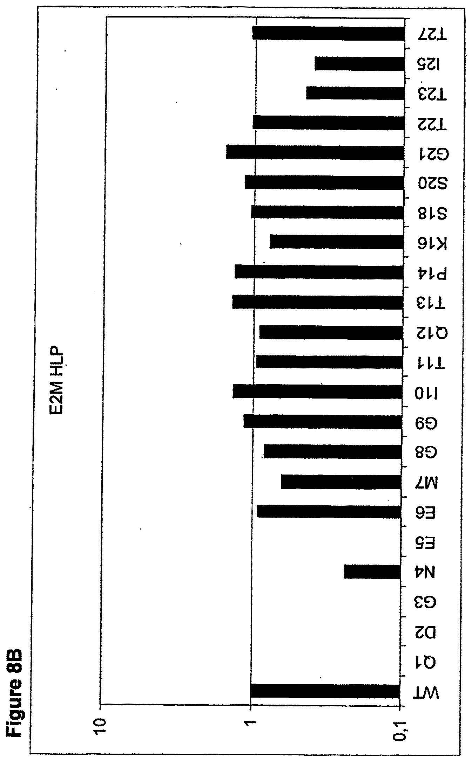

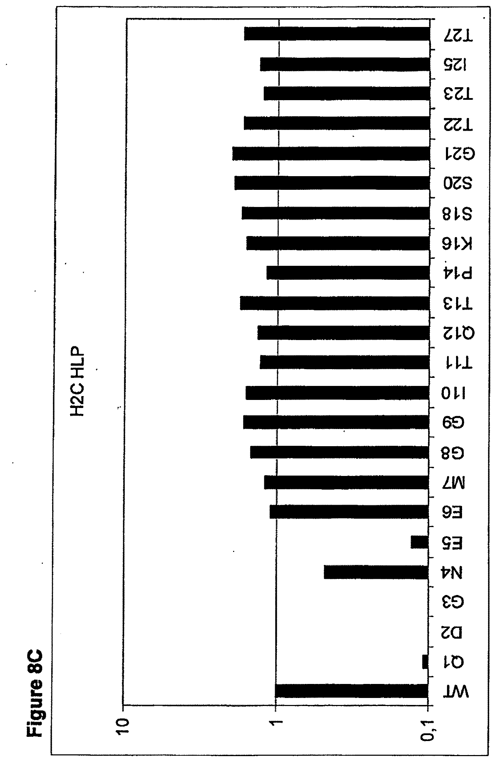

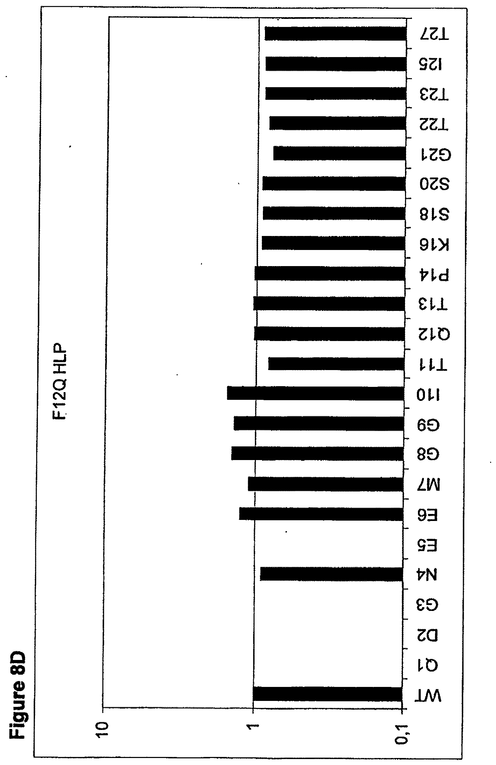

Chimeric IgG antibodies as described in 2) and cross-species specific single chain antibodies specific for CD3 epsilon were tested in alanine-scanning experiment. Binding of the antibodies to the EL4 cell lines transfected with the alanine-mutant constructs of human CD3 epsilon as described in 3) was tested by FACS assay according to standard protocols. $2,5 \times 10^5$ cells of the respective transfectants were incubated with 50 μl of cell culture supernatant containing the chimeric IgG antibodies or with 50 μl of crude preparations of periplasmatically expressed single-chain antibodies. For samples incubated with crude preparations of periplasmatically expressed single-chain antibodies the anti-Flag M2 antibody (Sigma-Aldrich Chemie GmbH, Taufkirchen, Germany) was used as secondary antibody at 5 μg/ml in 50 μl PBS with 2% FCS. For samples incubated with the chimeric IgG antibodies a secondary antibody was not necessary. For all samples the binding of the antibody molecules was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACSCalibur (BD biosciences, Heidelberg, Germany). Differential binding of chimeric IgG molecules or cross-species specific single-chain antibodies to the EL4 cell lines transfected with the alanine-mutants of human CD3 epsilon was detected. As negative control either an isotype control or a crude preparation of a periplasmatically expressed single-chain antibody of irrelevant specificity was used respectively. UCHT-1 antibody was used as positive control for the expression level of the alanine-mutants of human CD3 epsilon. The EL4 cell lines transfected with the alanine-mutants for the amino acids tyrosine at position 15, valine at position 17, isoleucine at position 19, valine at position 24 or leucine at position 26 of the mature CD3 epsilon chain were not evaluated due to very low expression levels (data not shown). Binding of the cross-species specific single chain antibodies and the single chain antibodies in chimeric IgG format to the EL4 cell lines transfected with the alanine-mutants of human CD3 epsilon is shown in Figure 8 (A-D) as relative binding in arbitrary units with the geometric mean fluorescence values of the respective negative controls subtracted from all respective geometric mean fluorescence sample values. To compensate for different expression levels all sample values for a certain transfectant were then divided through the geometric mean fluorescence value of the UCHT-1 antibody for the respective transfectant. For comparison with the wild-type sample value of a specificity all sample values of the respective specificity were finally divided through the wild-type sample value, thereby setting the wild-type sample value to 1 arbitrary unit of binding.

The calculations used are shown in detail in the following formula:

$$value\_Sample(x,y) = \frac{Sample(x,y) - neg\_Contr.(x)}{(UCHT-1(x) - neg\_Contr.(x)) * \dfrac{WT(y) - neg\_Contr.(wt)}{UCHT-1(wt) - neg\_Contr.(wt)}}$$

In this equation *value_Sample* means the value in arbitrary units of binding depicting the degree of binding of a specific anti-CD3 antibody to a specific alanine-mutant as shown in Figure 8 (A-D), *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT-1* means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, *x* specifies the respective transfectant, *y* specifies the respective anti-CD3 antibody and *wt* specifies that the respective transfectant is the wild-type.

As can be seen in Figure 8 (A-D) the IgG antibody A2J HLP showed a pronounced loss of binding for the amino acids asparagine at position 4, threonine at position 23 and isoleucine at position 25 of the mature CD3 epsilon chain. A complete loss of binding of IgG antibody A2J HLP was observed for the amino acids glutamine at position 1, aspartate at position 2, glycine at position 3 and glutamate at position 5 of the mature CD3 epsilon chain. IgG antibody E2M HLP showed a pronounced loss of binding for the amino acids asparagine at position 4, threonine at position 23 and isoleucine at position 25 of the mature CD3 epsilon chain. IgG antibody E2M HLP showed a complete loss of binding for the amino acids glutamine at position 1, aspartate at position 2, glycine at position 3 and glutamate at position 5 of the mature CD3 epsilon chain. IgG antibody H2C HLP showed an intermediate loss of binding for the amino acid asparagine at position 4 of the mature CD3 epsilon chain and it showed a complete loss of binding for the amino acids glutamine at position 1, aspartate at position 2, glycine at position 3 and glutamate at position 5 of the mature CD3 epsilon chain. Single chain antibody F12Q HLP showed an essentially complete loss of binding for the amino acids glutamine at position 1, aspartate at position 2, glycine at position 3 of the mature CD3 epsilon chain and glutamate at position 5 of the mature CD3 epsilon chain.

**6. Binding analysis of the cross-species specific anti-CD3 binding molecule H2C HLP to the human CD3 epsilon chain with and without N-terminal His6 tag transfected into the murine T cell line EL4**

**6.1. Cloning and expression of the human CD3 epsilon chain with N-terminal six histidine tag (His6 tag)**

A cDNA fragment coding for the human CD3 epsilon chain with a N-terminal His6 tag was obtained by gene synthesis. The gene synthesis fragment was designed as to contain first a Kozak site for eukaryotic expression of the construct, which is followed in frame by the coding sequence of a 19 amino acid immunoglobulin leader peptide, which is followed in frame by the coding sequence of a His6 tag which is followed in frame by the coding sequence of the mature human CD3 epsilon chain (the cDNA and amino acid sequences of the construct are listed as SEQ ID NOs 380 and 379). The gene synthesis fragment was also designed as to contain restriction sites at the beginning and the end of the cDNA. The introduced restriction sites EcoRI at the 5' end and SalI at the 3' end, were used in the following cloning procedures. The gene synthesis fragment was then cloned via EcoRI and SalI into a plasmid designated pEF-NEO (as described above) following standard protocols. A sequence verified plasmid was used to transfect the murine T cell line EL4. Transfection and selection of the transfectants were performed as described above. After 34 days of cell culture the transfectants were used for the assay described below.

**6.2. Binding of the cross-species specific anti-CD3 binding molecule H2C HLP to the human CD3 epsilon chain with and without N-terminal His6 tag**

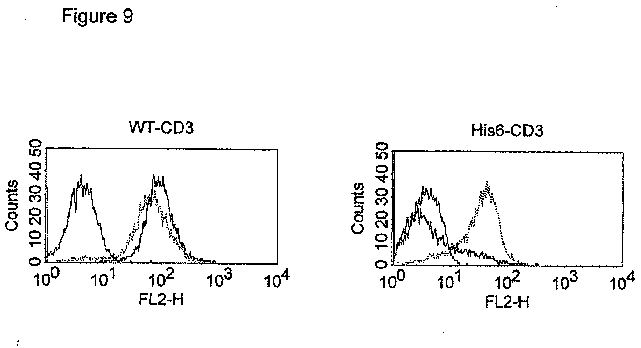

A chimeric IgG antibody with the binding specificity H2C HLP specific for CD3 epsilon was tested for binding to human CD3 epsilon with and without N-terminal His6 tag. Binding of the antibody to the EL4 cell lines transfected the His6-human CD3 epsilon and wild-type human CD3 epsilon respectively was tested by an FACS assay according to standard protocols. $2,5 \times 10^5$ cells of the transfectants were incubated with 50 $\mu$l of cell culture supernatant containing the chimeric IgG antibody or 50 $\mu$l of the respective control antibodies at 5$\mu$g/ml in PBS with 2% FCS. As negative control an appropriate isotype control and as positive control for expression of the constructs the CD3 specific antibody UCHT-1 were used respectively. The binding of the antibodies was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACSCalibur (BD biosciences, Heidelberg, Germany). Compared to the EL4 cell line transfected with wild-type human CD3 epsilon a clear loss of binding of the chimeric IgG with binding specificity H2C HLP to human-CD3 epsilon with an N-terminal His6 tag was detected. These results showed that a free N-terminus of CD3 epsilon is essential for binding of the cross-species specific anti-CD3 binding specificity H2C HLP to the human CD3 epsilon chain (Figure 9).

**7. Determination of the binding constant KD of bispecific single chain antibody cross-species specific for primate EGFR and primate CD3 (EGFR LH × H2C HLP) to the fusion protein 1-27 CD3-Fc by Plasmon Surface Resonance measurement compared to binding to CD3 expressing PBMC measured by a Fluorescence Activated Cell Sorter (FACS)**

**7.1. Plasmon Surface Resonance Measurement**

To determine the binding affinity of the fully cross-species specific bispecific single chain antibody EGFR-21-63 LH × H2C HLP to amino acids 1-27 of the N-terminus of the human CD3 epsilon chain a Surface Plasmon Resonance measurement was performed with a recombinant fusion protein consisting of the N-terminal amino acids 1-27 of the mature human CD3 epsilon chain fused to a Fc-part of human IgG1 (1-27 CD3-Fc). To this end a Biacore Carboxymethyl-Dextran CM5 chip (Biacore, Uppsala, Sweden) was installed on a Biacore 2000® system (Biacore, Uppsala, Sweden). One flow cell was activated by a *N*-(3-Dimethylaminopropyl)-*N'*-ethylcarbodiimide hydrochloride / N-Hydroxysuccinimide solution according to standard procedures. A solution of the fusion protein 1-27 CD3-Fc was added afterwards resulting in stable covalent linkage of the protein to the dextran layer of the Biacore chip. Unbound protein was removed by extensive washing followed by blocking of unreacted remaining NHS-activated carboxy groups by adding an ethanolamine solution. Success of protein coupling was confirmed by a higher signal measured as Response Units compared to the signal prior to coupling. A reference cell was prepared as described but without adding a protein solution.

Purified bispecific antibody EGFR-21-63 LH × H2C HLP was extensively dialyzed against HBS-EP buffer (Biacore, Uppsala, Sweden) in a Slide-A-Lyzer® Mini Dialysis Unit (Pierce, Rockford-II, USA). Protein concentration after dialysis was determined by UV280 nm absorption resulting in a concentration of 43 $\mu$g/ml.

The protein solution was transferred into a 96 well plate and serially diluted with HBS-EP buffer at a 1:1 ratio to 10 further wells.

Surface Plasmon Resonance Measurements were done by separately sampling all 11 wells. The flow cells were regenerated with acetate buffer between measurements to release bound protein.

Binding signals of bispecific antibody molecules were obtained by subtraction of the signal of the reference cell from the signal of the measurement cell conjugated with the 1-27 CD3-Fc protein. Association and dissociation curves were measured as Response Units and recorded. The binding constants were calculated using the Biacore® curve fitting software based on the Langmuir model.

The calculated binding constant KD over the first five concentrations was determined to be $1,52 \times 10^{-7}$ M.

**7.2. Determination of CD3 binding constant by FACS measurement**

In order to test the affinity of the cross-species specific bispecific antibody molecules with regard to the binding strength to native human CD3, an additional saturation FACS binding analysis was performed. The chosen bispecific antibody molecule EGFR-21-63 LH × H2C HLP was used to set up a dilution row with a factor of 1:1.5 and a starting concentration of 63.3 $\mu$g/ml. The bispecific antibody molecule was incubated at these different concentrations with 1.25

$\times 10^5$ human PBMCs each for 1 hour a 4°C followed by two washing steps in PBS at 4°C. The detection of the bound bispecific antibody molecules was carried out by using a Penta-His antibody (Qiagen GmbH, Hildesheim, Germany) at 5 μg/ml in 50 μl PBS with 2% FCS. After incubation for 45 minutes at 4°C and two washing steps the binding of the Penta-His antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Flow cytometry was performed on a FACS-Canto II apparatus, the FACS Diva software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002). The acquired fluorescence intensity mean values were plotted as a function of the used bispecific antibody molecule concentration and analyzed by the biomathematical software Prism in a one side binding analysis (hyperbola). The software calculated the corresponding KD value that described the binding of a ligand (the bispecific antibody molecule) to a receptor (the CD3 positive PBMC subfraction) that follows the law of mass action. The underlying formula is as follows: Y = Bmax $\times$ X / (Kd+X) with Bmax being the maximal binding. KD is the concentration of ligand required to reach half-maximal binding. The FACS staining was carried out in duplicates, the $R^2$ values were better than 0.95.

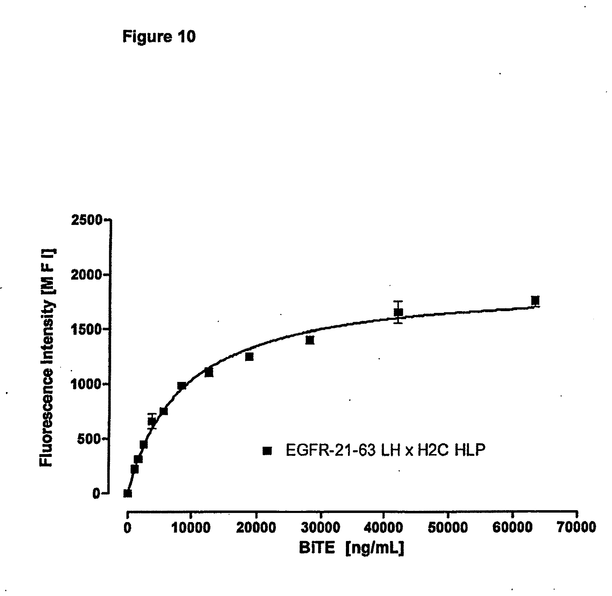

The determined half-maximal binding for the bispecific antibody molecule EGFR-21-63 LH $\times$ H2C HLP was reached at a concentration of 8472 ng/ml which corresponds to 154 nM (1.54 $\times$ 10$^{-7}$ M) at a given molecular mass of 55000 Dalton (Figure 10). Thus, the affinity of EGFR-21-63 LH $\times$ H2C HLP to the N-terminal amino acids 1-27 of the human CD3 epsilon chain separated from their native CD3-context proved to be equal to the affinity of EGFR-21-63 LH $\times$ H2C HLP to native CD3 on intact T cells.

## 8. Generation of CHO cells transfected with human EGFR

The cell line positive for human EGFR, A431 (epidermoid carcinoma cell line, CRL-1555, American Type Culture Collection, Rockville, MD) was used to obtain the total RNA that was isolated according to the instructions of the kit manual (Qiagen, RNeasy Mini Kit, Hilden, Germany). The obtained RNA was used for cDNA synthesis by random-primed reverse transcription. For cloning of the full length sequence of the human EGFR antigen the following oligonucleotides were used:

    5' EGFR AG XbaI 5'-GGTCTAGAGCATGCGACCCTCCGGGACGGCCGGG-3'
    3' EGFR AG SalI 5'-TTTTAAGTCGACTCATGCTCCAATAAATTCACTGCT-3'

The coding sequence was amplified by PCR (denaturation at 94 °C for 5 min, annealing at 58°C for 1 min, elongation at 72°C for 2 min for the first cycle; denaturation at 94°C for 1 min, annealing at 58°C for 1 min, elongation at 72°C for 2 min for 30 cycles; terminal extension at 72°C for 5 min). The PCR product was subsequently digested with XbaI and SalI, ligated into the appropriately digested expression vector pEF-DHFR (Raum et al., Cancer Immunol. Immunother. 2001; 50: 141-150), and transformed into E.coli. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence (SEQ ID 370, Amino acid sequence SEQ ID 369) was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

## 9. Generation of CHO cells expressing the extracellular domain of cynomolgus EGFR

The cDNA sequence of the extracellular domain of cynomolgus EGFR was obtained by a set of two PCRs on cynomolgus monkey colon cDNA (Cat#: C1534090-Cy-BC; obtained from BioCat GmbH, Heidelberg, Germany) using the following reaction conditions: 1 cycle at 94°C for 3 minutes followed by 35 cycles with 94°C for 1 minute, 53°C for 1 minute and 72°C for 2 minutes followed by a terminal cycle of 72°C for 3 minutes. The following primers were used:

    1. forward primer: 5'- CGCTCTGCCCGGCGAGTCGGGC -3'
    reverse primer: 5'- CCGTCTTCCTCCATCTCATAGC -3'
    2. forward primer: 5'- ACATCCGGAGGTGACAGATCACGGCTCGTGC -3'
    reverse primer: 5'- CAGGATATCCGAACGATGTGGCGCCTTCGC -3'

Those PCRs generated two overlapping fragments (A: 1-869, B: 848-1923), which were isolated and sequenced according to standard protocols using the PCR primers, and thereby provided a 1923 bp portion of the cDNA sequence of cynomolgus EGFR from the third nucleotide of codon +1 of the mature protein to the 21st codon of the transmembrane domain. To generate a construct for expression of cynomolgus EGFR a cDNA fragment was obtained by gene synthesis according to standard protocols (the cDNA and amino acid sequence of the construct is listed under SEQ ID Nos 372 and 371). In this construct the coding sequence for cynomolgus EGFR from amino acid +2 to +641 of the mature EGFR protein was fused into the coding sequence of human EGFR replacing the coding sequence of the amino acids +2 to +641. The gene synthesis fragment was also designed as to contain a Kozak site for eukaryotic expression of the construct and restriction sites at the beginning and the end of the cDNA coding for essentially the extracellular domain of cynomolgus EGFR fused to the transmembrane and intracellular domains of human EGFR. Furthermore a conservative mutation was introduced at amino acid 627 (4th amino acid of the transmembrane domain) mutating valine into leucine to generate a restriction site (SphI) for cloning purposes. The introduced restriction sites Xbal at the 5' end and Sall at the 3' end, were utilised in the following cloning procedures. The gene synthesis fragment was then cloned via Xbal and Sall into a plasmid designated pEF-DHFR (pEF-DHFR is described in Mack et al. Proc. Natl. Acad. Sci. USA 92 (1995) 7021-7025). A sequence verified clone of this plasmid was used to transfect CHO/dhfr- cells as described above.

## 10. Generation of EGFR and CD3 cross-species specific bispecific single chain molecules

### 10.1. Cloning of cross-species specific binding molecules

Generally, bispecific single chain antibody molecules, each comprising a domain with a binding specificity cross-species specific for human and non-chimpanzee primate CD3 epsilon as well as a domain with a binding specificity cross-species specific for human and non-chimpanzee primate EGFR, were designed as set out in the following Table 1:

Table 1: Formats of anti-CD3 and anti-EGFR cross-species specific bispecific single chain antibody molecules

| SEQ ID (nucl/prot) | Formats of protein constructs (N → C) |
|---|---|
| 294/293 | EGFR-21-63 LH × H2C HL |
| 296/295 | EGFR-21-63 LH × H2C HLP |
| 302/301 | EGFR-21-63 LH × A2J HLP |
| 298/297 | EGFR-21-63 LH × H1E HLP |
| 306/305 | EGFR-21-63 LH × E2M HLP |
| 308/307 | EGFR-21-63 LH × F7O HLP |
| 864/863 | EGFR-21-63 LH × I2C HL |
| 900/899 | E291C4 HL × I2C HL |
| 898/897 | E291C4 HL × F12Q HL |
| 896/895 | E291C4 HL × H2C HL |
| 882/881 | E291H8 HL × I2C HL |
| 918/917 | E292F3 HL × I2C HL |
| 936/935 | E98H5 HL × I2C HL |
| 932/931 | E98H5 HL × H2C HL |
| 934/933 | E98H5 HL × F12Q HL |
| 972/971 | E97H9 HL × I2C HL |
| 970/969 | E97H9 HL × F12Q HL |
| 968/967 | E97H9 HL × H2C HL |
| 954/953 | E98C3 HL × I2C HL |
| 952/951 | E98C3 HL × F12Q HL |
| 950/949 | E98C3 HL × H2C HL |
| 990/989 | E97B11 HL × 12C HL |
| 988/987 | E97B11 HL × F12Q HL |

(continued)

| SEQ ID (nucl/prot) | Formats of protein constructs (N → C) |
|---|---|
| 986/985 | E97B11 HL × H2C HL |
| 1018/1017 | E12E3 HL × I2C HL |
| 1032/1031 | E12B7 HL × I2C HL |
| 1046/1045 | E12E6 HL × I2C HL |
| 1060/1059 | E12F12 HL × I2C HL |
| 1004/1003 | E13B4 HL × I2C HL |
| 1074/1073 | E13H7 HL × I2C HL |
| 1088/1087 | E13E6 HL × I2C HL |
| 1102/1101 | E13E2 HL × I2C HL |

The aforementioned constructs containing the variable light-chain (L) and variable heavy-chain (H) domains cross-species specific for human and cynomolgus EGFR were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilized in the following cloning procedures. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into dihydrofolate reductase (DHFR) deficient Chinese hamster ovary (CHO) cells for eukaryotic expression of the construct.

The constructs were transfected stably or transiently into DHFR-deficient CHO-cells (ATCC No. CRL 9096) by electroporation or alternatively into HEK 293 (human embryonal kidney cells, ATCC Number: CRL-1573) in a transient manner according to standard protocols.

## 10.2. Expression and purification of the bispecific single chain antibody molecules

The bispecific single chain antibody molecules were expressed in chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by increasing final concentrations of MTX up to 20 nM. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Akta® Explorer System (GE Health Systems) and Unicorn® Software were used for chromatography. Immobilized metal affinity chromatography ("IMAC") was performed using a Fractogel EMD chelate® (Merck) which was loaded with ZnCl2 according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazol) according to the following:

Step 1: 20% buffer B in 6 column volumes
Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrat, 200 mM Lysin, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein was >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. The antibodies used were directed against the His Tag (Penta His, Qiagen) and Goat-anti-mouse Ig labeled with alkaline phosphatase (AP) (Sigma), and BCIP/NBT . (Sigma) as substrate. A single band was detected at 52 kD corresponding to the purified bispecific single chain antibody.

**11. Determination of the binding constant KD of fully cross-species specific bispecific single chain antibodies to the fusion protein 1-27 CD3-Fc by Surface Plasmon Resonance measurement**

To determine the binding affinities of bispecific single chain antibody molecules cross-species specific to primate EGFR and primate CD3 to the amino acids 1-27 of the N-terminus of the mature human CD3 epsilon chain a Surface Plasmon Resonance measurement was performed with a recombinant fusion protein consisting of the N-terminal amino acids 1-27 of the human CD3 epsilon chain fused to a Fc-part of human IgG1 (1-27 CD3-Fc). To this end a Biacore Carboxymethyl-Dextran CM5 chip (Biacore, Uppsala, Sweden) was installed on a Biacore 2000® system (Biacore, Uppsala, Sweden). A flow cell was activated by a *N*-(3-Dimethylaminopropyl)-*N*'-ethylcarbodiimide hydrochloride / N-Hydroxysuccinimide solution according to standard procedures. A solution of the fusion protein 1-27 CD3-Fc was added afterwards resulting in stable covalent linkage of the protein to the dextran layer of the Biacore chip. Unbound protein was removed by extensive washing followed by blocking of remaining unreacted NHS-activated carboxy groups by adding an ethanolamine solution. Success of protein coupling was confirmed by detection of a higher signal measured as Response Units compared to the signal prior to coupling. A reference cell was prepared as described but without adding the protein solution.

Purified bispecific single chain antibodies listed below were adjusted to 5 $\mu$g/ml with HBS-EP buffer (Biacore, Uppsala, Sweden) and transferred into a 96 well plate each at a volume of 150 $\mu$l.

Surface Plasmon Resonance Measurements were performed for all samples and the flow cells were regenerated with acetate buffer between measurements to release bound protein (all according to standard protocols).

Binding signals of the bispecific single chain antibodies were obtained by subtraction of the signal of the reference cell from the signal of the measurement cell conjugated with the 1-27 CD3-Fc protein.

Association and dissociation curves were measured as Response Units and recorded. The binding constants were calculated using the Biacore® curve-fitting software based on the Langmuir model. The calculated affinities for the tested fully cross-species specific bispecific single chain molecules to the N-terminal amino acids 1-27 of the human CD3 epsilon are given as KD values below and range from $2,54 \times 10^{-6}$ M to $2,49 \times 10^{-7}$ M. "LH" refers to an arrangement of variable domains in the order VL-VH. "HL" refers to an arrangement of variable domains in the order VH-VL. G4H, F70, A2J, E1L, E2M, H1E and F6A refer to different cross-species specific CD3 binding molecules.

| Bispecific antibody molecule | KD(M) |
|---|---|
| EGFR LH $\times$ F7O HLP | $1.01 \times 10^{-6}$ |
| EGFR LH $\times$ A2J HLP | $2.49 \times 10^{-7}$ |
| EGFR LH $\times$ E2M HLP | $2.46 \times 10^{-6}$ |
| EGFR LH $\times$ H1E HLP | $2.54 \times 10^{-6}$ |

**12. Flow cytometric binding analysis of the EGFR and CD3 cross-species specific bispecific antibodies**

In order to test the functionality of the cross-species specific bispecific antibody constructs with regard to binding capability to human and cynomolgus EGFR and CD3, respectively, a FACS analysis was performed. For this purpose CHO cells transfected with human EGFR as described in Example 8 and human CD3 positive T cell leukemia cell line HPB-ALL (DSMZ, Braunschweig, ACC483) were used to test the binding to human antigens. The binding reactivity to cynomolgus antigens was tested by using the generated cynomolgus EGFR transfectant described in Example 9 and a macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61) 200.000 cells of the respective cell population were incubated for 30 min on ice with 50 $\mu$l of the purified protein of the cross-species specific bispecific antibody constructs (2 $\mu$g/ml). Alternatively, the cell culture supernatant of transiently produced proteins was used. The cells were washed twice in PBS and binding of the construct was detected with a murine Penta His antibody (Qiagen; diluted 1:20 in 50 $\mu$l PBS with 2% FCS). After washing, bound anti His antibodies were detected with an Fc gamma-specific antibody (Dianova) conjugated to phycoerythrin, diluted 1:100 in PBS with 2% FCS. Fresh culture medium was used as a negative control.

Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

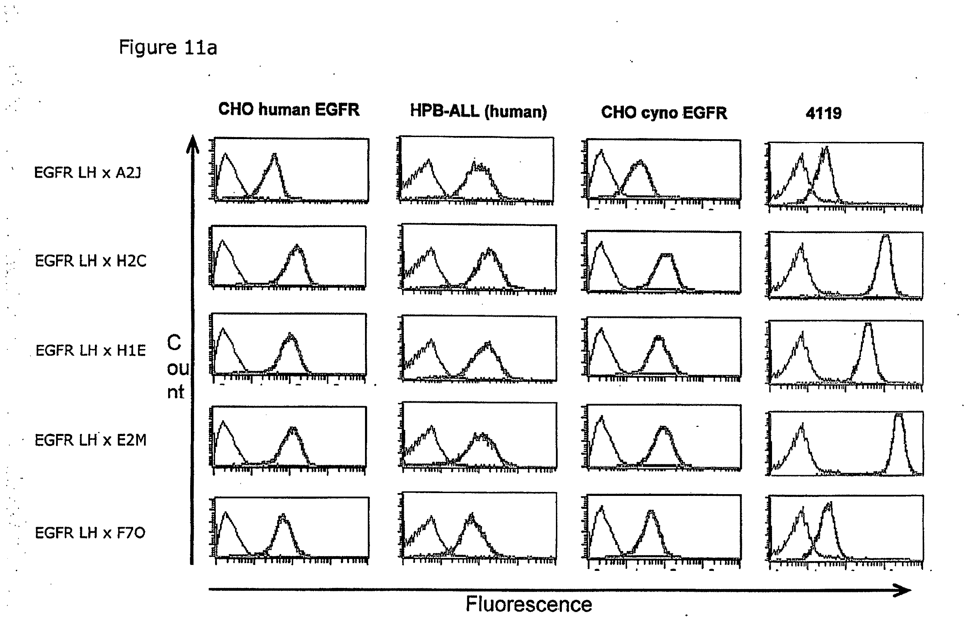

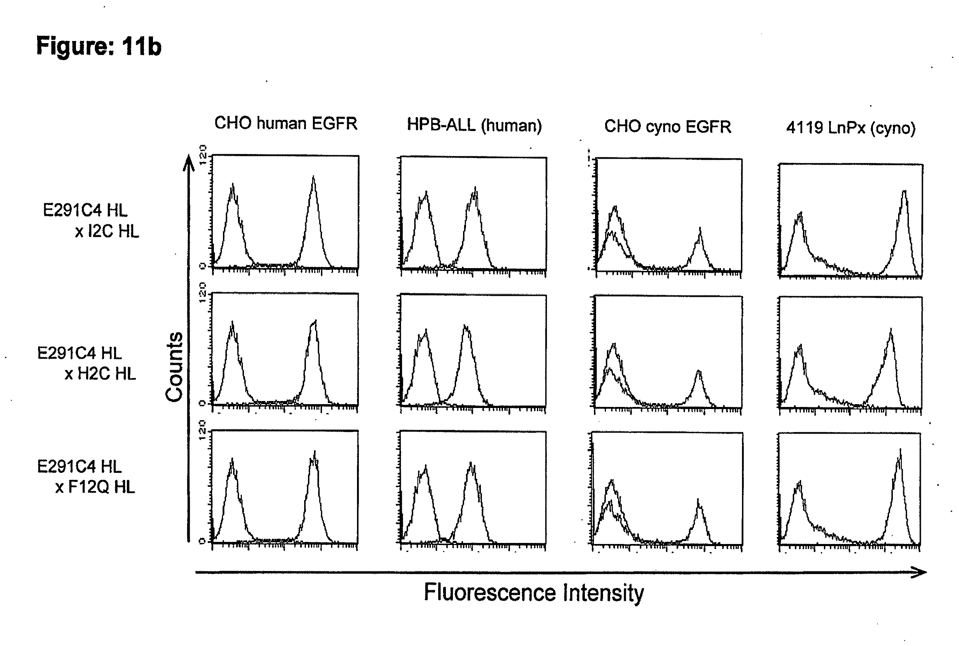

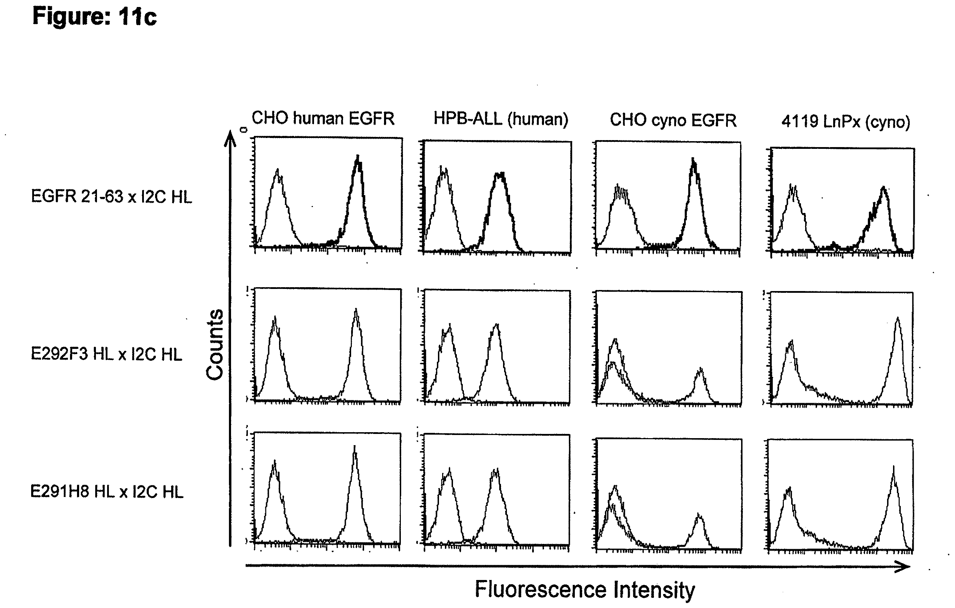

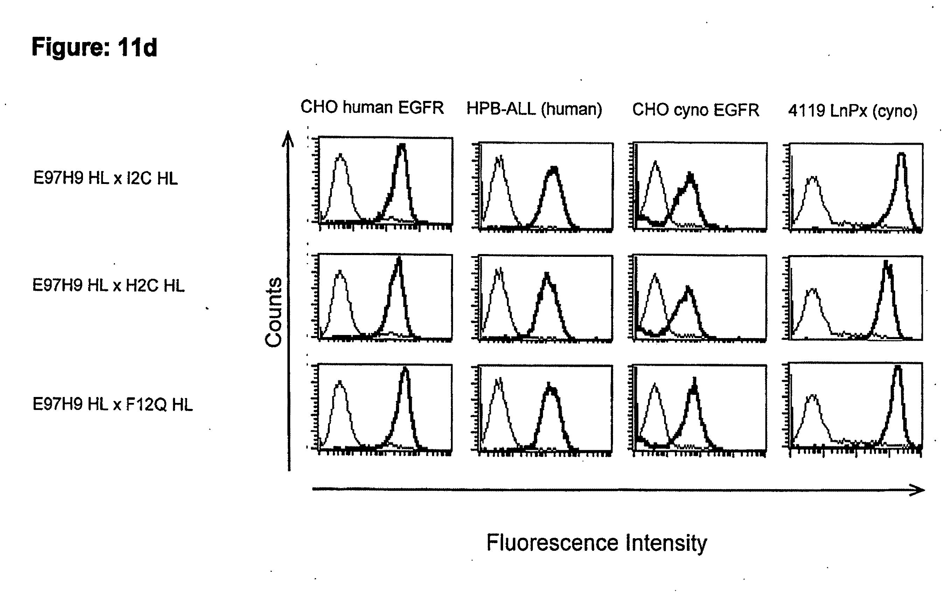

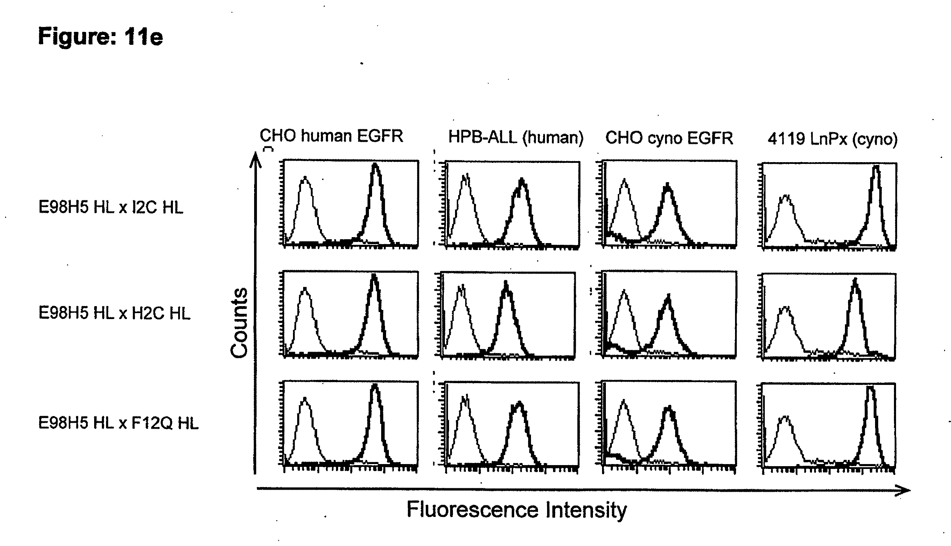

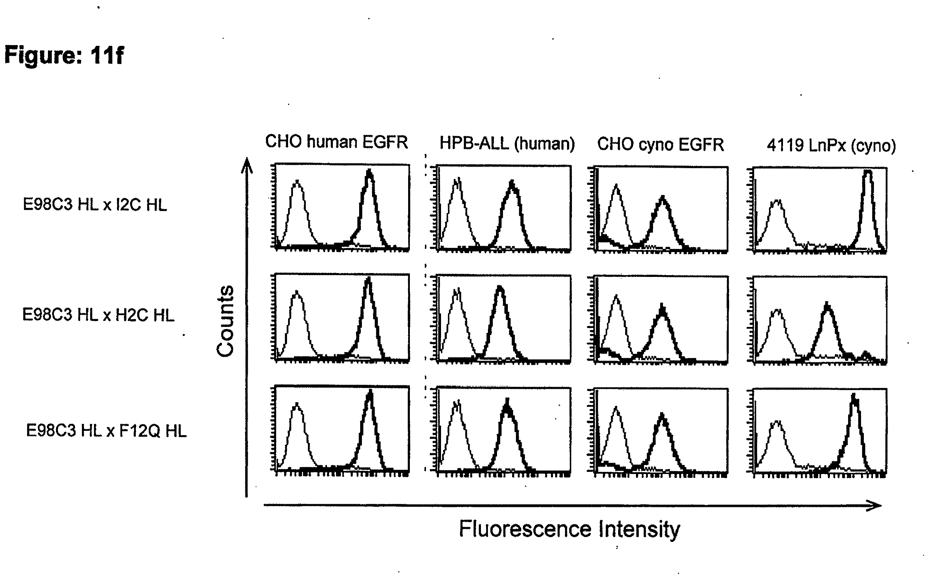

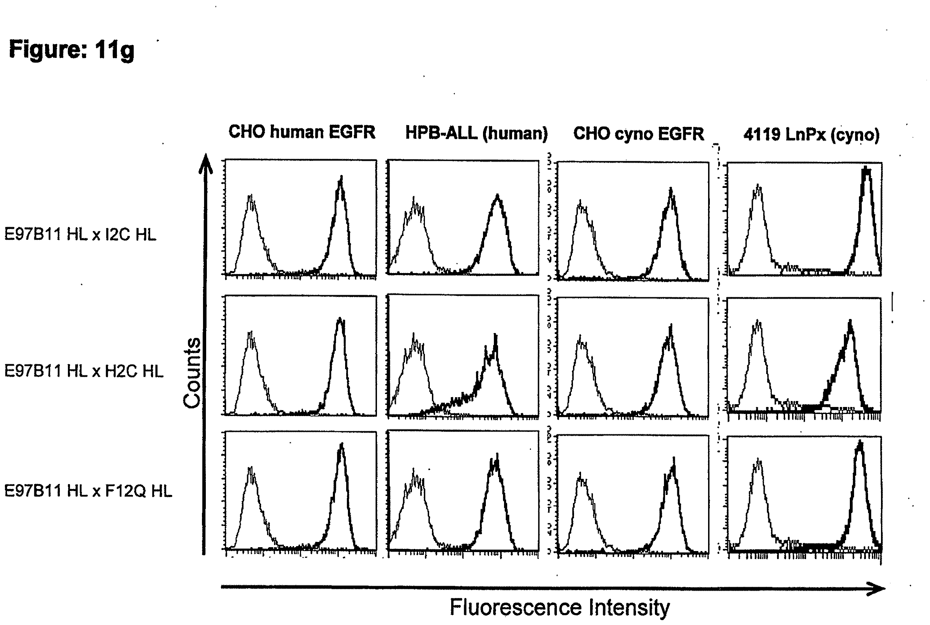

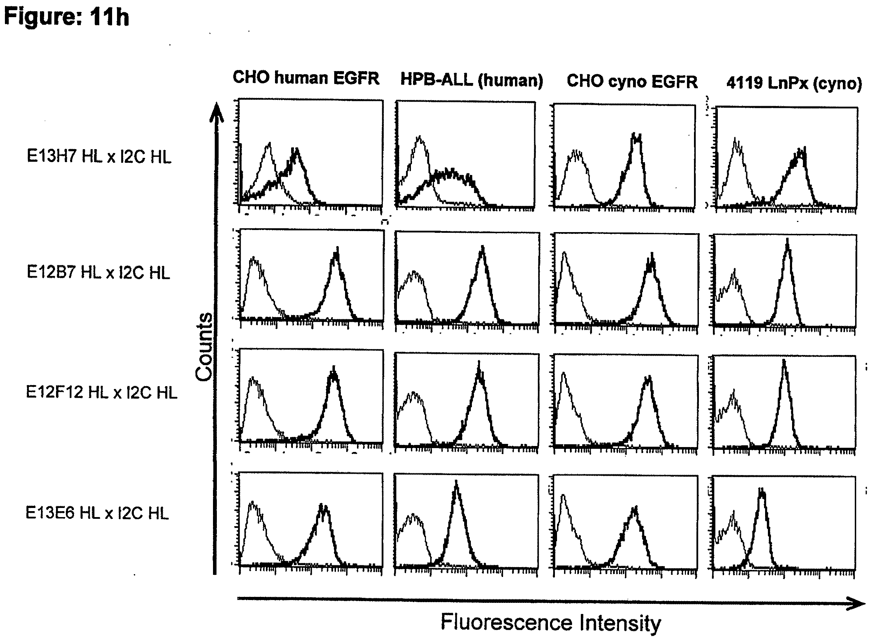

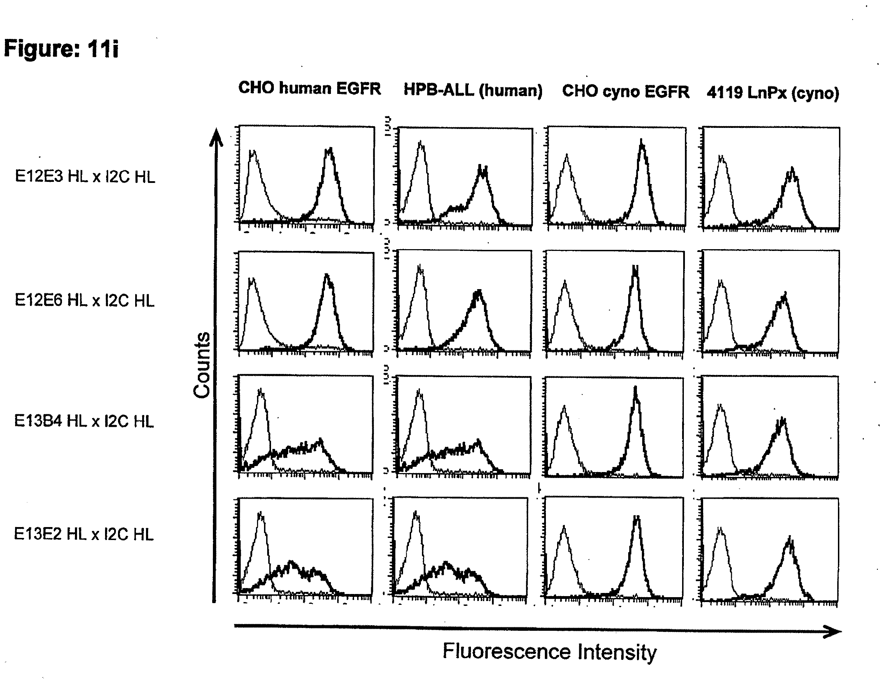

The binding ability of several bispecific single chain molecules which are specific for EGFR and cross-species specific for human and non-chimpanzee primate CD3 were clearly detectable as shown in Figure 11. In the FACS analysis, all constructs showed binding to CD3 and EGFR compared to culture medium and first and second detection antibody as the negative controls. Cross-species specificity of the bispecific antibody to human and cynomolgus CD3 and EGFR antigens was demonstrated.

## 13. Bioactivity of EGFR and CD3 cross-species specific bispecific single chain antibodies

Bioactivity of the generated bispecific single chain antibodies was analyzed by chromium 51 ($^{51}$Cr) release in vitro cytotoxicity assays using the EGFR positive cell lines described in Examples 8 and 9. As effector cells stimulated human CD8 positive T cells or the macaque T cell line 4119LnPx were used, respectively.

Generation of the stimulated CD8+ T cells was performed as follows:

A Petri dish (145 mm diameter, Greiner) was pre-coated with a commercially available anti-CD3 specific antibody in a final concentration of 1 $\mu$g/ml for 1 hour at 37°C. Unbound protein was removed by one washing step with PBS. The fresh PBMC's were isolated from peripheral blood (30 - 50 ml human blood) by Ficoll gradient centrifugation according to standard protocols. 3 - 5 $\times$ 10$^7$ PBMCs were added to the precoated petri dish in 120 ml of RPMI 1640 / 10% FCS / IL-2 20 U/ml (Proleukin, Chiron) and stimulated for 2 days. At the third day the cells were collected, washed once with RPMI 1640. IL-2 was added to a final concentration of 20 U/ml and cultivated again for one day. CD8+ cytotoxic T lymphocytes (CTLs) were isolated by depletion of CD4+ T cells and CD56+ NK cells.

Target cells were washed twice with PBS and labeled with 11,1 MBq $^{51}$Cr in a final volume of 100$\mu$l RPMI with 50% FCS for 45 minutes at 37°C. Subsequently the labeled target cells were washed 3 times with 5 ml RPMI and then used in the cytotoxicity assay. The assay was performed in a 96 well plate in a total volume of 250$\mu$l supplemented RPMI (as above) with an E:T ratio of 10:1. 1 $\mu$g/ml of the cross-species specific bispecific single chain antibody molecules and 20 threefold dilutions thereof were applied. Alternatively cell culture supernatant of transiently produced proteins was serially diluted in 1:2 steps. The assay time is 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gammacounter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically had R$^2$ values >0.90 as determined by the software. EC$_{50}$ values calculated by the analysis program were used for comparison of bioactivity.

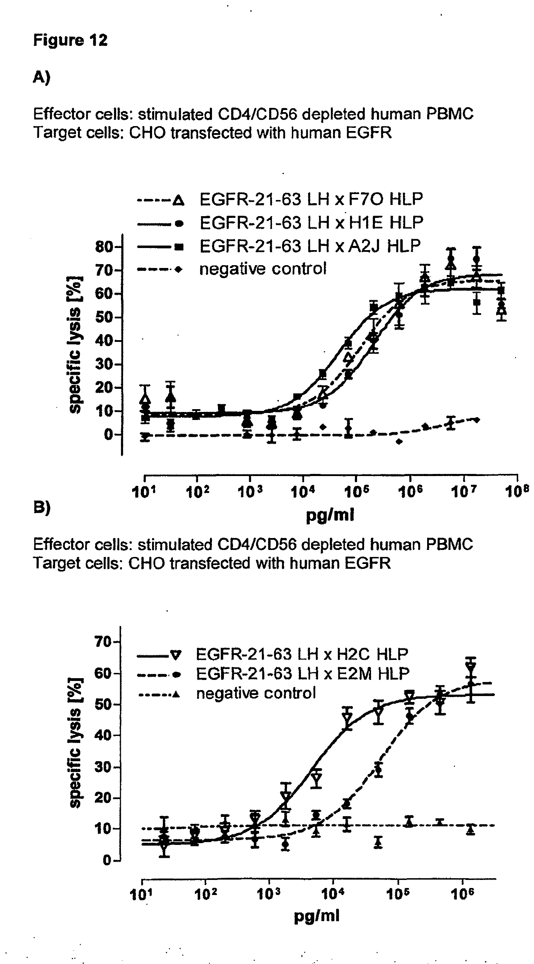

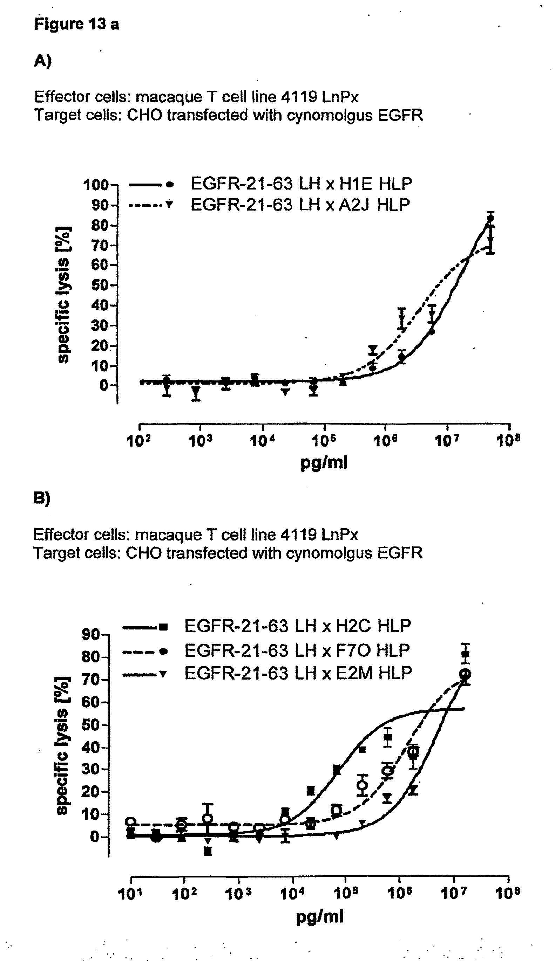

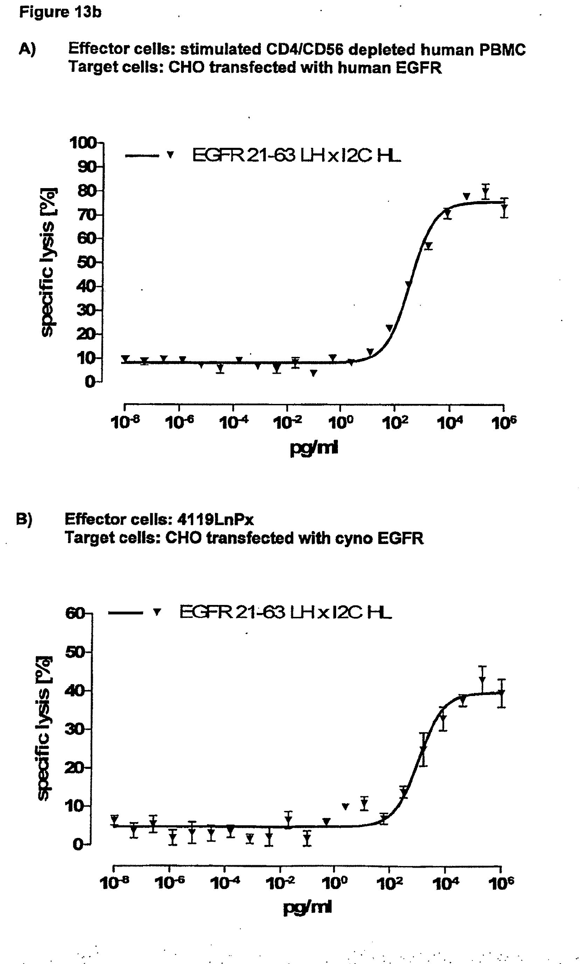

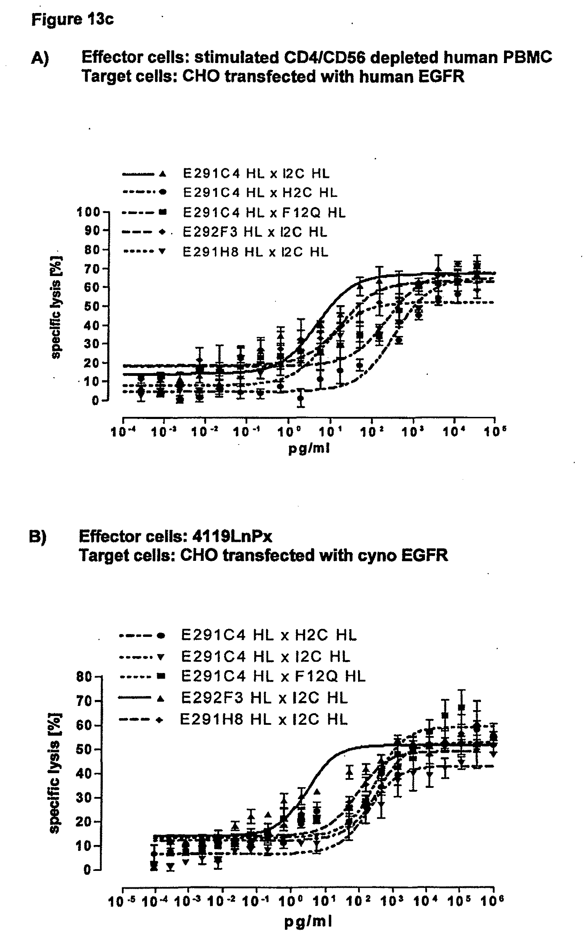

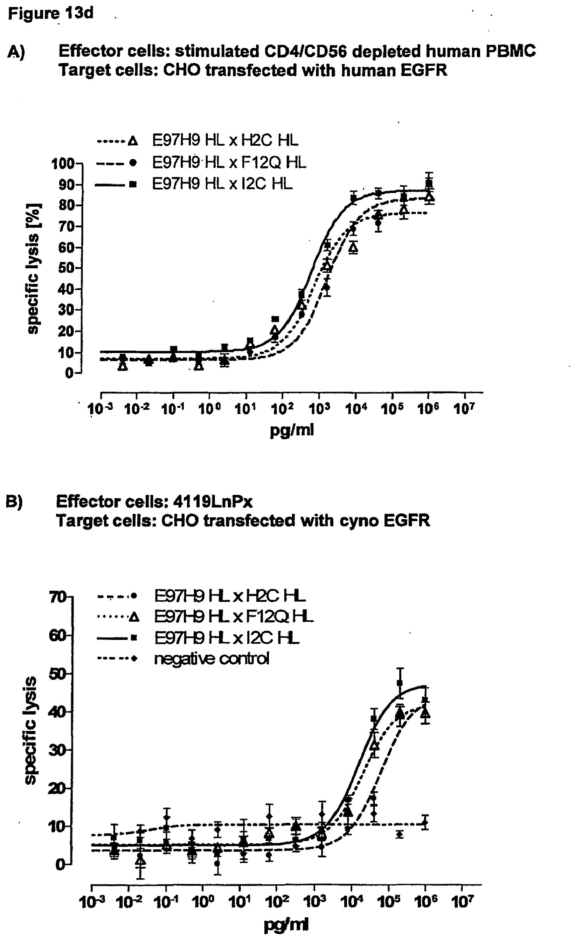

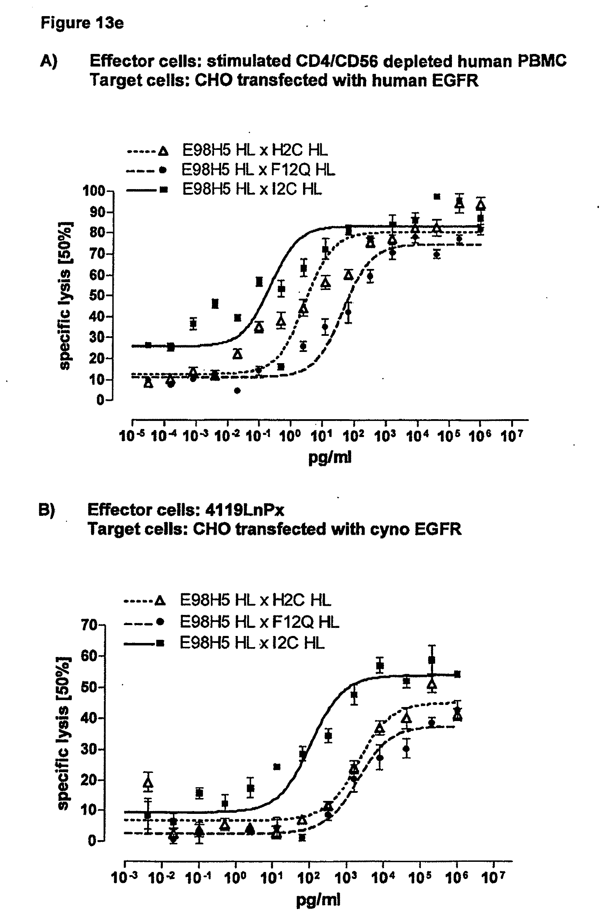

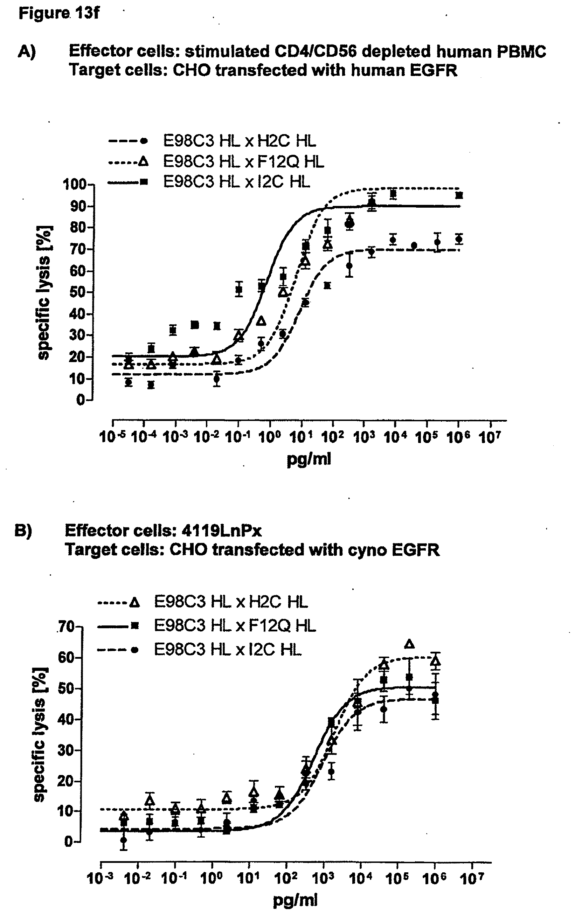

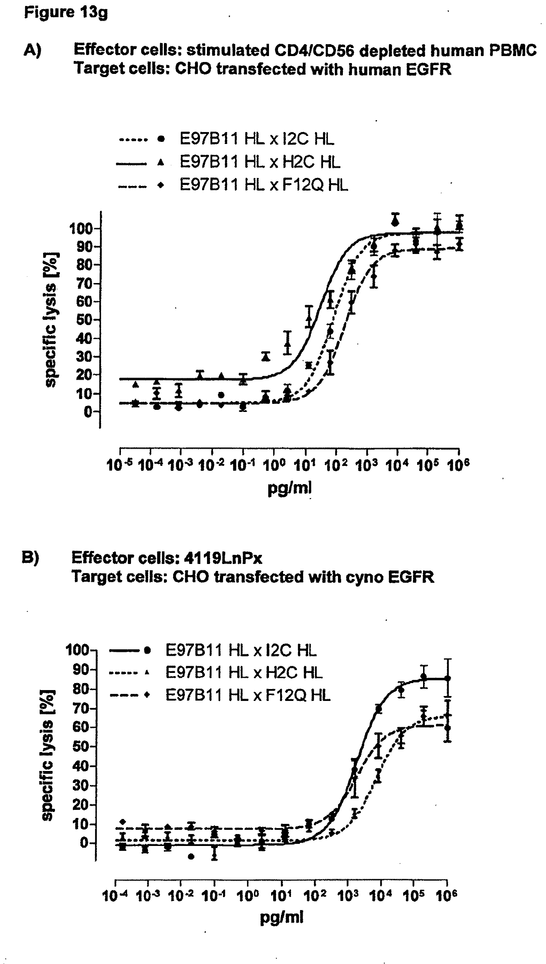

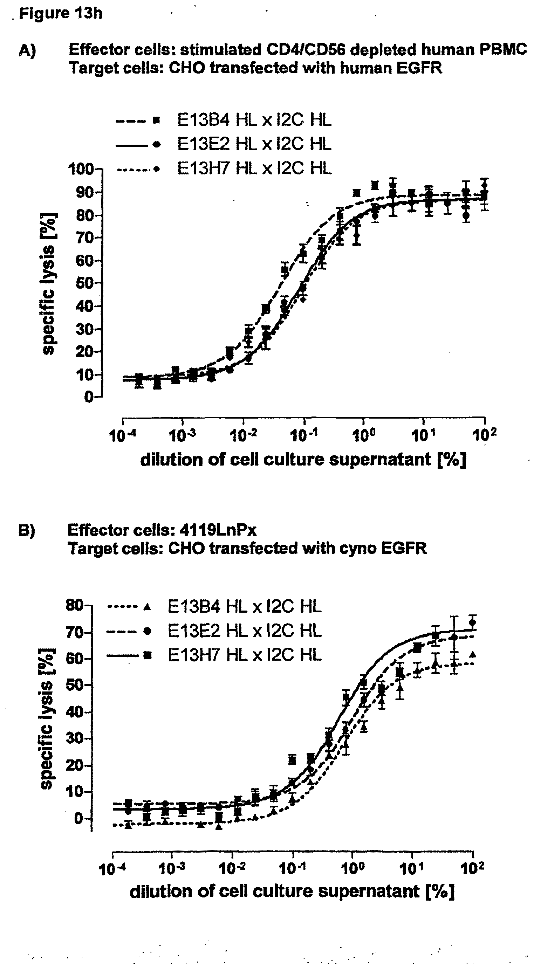

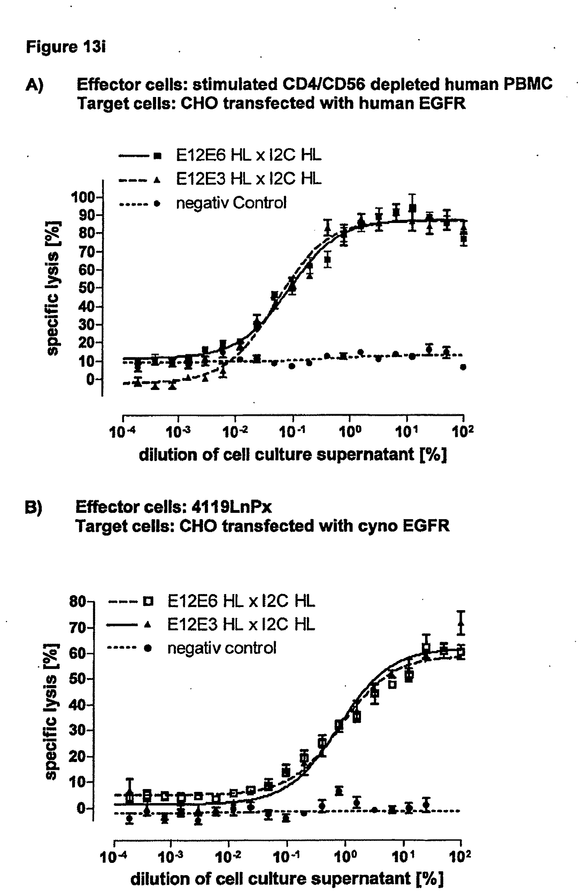

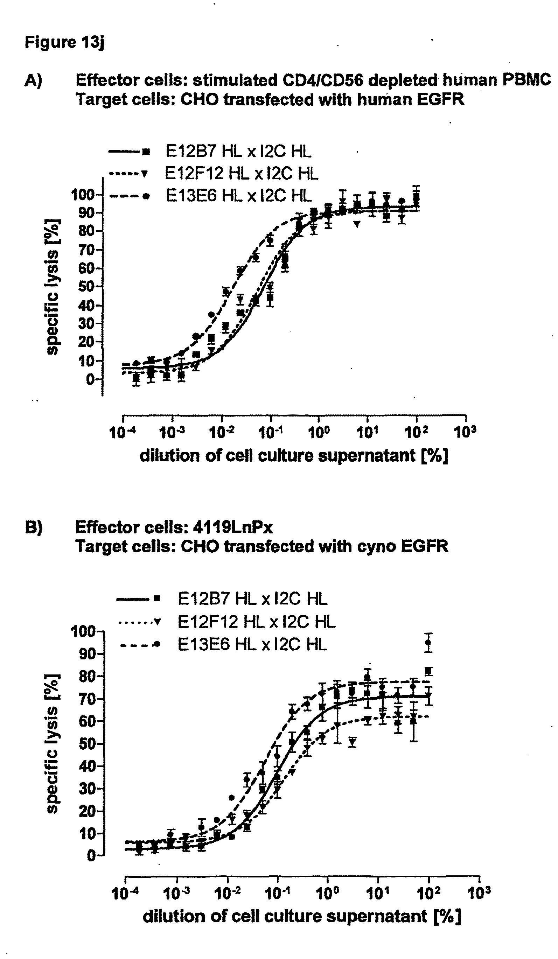

As shown in Figures 12 and 13 all of the generated cross-species specific bispecific single chain antibody constructs revealed cytotoxic activity against human EGFR positive target cells elicited by human CD8+ cells and cynomolgus EGFR positive target cells elicited by the macaque T cell line 4119LnPx. A bispecific single chain antibody with different target specificity was used as negative control.

## 14. Cloning and expression of the C-terminal, transmembrane and truncated extracellular domains of human MCSP

The coding sequence of the C-terminal, transmembrane and truncated extracellular domain of human MCSP (amino acids 1538 - 2322) was obtained by gene synthesis according to standard protocols (cDNA sequence and amino acid sequence of the recombinant construct for expression of the C-terminal, transmembrane and truncated extracellular domain of human MCSP (designated as human D3) are listed under SEQ ID NOs 374 and 373). The gene synthesis fragment was designed as to contain first a Kozak site to allow eukaryotic expression of the construct followed by the coding sequence of an 19 amino acid immunoglobulin leader peptide followed in frame by a FLAG tag, followed in frame by a sequence containing several restriction sites for cloning purposes and coding for a 9 amino acid artificial linker (SRTRSGSQL), followed in frame by the coding sequence of the C-terminal, transmembrane and truncated extracellular domain of human MCSP and a stop codon. Restriction sites were introduced at the beginning and at the end of the DNA fragment. The restriction sites EcoRI at the 5' end and Sail at the 3' end were used in the following cloning procedures. The fragment was digested with EcoRI and Sall and cloned into pEF-DHFR (pEF-DHFR is described in Mack et al. Proc. Natl. Acad. Sci. USA 92 (1995) 7021-7025) following standard protocols. A sequence verified plasmid was used to transfect CHO/dhfr- cells (ATCC No. CRL 9096). Cells were cultivated in RPMI 1640 with stabilized glutamine, supplemented with 10% FCS, 1% penicillin/streptomycin (all obtained from Biochrom AG Berlin, Germany) and nucleosides from a stock solution of cell culture grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37 °C, 95% humidity and 7% $CO_2$. Transfection was performed using the PolyFect Transfection Reagent (Qiagen GmbH, Hilden, Germany) and 5 $\mu$g of plasmid DNA according to the manufacturer's protocol. After cultivation for 24 hours cells were washed once with PBS and cultivated again in RPMI 1640 with stabilized glutamine and 1% penicillin/streptomycin. Thus the cell culture medium did not contain nucleosides and thereby selection was applied on the transfected cells. Approximately 14 days after transfection the outgrowth of resistant cells was observed. After an additional 7 to 14 days the transfectants were tested for expression of the construct by FACS analysis. $2,5 \times 10^5$ cells were incubated with 50 $\mu$l of an anti-Flag-M2 antibody (Sigma-Aldrich Chemie GmbH, Taufkirchen, Germany) diluted to 5 $\mu$g/ml in PBS with 2% FCS. The binding of the antibody was detected with a R-Phycoerythrin-conjugated affinity purified F(ab')2 fragment, goat anti-mouse IgG, Fc-gamma fragment specific diluted 1:100 in PBS with 2% FCS (ImmunoResearch Europe Ltd., Newmarket, Suffolk, UK). The samples were measured on a FACScalibur (BD biosciences, Heidelberg, Germany).

**15. Cloning and expression of the C-terminal, transmembrane and truncated extracellular domains of macaque MCSP**

The cDNA sequence of the C-terminal, transmembrane and truncated extracellular domains of macaque MCSP (designated as macaque D3) was obtained by a set of three PCRs on macaque skin cDNA (Cat No. C1534218-Cy-BC; BioCat GmbH, Heidelberg, Germany) using the following reaction conditions: 1 cycle at 94°C,3 min., 40 cycles with 94°C for 0,5 min., 52°C for 0,5 min. and 72°C for 1,75 min., terminal cycle of 72°C for 3 min.. The following primers were used:

forward primer: 5'-GATCTGGTCTACACCATCGAGC-3'
reverse primer: 5'-GGAGCTGCTGCTGGCTCAGTGAGG-3'
forward primer: 5'- TTCCAGCTGAGCATGTCTGATGG-3'
reverse primer: 5'- CGATCAGCATCTGGGCCCAGG-3'
forward primer: 5'- GTGGAGCAGTTCACTCAGCAGGACC-3'
reverse primer: 5'- GCCTTCACACCCAGTACTGGCC-3'

Those PCRs generated three overlapping fragments (A: 1-1329, B: 1229-2428, C: 1782-2547) which were isolated and sequenced according to standard protocols using the PCR primers and thereby provided a 2547 bp portion of the cDNA sequence of macaque MCSP (the cDNA sequence and amino acid sequence of this portion of macaque MCSP are listed under SEQ ID NOs 376 and 375) from 74 bp upstream of the coding sequence of the C-terminal domain to 121 bp downstream of the stop codon. Another PCR using the following reaction conditions: 1 cycle at 94°C for 3 min, 10 cycles with 94°C for 1 min, 52°C for 1 min and 72°C for 2,5 min, terminal cycle of 72°C for 3 min was used to fuse the PCR products of the aforementioned reactions A and B. The following primers are used:

forward primer: 5'-tcccgtacgagatctggatcccaattggatggcggactcgtgctgttctcacacagagg-3'
reverse primer: 5'-agtgggtcgactcacacccagtactggccattcttaagggcaggg-3'

The primers for this PCR were designed to introduce restriction sites at the beginning and at the end of the cDNA fragment coding for the C-terminal, transmembrane and truncated extracellular domains of macaque MCSP. The introduced restriction sites Mfel at the 5' end and Sall at the 3' end, were used in the following cloning procedures. The PCR fragment was then cloned via Mfel and Sall into a Bluescript plasmid containing the EcoRI/Mfel fragment of the aforementioned plasmid pEF-DHFR (pEF-DHFR is described in Raum et al. Cancer Immunol Immunother 50 (2001) 141-150) by replacing the C-terminal, transmembrane and truncated extracellular domains of human MCSP. The gene synthesis fragment contained the coding sequences of the immunoglobulin leader peptide and the Flag tag as well as the artificial linker (SRTRSGSQL) in frame to the 5' end of the cDNA fragment coding for the C-terminal, transmembrane and truncated extracellular domains of macaque MCSP. This vector was used to transfect CHO/dhfr- cells (ATCC No. CRL 9096). Cells were cultivated in RPMI 1640 with stabilized glutamine supplemented with 10% FCS, 1% penicillin/streptomycin (all from Biochrom AG Berlin, Germany) and nucleosides from a stock solution of cell culture grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37 °C, 95% humidity and 7% CO2. Transfection was performed with PolyFect Transfection Reagent (Qiagen GmbH, Hilden, Germany) and 5 $\mu$g of plasmid DNA according to the manufacturer's protocol. After cultivation for 24 hours cells were washed once with PBS and cultivated again in RPMI 1640 with stabilized glutamine and 1% penicillin/streptomycin. Thus the cell culture medium did not contain nucleosides and thereby selection was applied on the transfected cells. Approximately 14 days after transfection the outgrowth of resistant cells is observed. After an additional 7 to 14 days the transfectants were tested for expression of the recombinant construct via FACS. $2,5\times10^5$ cells were incubated with 50 $\mu$l of an anti-Flag-M2 antibody (Sigma-Aldrich Chemie GmbH, Taufkirchen, Germany) diluted to 5 $\mu$g/ml in PBS with 2% FCS. Bound antibody was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACScalibur (BD biosciences, Heidelberg, Germany).

## 16. Generation and characterisation of MCSP and CD3 cross-species specific bispecific single chain molecules

Bispecific single chain antibody molecules each comprising a binding domain cross-species specific for human and non-chimpanzee primate CD3 epsilon as well as a binding domain cross-species-specific for human and non-chimpanzee primate MCSP, are designed as set out in the following Table 2:

Table 2: Formats of MCSP and CD3 cross-species specific bispecific single chain antibodies

| SEQ ID (nucl/prot) | Formats of protein constructs (N → C) |
|---|---|
| 310/309 | MCSP-G4 HL × H2C HL |
| 312/311 | MCSP-G4 HL × F12Q HL |
| 314/313 | MCSP-G4 HL × I2C HL |
| 316/315 | MCSP-G4 HLP × F6A HLP |
| 318/317 | MCSP-G4 HLP × H2C HLP |
| 322/321 | MCSP-G4 HLP × G4H HLP |
| 326/325 | MCSP-G4 HLP × E1L HLP |
| 328/327 | MCSP-G4 HLP × E2M HLP |
| 332/331 | MCSP-G4 HLP × F12Q HL |
| 334/333 | MCSP-G4 HLP × I2C HL |
| 336/335 | MCSP-D2 HL × H2C HL |
| 338/337 | MCSP-D2 HL × F12Q HL |
| 340/339 | MCSP-D2 HL × I2C HL |
| 342/341 | MCSP-D2 HLP × H2C HLP |
| 344/343 | MCSP-F9 HL × H2C HL |
| 346/345 | MCSP-F9 HLP × H2C HLP |
| 348/347 | MCSP-F9 HLP × G4H HLP |
| 1355/1354 | MCSP-A9 HL × H2C HL |
| 1357/1356 | MCSP-A9 HL × F12Q HL |
| 1359/1358 | MCSP-A9 HL × I2C HL |
| 1373/1372 | MCSP-C8 HL × I2C HL |
| 1401/1400 | MCSP-B7 HL × I2C HL |
| 1387/1386 | MCSP-B8 HL × I2C HL |
| 1415/1414 | MCSP-G8 HL × I2C HL |

(continued)

| SEQ ID (nucl/prot) | Formats of protein constructs (N → C) |
| --- | --- |
| 1429/1428 | MCSP-D5 HL × I2C HL |
| 1443/1442 | MCSP-F7 HL × I2C HL |
| 1457/1456 | MCSP-G5 HL × I2C HL |
| 1471/1470 | MCSP-F8 HL × I2C HL |
| 1485/1484 | MCSP-G10 HL × I2C HL |

The aforementioned constructs containing the variable heavy-chain (VH) and variable light-chain (VL) domains cross-species specific for human and macaque MCSP D3 and the VH and VL domains cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a histidine$_6$-tag and a stop codon. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were transfected stably or transiently into DHFR-deficient CHO-cells (ATCC No. CRL 9096) by electroporation or alternatively into HEK 293 (human embryonal kidney cells, ATCC Number: CRL-1573) in a transient manner according to standard protocols.

Eukaryotic protein expression in DHFR deficient CHO cells was performed as described by Kaufmann R.J. (1990) Methods Enzymol. 185, 537-566. Gene amplification of the constructs was induced by addition of increasing concentrations of methothrexate (MTX) up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein is stored at -20°C.

Äkta® Explorer System (GE Health Systems) and Unicorn® Software were used for chromatography. Immobilized metal affinity chromatography ("IMAC") was performed using a Fractogel EMD chelate® (Merck) which was loaded with ZnCl$_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in phosphate buffered saline (PBS). All constructs were purified according to this method.

Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

Alternatively, constructs were transiently expressed in DHFR deficient CHO cells. In brief, $4 \times 10^5$ cells per construct were cultivated in 3 ml RPMI 1640 all medium with stabilized glutamine supplemented with 10% fetal calf serum, 1% penicillin/streptomycin and nucleosides from a stock solution of cell culture grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37 °C, 95% humidity and 7% CO2 one day before transfection. Transfection was performed with Fugene 6 Transfection Reagent (Roche, # 11815091001) according to the manufacturer's protocol. 94 $\mu$l OptiMEM medium (Invitrogen) and 6 $\mu$l Fugene 6 are mixed and incubated for 5 minutes at room temperature. Subsequently, 1.5 $\mu$g DNA per construct were added, mixed and incubated for 15 minutes at room temperature. Meanwhile, the DHFR deficient CHO cells were washed with 1x PBS and resuspended in 1.5 ml RPMI 1640 all medium. The transfection mix was diluted with 600 $\mu$l RPMI 1640 all medium, added to the cells and incubated overnight at 37 °C, 95% humidity and 7% CO2. The day after transfection the incubation volume of each approach was extended to 5 ml RPMI 1640 all medium. Supernatant was harvested after 3 days of incubation.

## 17. Flow cytometric binding analysis of the MCSP and CD3 cross-species specific bispecific antibodies

In order to test the functionality of the cross-species specific bispecific antibody constructs regarding the capability to bind to human and macaque MCSP D3 and CD3, respectively, a FACS analysis was performed. For this purpose CHO cells transfected with human MCSP D3 (as described in Example 14) and the human CD3 positive T cell leukemia cell line HPB-ALL (DSMZ, Braunschweig, ACC483) were used to test the binding to human antigens. The binding reactivity to macaque antigens was tested by using the generated macaque MCSP D3 transfectant (described in Example 15) and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 $\mu$l of the purified protein of the cross-species specific bispecific antibody constructs (2 $\mu$g/ml) or cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 $\mu$l PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines. A single chain construct with irrelevant target specificity was used as negative control for binding to the MCSP-D3 transfected CHO cells.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

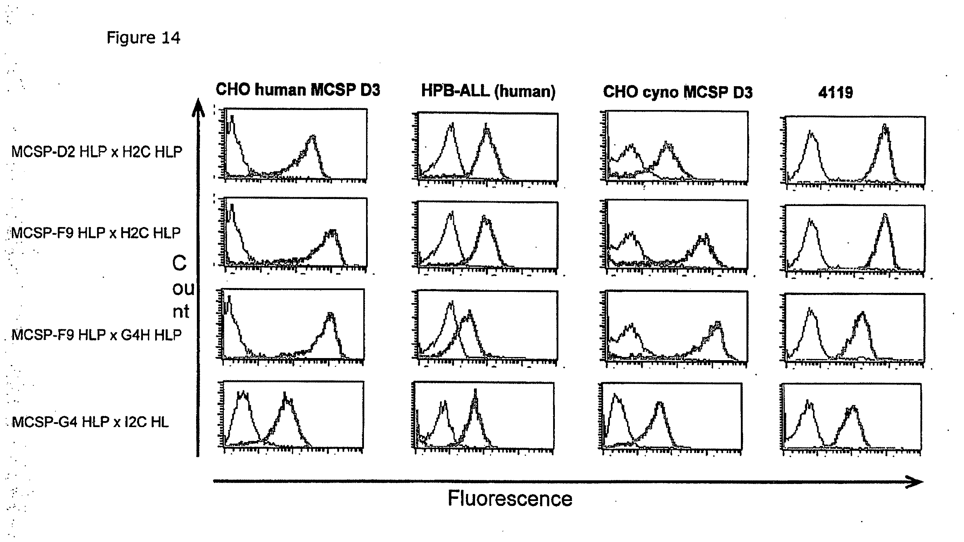

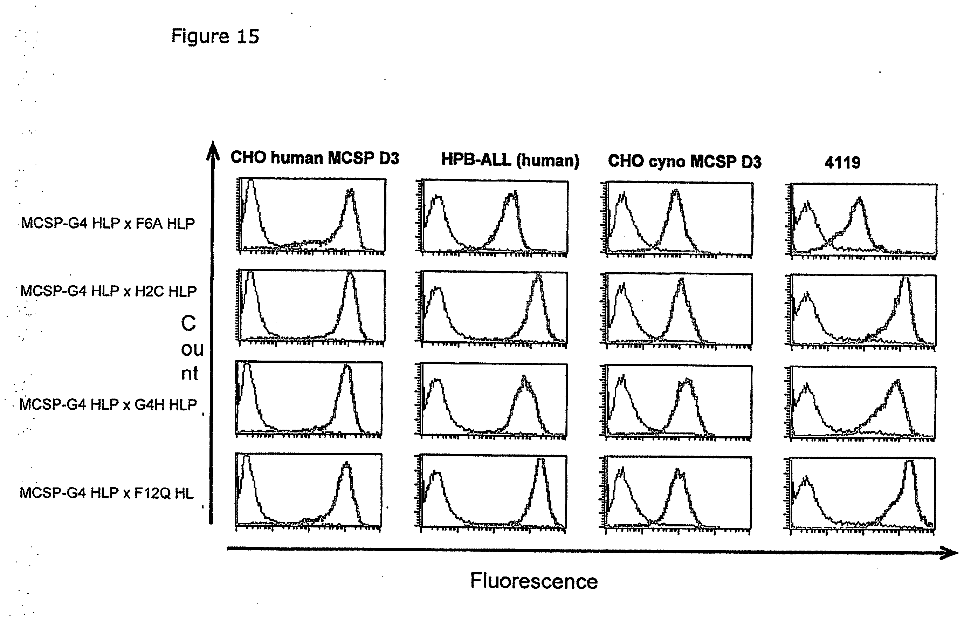

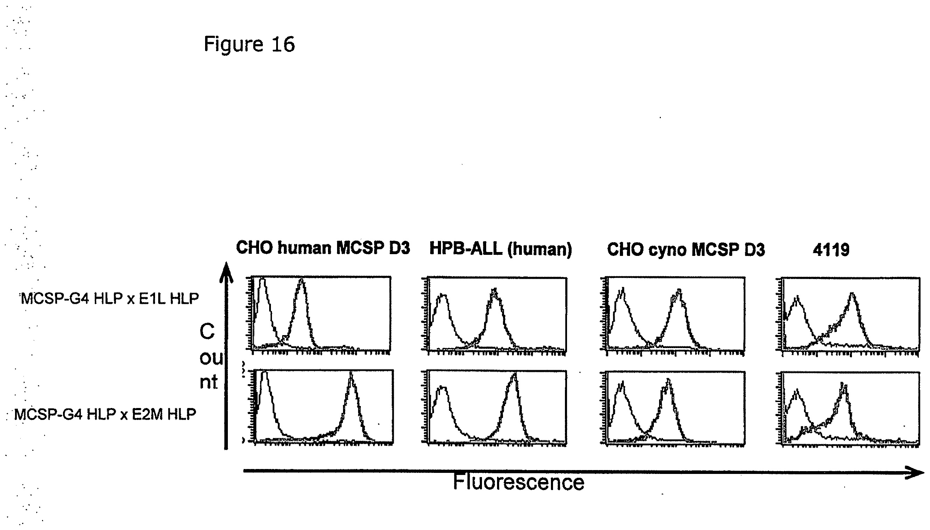

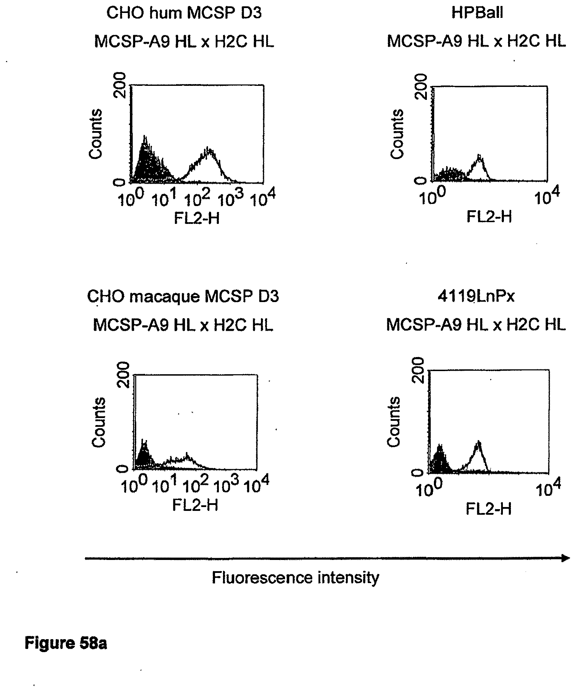

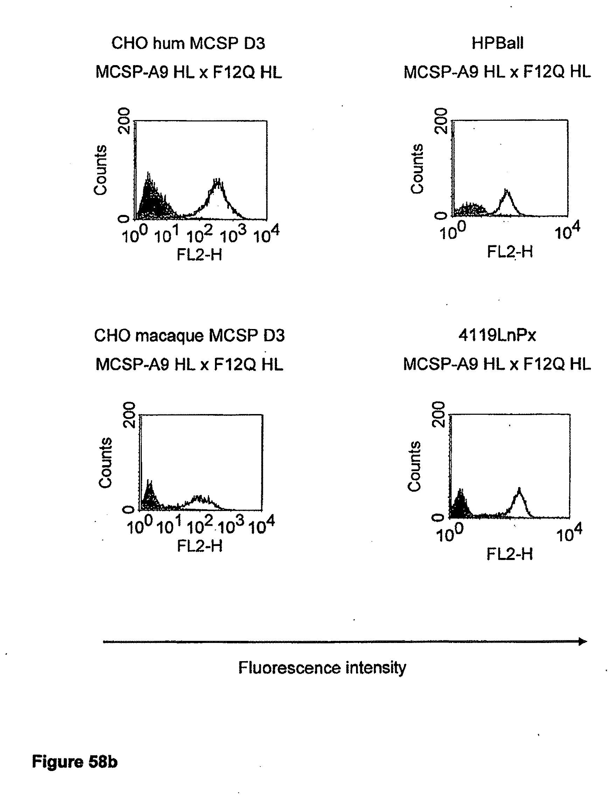

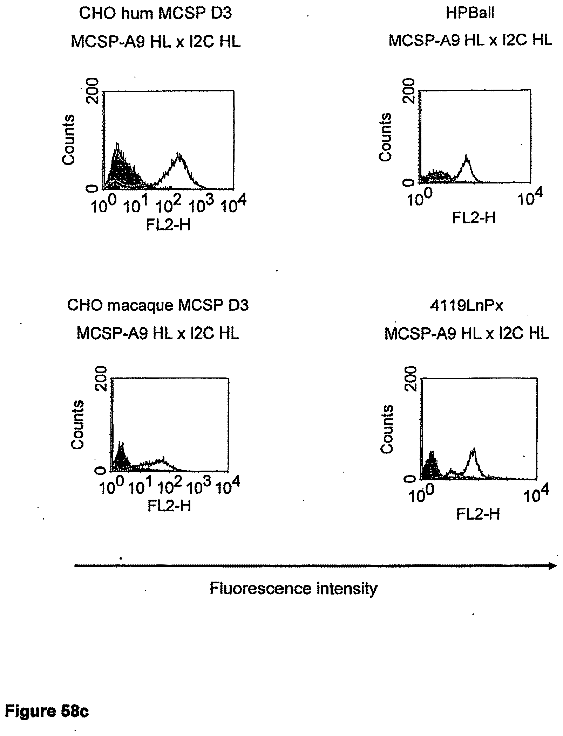

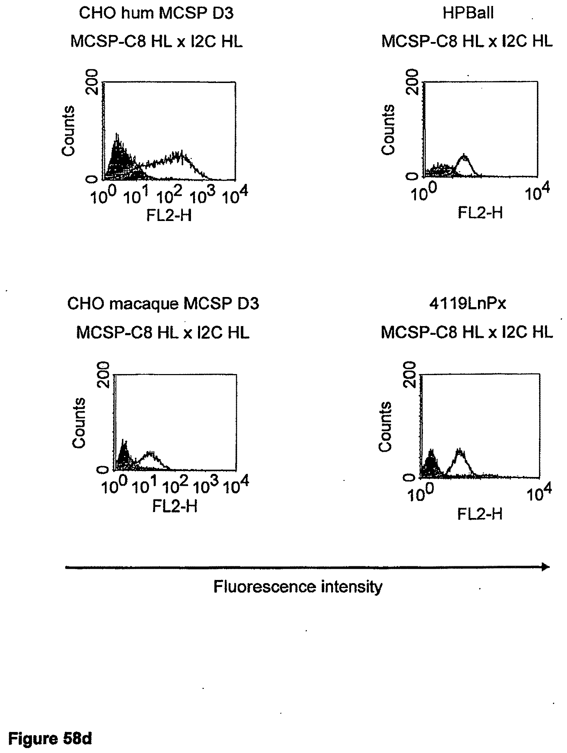

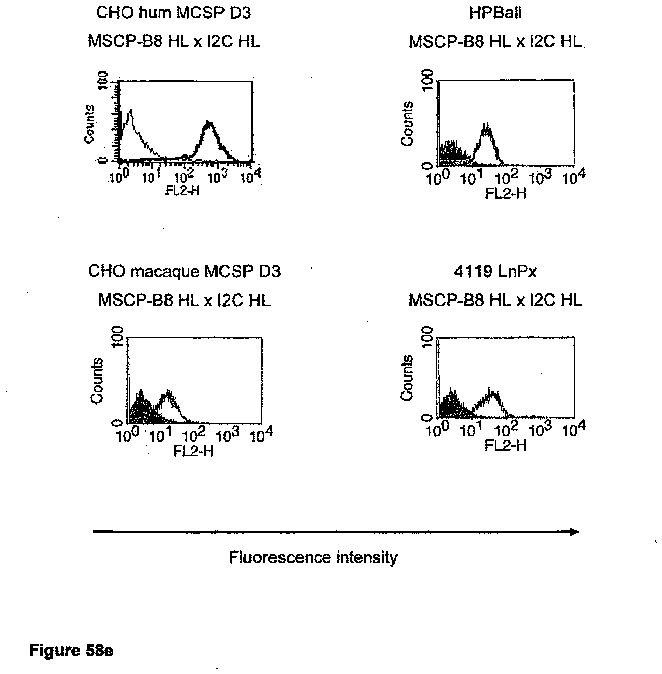

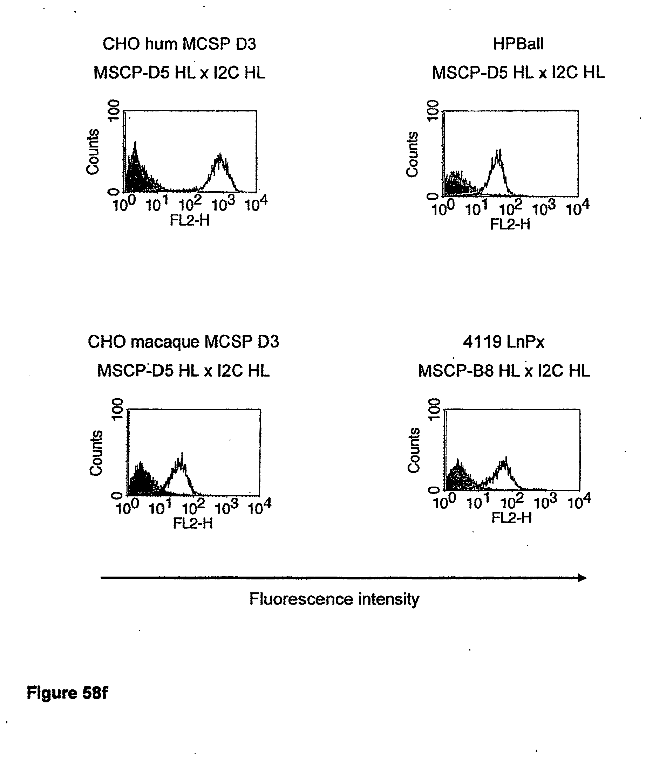

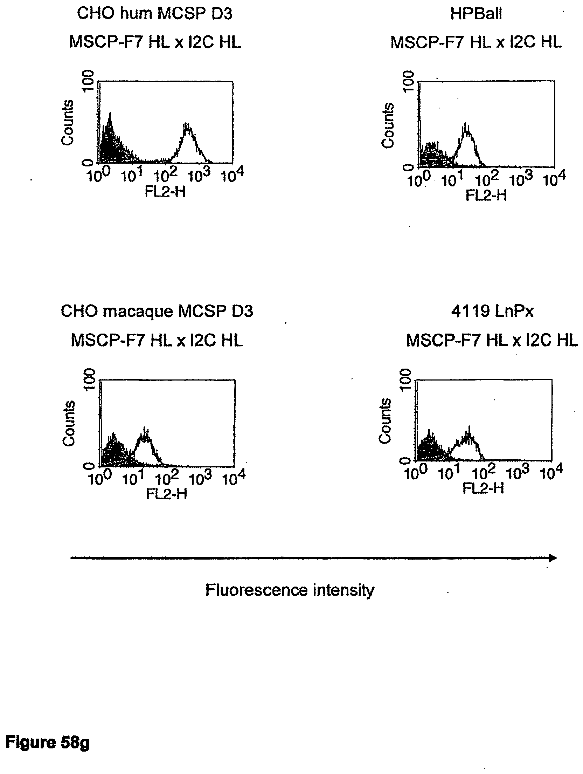

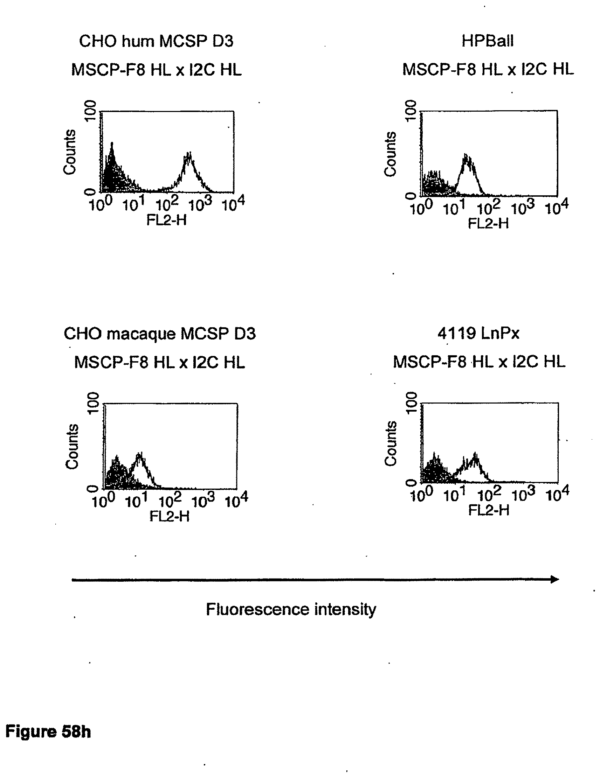

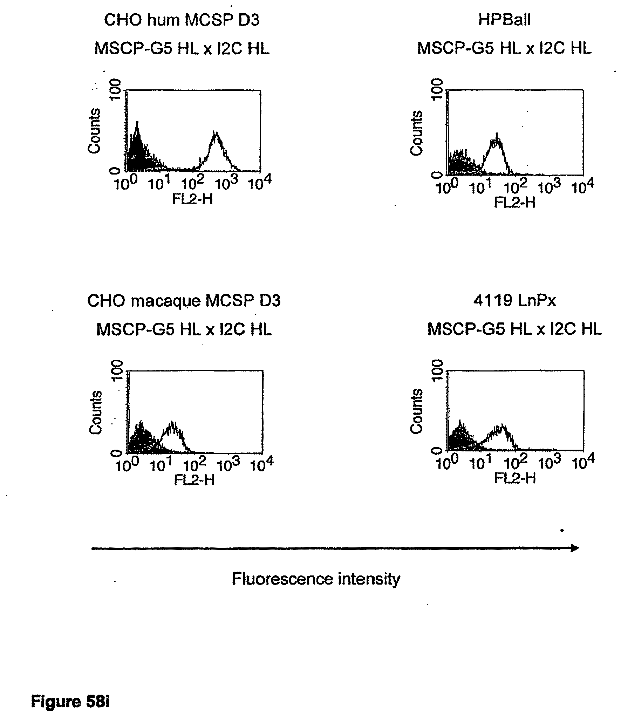

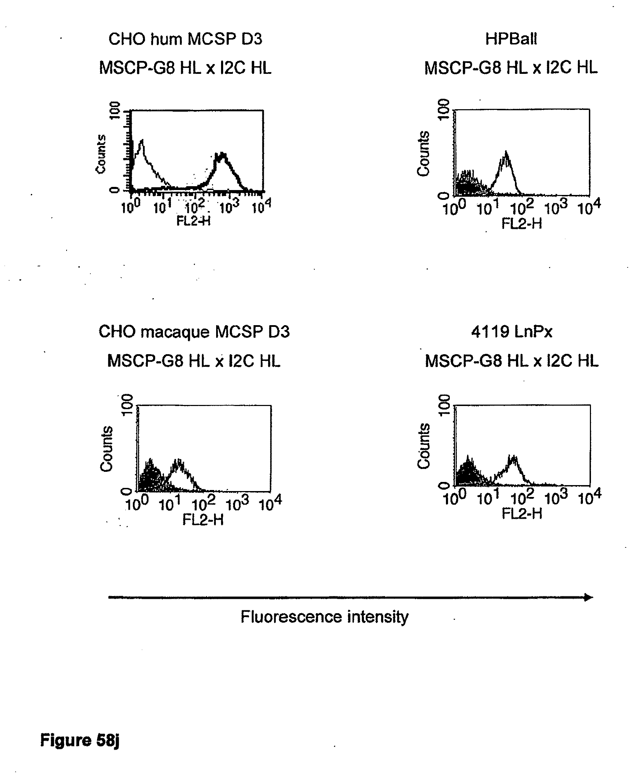

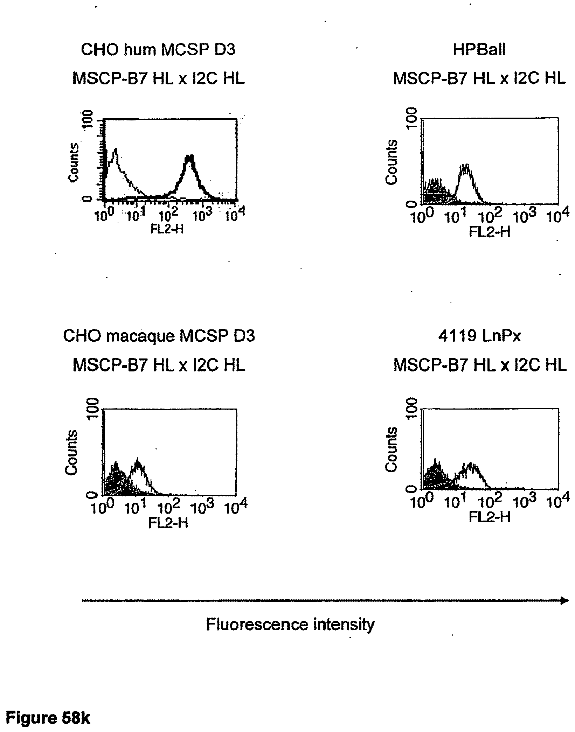

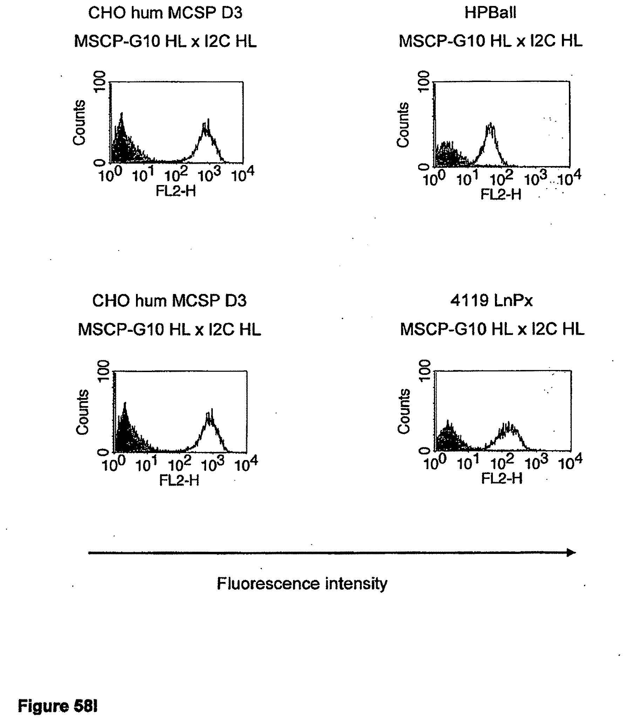

The bispecific binding of the single chain molecules listed above, which are cross-species specific for MCSP D3 and cross-species specific for human and macaque CD3 was clearly detectable as shown in Figures 14, 15, 16 and 58. In the FACS analysis all constructs showed binding to CD3 and MCSP D3 as compared to the respective negative controls. Cross-species specificity of the bispecific antibodies to human and macaque CD3 and MCSP D3 antigens was demonstrated.

## 18. Bioactivity of MCSP and CD3 cross-species specific bispecific single chain antibodies

Bioactivity of the generated bispecific single chain antibodies was analyzed by chromium 51 ($^{51}$Cr) release in vitro cytotoxicity assays using the MCSP D3 positive cell lines described in Examples 14 and 15. As effector cells stimulated human CD4/CD56 depleted PBMC, stimulated human PBMC or the macaque T cell line 4119LnPx are used as specified in the respective figures.

Generation of the stimulated CD4/CD56 depleted PBMC was performed as follows:

Coating of a Petri dish (145 mm diameter, Greiner bio-one GmbH, Kremsmünster) was carried out with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 $\times 10^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above. By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}$Cr in a final volume of 100 $\mu$l RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250$\mu$l supplemented RPMI (as above) with an E:T ratio 10:1. 1 $\mu$g/ml of the cross-species specific bispecific single chain antibody molecules and 20 threefold dilutions thereof were applied. If using supernatant containing the cross-species specific bispecific single chain antibody molecules, 21 two- and 20 threefold dilutions thereof were applied for the macaque and the human cytotoxicity assay, respectively. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

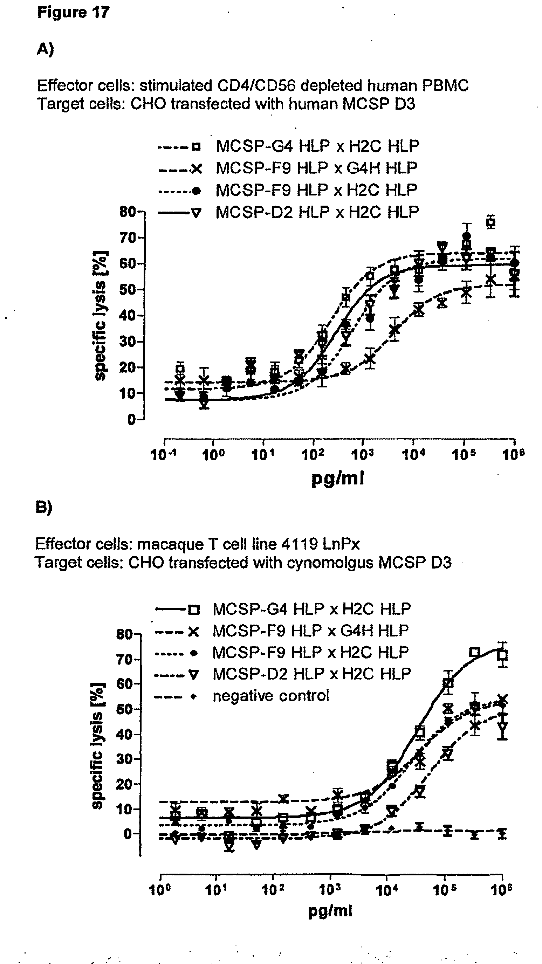

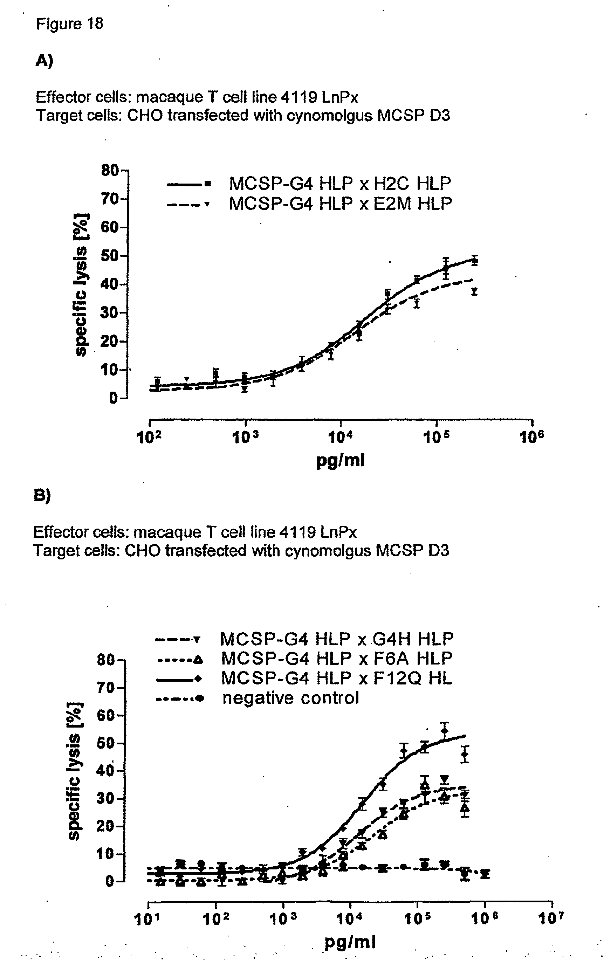

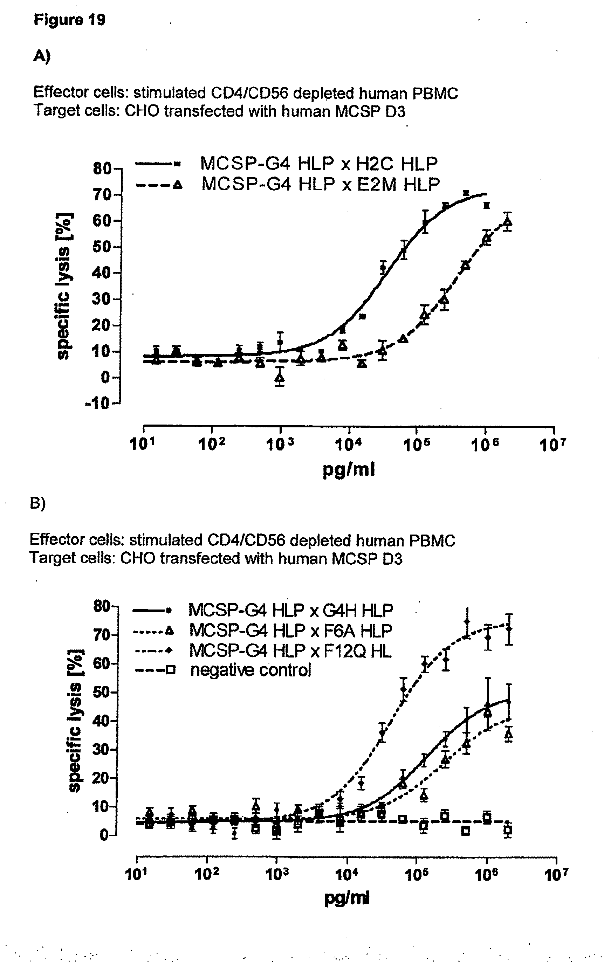

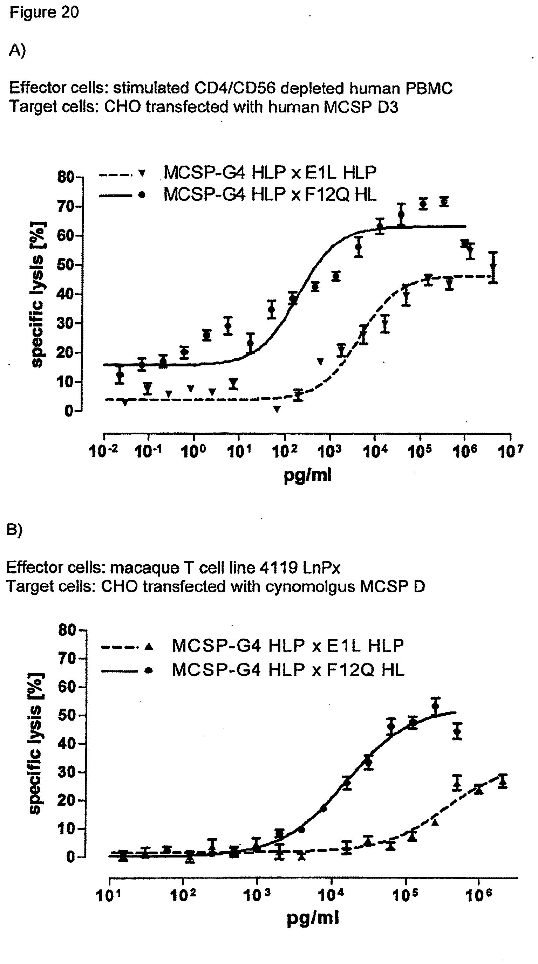

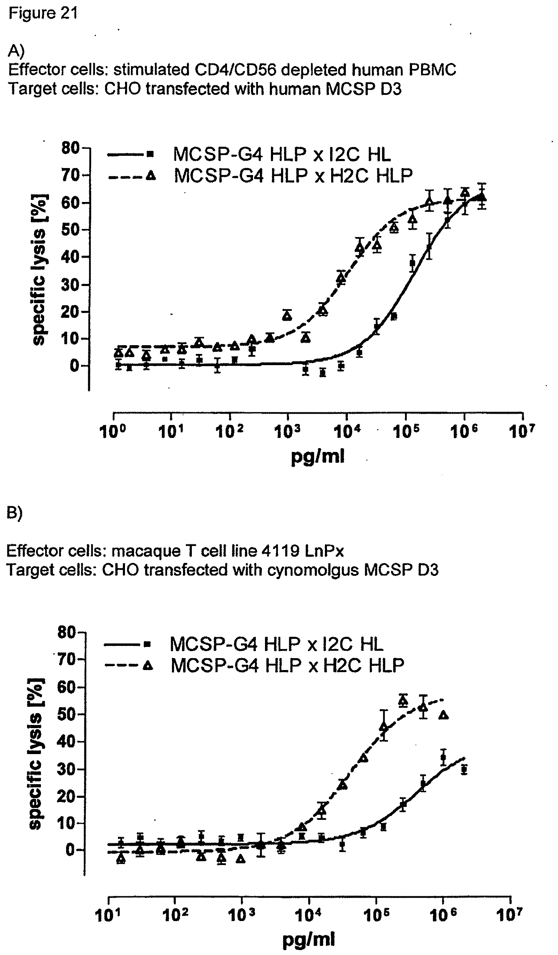

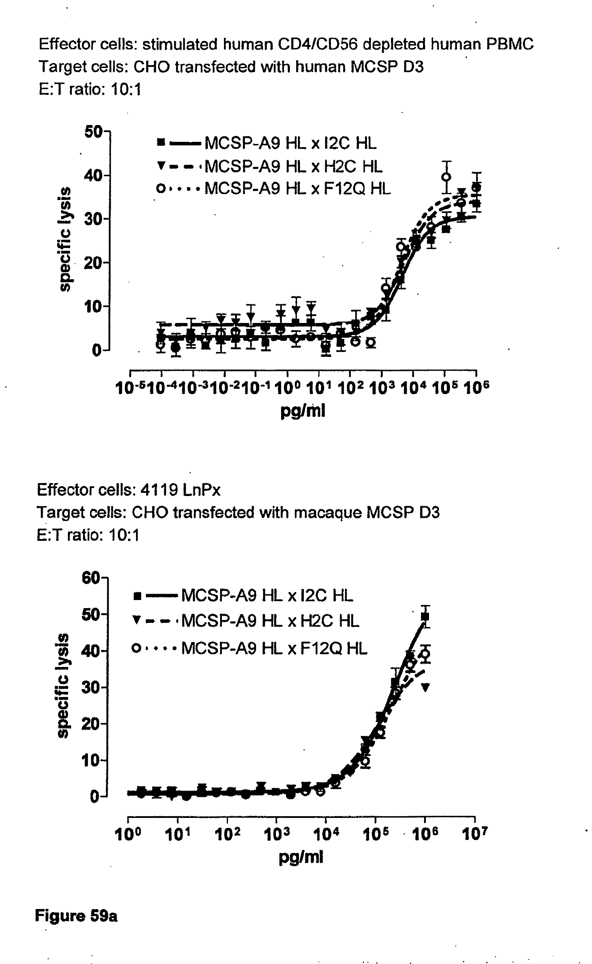

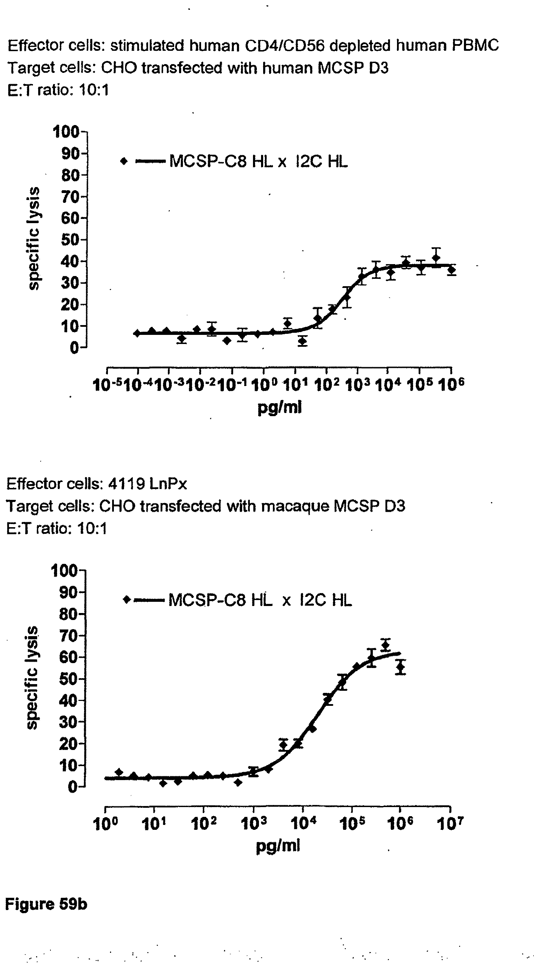

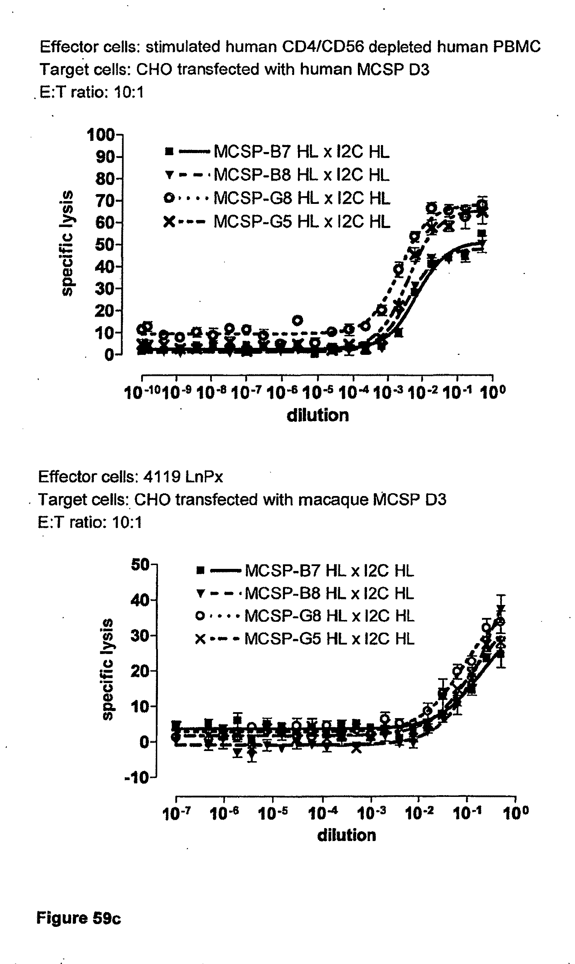

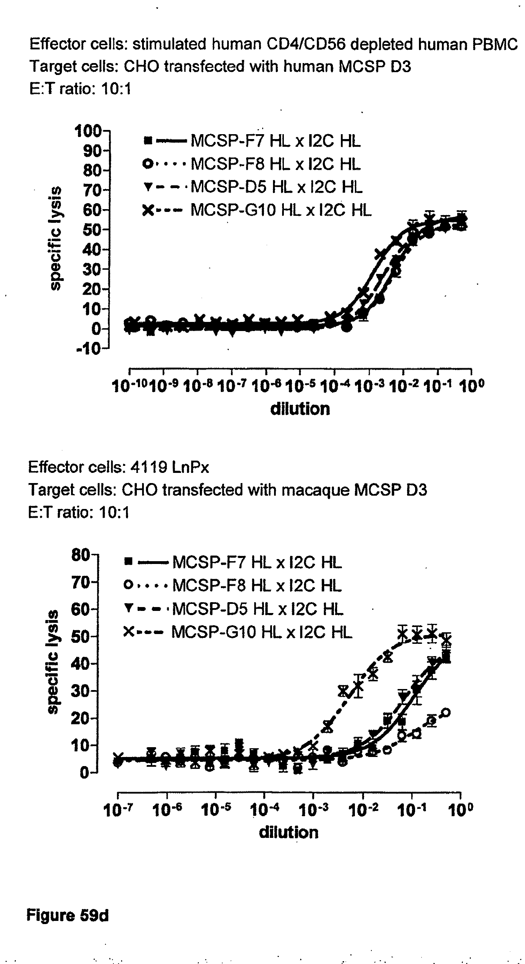

As shown in Figures 17 to 21 and 59, all of the generated cross-species specific bispecific single chain antibody constructs demonstrate cytotoxic activity against human MCSP D3 positive target cells elicited by stimulated human CD4/CD56 depleted PBMC or stimulated PBMC and against macaque MCSP D3 positive target cells elicited by the macaque T cell line 4119LnPx.

**19. Plasma stability of MCSP and CD3 cross-species specific bispecific single chain antibodies**

Stability of the generated bispecific single chain antibodies in human plasma was analyzed by incubation of the bispecific single chain antibodies in 50% human Plasma at 37°C and 4°C for 24 hours and subsequent testing of bioactivity. Bioactivity was studied in a chromium 51 ($^{51}$Cr) release in vitro cytotoxicity assay using a MCSP positive CHO cell line (expressing MCSP as cloned according to example 14 or 15) as target and stimulated human CD8 positive T cells as effector cells.

$EC_{50}$ values calculated by the analysis program as described above were used for comparison of bioactivity of bispecific single chain antibodies incubated with 50% human plasma for 24 hours at 37°C and 4°C respectively with bispecific single chain antibodies without addition of plasma or mixed with the same amount of plasma immediately prior to the assay.

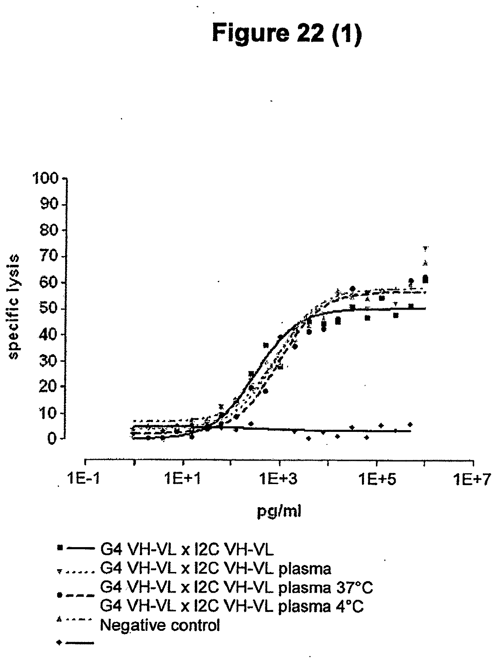

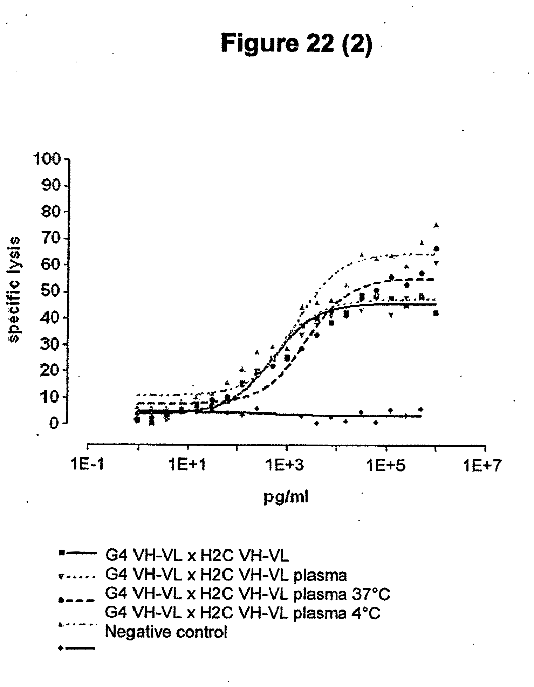

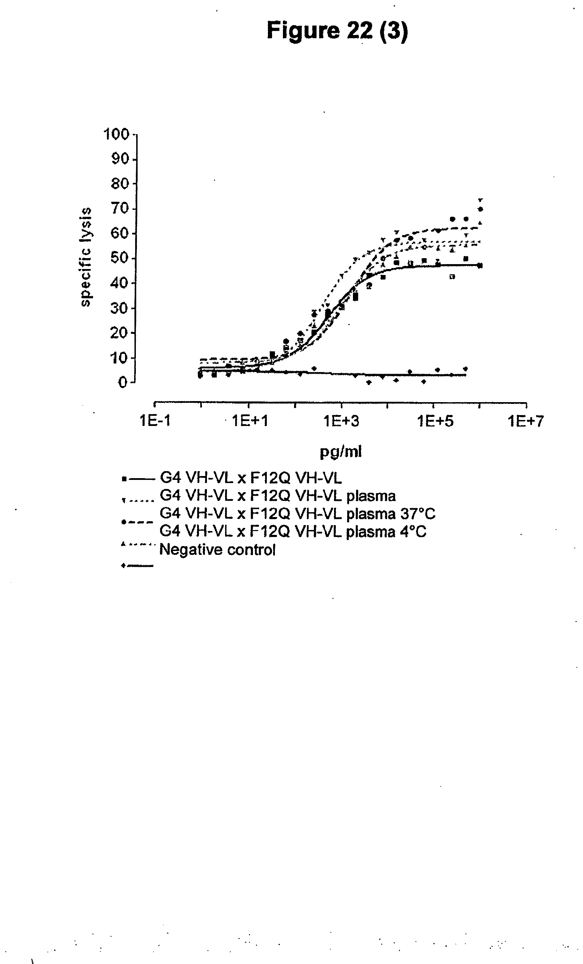

As shown in Figure 22 and Table 3 the bioactivity of the G4 H-L × I2C H-L, G4 H-L × H2C H-L and G4 H-L × F12Q H-L bispecific antibodies was not significantly reduced as compared with the controls without the addition of plasma or with addition of plasma immediately before testing of bioactivity.

Table 3: bioactivity of the bispecific antibodies without or with the addition of Plasma

| Construct | Without plasma | With plasma | Plasma 37°C | Plasma 4°C |
|---|---|---|---|---|
| G4 H-L × I2C H-L | 300 | 796 | 902 | 867 |
| G4 H-L × H2C H-L | 496 | 575 | 2363 | 1449 |
| G4 H-L × F12Q H-L | 493 | 358 | 1521 | 1040 |

**20. Redistribution of circulating T cells in the absence of circulating target cells by first exposure to CD3 binding molecules directed at conventional i.e. context dependent CD3 epitopes is a major risk factor for adverse events related to the initiation of treatment**

**T cell redistribution in patients with B-cell Non-Hodgkin-Lymphoma (B-NHL) following initiation of treatment with the conventional CD3 binding molecule**

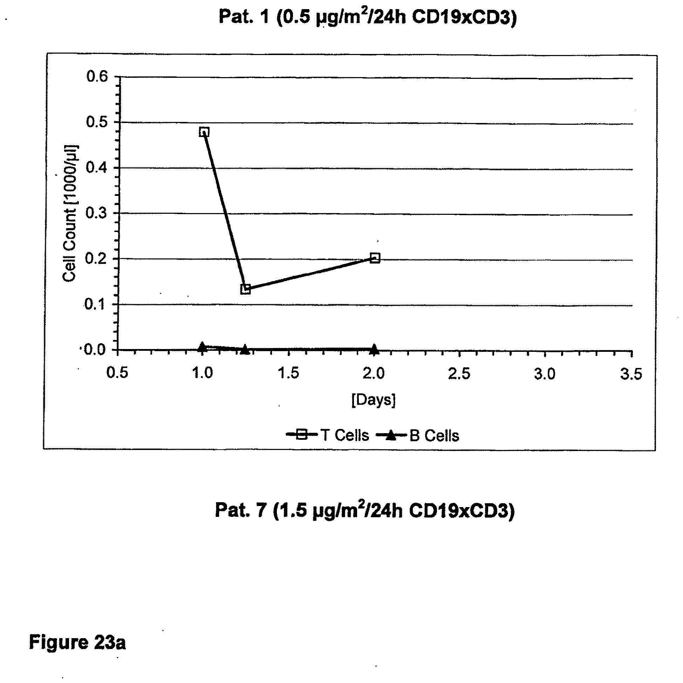

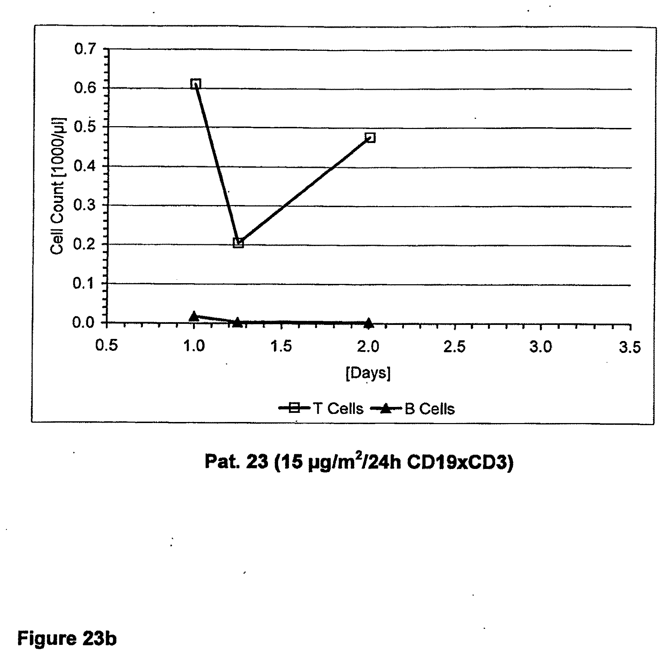

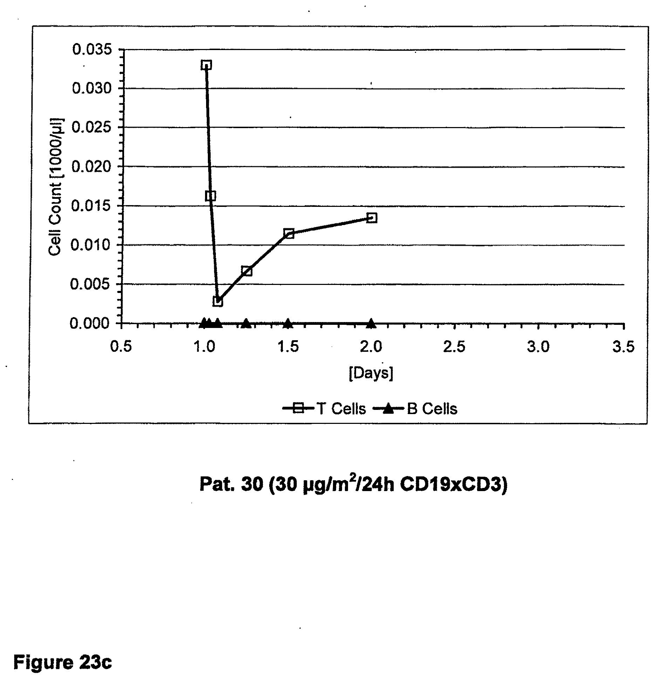

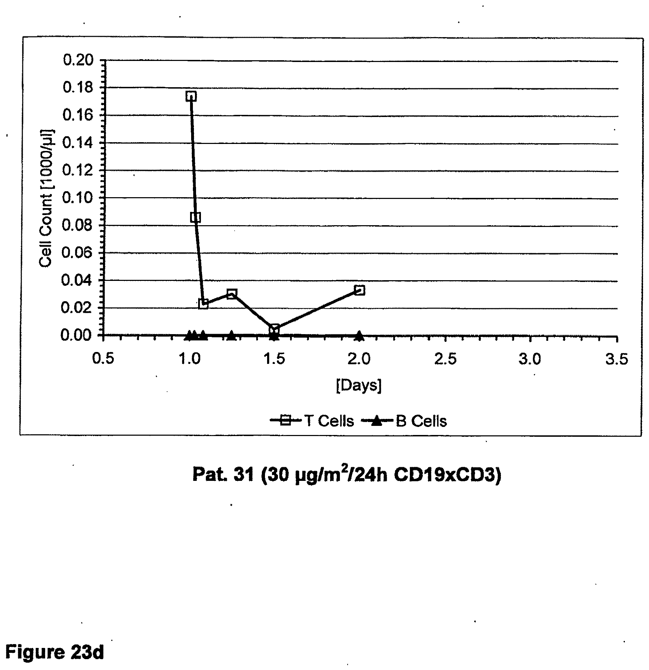

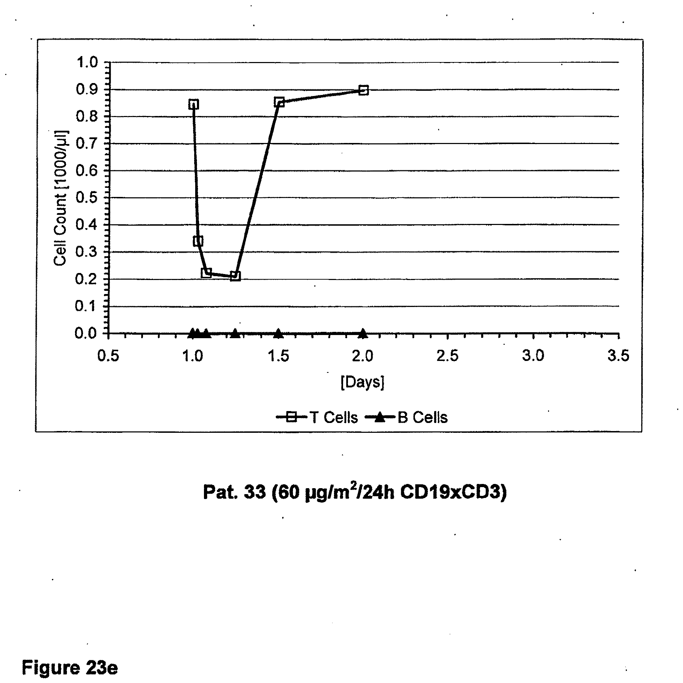

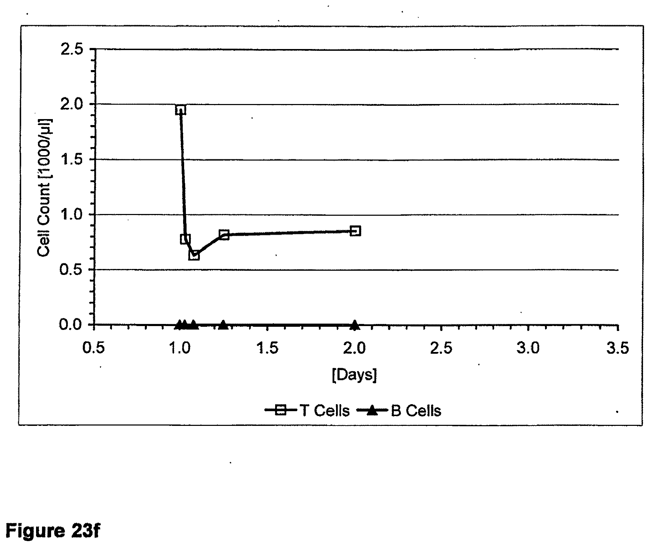

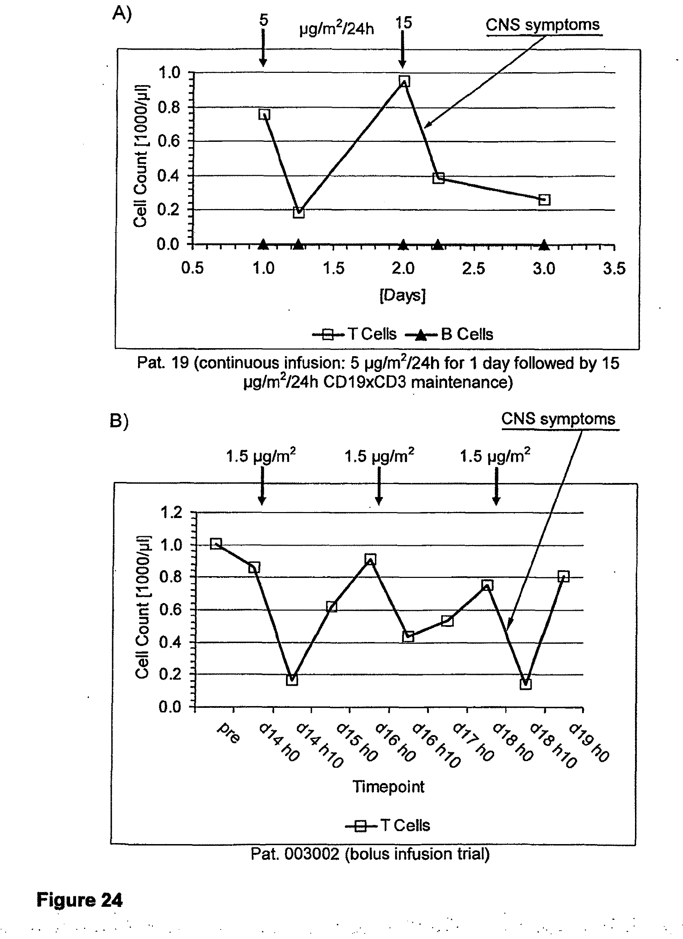

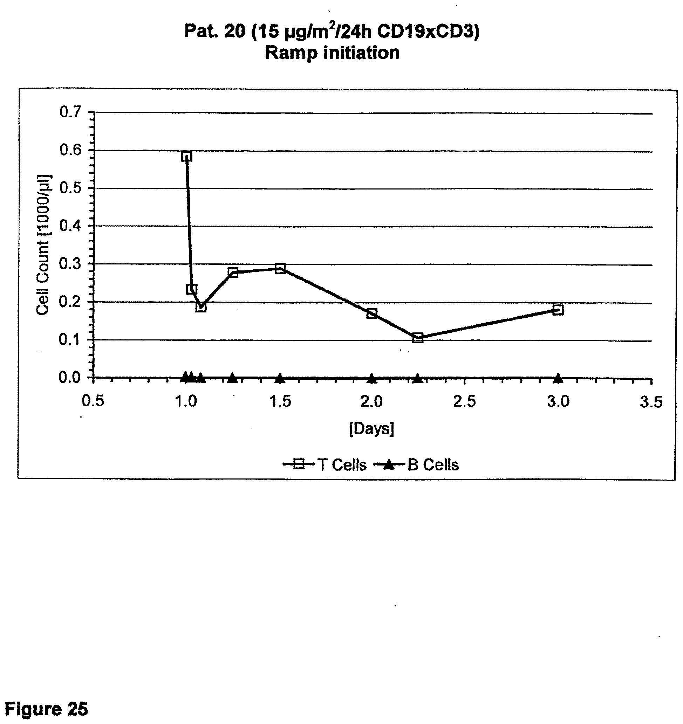

A conventional CD19xCD3 binding molecules is a CD3 binding molecule of the bispecific tandem scFv format (Loffler (2000, Blood, Volume 95, Number 6) or WO 99/54440). It consists of two different binding portions directed at (i) CD19 on the surface of normal and malignant human B cells and (ii) CD3 on human T cells. By crosslinking CD3 on T cells with CD19 on B cells, this construct triggers the redirected lysis of normal and malignant B cells by the cytotoxic activity of T cells. The CD3 epitope recognized by such a conventional CD3 binding molecule is localized on the CD3 epsilon chain, where it only takes the correct conformation if it is embedded within the rest of the epsilon chain and held in the right position by heterodimerization of the epsilon chain with either the CD3 gamma or delta chain. Interaction of this highly context dependent epitope with a conventional CD3 binding molecule (see e.g. Loffler (2000, Blood, Volume 95, Number 6) or WO 99/54440) - even when it occurs in a purely monovalent fashion and without any crosslinking - can induce an allosteric change in the conformation of CD3 leading to the exposure of an otherwise hidden proline-rich region within the cytoplasmic domain of CD3 epsilon. Once exposed, the proline-rich region can recruit the signal transduction molecule Nck2, which is capable of triggering further intracellular signals. Although this is not sufficient for full T cell activation, which definitely requires crosslinking of several CD3 molecules on the T cell surface, e.g. by crosslinking of several anti-CD3 molecules bound to several CD3 molecules on a T cell by several CD19 molecules on the surface of a B cell, pure monovalent interaction of conventional CD3 binding molecules to their context dependent epitope on CD3 epsilon is still not inert for T cells in terms of signaling. Without being bound by theory, monovalent conventional CD3 binding molecules (known in the art) may induce some T cell reactions when infused into humans even in those cases where no circulating target cells are available for CD3 crosslinking. An important T cell reaction to the intravenous infusion of monovalent conventional CD19xCD3 binding molecule into B-NHL patients who have essentially no circulating CD19-positive B cells is the redistribution of T cells after start of treatment. It has been found in a phase I clinical trial that this T cell reaction occurs during the starting phase of intravenous CD19xCD3 binding molecule infusion in all individuals without circulating CD19-positive target B cells essentially independent of the CD19xCD3 binding molecule dose (Fig. 23). However, sudden increases in CD19xCD3 binding molecule exposure have been found to trigger virtually the same redistributional T cell reaction in these patients as the initial exposure of T cells to CD19xCD3 binding molecule at treatment start (Fig. 24 A) and even gradual increases in CD19xCD3 binding molecule exposure still can have redistributional effects on circulating T cells (Fig. 25). Moreover, it has been found that this essentially dose-independent redistributional T cell reaction in the absence of circulating target cells as triggered by conventional CD3 binding molecules like the CD19xCD3 binding molecule (e.g. disclosed in WO 99/54440) in 100% of all treated individuals is a major risk factor for adverse events related to the initiation of treatment.

According to the study protocol, patients with relapsed histologically confirmed indolent B-cell Non-Hodgkin-Lymphoma (B-NHL) including mantle cell lymphoma were recruited in an open-label, multi-center phase I interpatient dose-escalation trial. The study protocol was approved by the independent ethics committees of all participating centers and sent for notification to the responsible regulatory authority. Measurable disease (at least one lesion $\geq$ 1.5 cm) as documented by CT scan was required for inclusion into the study. Patients received conventional CD19xCD3 binding molecule by continuous intravenous infusion with a portable minipump system over four weeks at constant flow rate (i.e. dose level). Patients were hospitalized during the first two weeks of treatment before they were released from the hospital and continued treatment at home. Patients without evidence of disease progression after four weeks were offered to continue treatment for further four weeks. So far six different dose levels were tested without reaching a maximum tolerated dose (MTD): 0.5, 1.5, 5, 15, 30 and 60 $\mu$g/m$^2$/24h. Cohorts consisted of three patients each if no adverse events defined by the study protocol as DLT (dose limiting toxicity) were observed. In case of one DLT among the first three patients the cohort was expanded to six patients, which - in the absence of a second DLT - allowed further dose escalation. Accordingly, dose levels without DLT in cohorts with 3 patients or with one DLT in cohorts with 6 patients were regarded as safe. Study treatment was stopped in all patients who developed a DLT. At 15 and 30 $\mu$g/m/24h different modes of treatment initiation during the first 24h were tested in several additional cohorts: (i) Stepwise increase after 5 $\mu$g/m$^2$/24h for the first 24h to 15 $\mu$g/m$^2$/24h maintenance dose (patient cohort 15-step), (ii) even continuous increase of flow-rate from almost zero to 15 or 30 $\mu$g/m$^2$/24h (patient cohorts 15-ramp and 30-ramp) and (iii) start with the maintenance dose from the very beginning (patient cohorts 15-flat, 30-flat and 60-flat). Patient cohorts at dose levels 0.5, 1.5 and 5 $\mu$g/m$^2$/24h were all started with the maintenance dose from the very beginning (i.e. flat initiation).

Time courses of absolute B- and T-cell counts in peripheral blood were determined by four color FACS analysis as follows:

**Collection of blood samples and routine analysis**

In patient cohorts 15-ramp, 15-flat, 30-ramp, 30-flat and 60-flat blood samples (6 ml) were obtained before and 0.75, 2, 6, 12, 24, 30, 48 hours after start of CD19xCD3 binding molecule (as disclosed in WO 99/54440) infusion as well as on treatment days 8, 15, 17, 22, 24, 29, 36, 43, 50, 57 and 4 weeks after end of conventional CD19xCD3 binding molecule infusion using EDTA-containing Vacutainer™ tubes (Becton Dickinson) which were shipped for analysis at 4°C. In patient cohorts 15-step blood samples (6 ml) were obtained before and 6, 24, 30, 48 hours after start of CD19xCD3 binding molecule infusion as well as on treatment days 8, 15, 22, 29, 36, 43, 50, 57 and 4 weeks after end of CD19xCD3 binding molecule infusion. At dose levels 0.5, 1.5 and 5 $\mu$g/m$^2$/24h blood samples (6 ml) were obtained before and 6, 24, 48 hours after start of CD19xCD3 binding molecule infusion as well as on treatment days 8, 15, 22, 29, 36, 43, 50, 57 and 4 weeks after end of CD19xCD3 binding molecule infusion. In some cases slight variations of these time points occurred for operational reasons. FACS analysis of lymphocyte subpopulations was performed within 24 - 48 h after blood sample collection. Absolute numbers of leukocyte subpopulations in the blood samples were determined through differential blood analysis on a CoulterCounter™ (Coulter).

**Isolation of PBMC from blood samples**

PBMC (peripheral blood mononuclear cells) isolation was performed by an adapted Ficoll™ gradient separation protocol. Blood was transferred at room temperature into 10 ml Leucosep™ tubes (Greiner) pre-loaded with 3 ml Biocoll™ solution (Biochrom).

Centrifugation was carried out in a swing-out rotor for 15 min at 1700xg and 22°C without deceleration. The PBMC above the Biocoll™ layer were isolated, washed once with FACS buffer (PBS / 2% FBS [Foetal Bovine Serum; Biochrom]), centrifuged and resuspended in FACS buffer. Centrifugation during all wash steps was carried out in a swing-out rotor for 4 min at 800xg and 4°C. If necessary, lysis of erythrocytes was performed by incubating the isolated PBMC in 3 ml erythrocyte lysis buffer (8.29 g $NH_4Cl$, 1.00 g $KHCO_3$, 0.037 g EDTA, ad 1.0 l $H_2O_{bidest}$, pH 7.5) for 5 min at room temperature followed by a washing step with FACS buffer.

**Staining of PBMC with fluorescence-labeled antibodies against cell surface molecules**

Monoclonal antibodies were obtained from Invitrogen ([1]Cat. No. MHCD1301, [2]Cat. No. MHCD1401), Dako ([5]Cat. No. C7224) or Becton Dickinson ([3]Cat. No. 555516, [4]Cat. No. 345766) used according to the manufacturers recommendations. $5 \times 10^5$ - $1 \times 10^6$ cells were stained with the following antibody combination: anti-CD13[1] / anti-CD14[2] (FITC) $\times$ anti-CD56[3] (PE) $\times$ anti-CD3[4] (PerCP) $\times$ anti-CD19[5] (APC). Cells were pelleted in V-shaped 96 well multititer plates (Greiner) and the supernatant was removed. Cell pellets were resuspended in a total volume of 100 $\mu$l containing the specific antibodies diluted in FACS buffer. Incubation was carried out in the dark for 30 min at 4°C. Subsequently, samples were washed twice with FACS buffer and cell pellets were resuspended in FACS buffer for flowcytometric analysis.

**Flowcytometric detection of stained lymphocytes by FACS**

Data collection was performed with a 4 color BD FACSCalibur™ (Becton Dickinson). For each measurement $1 \times 10^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding any myeloid cells via CD13/14-staining) as determined by FACS were correlated with the lymphocyte count from the differential blood analysis to calculate absolute cell numbers of T cells (CD3[+], CD56[-], CD13/14[-]) and B cells (CD19[+], CD13/14[-]).

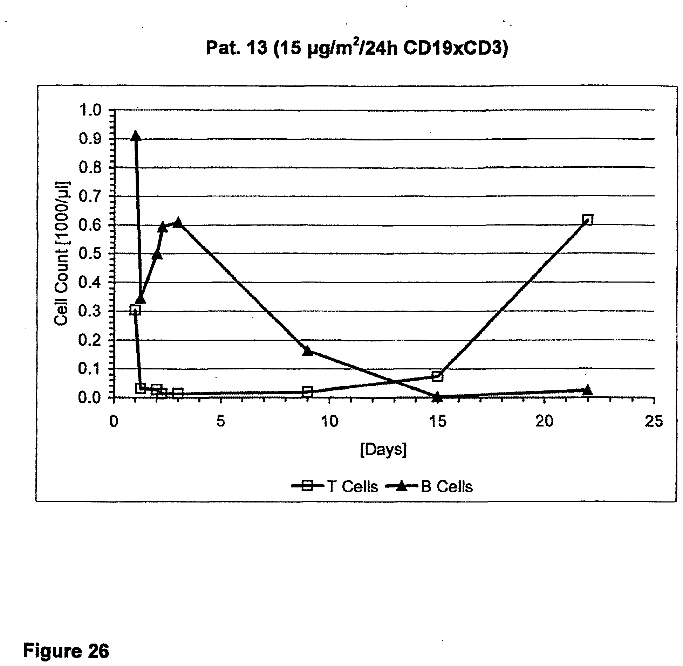

T cell redistribution during the starting phase of conventional CD19xCD3 binding molecule (e.g. disclosed in WO 99/54440) treatment in all those patients who had essentially no circulating CD19-positive B cells at treatment start is shown in (Fig. 23). For comparison, a representative example of T cell redistribution during the starting phase of CD19xCD3 binding molecule treatment in a patient with a significant number of circulating CD19-positive B cells is shown in Fig. 26.

In both cases (i.e. essentially no or many circulating B cells) circulating T cell counts rapidly decrease upon treatment start. However, in the absence of circulating B cells T cells tend to return into the circulating blood very early, while the return of T cells into the circulating blood of those patients who have a significant number of circulating B cells at treatment start is usually delayed until these circulating B cells are depleted. Thus, the T cell redistribution patterns mainly differ in the kinetics of T cell reappearance in the circulating blood.

Assessment of efficacy based on CT scan was carried out by central reference radiology after 4 weeks of treatment and in patients receiving additional 4 weeks also after 8 weeks of treatment plus in all cases four weeks after end of treatment. Disappearance and/or normalization in size of all known lesions (including an enlarged spleen) plus clearance of bone marrow from lymphoma cells in cases of bone marrow infiltration was counted as complete response (CR). Reduction by at least 50% from baseline of the sum of products of the two biggest diameters (SPD) of each predefined target lesion was defined as partial response (PR); a reduction by at least 25% was regarded a minimal response (MR). Progressive disease (PD) was defined as $\geq$ 50% increase of SPD from baseline. SPD deviations from baseline between +50% and -25% were regarded as stable disease (SD).

Patient demographics, doses received and clinical outcome in 34 patients are summarized in Table 4. Clinical anti-tumor activity of the CD19xCD3 binding molecule was clearly dose dependent: Consistent depletion of circulating CD19-positive B (lymphoma) cell from peripheral blood was observed from 5 $\mu$g/m²/24h onwards. At 15 $\mu$g/m²/24h and 30 $\mu$g/m²/24h first objective clinical responses (PRs and CRs) were recorded as well as cases of partial and complete elimination of B lymphoma cells from infiltrated bone marrow. Finally, at 60 $\mu$g/m²/24h the response rate increased to 100% (PRs and CRs) and bone marrow clearance from B lymphoma cells was complete in all evaluable cases.

The CD19xCD3 binding molecule was well tolerated by the majority of patients. Most frequent adverse events of grades 1-4 in 34 patients, regardless of causality are summarized in Table 5. CD19xCD3 binding molecule-related adverse events usually were transient and fully reversible. In particular, there were 2 patients (patients # 19 and # 24 in Table 4) essentially without circulating CD19-positive B cells whose treatment was stopped early because of CNS adverse events (lead symptoms: confusion and disorientation) related to repeated T cell redistribution during the starting phase of CD19xCD3 binding molecule infusion.

One of these patients (#19) was in cohort 15-step. He received 5 μg/m²/24h CD19xCD3 binding molecule for the first 24h followed by sudden increase to 15 μg/m²/24h maintenance dose. The corresponding T cell redistribution pattern shows that circulating T cell counts rapidly decreased upon start of infusion at 5 μg/m²/24h followed by early reappearance of T cells in the circulating blood essentially without circulating CD19-positive B cells. As a consequence, the peripheral T cell counts had fully recovered when the CD19xCD3 binding molecule dose was increased after 24h from 5 to 15 μg/m²/24h. Therefore the dose step could trigger a second episode of T cell redistribution as shown in Fig. 24 A. This repeated T cell redistribution was related with CNS side effects (lead symptoms: confusion and disorientation) in this patient, which led to the stop of infusion. The relationship between repeated T cell redistribution and such CNS adverse events was also observed in previous phase I clinical trials in B-NHL patients who received CD19xCD3 binding molecule (e.g. disclosed in WO 99/54440) as repeated bolus infusion for 2 to 4 hours each usually followed by 2 days of treatment free interval (Fig. 24 B). Every single bolus infusion triggered one episode of T cell redistribution consisting of a fast decrease in circulating T cell counts and T cell recovery prior to the next bolus infusion. In total, CNS adverse events related to repeated T cell redistribution were observed in 5 out of 21 patients. Fig. 24 B shows the representative example of one patient from the bolus infusion trials, who developed CNS symptoms after the third episode of T cell redistribution. Typically, patients with CNS adverse events in the bolus infusion trials also had low circulating B cell counts.

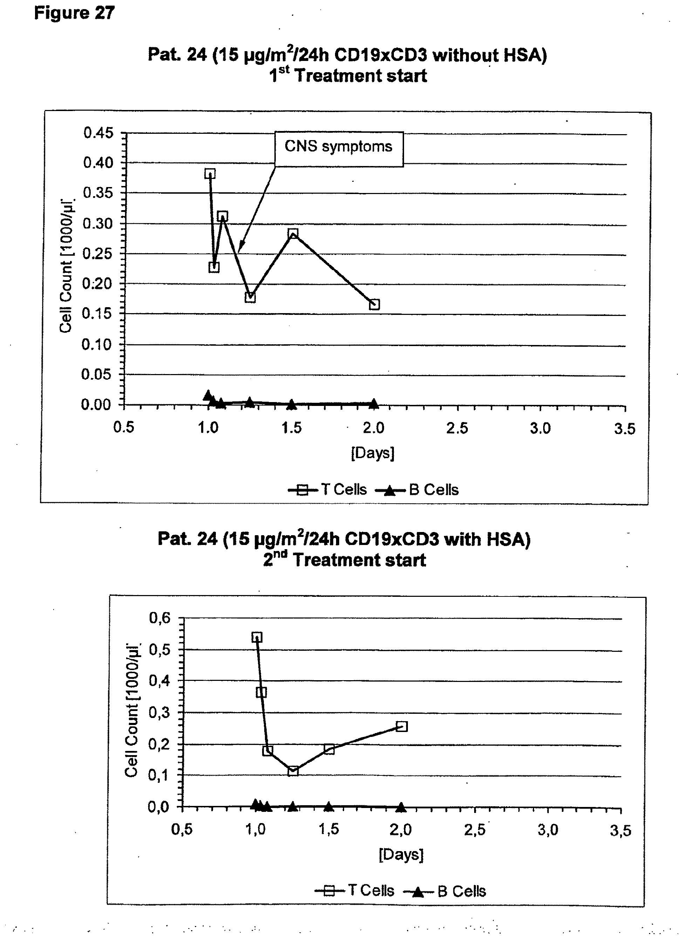

The second patient (#24) from the continuous infusion trial, whose treatment was stopped early because of CNS adverse events (lead symptoms: confusion and disorientation) related to repeated T cell redistribution during the starting phase of CD19xCD3 binding molecule infusion, was in cohort 15-flat. By mistake, this patient received an CD19xCD3 binding molecule infusion without additional HSA as required for stabilization of the drug. The resulting uneven drug flow triggered repeated episodes of T cell redistribution instead of only one (Fig. 27 A) with the consequence that the infusion had to be stopped because of developing CNS symptoms. Yet, when the same patient was restarted correctly with CD19xCD3 binding molecule solution containing additional HSA for drug stabilization (e.g. disclosed in WO 99/54440), no repeated T cell redistribution was observed and the patient did not again develop any CNS symptoms (Fig. 27 B). Because this patient also had essentially no circulating B cells, the circulating T cells could react with fast redistribution kinetics even to subtle changes in drug exposure as observed. The CNS adverse events related to T cell redistribution in patients who have essentially no circulating target cells can be explained by a transient increase of T cell adhesiveness to the endothelial cells followed by massive simultaneous adhesion of circulating T cells to the blood vessel walls with a consecutive drop of T cell numbers in the circulating blood as observed. The massive simultaneous attachment of T cells to the blood vessel walls can cause an increase in endothelial permeability and endothelial cell activation. The consequences of increased endothelial permeability are fluid shifts from the intravascular compartment into interstitial tissue compartments including the CNS interstitium. Endothelial cell activation by attached T cells can have procoagulatory effects (Monaco et al. J Leukoc Biol 71 (2002) 659-668) with possible disturbances in blood flow (including cerebral blood flow) particularly with regard to capillary microcirculation. Thus, CNS adverse events related to T cell redistribution in patients essentially without circulating target cells can be the consequence of capillary leak and/or disturbances in capillary microcirculation through adherence of T cells to endothelial cells. The endothelial stress caused by one episode of T cell redistribution is tolerated by the majority of patients, while the enhanced endothelial stress caused by repeated T cell redistribution frequently causes CNS adverse events. More than one episode of T cell redistribution may be less risky only in patients who have low baseline counts of circulating T cells. However, also the limited endothelial stress caused by one episode of T cell redistribution can cause CNS adverse events in rare cases of increased susceptibility for such events as observed in 1 out of 21 patients in the bolus infusion trials with the CD19xCD3 binding molecule.

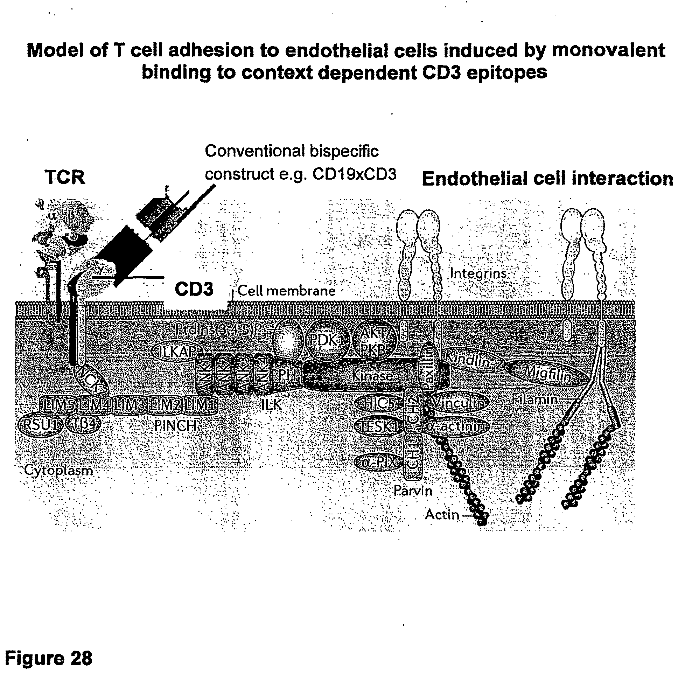

Without being bound by theory, the transient increase of T cell adhesiveness to the endothelial cells in patients who have essentially no circulating target cells can be explained as T cell reaction to the monovalent interaction of a conventional CD3 binding molecule, like the CD19xCD3 binding molecule (e.g. WO 99/54440), to its context dependent epitope on CD3 epsilon resulting in an allosteric change in the conformation of CD3 followed by the recruitment of Nck2 to the cytoplasmic domain of CD3 epsilon as described above. As Nck2 is directly linked to integrins via PINCH and ILK (Fig 28), recruitment of Nck2 to the cytoplasmic domain of CD3 epsilon following an allosteric change in the conformation of CD3 through binding of a conventional CD3 binding molecule, like the CD19xCD3 binding molecule, to its context dependent epitope on CD3 epsilon, can increase the adhesiveness of T cells to endothelial cells by transiently switching integrins on the T cell surface into their more adhesive isoform via inside-out-signalling.

Table 4. Patient demographics and clinical outcome

| Cohort | Patient | Age/Sex | Disease (Ann Arbor Classification) | Dose Level [mg/m²/Day] | Clearance of Bone Marrow | Best Response* (CR Duration in Months or Weeks) |
|---|---|---|---|---|---|---|
| 1 | 1 | 71/m | IC, Binet C | 0.0005 | None | SD |
| | 2 | 67/f | MCL, Stage IV/A/E | 0.0005 | n.d. | PD |
| | 3 | 67/m | CLL, Stage IV/B/E | 0.0005 | n.d. | MR |
| 2 | 4 | 69/m | MCL, Stage IV/B | 0.0015 | n.i. | SD |
| | 5 | 49/m | MCL, Stage IV/A/S | 0.0015 | n.d. | SD |
| | 6 | 71/m | MCL, Stage IV/B/E | 0.0015 | n.i. | PD |
| | 7 | 77/m | MCL, Stage IV /B/E/S | 0.0015 | n.i. | SD |
| | 8 | 65/m | CLL, Stage IV/B/E/S | 0.0015 | n.d. | PD |
| | 9 | 75/m | FL, Stage II/B | 0.0015 | n.i. | SD |
| 3 | 10 | 58/m | MCL, Stage III/B/S | 0.005 | n.i. | PD |
| | 11 | 68/f | FL, Stage IV/B | 0.005 | n.d. | SD |
| | 12 | 65/m | MCL, Stage III/A/E | 0.005 | n.i. | SD |
| 4ᵃ | 13 | 60/m | SLL, Stage IV/B/S | 0.015 | Complete | **PR** |
| | 14 | 73/m | MCL, Stage II/A/E | 0.015 | n.i. | SD |
| | 15 | 44/m | FL, Stage IV/B/E/S | 0.015 | Partial | **PR** |
| | 16 | 61/m | FL, Stage IV/A/S | 0.015 | Complete | **CR** (7mo) |
| | 17 | 67/m | MZL, Stage IV/B/S | 0.015 | n.i. | n.e. |
| | 18 | 64/m | FL, Stage IV/A/E | 0.015 | n.i. | PD |
| | 19 | 75/m | MCL, Stage III/A | 0.015 | n.i. | n.e. |
| | 20 | 65/f | FL; Stage III/A | 0.015 | n.i. | SD |
| | 21 | 60/m | MCL, Stage IV/A/E | 0.015 | None | SD |
| | 22 | 67/f | FL, Stage IV/B | 0.015 | Complete | MR |
| | 23 | 67/m | DLBCL, Stage III/B | 0.015 | n.i. | n.e. |
| | 24 | 65/f | FL, Stage III/A | 0.015 | n.d. | SD |
| | 25 | 74/f | WD, Stage IV/B | 0.015 | Partial | SD |
| 5 | 26 | 67/m | MCL, Stage IV/A | 0.03 | Complete | SD |
| | 27 | 48/m | FL, Stage III/A | 0.03 | n.i. | PD |
| | 28 | 58/m | MCL, Stage III/A | 0.03 | n.i. | **CR** (10mo+) |
| | 29 | 45/f | MCL, Stage IV/B | 0.03 | Partial | PD |
| | 30 | 59/m | MZL, Stage III/A | 0.03 | n.i. | n.e. |
| | 31 | 43/m | FL, Stage III/A | 0.03 | n.i. | MR |
| 6 | 32 | 72/m | MCL, Stage IV/A | 0.06 | Complete | **PR** |
| | 33 | 55/m | MCL, Stage IV/B | 0.06 | Complete | **CR** (4mo+) |

(continued)

| Cohort | Patient | Age/Sex | Disease (Ann Arbor Classification) | Dose Level [mg/m$^2$/Day] | Clearance of Bone Marrow | Best Response* (CR Duration in Months or Weeks) |
|---|---|---|---|---|---|---|
| | 34 | 52/m | FL, Stage IV/A | 0.06 | n.i. | **CR**[b] (1w+) |

*Centrally confirmed complete (CR) and partial (PR) responses by Cheson criteria in bold; MR, minimal response ($\geq$25 to <50%); SD, stable disease; PD, progressive disease; duration from first documentation of response in parentheses; + denotes an ongoing response

[a]Cohort 4 was expanded to study three different schedules of treatment initiation

[b]PR after 8 weeks of treatment that turned into a CR after an additional treatment cycle of 4 weeks at the same dose following 7 weeks of treatment free interval n.e.: not evaluable, because of treatment period <7 d n.d.: not determined (infiltrated, but no second biopsy performed at end of treatment) n.i.: not infiltrated at start of treatment

Table 5. Incidence of adverse events observed during treatment

| Adverse events regardless of relationship, occuring in $\geq$ 3 patients (N=34) | Grade 1-4 N (%) | Grade 3-4 N (%) |
|---|---|---|
| Pyrexia | 22 (64.7) | 2 (5.9) |
| Leukopenia | 21 (61.8) | 11 (32.4) |
| Lymphopenia | 21 (61.8) | 21 (61.8 |
| Coagulopathy (increase in D-dimers) | 16 (47.1) | 6 (17.6) |
| Enzyme abnormality (AP, LDH, CRP) | 16 (47.1 | 10 (29.4) |
| Hepatic function abnormality (ALT, AST, GGT) | 16 (47.1) | 1 (2.9) |
| Anaemia | 13 (38.2) | 5 (14.7) |
| Chills | 13 (38.2) | 0 (0.0) |
| Headache | 12 (35.3) | 1 (2.9) |
| Hypokalaemia | 12 (35.3) | 2 (5.9) |
| Thrombocytopenia | 12 (35.3) | 6 (17.6) |
| Weight increased | 12 (35.3) | 0 (0.0) |
| Hyperglycaemia | 11 (32.4) | 2 (5.9) |
| Neutropenia | 11 32.4 | 8 (23.5) |
| Haematuria | 10 29.4 | 0 (0.0) |
| Oedema peripheral | 10 (29.4) | 2 (5.9) |
| Anorexia | 9 (26.5) | 1 (2.9) |
| Diarrhoea | 9 (26.5) | 0 (0.0) |
| Weight decreased | 9 (26.5 | 0 (0.0) |
| Fatigue | 8 (23.5) | 1 (2.9 |
| Proteinuria | 8 23.5 | 0 (0.0) |
| Hypocalcaemia | 7 (20.6) | 2 (5.9) |
| Pancreatic enzyme abnormality | 7 (20.6 | 0 (0.0) |
| Cough | 6 (17.6) | 0 (0.0) |
| Dyspnoea | 6 (17.6) | 0 (0.0) |
| Back pain | 5 (14.7) | 0 (0.0) |

(continued)

| Adverse events regardless of relationship, occuring in ≥ 3 patients (N=34) | Grade 1-4 N (%) | Grade 3-4 N (%) |
|---|---|---|
| Catheter site pain | 5 (14.7) | 0 (0.0) |
| Hyperbilirubinaemia | 5 (14.7) | 2 (5.9) |
| Hypoalbuminaemia | 5 (14.7) | 0 (0.0) |
| Hypogammaglobulinaemia | 5 (14.7) | 1 (2.9) |
| Hypoproteinaemia | 5 (14.7) | 0 (0.0) |
| Pleural effusion | 5 (14.7) | 1 (2.9) |
| Vomiting | 5 (14.7 | 0 (0.0) |
| Asthenia | 4(11.8) | 1 (2.9) |
| Confusional state | 4 (11.8) | 0 (0.0) |
| Constipation | 4 (11.8) | 0 (0.0) |
| Dizziness | 4 (11.8 | 0 (0.0) |
| Hypertension | 4 (11.8) | 0 (0.0) |
| Hyponatraemia | 4 (11.8) | 2 (5.9) |
| Mucosal dryness | 4 (11.8) | 0 (0.0) |
| Muscle spasms | 4 (11.8) | 0 (0.0) |
| Nausea | 4(11.8) | 0 (0.0) |
| Night sweats | 4 (11.8) | 0 (0.0) |
| Abdominal pain | 3 (8.8) | 1 (2.9) |
| Ascites | 3 (8.8) | 0(0.0) |
| Hypercoagulation | 3 (8.8) | 0 (0.0) |
| Hyperhidrosis | 3 (8.8) | 0 (0.0) |
| Hypoglobulinaemia | 3 (8.8) | 0 (0.0) |
| Insomnia | 3 (8.8) | 0 (0.0) |
| Liver disorder | 3 (8.8) | 1 (2.9) |
| Nasopharyngitis | 3 (8.8) | 0 (0.0) |
| Pruritus | 3 (8.8) | 0 (0.0) |

Abbreviations used are: AE, adverse event; AP, alkaline phosphatase; LDH, lactate dehydrogenase; CRP, C-reactive protein; ALT, alanine transaminase; AST, aspartate transaminase; GGT, gamma-glutamyl transferase; AE data from the additional treatment cycle of patient 34 not yet included.

As explained above, conventional CD3 binding molecules (e.g. disclosed in WO 99/54440) capable of binding to a context-dependent epitope, though functional, lead to the undesired effect of T cell redistribution in patients causing CNS adverse events. In contrast, binding molecules of the present invention, by binding to the context-independent N-terminal 1-27 amino acids of the CD3 epsilon chain, do not lead to such T cell redistribution effects. As a consequence, the CD3 binding molecules of the invention are associated with a better safety profile compared to conventional CD3 binding molecules.

**21. Bispecific CD3 binding molecules of the invention inducing T cell mediated target cell lysis by recognizing a surface target antigen deplete target antigen positive cells in vivo**

**A bispecific CD3 binding molecule of the invention recognizing CD33 as target antigen depletes CD33-positive circulating monocytes from the peripheral blood of cynomolgus monkeys**

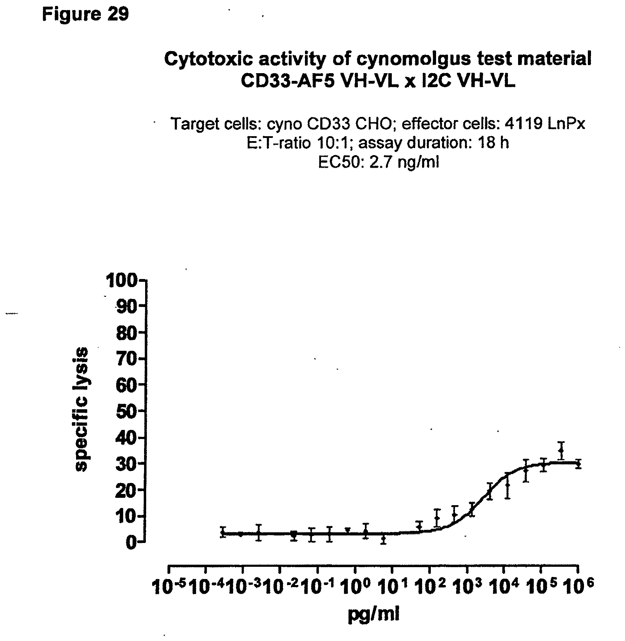

CD33-AF5 VH-VL × I2C VH-VL (amino acid sequence: SEQ ID NO.415) was produced by expression in CHO cells using the coding nucleotide sequence SEQ ID NO. 416. The coding sequences of (i) an N-terminal immunoglobulin heavy chain leader comprising a start codon embedded within a Kozak consensus sequence and (ii) a C-terminal HiS$_6$-tag followed by a stop codon were both attached in frame to the nucleotide sequence SEQ ID NO 416 prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the CD3 binding molecule CD33-AF5 VH-VL × I2C VH-VL into the culture supernatant and gene amplification with methotrexat for increasing expression levels were carried out as described (Mack et al. Proc. Natl. Acad. Sci. USA 92 (1995) 7021-7025). Test material for treatment of cynomolgus monkeys was produced in a 20-liter fermenter. Protein purification from the harvest of 3 production runs was based on IMAC affinity chromatography targeting the C-terminal His6-tag of CD33-AF5 VH-VL × I2C VH-VL followed by preparative size exclusion chromatography (SEC). The total yield of final endotoxin-free test material was 60 mg. The analytical SEC-profile of CD33-AF5 VH-VL × I2C VH-VL for use in cynomolgus monkeys revealed that the test material almost exclusively consisted of monomer. The potency of the test material was measured in a cytotoxicity assay as described in example 23.5 using CHO cells transfected with cynomolgus CD33 as target cells and the macaque T cell line 4119LnPx as source of effector cells (FIG. 29). The concentration of CD33-AF5 VH-VL × I2C VH-VL required for half-maximal target cell lysis by the effector T cells (EC50) was determined to be 2.7 ng/ml.

Young (approx. 3 years old) adult cynomolgus monkeys (Macaca fascicularis) were treated by continuous intravenous infusion of CD3 binding molecule CD33-AF5 VH-VL × I2C VH-VL at different flow-rates (i.e. dose levels) to study depletion of circulating CD33-positive monocytes from the peripheral blood. This situation is equivalent to the treatment with the conventional CD3 binding molecule CD19xCD3 (specific for CD19 on B cells and CD3 on T cells) of those B-NHL patients, who have circulating CD19-positive target B cells (see e.g. WO99/54440). Depletion of circulating CD19-positive target B cells from the peripheral blood had turned out as a valid surrogate for the general clinical efficacy of the conventional CD3 binding molecule (CD19xCD3 as provided in WO99/54440) in patients with CD19-positive B-cell malignomas like B-NHL. Likewise, depletion of circulating CD33-positive monocytes from the peripheral blood is regarded as a valid surrogate of the general clinical efficacy of CD33-directed bispecific CD3 binding molecules of the invention like CD33-AF5 VH-VL × I2C VH-VL in patients with CD33-positive myeloid malignomas like AML (acute myeloid leukemia).

Continuous infusion was carried out according to the Swivel method as follows: The monkeys are catheterized via the vena femoralis into the vena cava caudalis using a vein catheter. The catheter is tunneled subcutaneously to the dorsal shoulder region and exteriorized at the caudal scapula. Then a tube is passed through a jacket and a protection spring. The jacket is fastened around the animal and the catheter, via the tube, is connected to an infusion pump.

Administration solution (1.25 M lysine, 0.1% tween 80, pH 7) without test material was infused continuously at 48 ml/24h for 7 days prior to treatment start to allow acclimatization of the animals to the infusion conditions. Treatment was started by adding CD33-AF5 VH-VL × I2C VH-VL test material to the administration solution at the amount required for each individual dose level to be tested (i.e. flow rate of CD33-AF5 VH-VL × I2C VH-VL). The infusion reservoir was changed every day throughout the whole acclimatization and treatment phase. Planned treatment duration was 7 days except for the 120ug/m$^2$/24h dose level, where animals received 14 days of treatment.

Time courses of absolute counts in circulating T cells and CD33-positive monocytes were determined by 4- or 3-colour FACS analysis, respectively:

**Collection of blood samples and routine analysis**

Blood samples (1 ml) were obtained before and 0.75, 2, 6, 12, 24, 30, 48, 72 hours after start of continuous infusion with MCSP-G4 VH-VL × I2C VH-VL as well as after 7 and 14 days (and after 9 days at the 120 $\mu$g/m$^2$/24h dose level) of treatment using EDTA-containing Vacutainer™ tubes (Becton Dickinson) which were shipped for analysis at 4°C. In some cases slight variations of these time points occurred for operational reasons. FACS analysis of lymphocyte subpopulations was performed within 24 - 48 h after blood sample collection. Absolute numbers of leukocyte subpopulations in the blood samples were determined through differential blood analysis in a routine veterinary lab.

**Isolation of PBMC from blood samples**

PBMC (peripheral blood mononuclear cells) isolation was performed by an adapted Ficoll™ gradient separation protocol. Blood was transferred at room temperature into 5 ml Falcon™ tubes pre-loaded with 1 ml Biocoll™ solution (Biochrom). Centrifugation was carried out in a swing-out rotor for 15 min at 1700xg and 22°C without deceleration. The PBMC above the Biocoll™ layer were isolated, washed once with FACS buffer (PBS / 2% FBS [Foetal Bovine Serum; Biochrom]), centrifuged and resuspended in FACS buffer. Centrifugation during all wash steps was carried out in a swing-out rotor for 4 min at 800xg and 4°C. If necessary, lysis of erythrocytes was performed by incubating the isolated PBMC in 3 ml erythrocyte lysis buffer (8.29 g $NH_4Cl$, 1.00 g $KHCO_3$, 0.037 g EDTA, ad 1.0 l $H_2O_{bidest}$, pH 7.5) for 5 min at room temperature followed by a washing step with FACS buffer.

**Staining of PBMC with fluorescence-labeled antibodies against cell surface molecules**

Monoclonal antibodies reactive with cynomolgus antigens were obtained from Becton Dickinson ([1]Cat. No. 345784, [2]Cat. No. 556647, [3]Cat. No. 552851, [6]Cat. No. 557710), Beckman Coulter ([4]Cat. No. IM2470) and Miltenyi ([5]Cat. No. 130-091-732) and used according to the manufacturers' recommendations. $5 \times 10^5$ - $1 \times 10^6$ cells were stained with the following antibody combinations: anti-CD14[1] (FITC) × anti-CD56[2] (PE) × anti-CD3[3] (PerCP) × anti-CD19[4] (APC) and anti-CD14[1] (FITC) × anti-CD33[5] (PE) × anti-CD16[6] (Alexa Fluor 647™). Cells were pelleted in V-shaped 96 well multititer plates (Greiner) and the supernatant was removed. Cell pellets were resuspended in a total volume of 100 μl containing the specific antibodies diluted in FACS buffer. Incubation was carried out in the dark for 30 min at 4°C. Subsequently, samples were washed twice with FACS buffer and cell pellets were resuspended in FACS buffer for flowcytometric analysis.

**Flowcytometric detection of stained lymphocytes by FACS**

Data collection was performed with a 4 color BD FACSCalibur™ (Becton Dickinson). For each measurement $1 \times 10^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding myeloid cells via CD14-staining) as determined by FACS were correlated with the lymphocyte count from the differential blood analysis to calculate absolute cell numbers of T cells (CD3[+], CD56[-], CD14[-]). Absolute numbers of CD33-positive monocytes were calculated by multiplying the monocyte counts from the differential blood analysis with the corresponding ratios of CD33-positive monocytes (CD33[+], CD14[+]) to all monocytes (CD14[+]) as determined by FACS.

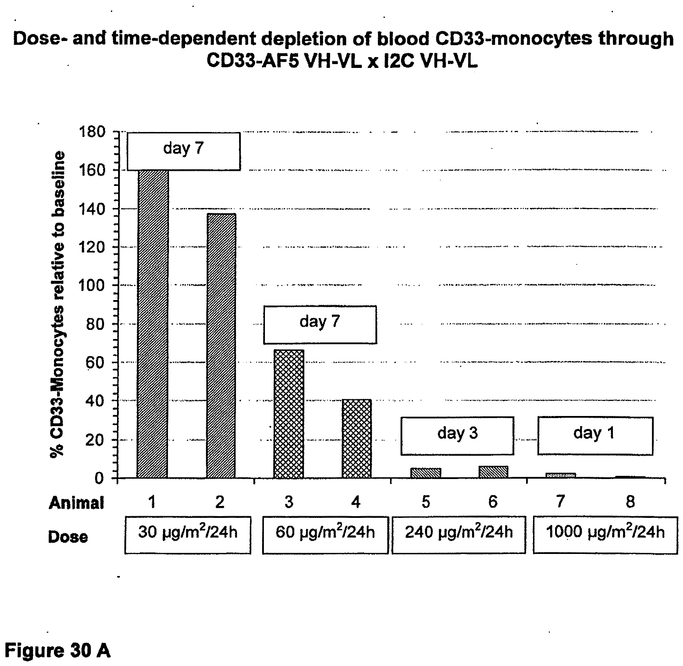

The percentage compared to baseline (i.e. 100%) of absolute circulating CD33-positive monocyte counts at the end of treatment with CD33-AF5 VH-VL × I2C VH-VL in 4 cohorts of 2 cynomolgus monkeys with inter-cohort dose escalation from 30 over 60 and 240 to 1000 μg/m$^2$/24h are shown in FIG 30 A.

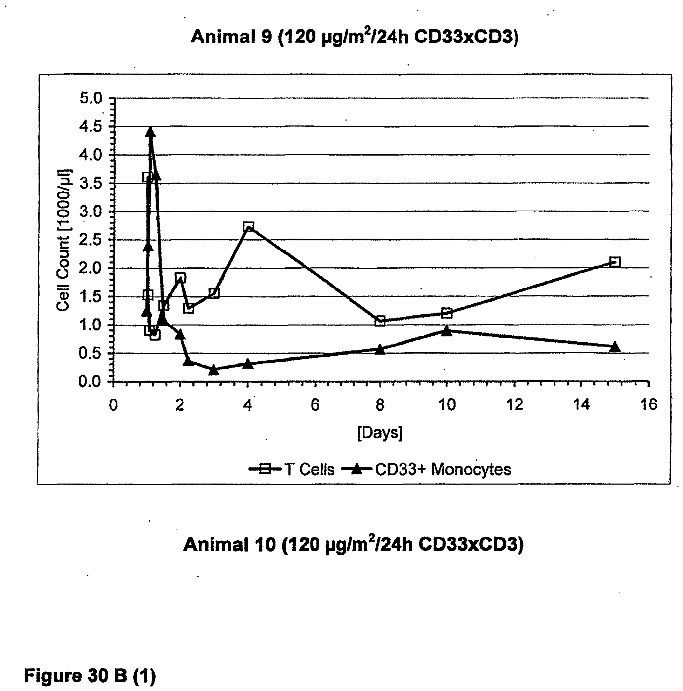

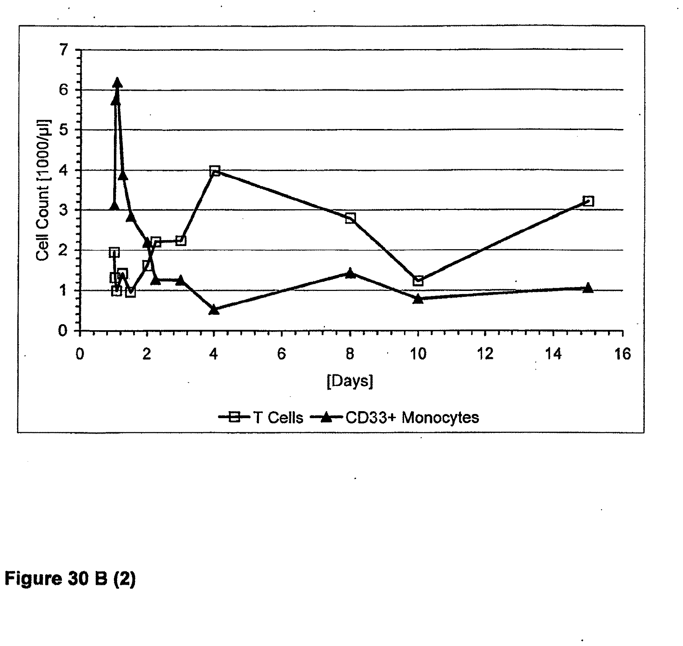

As shown in FIG 30 A, continuous intravenous infusion of CD33-AF5 VH-VL × I2C VH-VL induces depletion of circulating CD33-positive monocytes in a dose-dependent manner. While there was still no detectable depletion of circulating CD33-positive monocytes at 30 μg/m$^2$/24h, a first trend towards a reduction of CD33-positive monocyte counts became visible at 60 μg/m$^2$/24h after 7 days of treatment. At 240 μg/m$^2$/24h circulating CD33-positive monocytes were almost completely depleted from the peripheral blood after 3 days of treatment. This was reached even faster at 1000 μg/m$^2$/24h, where depletion of the circulating CD33-positive monocytes from the peripheral blood was completed already after 1 day of treatment. This finding was confirmed by the results shown in Figure 30 B demonstrating depletion of circulating CD33-positive monocytes by two thirds and 50% compared to the respective baseline in two cynomolgus monkeys treated by continuous infusion with CD33-AF5 VH-VL × I2C VH-VL at 120 μg/m$^2$/24h for 14 days.

This outcome is a clear signal clinical efficacy of the CD3 binding molecules of the invention in general and of bispecific CD33-directed CD3 binding molecules of the invention for the treatment of CD33-positive malignomas like AML in particularly. Moreover, the T cell redistribution during the starting phase of treatment with CD33-AF5 VH-VL × I2C VH-VL in the presence of circulating target cells (i.e. CD33-positive monocytes) seems to be less pronounced than T cell redistribution during the starting phase of treatment with conventional CD19xCD3 constructs, as described in WO99/54440 in B-NHL patients with a significant number of circulating target cells (i.e. CD19-positive B cells) as shown in Fig 26. While T cells disappear completely from the circulation upon start of CD19xCD3 infusion and do not reappear until the circulating CD19-positive target B cells are depleted from the peripheral blood (FIG 26), initial disappearance of circulating T cells is incomplete upon infusion start with CD33-AF5 VH-VL × I2C VH-VL and T cell counts recover still during the presence of circulating CD33-positive target cells (FIG 30 B). This confirms that CD3 binding molecules of the invention (directed against and generated against an epitope of human and non-chimpanzee primates CD3ε (epsilon) chain and being a part or fragment or the full length of the amino acid sequence as provided in SEQ ID Nos.

2, 4, 6, or 8) by recognizing a context-independent CD3 epitope show a more favorable T cell redistribution profile than conventional CD3 binding molecules recognizing a context-dependent CD3 epitope, like the binding molecules provided in WO99/54440.

## 22. CD3 binding molecules of the invention directed at essentially context independent CD3 epitopes by inducing less redistribution of circulating T cells in the absence of circulating target cells reduce the risk of adverse events related to the initiation of treatment

### Reduced T cell redistribution in cynomolgus monkeys following initiation of treatment with a representative cross-species specific CD3 binding molecule of the invention

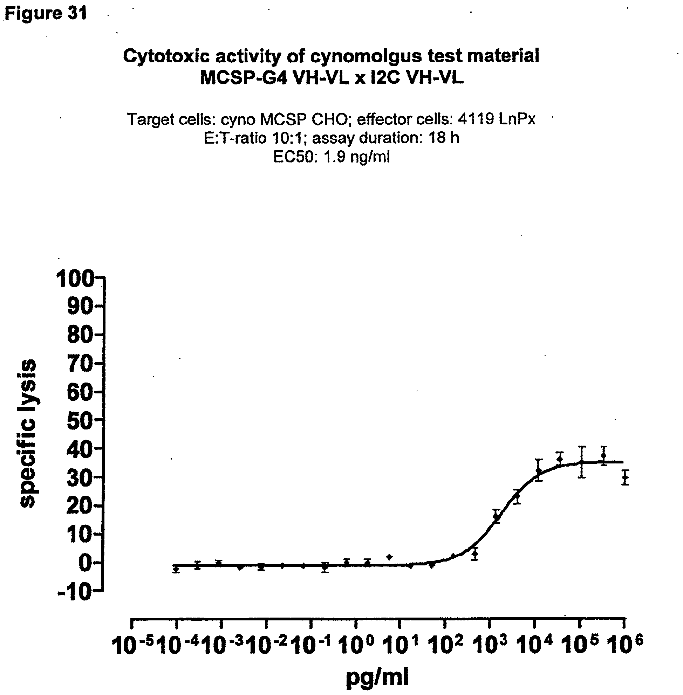

MCSP-G4 VH-VL × I2C VH-VL (amino acid sequence: SEQ ID NO. 313) was produced by expression in CHO cells using the coding nucleotide sequence SEQ ID NO. 314. The coding sequences of (i) an N-terminal immunoglobulin heavy chain leader comprising a start codon embedded within a Kozak consensus sequence and (ii) a C-terminal His6-tag followed by a stop codon were both attached in frame to the . nucleotide sequence SEQ ID NO. 314 prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the CD3 binding molecule MCSP-G4 VH-VL × I2C VH-VL into the culture supernatant and gene amplification with methotrexat for increasing expression levels were carried out as described (Mack et al. Proc. Natl. Acad. Sci. USA 92 (1995) 7021-7025). Test material for treatment of cynomolgus monkeys was produced in a 200-liter fermenter. Protein purification from the harvest was based on IMAC affinity chromatography targeting the C-terminal His6-tag of MCSP-G4 VH-VL × I2C VH-VL followed by preparative size exclusion chromatography (SEC). The total yield of final endotoxin-free test material was 40 mg. The test material consisted of 70 % monomer, 30% dimer and a small contamination of higher multimer. The potency of the test material was measured in a cytotoxicity assay as described in example 18 using CHO cells transfected with cynomolgus MCSP as target cells and the macaque T cell line 4119LnPx as source of effector cells (FIG 31). The concentration of MCSP-G4 VH-VL × I2C VH-VL required for half-maximal target cell lysis by the effector T cells (EC50) was determined to be 1.9 ng/ml.

Young (approx. 3 years old) adult cynomolgus monkeys (Macaca fascicularis) were treated by continuous intravenous infusion of CD3 binding molecule MCSP-G4 VH-VL × I2C VH-VL at different flow-rates (i.e. dose levels) to study redistribution of circulating T cells following initiation of treatment in the absence of circulating target cells. Although the CD3 binding molecule MCSP-G4 VH-VL × I2C VH-VL can recognize both cynomolgus MCSP and cynomolgus CD3, there are no circulating blood cells expressing MCSP. Therefore, the only interaction possible in the circulating blood is binding of the CD3-specific arm of MCSP-G4 VH-VL × I2C VH-VL to CD3 on T cells. This situation is equivalent to the treatment with the conventional CD3 binding molecule (CD19xCD3 binding molecule specific for CD19 on B cells and CD3 on T cells) of those B-NHL patients, who have no circulating CD19-positive target B cells as described in example 20.

Continuous infusion was carried out according to the Swivel method as follows: The monkeys are catheterized via the vena femoralis into the vena cava caudalis using a vein catheter. The catheter is tunneled subcutaneously to the dorsal shoulder region and exteriorized at the caudal scapula. Then a tube is passed through a jacket and a protection spring. The jacket is fastened around the animal and the catheter, via the tube, is connected to an infusion pump.

Administration solution (1.25 M lysine, 0.1% tween 80, pH 7) without test material was infused continuously at 48 ml/24h for 7 days prior to treatment start to allow acclimatization of the animals to the infusion conditions. Treatment was started by adding MCSP-G4 VH-VL × I2C VH-VL test material to the administration solution at the amount required for each individual dose level to be tested (i.e. flow rate of MCSP-G4 VH-VL × I2C VH-VL). The infusion reservoir was changed every day throughout the whole acclimatization and treatment phase. Treatment duration was 7 days.

Time courses of absolute T-cell counts in peripheral blood were determined by four color FACS analysis as follows:

### Collection of blood samples and routine analysis

Blood samples (1 ml) were obtained before and 0.75, 2, 6, 12, 24, 30, 48, 72 hours after start of continuous infusion with MCSP-G4 VH-VL × I2C VH-VL as well as after 7 days of treatment using EDTA-containing Vacutainer™ tubes (Becton Dickinson) which were shipped for analysis at 4°C. In some cases slight variations of these time points occurred for operational reasons. FACS analysis of lymphocyte subpopulations was performed within 24 - 48 h after blood sample collection. Absolute numbers of leukocyte subpopulations in the blood samples were determined through differential blood analysis in a routine veterinary lab.

**Isolation of PBMC from blood samples**

PBMC (peripheral blood mononuclear cells) isolation was performed by an adapted Ficoll™ gradient separation protocol. Blood was transferred at room temperature into 5 ml Falcon™ tubes pre-loaded with 1 ml Biocoll™ solution (Biochrom). Centrifugation was carried out in a swing-out rotor for 15 min at 1700xg and 22°C without deceleration. The PBMC above the Biocoll™ layer were isolated, washed once with FACS buffer (PBS / 2% FBS [Foetal Bovine Serum; Biochrom]), centrifuged and resuspended in FACS buffer. Centrifugation during all wash steps was carried out in a swing-out rotor for 4 min at 800xg and 4°C. If necessary, lysis of erythrocytes was performed by incubating the isolated PBMC in 3 ml erythrocyte lysis buffer (8.29 g $NH_4Cl$, 1.00 g $KHCO_3$, 0.037 g EDTA, ad 1.0 l $H_2O_{bidest}$, pH 7.5) for 5 min at room temperature followed by a washing step with FACS buffer.

**Staining of PBMC with fluorescence-labeled antibodies against cell surface molecules**

Monoclonal antibodies reactive with cynomolgus antigens were obtained from Becton Dickinson ([1]Cat. No. 345784, [2]Cat. No. 556647, [3]Cat. No. 552851) and Beckman Coulter ([4]Cat. No. IM2470) used according to the manufacturers' recommendations. $5 \times 10^5$ - $1 \times 10^6$ cells were stained with the following antibody combination: anti-CD14[1] (FITC) $\times$ anti-CD56[2] (PE) $\times$ anti-CD3[3] (PerCP) $\times$ anti-CD19[4] (APC). Cells were pelleted in V-shaped 96 well multititer plates (Greiner) and the supernatant was removed. Cell pellets were resuspended in a total volume of 100 $\mu$l containing the specific antibodies diluted in FACS buffer. Incubation was carried out in the dark for 30 min at 4°C. Subsequently, samples were washed twice with FACS buffer and cell pellets were resuspended in FACS buffer for flowcytometric analysis.

**Flowcytometric detection of stained lymphocytes by FACS**

Data collection was performed with a 4 color BD FACSCalibur™ (Becton Dickinson). For each measurement $1 \times 10^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding myeloid cells via CD14-staining) as determined by FACS were correlated with the lymphocyte count from the differential blood analysis to calculate absolute cell numbers of T cells ($CD3^+$, $CD56^-$, $CD14^-$).

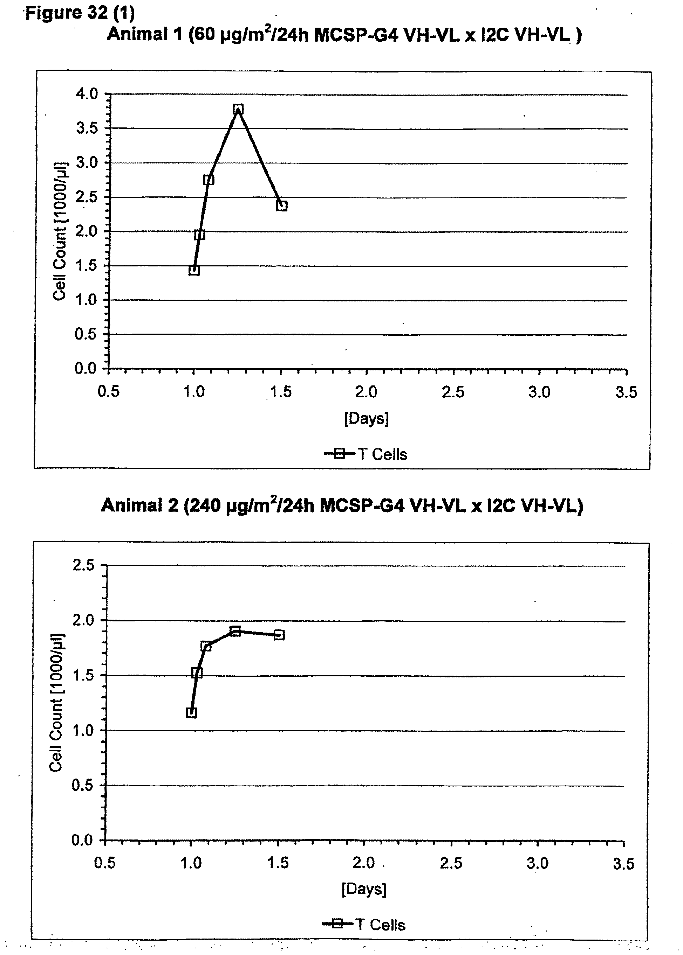

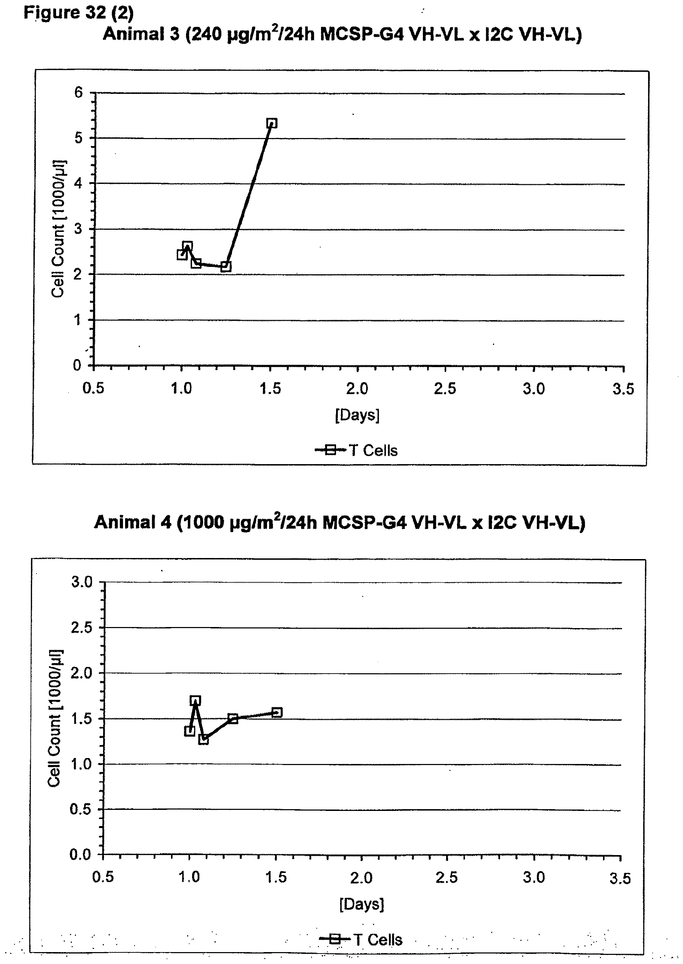

T cell redistribution during the starting phase of treatment with MCSP-G4 VH-VL $\times$ I2C VH-VL in cynomolgus monkeys at dose levels of 60, 240 and 1000 $\mu$g/m²/24h is shown in Fig 32. These animals showed no signs at all of any T cell redistribution during the starting phase of treatment, i.e. T cell counts rather increased than decreased upon treatment initiation. Given that T cell redistribution is consistently observed in 100% of all patients without circulating target cells, upon treatment initiation with the conventional CD3 binding molecule (e.g. CD19xCD3 construct as described in WO 99/54440) against a context dependent CD3 epitope, it was demonstrated that substantially less T cell redistribution in the absence of circulating target cells upon treatment initiation can be observed with a CD3 binding molecule of the invention directed and generated against an epitope of human an non-chimpanzee primate CD3 epsilon chain as defined by the amino acid sequence of anyone of SEQ ID NOs: 2, 4, 6, or 8 or a fragment thereof. This is in clear contrast to CD3-binding molecules directed against a context-dependent CD3 epitope, like the constructs described in WO 99/54440, The binding molecules against context-independent CD3 epitopes, as (inter alia) provided in any one of SEQ ID NOs: 2, 4, 6, or 8 (or fragments of these sequences) provide for this substantially less (detrimental and non-desired) T cell redistribution. Because T cell redistribution during the starting phase of treatment with CD3 binding molecules is a major risk factor for CNS adverse events, the CD3 binding molecules provided herein and capable of recognizing a context independent CD3 epitope have a substantial advantage over the CD3 binding molecules known in the art and directed against context-dependent CD3 epitopes. Indeed none of the cynomolgus monkeys treated with MCSP-G4 VH-VL $\times$ I2C VH-VL showed any signs of CNS symptoms.

The context-independence of the CD3 epitope is provided in this invention and corresponds to the first 27 N-terminal amino acids of CD3 epsilon) or fragments of this 27 amino acid stretch. This context-independent epitope is taken out of its native environment within the CD3 complex and fused to heterologous amino acid sequences without loss of its structural integrity. Anti-CD3 binding molecules as provided herein and generated (and directed) against a context-independent CD3 epitope provide for a surprising clinical improvement with regard to T cell redistribution and, thus, a more favorable safety profile. Without being bound by theory, since their CD3 epitope is context-independent, forming an autonomous selfsufficient subdomain without much influence on the rest of the CD3 complex, the CD3 binding molecules provided herein induce less allosteric changes in CD3 conformation than the conventional CD3 binding molecules (like molecules provided in WO 99/54440), which recognize context-dependent CD3 epitopes like molecules provided in WO 99/54440. As a consequence (again without being bound by theory), the induction of intracellular NcK2 recruitment by the CD3 binding molecules provided herein is also reduced resulting in less isoform switch of T cell integrins and less adhesion of T cells to endothelial cells. It is preferred that preparations of CD3 binding molecules of the invention (directed against and generated against a context-independent epitope as defined herein) essentially consists of monomeric molecules. These monomeric molecules are even more efficient (than dimeric or multimeric molecules) in avoiding T cell redistribution and thus the risk of CNS adverse events during the starting phase of treatment.

**23. Generation and characterization of CD33 and CD3 cross-species specific bispecific single chain molecules**

**23.1. Generation of CHO cells expressing human CD33**

The coding sequence of human CD33 as published in GenBank (Accession number NM_001772) was obtained by gene synthesis according to standard protocols. The gene synthesis fragment was designed as to contain first a Kozak site for eukaryotic expression of the construct, followed by a 19 amino acid immunoglobulin leader peptide, followed in frame by the coding sequence of the mature human CD33 protein, followed in frame by the coding sequence of serine glycine dipeptide, a histidine$_6$-tag and a stop codon (the cDNA and amino acid sequence of the construct is listed under SEQ ID Nos 525 and 526). The gene synthesis fragment was also designed as to introduce restriction sites at the beginning and at the end of the fragment. The introduced restriction sites, EcoRI at the 5' end and Sail at the 3' end, were utilised in the following cloning procedures. The gene synthesis fragment was cloned via EcoRI and Sail into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

**23.2. Generation of CHO cells expressing the extracellular domain of macaque CD33**

The cDNA sequence of macaque CD33 was obtained by a set of 3 PCRs on cDNA from macaque monkey bone marrow prepared according to standard protocols. The following reaction conditions: 1 cycle at 94°C for 3 minutes followed by 35 cycles with 94°C for 1 minute, 53°C for 1 minute and 72°C for 2 minutes followed by a terminal cycle of 72°C for 3 minutes and the following primers were used:

3. forward primer: 5' -gaggaattcaccatgccgctgctgctactgctgcccctgctgtgggcaggggccctggctatgg-3'
reverse primer: 5'-gatttgtaactgtatttggtacttcc-3'
4. forward primer: 5'-attccgcctccttgggggatcc-3'
reverse primer: 5'-gcataggagacattgagctggatgg-3'
5. forward primer: 5'-gcaccaacctgacctgtcagg-3'
reverse primer: 5'-agtgggtcgactcactgggtcctgacctctgagtattcg-3'

Those PCRs generate three overlapping fragments, which were isolated and sequenced according to standard protocols using the PCR primers, and thereby provided a portion of the cDNA sequence of macaque CD33 from the second nucleotide of codon +2 to the third nucleotide of codon +340 of the mature protein. To generate a construct for expression of macaque CD33 a cDNA fragment was obtained by gene synthesis according to standard protocols (the cDNA and amino acid sequence of the construct is listed under SEQ ID Nos 527 and 528). In this construct the coding sequence of macaque CD33 from amino acid +3 to +340 of the mature CD33 protein was fused into the coding sequence of human CD33 replacing the human coding sequence of the amino acids +3 to +340. The gene synthesis fragment was also designed as to contain a Kozak site for eukaryotic expression of the construct and restriction sites at the beginning and the end of the fragment containing the cDNA coding for essentially the whole extracellular domain of macaque CD33, the macaque CD33 transmembrane domain and a macaque-human chimeric intracellular CD33 domain. The introduced restriction sites Xbal at the 5' end and Sail at the 3' end, were utilised in the following cloning procedures. The gene synthesis fragment was then cloned via Xbal and Sall into a plasmid designated pEF-DHFR (pEF-DHFR is described in Raum et al. Cancer Immunol Immunother 50 (2001) 141-150). A sequence verified clone of this plasmid was used to transfect CHO/dhfr- cells as described above.

**23.3. Generation of CD33 and CD3 cross-species specific bispecific antibody molecules**

**Cloning of cross-species specific binding molecules**

Generally, bispecific antibody molecules, each comprising a domain with a binding specificity cross-species specific for human and non-chimpanzee primate CD3 epsilon as well as a domain with a binding specificity cross-species specific for human and non-chimpanzee primate CD33, were designed as set out in the following Table 6:

Table 6: Formats of anti-CD3 and anti-CD33 cross-species specific bispecific molecules

| SEQ ID (nucl/prot) | Formats of protein constructs (N → C) |
|---|---|
| 448/447 | AH11HLxH2CHL |
| 394/393 | AH3HLxH2CHL |
| 430/429 | AC8HLxH2CHL |
| 412/411 | AF5HLxH2CHL |
| 484/483 | F2HLxH2CHL |
| 520/519 | E11HLxH2CHL |
| 466/465 | B3HLxH2CHL |
| 502/501 | B10HL×H2CHL |
| 450/449 | AH11HL×F12QHL |
| 396/395 | AH3HL×F12QHL |
| 432/431 | AC8HLxF12QHL |
| 414/413 | AF5HLxF12QHL |
| 486/485 | F2HLxF12QHL |
| 522/521 | E11HLxF12QHL |
| 468/467 | B3HLxF12QHL |
| 504/503 | B10HLxF12QHL |
| 452/451 | AH11HL×ICHL |
| 398/397 | AH3HL×I2CHL |
| 434/433 | AC8HL×I2CHL |
| 416/415 | AF5HL×I2CHL |
| 488/487 | F2HL×I2CHL |
| 524/523 | E11HL×I2CHL |
| 470/469 | B3HL×I2CHL |
| 506/505 | B10HL×I2CHL |

The aforementioned constructs containing the variable light-chain (L) and variable heavy-chain (H) domains cross-species specific for human and macaque CD33 and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific antibody molecule, followed in frame by the coding sequence of a histidine$_6$-tag and a stop codon. The gene synthesis fragment was also designed as to introduce suitable restriction sites at the beginning and at the end of the fragment. The introduced restriction sites were utilized in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

**Expression and purification of the bispecific antibody molecules**

The bispecific antibody molecules are expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

    Step 1: 20% buffer B in 6 column volumes
    Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific antibody protein was analyzed in SDS PAGE under reducing conditions performed with precast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific antibody.

**23.4. Flow cytometric binding analysis of the CD33 and CD3 cross-species specific bispecific antibodies**

In order to test the functionality of the cross-species specific bispecific antibody constructs regarding the capability to bind to human and macaque CD33 and CD3, respectively, a FACS analysis was performed. For this purpose CHO cells transfected with human CD33 as described in Example 23.1 and the human CD3 positive T cell leukemia cell line HPB-ALL (DSMZ, Braunschweig, ACC483) were used to test the binding to human antigens. The binding reactivity to macaque antigens was tested by using the generated macaque CD33 transfectant described in Example 23.2 and macaque PBMC (preparation of macaque PBMC was performed by Ficoll gradient centrifugation of peripheral blood from macaque monkeys according to standard protocolls). 200.000 cells of the respective cell lines of PBMC were incubated for 30 min. on ice with 50 μl of the purified protein of the cross-species specific bispecific antibody constructs (5 μg/ml) or cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

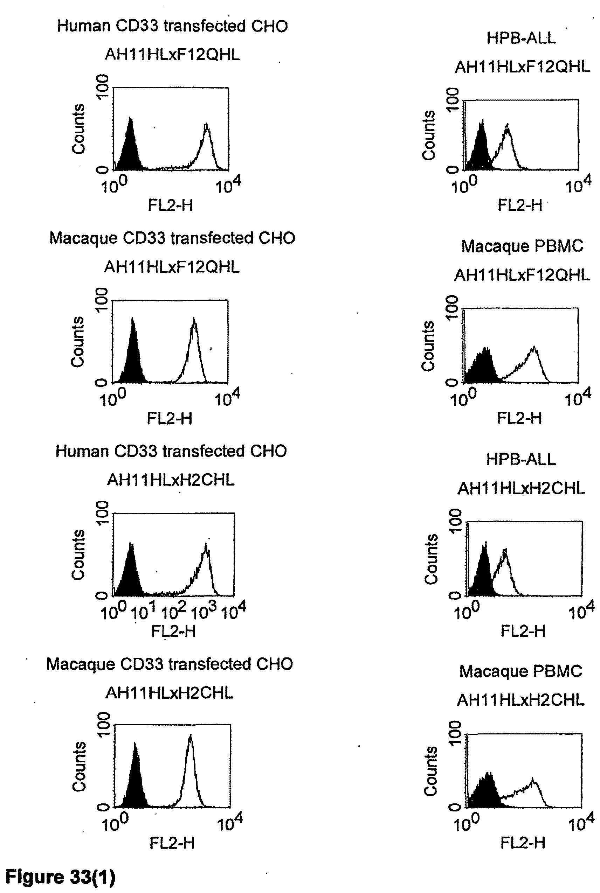

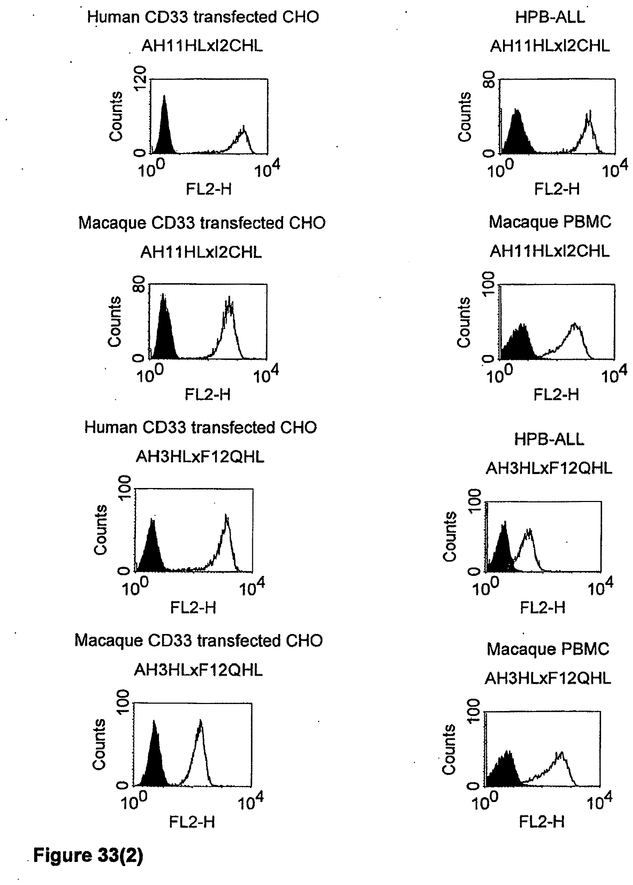

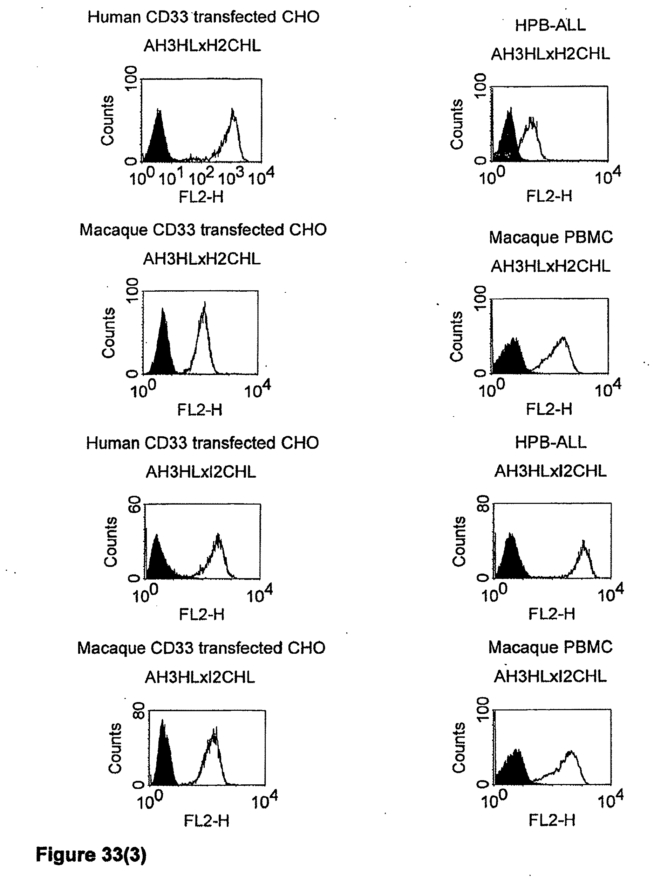

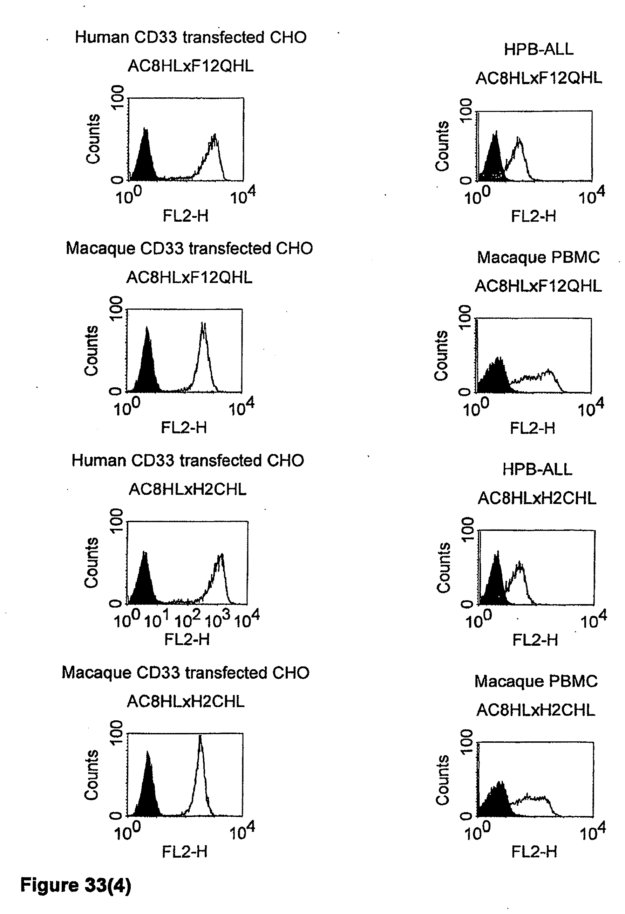

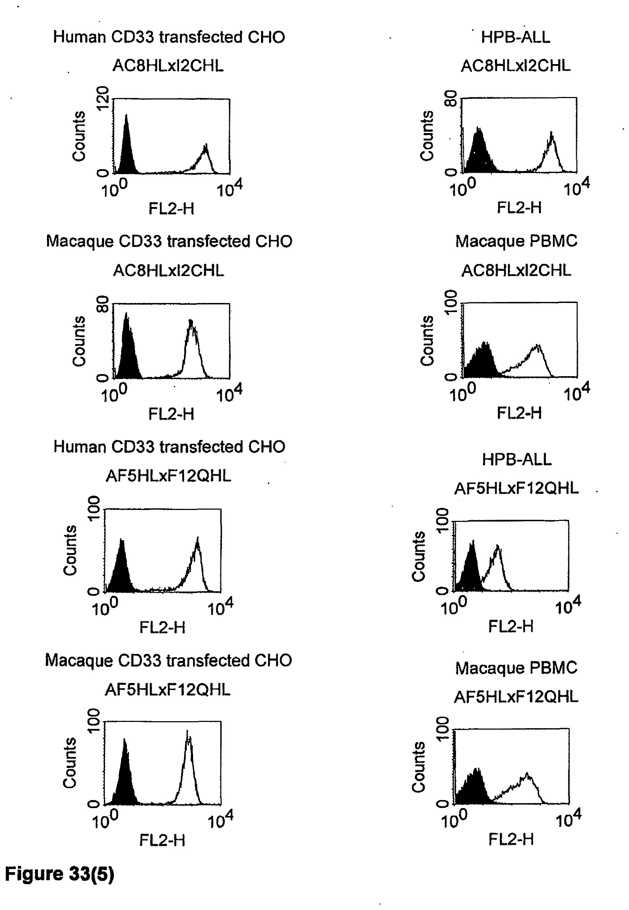

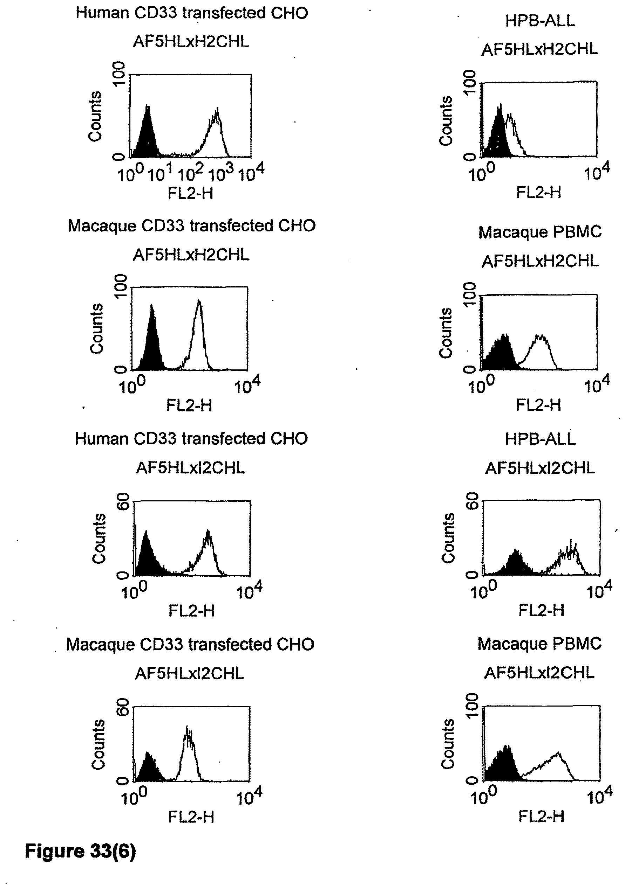

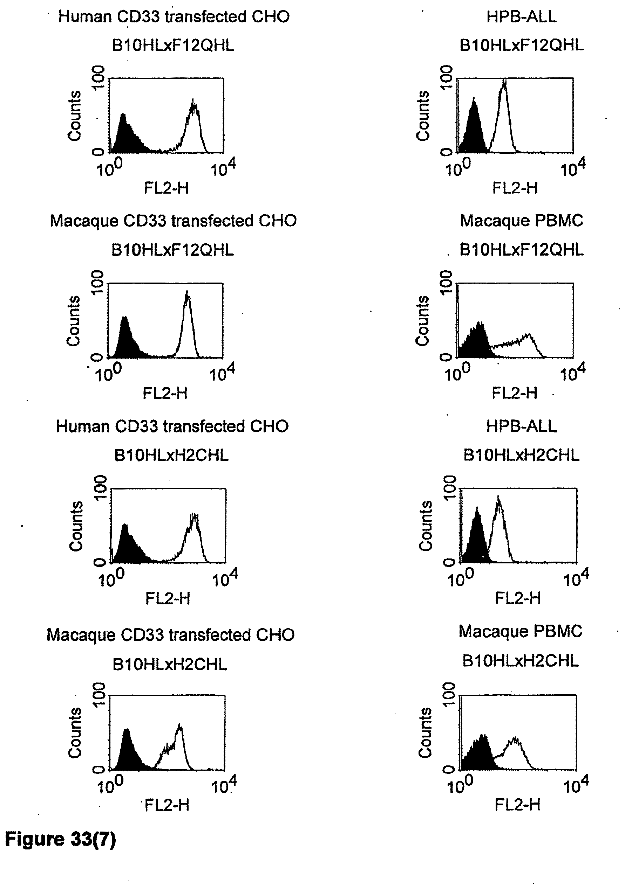

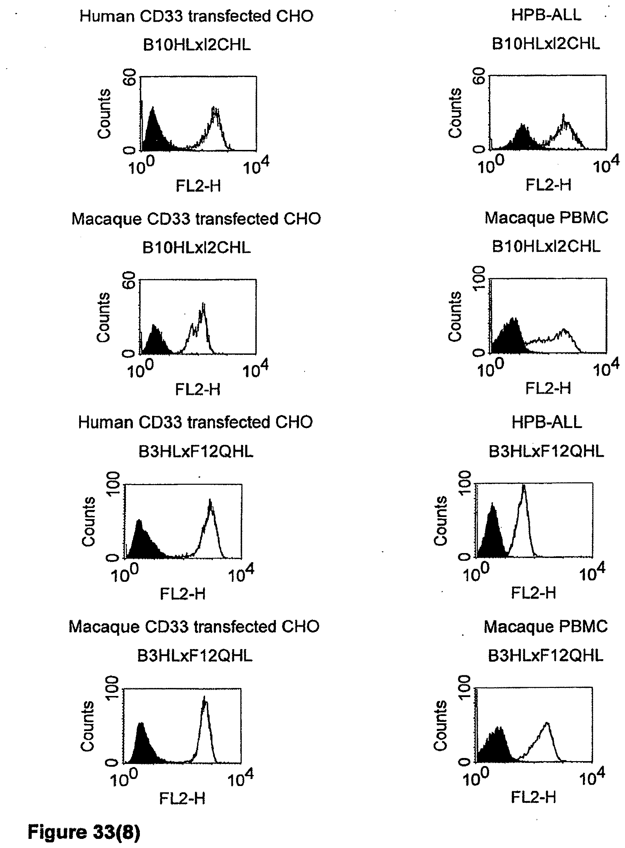

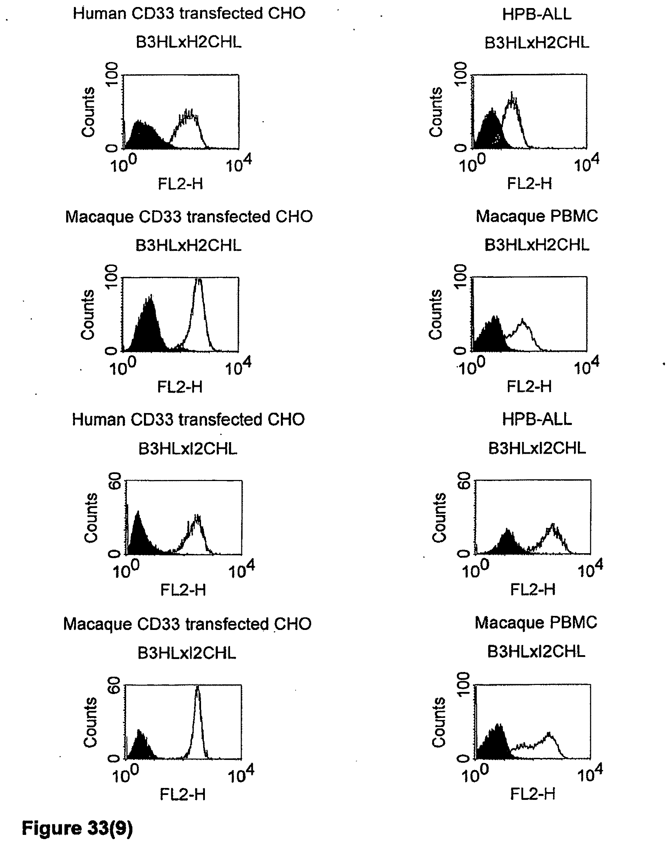

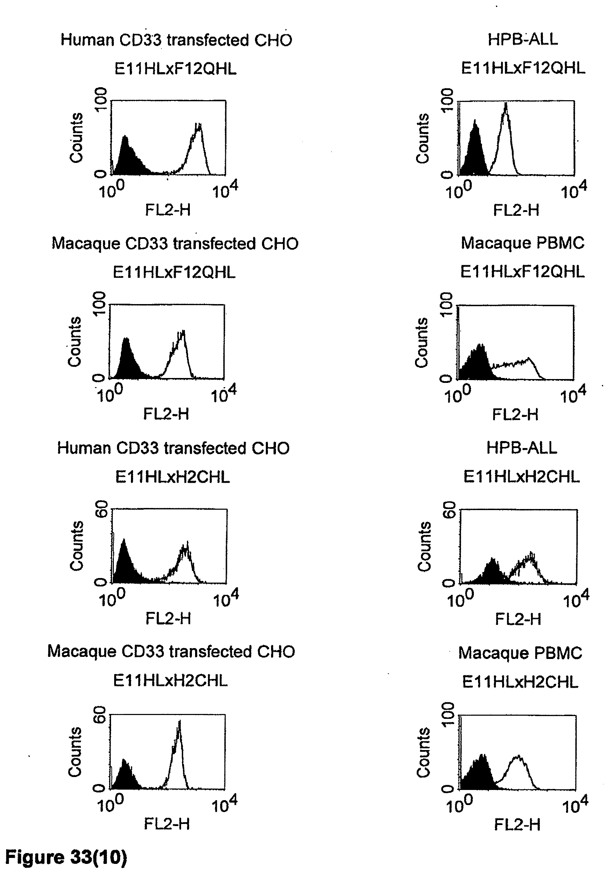

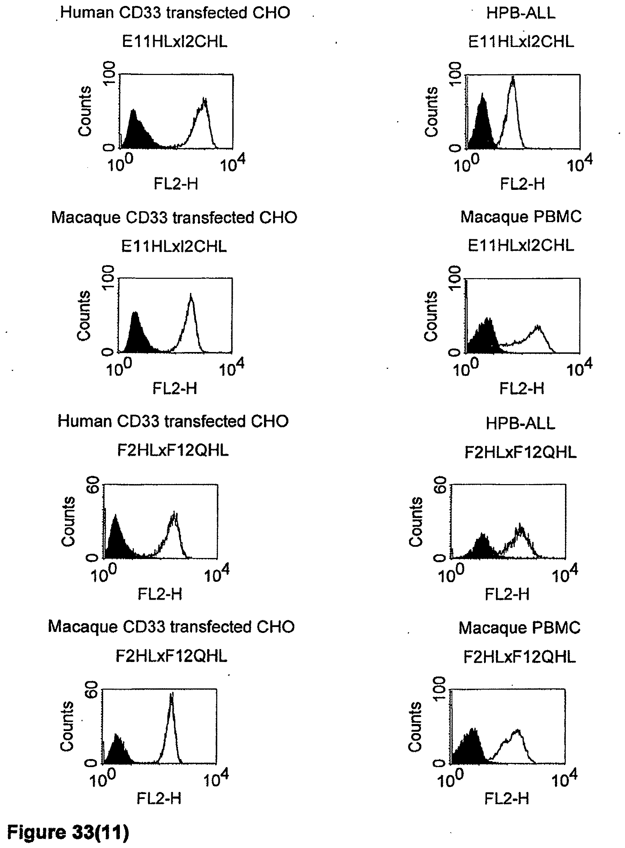

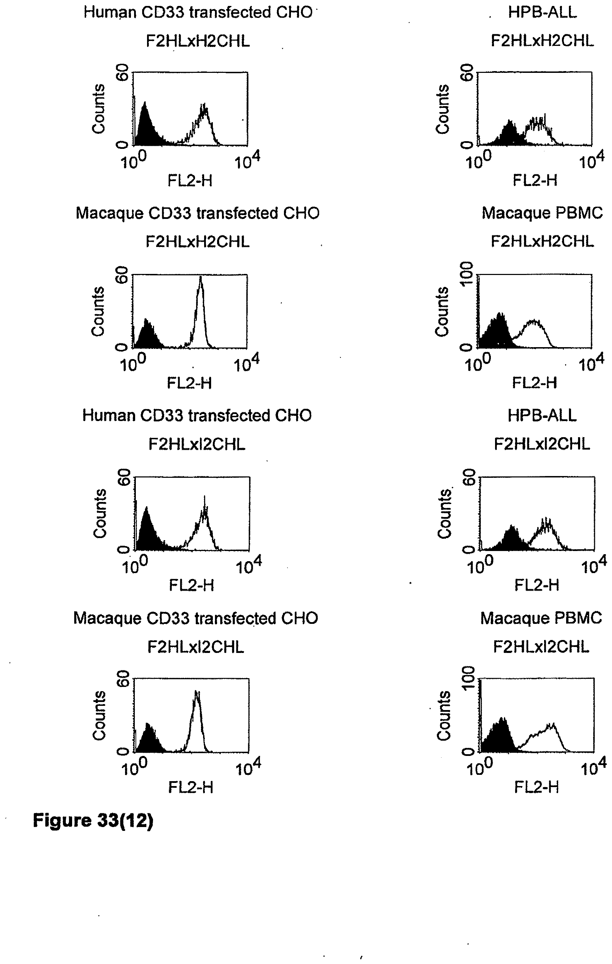

The specific binding of human and non-chimpanzee primate CD3 of the CD3 binding molecules of the invention was clearly detectable as shown in Figure 33. In the FACS analysis all constructs show binding to CD3 and CD33 as compared to the respective negative controls. Cross-species specificity of the bispecific antibodies to human and macaque CD3 and CD33 antigens is demonstrated.

### 23.5. Bioactivity of CD33 and CD3 cross-species specific bispecific antibodies

Bioactivity of the generated bispecific antibodies was analyzed by chromium 51 ($^{51}$Cr) release in vitro cytotoxicity assays using the CD33 positive cell lines described in Examples 23.1 and 23.2. As effector cells stimulated human CD4/CD56 depleted PBMC or the macaque T cell line 4119LnPx were used as specified in the respective figures.

Generation of stimulated human PBMC was performed as follows:

A Petri dish (85 mm diameter, Nunc) was coated with a commercially available anti-CD3 specific antibody (e.g. OKT3, Othoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC .were added to the precoated petri dish in 50 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

Target cells were washed twice with PBS and labeled with 11.1 MBq $^{51}$Cr in a final volume of 100 μl RPMI with 50% FCS for 45 minutes at 37°C. Subsequently the labeled target cells were washed 3 times with 5 ml RPMI and then used in the cytotoxicity assay. The assay was performed in a 96 well plate in a total volume of 250μl supplemented RPMI (as above) with an E:T ratio of 10:1. 1 μg/ml of the cross-species specific bispecific antibody molecules and 20 threefold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have R$^2$ values >0.90 as determined by the software. EC$_{50}$ values calculated by the analysis program were used for comparison of bioactivity.

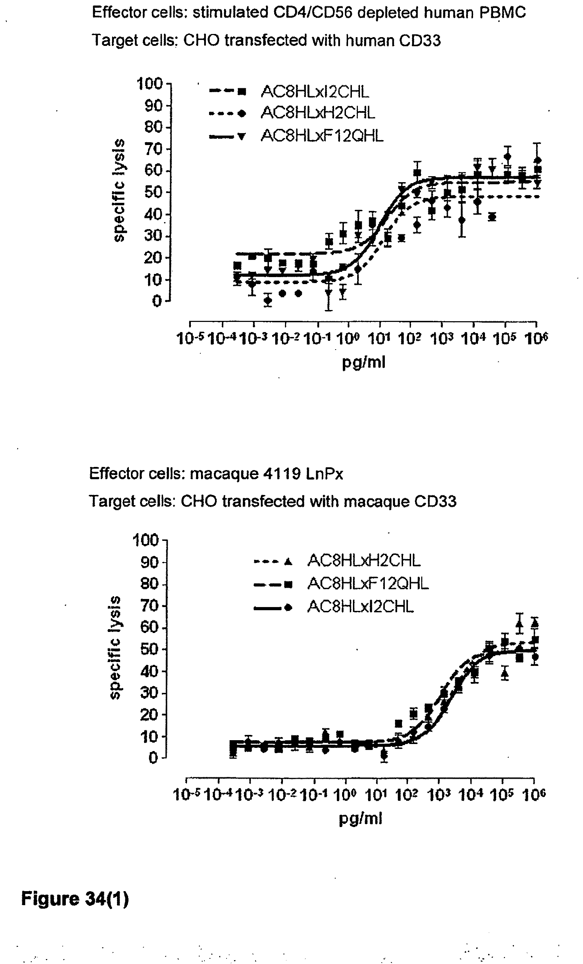

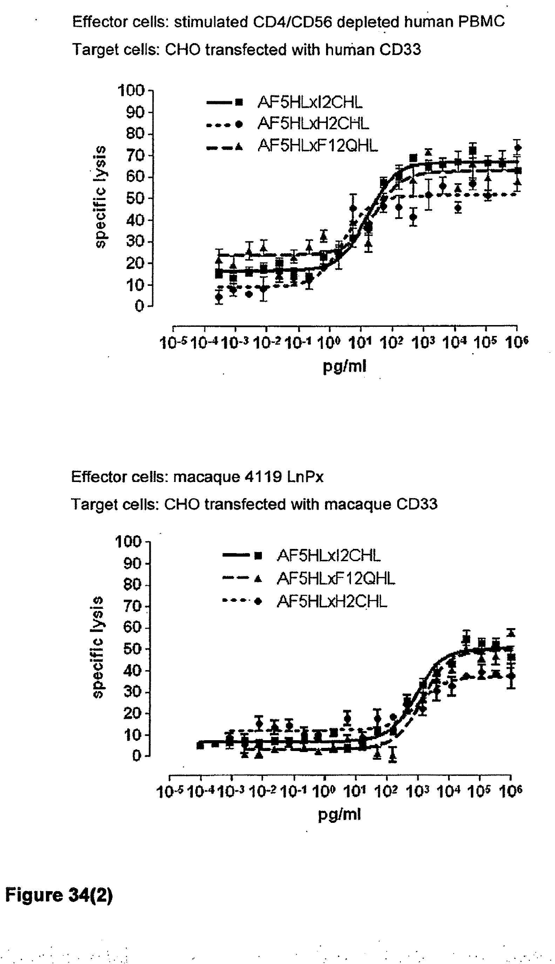

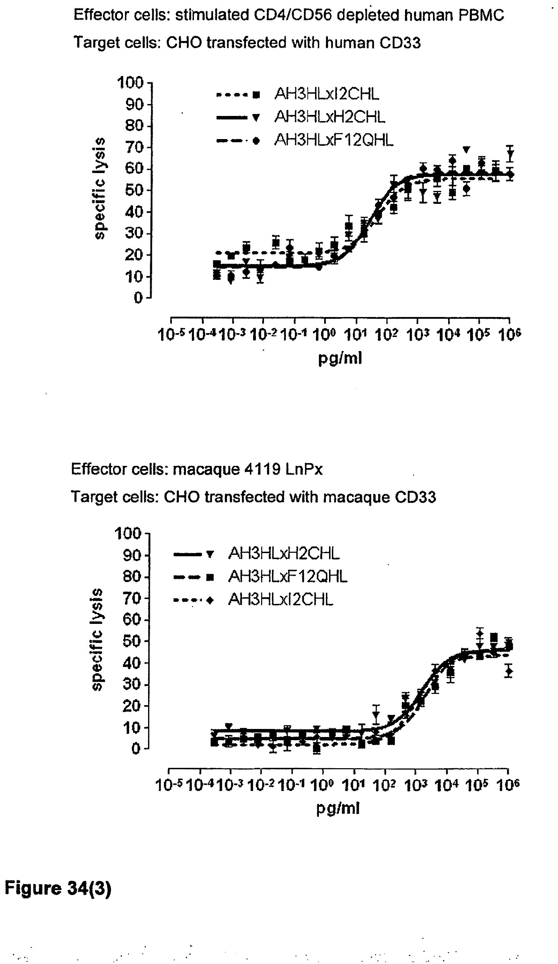

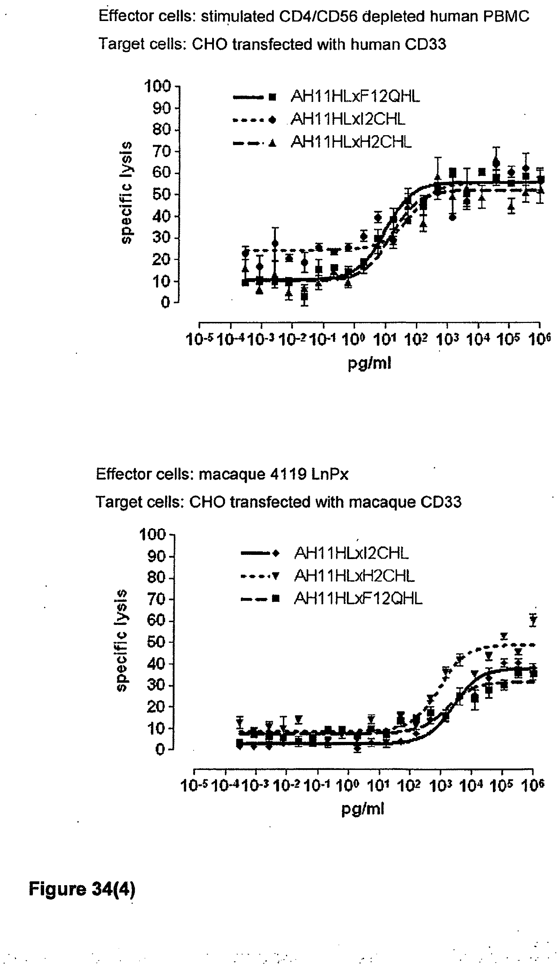

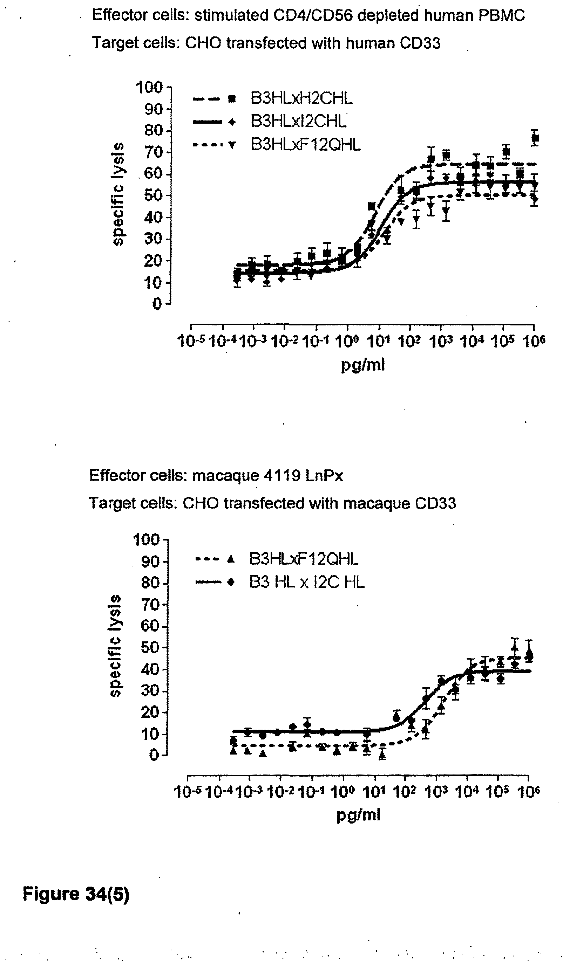

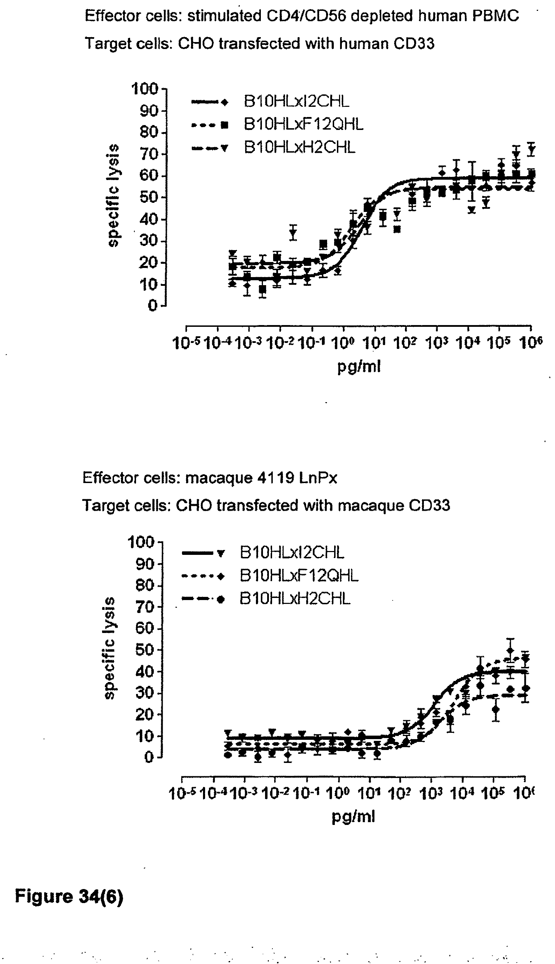

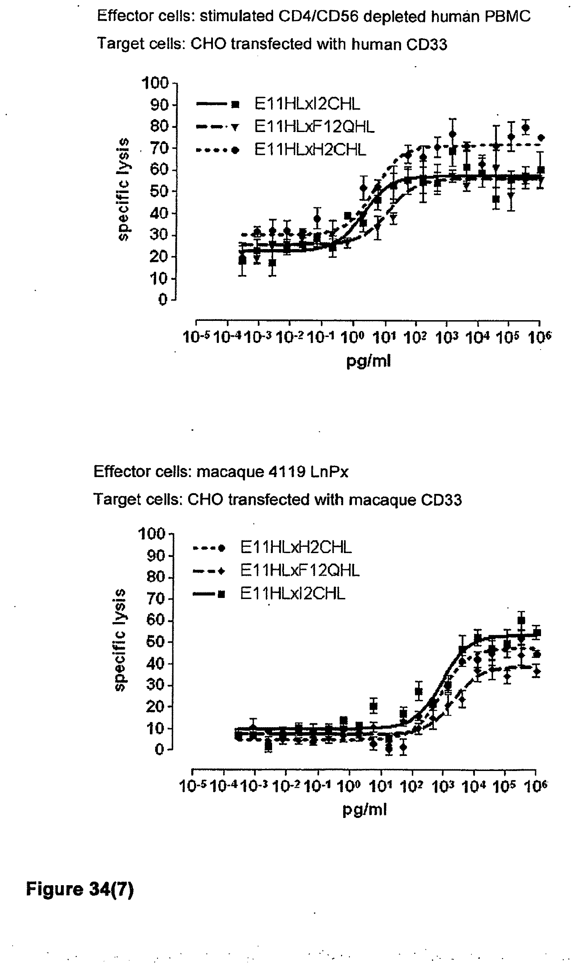

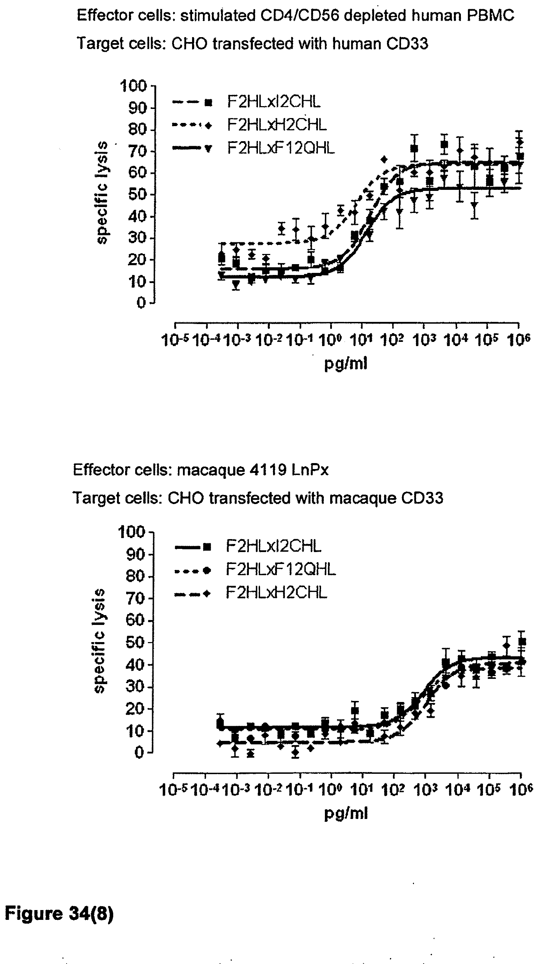

As shown in Figure 34, all of the generated cross-species specific bispecific constructs demonstrate cytotoxic activity against human CD33 positive target cells elicited by stimulated human CD4/CD56 depleted PBMC and against macaque CD33 positive target cells elicited by the macaque T cell line 4119LnPx.

### 24. Generation and characterization of CAIX and CD3 cross-species specific bispecific single chain molecules

### 24.1 Generation of CHO cells expressing human CAIX

The coding sequence of human CAIX as published in GenBank (Accession number NM_001216) was obtained by gene synthesis according to standard protocols. The gene synthesis fragment was designed as to contain the coding sequence of the human CAIX protein including its leader peptide (the cDNA and amino acid sequence of the construct is listed under SEQ ID Nos 604 and 605). The gene synthesis fragment was also designed as to introduce restriction sites at the beginning and at the end of the fragment. The introduced restriction sites, Xbal at the 5' end and Sall at the 3' end, were utilized in the following cloning procedures. The gene synthesis fragment was cloned via Xbal and Sall into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**24.2 Generation of CHO cells expressing macaque CAIX**

The coding sequence of macaque CAIX as published in GenBank (Accession number XM_001088481) was obtained by gene synthesis according to standard protocols. The gene synthesis fragment was designed as to contain the coding sequence of the macaque CAIX protein including its leader peptide (the cDNA and amino acid sequence of the construct is listed under SEQ ID Nos 606 and 607). The gene synthesis fragment was also designed as to introduce restriction sites at the beginning and at the end of the fragment. The introduced restriction sites, Xbal at the 5' end and Sall at the 3' end, were utilized in the following cloning procedures. The gene synthesis fragment was cloned via Xbal and Sall into a plasmid designated pEF-DHFR (pEF-DHFR is described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**24.3 Generation of CAIX and CD3 cross-species specific bispecific single chain molecules**

**Cloning of cross-species specific binding molecules**

Generally, bispecific single chain antibody molecules, each comprising a domain with a binding specificity cross-species specific for human and non-chimpanzee primate CD3epsilon as well as a domain with a binding specificity cross-species specific for human and non-chimpanzee primate CAIX, were designed as set out in the following Table 7:

Table 7: Formats of anti-CD3 and anti-CAIX cross-species specific bispecific single chain antibody molecules

| SEQ ID (nucl/prot) | Formats of protein constructs (N → C) |
| --- | --- |
| 539/538 | CA9-A8 HL × H2C HL |
| 571/570 | CA9-B2 HL × H2C HL |
| 557/556 | CA9-E8 HL × I2C HL |
| 543/542 | CA9-A8 HL × I2C HL |
| 575/574 | CA9-B2 HL × I2C HL |
| 541/540 | CA9-A8 HL × F12Q HL |
| 573/572 | CA9-B2 HL × F12Q HL |

The aforementioned constructs containing the variable light-chain (L) and variable heavy-chain (H) domains cross-species specific for human and macaque CAIX and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilized in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

**24.4. Expression and purification of the bispecific single chain antibody molecules**

The bispecific single chain antibody molecules were expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

**24.5 Flow cytometric binding analysis of the CAIX and CD3 cross-species specific bispecific antibodies**

In order to test the functionality of the cross-species specific bispecific antibody constructs regarding the capability to bind to human and macaque CAIX and CD3, respectively, a FACS analysis was performed. For this purpose CHO cells transfected with human CAIX as described in Example 24.1 and the human CD3 positive T cell leukemia cell line HPB-ALL (DSMZ, Braunschweig, ACC483) were used to test the binding to human antigens. The binding reactivity to macaque antigens was tested by using the generated macaque CAIX transfectant described in Example 24.2 and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 $\mu$l of the purified protein of the cross-species specific bispecific antibody constructs (2 $\mu$g/ml) or cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 $\mu$l PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines. A single chain construct with irrelevant target specificity was used as negative control for binding to the CAIX transfected CHO cells.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

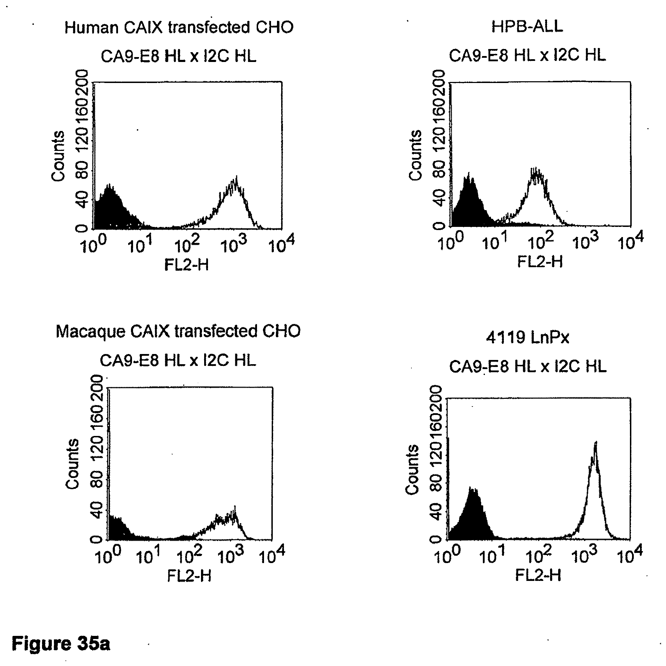

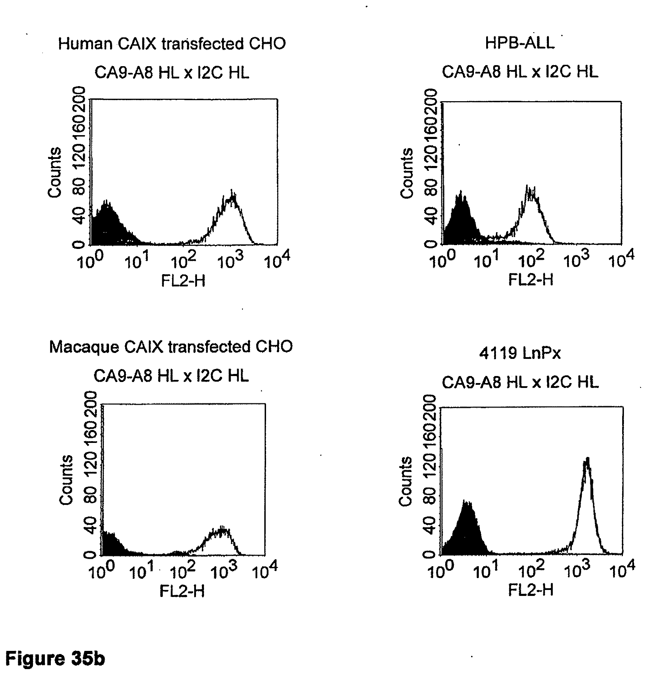

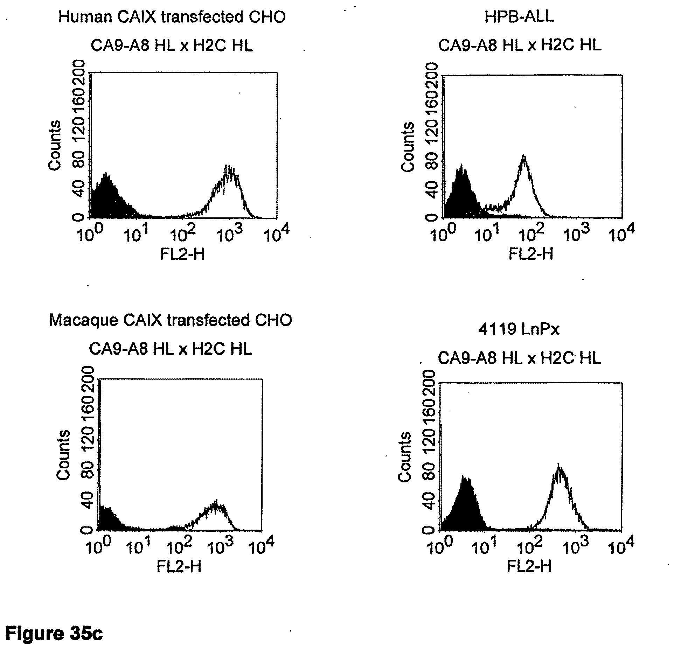

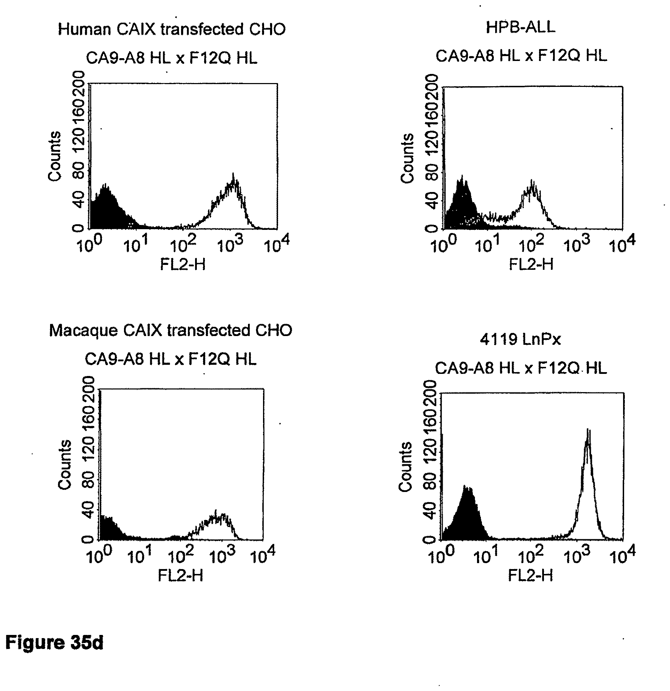

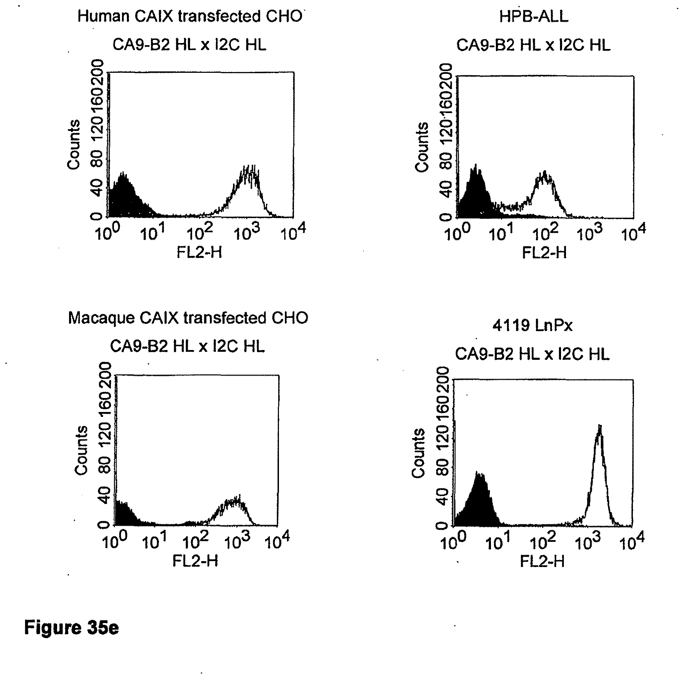

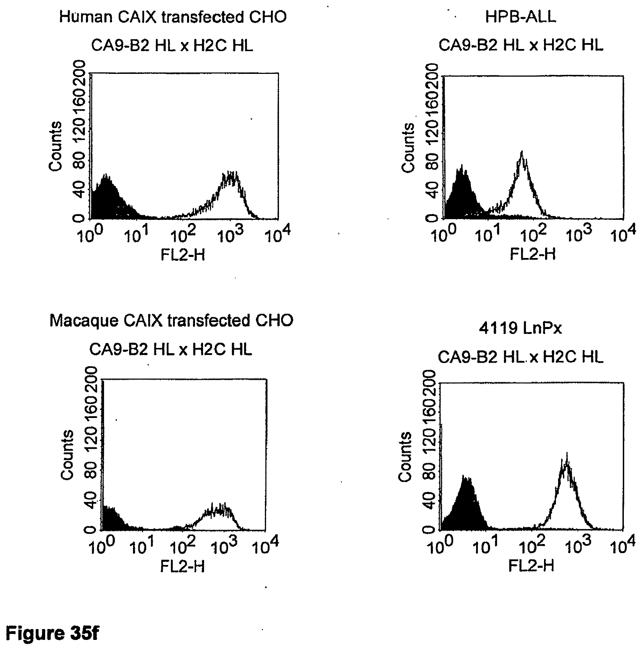

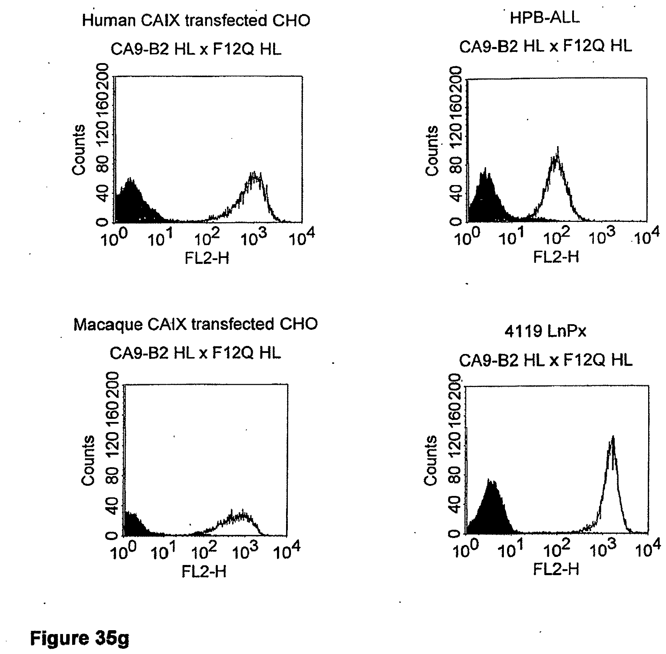

The bispecific binding of the single chain molecules listed above, which are cross-species specific for CAIX and cross-species specific for human and non-chimpanzee primate CD3 was clearly detectable as shown in Figure 35. In the FACS analysis all constructs showed binding to CD3 and CAIX as compared to the respective negative controls. Cross-species specificity of the bispecific antibodies to human and non-chimpanzee primate CD3 and CAIX antigens was demonstrated.

**24.6 Bioactivity of CAIX and CD3 cross-species specific bispecific single chain antibodies**

Bioactivity of the generated bispecific single chain antibodies was analyzed by chromium 51 ($^{51}$Cr) release in vitro cytotoxicity assays using the CAIX positive cell lines described in Examples 24.1 and 24.2. As effector cells stimulated human CD4/CD56 depleted PBMC, stimulated human PBMC or the macaque T cell line 4119LnPx were used as specified in the respective figures.

Generation of stimulated human PBMC was performed as follows:

A Petri dish (145 mm diameter, Greiner) was coated with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

Generation of the stimulated CD4/CD56 depleted PBMC was performed as follows:

A Petri dish (145 mm diameter, Greiner) was coated with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above. By depletion of CD4+ T cells and CD56+ NK cells according to standard protocols CD8+ cytotoxic T lymphocytes (CTLs) were enriched.

Target cells were washed twice with PBS and labeled with 11.1 MBq $^{51}$Cr in a final volume of 100 $\mu$l RPMI with 50% FCS for 45 minutes at 37°C. Subsequently the labeled target cells were washed 3 times with 5 ml RPMI and then used in the cytotoxicity assay. The assay was performed in a 96 well plate in a total volume of 250$\mu$l supplemented RPMI (as above) with differing E:T ratios from 5:1 to 50:1 which are specified in the respective figures. 1 $\mu$g/ml of the cross-species specific bispecific single chain antibody molecules and 20 threefold dilutions thereof were applied. The assay time was 16 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have R$^2$ values >0.90 as determined by the software. EC$_{50}$ values calculated by the analysis program were used for comparison of bioactivity.

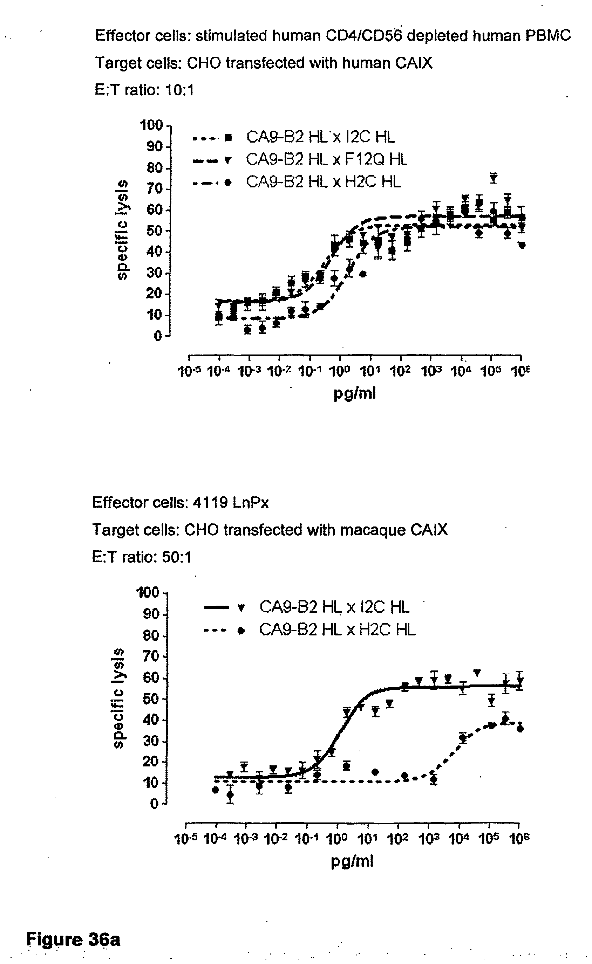

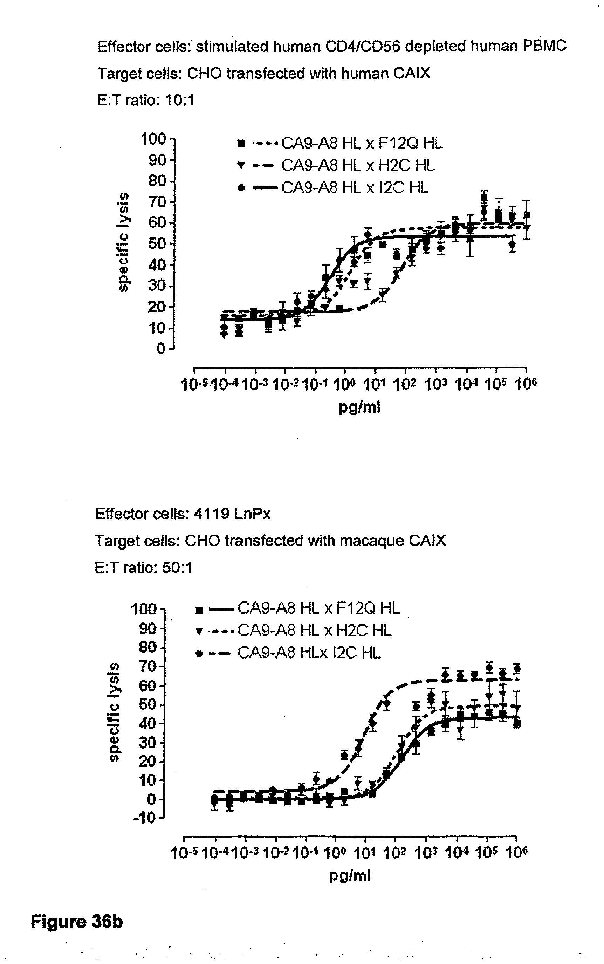

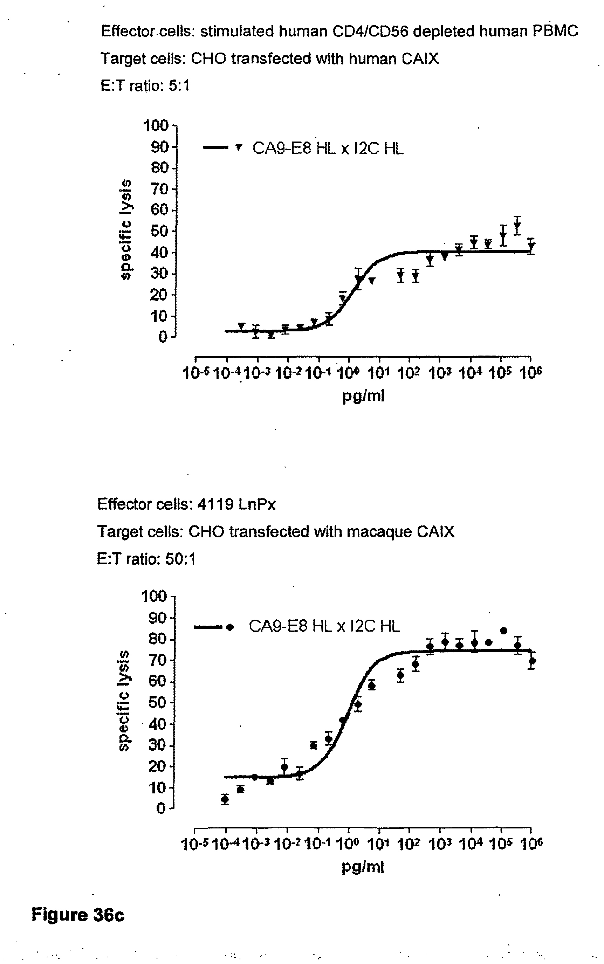

As shown in Figure 36, all of the generated cross-species specific bispecific single chain antibody constructs demonstrated cytotoxic activity against human CAIX positive target cells elicited by stimulated human CD4/CD56 depleted PBMC or stimulated PBMC and against macaque CAIX positive target cells elicited by the macaque T cell line 4119LnPx.

**25. Generation and characterization of EpCAM and CD3 cross-species specific bispecific single chain molecules**

**Construction of EpCAM-specific bispecific antibodies**

The basis for the construction of EpCAM-specific bispecific antibodies was the fully human, EpCAM specific antibody HD69 (Raum et al. Cancer Immunol Immunother (2001) 50: 141 ff). The VH and the VL were PCR amplified from HD69 DNA according to standard methods. VH and VL were cloned subsequently into a plasmid, thus forming a functional scFv (orientation VH-Linker-VL) according to standard protocols. This scFv construct was then PCR amplified using primers introducing a N-terminal BsrGI and a C-terminal BspE1 restriction site useful for functional cloning into the bispecific antibody format

(5'-HD69-BsrG1: 5'-gacaggtgtacactccgaggtgcagctgctcgagtctgg-3'; 3'-HD69-BspE1: 5'-tgatagtccggatttgatctccagcttggtcc-3'). The PCR product was then cloned into three suitable expression vectors, each comprising one CD3 specific VH and VL combination cross-species specific for human and macaque CD3 (H2C HL, F12Q HL and I2C, respectively), thus coding for three functional bispecific constructs HD69 HL x H2C HL, HD69 HL × F12Q HL and HD69 HL × I2C HL. The constructs were then transfected into DHFR-deficient CHO-cells by electroporation for stable transfection according to standard procedures, resulting in three transfected cell pools, each pool expressing one of the three bispecific products.

Table 8: Formats of anti-EpCAM bispecific single chain antibody molecules comprising a human-cynomolgus cross-specific anti-CD3 portion

| SEQ ID (nucl/prot) | Formats of protein constructs (N → C) |
| --- | --- |
| 589/588 | HD69 HL × H2C HL |
| 591/590 | HD69 HL x F12Q HL |
| 593/592 | HD69 HL × I2C HL |

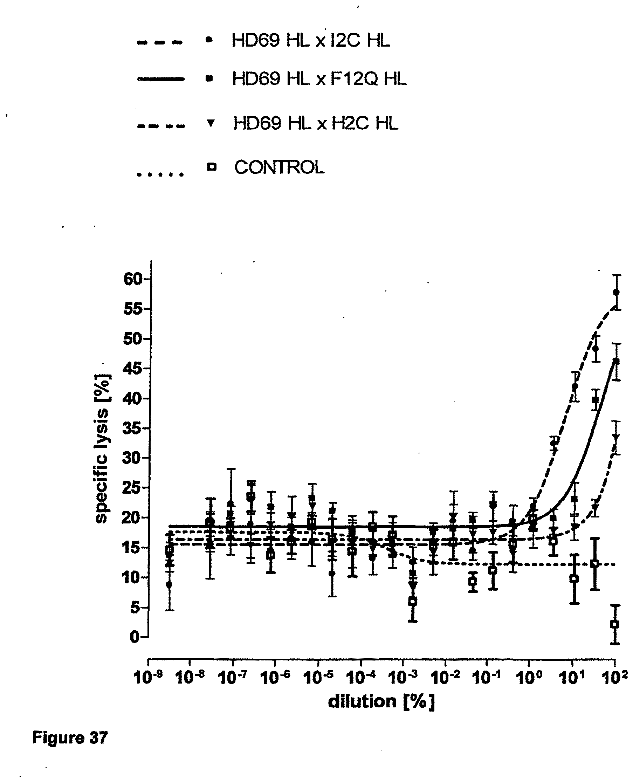

Bioactivity of the generated bispecific single chain antibodies (in culture supernatants of CHO cells expressing the respective bispecific constructs) was analyzed by chromium 51 release in vitro cytotoxicity assays using the transfected EpCAM-positive cell line CHO-EpCAM (expressing surface bound human EpCAM, published in Raum et al. Cancer Immunol Immunother (2001) 50: 141 ff) as target cells. As effector cells stimulated human CD8 positive T cells were used. As shown in Figure 37, all of the generated bispecific single chain antibody constructs revealed cytotoxic activity against human EpCAM-positive target cells elicited by human CD8+ cells. As a negative control, culture supernatant of untransfected CHO cells was used, which revealed no detectable cytotoxicity.

To demonstrate the cross-specific activity of the generated bispecific constructs on the CD3 arm, flow cytometric analysis were performed in which binding of the constructs to cells expressing human CD3 and cells expressing macaque CD3 was verified. Furthermore, specificity to EpCAM-positive cells was examined.

In brief, CHO cells transfected with human EpCAM and human CD3 positive T cells in isolated human PBMCs were used to test the binding to human antigens. The binding reactivity to macaque CD3 antigen was tested by using a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with cell culture supernatant of transfected cells expressing the CD3 cross-species specific bispecific antibody constructs. The cells were washed twice in PBS with 2% FCS and binding of the construct was detected with a murine anti-His antibody (Penta His antibody; Qiagen; diluted 1:20 in 50 $\mu$l PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines.

Flow cytometry was performed on a FACS-Calibur apparatus and CellQuest software used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

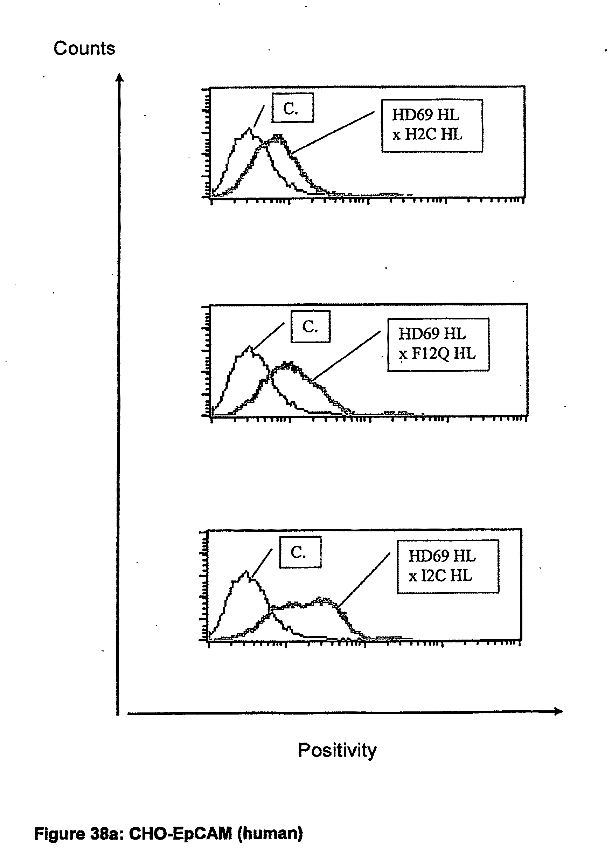

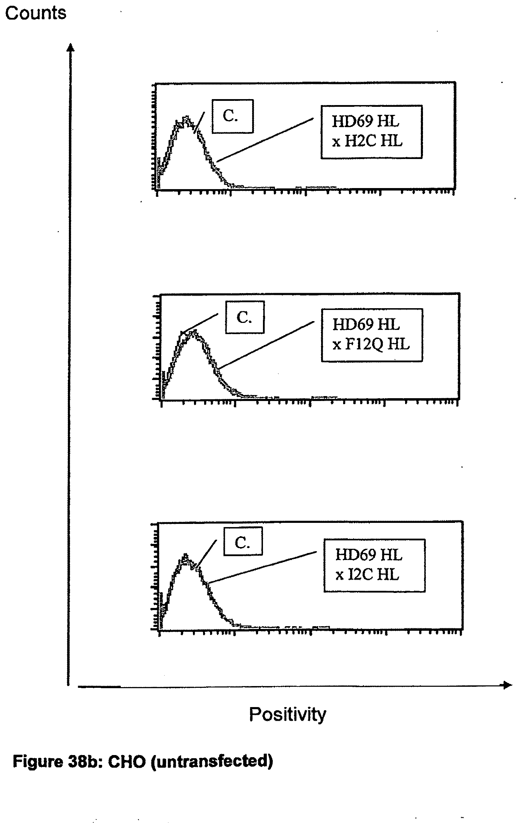

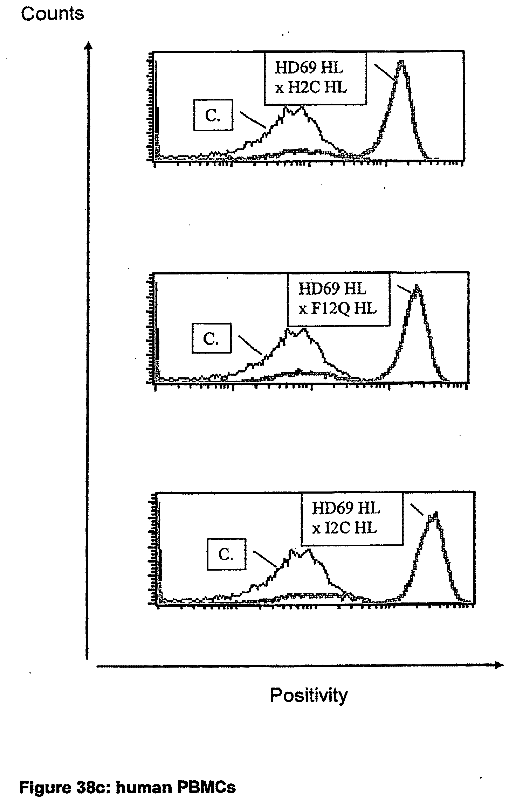

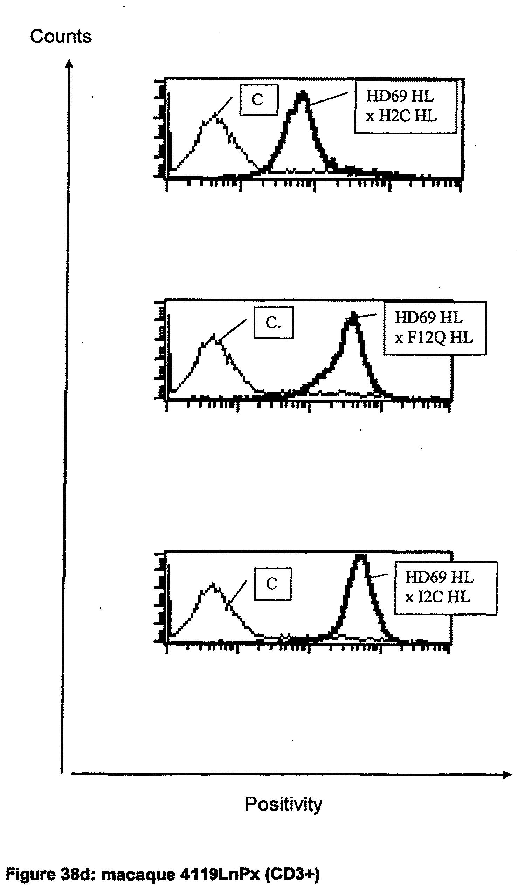

The bispecific binding of the single chain molecules, which are specific for human EpCAM and cross-species specific for human and macaque CD3 was clearly detectable as shown in Figure 38. In the flow cytometric analysis all constructs showed binding to CD3 and EpCAM as compared to the respective negative controls and the EpCAM/CD3 negative CHO cell line.

## 26 Generation and characterization of EGFRvIII and CD3 cross-species specific bispecific single chain molecules

### 26.1 Generation and expression of CHO cells transfected with human EGFRvIII

The coding sequence of the C-terminal, transmembrane and cytoplasmic domain of the mutant epidermal growth factor receptor (DeltaEGFR, de2-7 EGFR, or EGFRvIII are used as synonyms) containing a deletion of 267 amino acids of the extracellular domain (Kuan CT, Wikstrand CJ, Bigner DD: EGF mutant receptor vIII as a molecular target in cancer therapy; Endocr Relat Cancer. 2001 Jun;8(2):83-96) was obtained by gene synthesis according to standard protocols. The gene synthesis fragment was designed as to contain first a Kozak site to allow for eukaryotic expression of the construct followed by the coding sequence of an 24 amino acid leader peptide followed in frame by the coding sequence of the human C-terminal extracellular domain, the transmembrane and cytoplasmic domain and a stop codon. The gene synthesis fragment was also designed as to introduce restriction sites at the beginning and at the end of the DNA fragment. For cloning purposes a XbaI and SalI restriction enzyme recognition sites were added at the 5' and 3' end, respectively. The synthetic gene DNA was subsequently digested with Xbal and Sall, ligated into the appropriately digested expression vector pEF-DHFR, and transformed into E.coli. A clone with verified nucleotide sequence (cDNA sequence and amino acid sequence of the recombinant construct is listed under SEQ ID NOs 599 and 598) was transfected into DHFR deficient CHO cells for eukaryotic expression of the construct. Eukaryotic protein expression in DHFR deficient CHO cells was performed as described above.

### 26.2 Generation and expression of the of cynomolgus EGFR vIII

In analogy to example 26.1 the cynomolgus EGFR vIII variant was created by deletion of the 267 amino acids of the extracellular domain and insertion of the new amino acid glycine at the fusion site (amino acid no. 6). The gene synthesis fragment was designed as to contain first a Kozak site to allow for eukaryotic expression of the construct followed by the coding sequence of an 24 amino acid leader peptide followed in frame by the coding sequence of the cynomolgus C-terminal extracellular domain, the transmembrane and cytoplasmic domain (both human) and a stop codon. The gene synthesis fragment was also designed as to introduce restriction sites at the beginning and at the end of the DNA fragment. The introduced restriction sites Xbal at the 5' end and Sallat the 3' end, were utilized in the following cloning procedures. The fragment was digested with Xbal / Salland cloned into pEF-DHFR following standard protocols. A sequence verified plasmid was used to transfect CHO/dhfr- cells (ATCC No. CRL 9096; cultivated in RPMI 1640 with stabilized glutamine obtained from Biochrom AG Berlin, Germany, supplemented with 10% FCS, 1% penicillin/streptomycin all obtained from Biochrom AG Berlin, Germany and nucleosides from a stock solution of cell culture grade reagents obtained from Sigma-Aldrich Chemie GmbH, Taufkirchen, Germany, to a final concentration of 10 $\mu$g/ml Adenosine, 10 $\mu$g/ml Deoxyadenosine and 10 $\mu$g/ml Thymidine, in an incubator at 37 °C, 95% humidity and 7% CO2). Transfection was performed using the PolyFect Transfection Reagent (Qiagen GmbH, Hilden, Germany) and 5 $\mu$g of plasmid DNA according to the manufacturer's protocol. After a cultivation of 24 hours, cells were washed once with PBS and again cultivated in the aforementioned cell culture medium except that the medium was not supplemented with nucleosides and dialyzed FCS (obtained from Biochrom AG Berlin, Germany) was used. Thus the cell culture medium did not contain nucleosides and thereby selection was applied on the transfected cells. Approximately 14 days after transfection the outgrowth of resistant cells was observed. After an additional 7 to 14 days the transfectants were tested positive for expression of the construct via FACS.

### 26.3 Generation of EGFRvIII and CD3 cross-species specific bispecific single chain molecules

Generally, bispecific single chain antibody molecules, each comprising a domain with a binding specificity for the human and the non-chimpanzee primate CD3 antigen as well as a domain with a binding specificity for the human and the non-chimpanzee primate EGFRvIII antigen, were designed as set out in the following Table 9:

Table 9: Formats of anti-CD3 and anti-EGFRvIII cross-species specific bispecific single chain antibody molecules

| SEQ ID (nucl/prot) | Formats of protein constructs (N $\rightarrow$ C) |
| --- | --- |
| 708/707 | V90F5 HL $\times$ I2C HL |
| 680/679 | V96A9 HL $\times$ I2C HL |
| 694/693 | V98B11 HL $\times$ I2C HL |
| 722/721 | V11A11 HL $\times$ I2C HL |
| 736/735 | V11F6 HL $\times$ I2C HL |
| 750/749 | V11G7 HL $\times$ I2C HL |
| 764/763 | V17F5 HL $\times$ I2C HL |
| 778/777 | V11A4 HL $\times$ I2C HL |
| 792/791 | V17C8 HL $\times$ I2C HL |
| 806/805 | V17G9 HL $\times$ I2C HL |
| 820/819 | V17B11 HL $\times$ I2C HL |
| 834/833 | V17F11 HL $\times$ I2C HL |
| 848/847 | V16B7 HL $\times$ I2C HL |
| 862/861 | V17A4 HL $\times$ I2C HL |

(continued)

| SEQ ID (nucl/prot) | Formats of protein constructs (N → C) |
|---|---|
| 648/647 | V207C12 HL × I2C HL |

The aforementioned constructs containing the variable light-chain (L) and variable heavy-chain (H) domains cross-species specific for human and cynomolgus EGFR vIII and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilized in the following cloning procedures. The gene synthesis fragment was cloned via these restriction sites into a plasmid designated pEF-DHFR following standard protocols. A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX. Alternatively the constructs were transfected into DHFR-deficient CHO-cells in a transient manner according to standard protocols.

The FACS binding experiments were performed with the human **EGFRvIII** transfected CHO cell line to assess the binding capability to the human **EGFRvIII** antigen. The cross-species specificity to cynomolgus tissue was tested by deploying the CHO cells transfected with the cynomolgus EGFRvIII. The same changes in cell lines apply to the cytotoxicity assays performed with the EGFRvIII and CD3 cross-species specific bispecific single chain antibodies. Apart from this the assays were performed as described in examples 4 and 5.

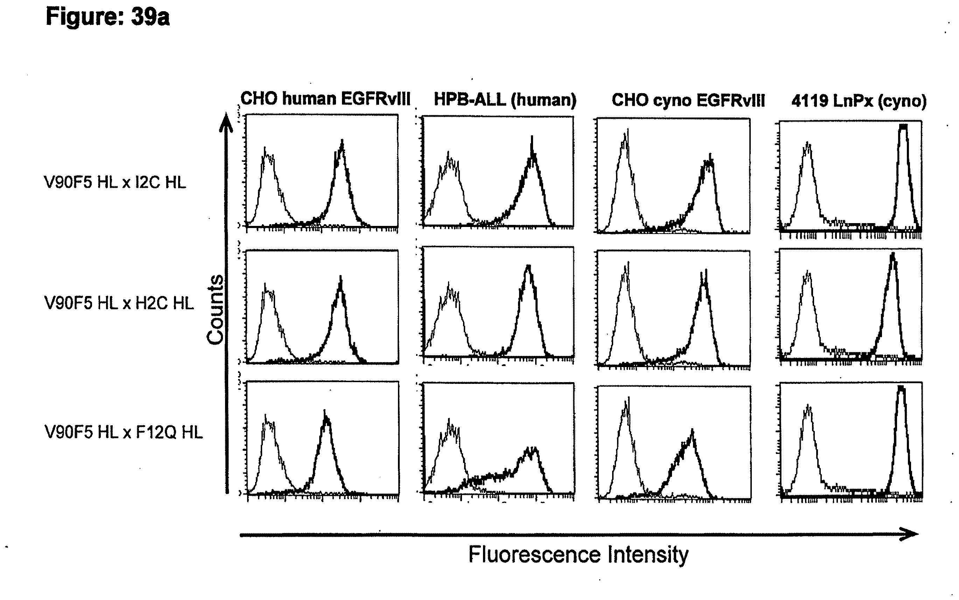

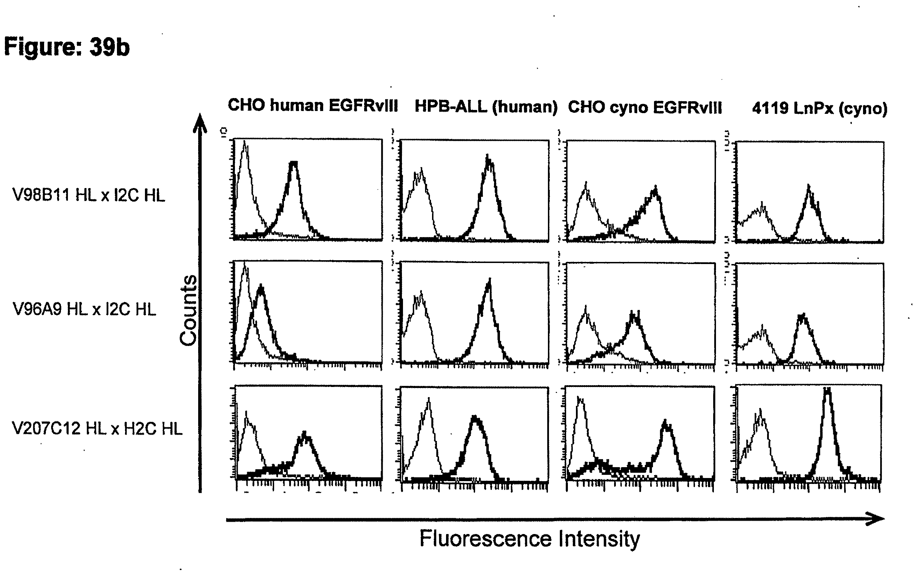

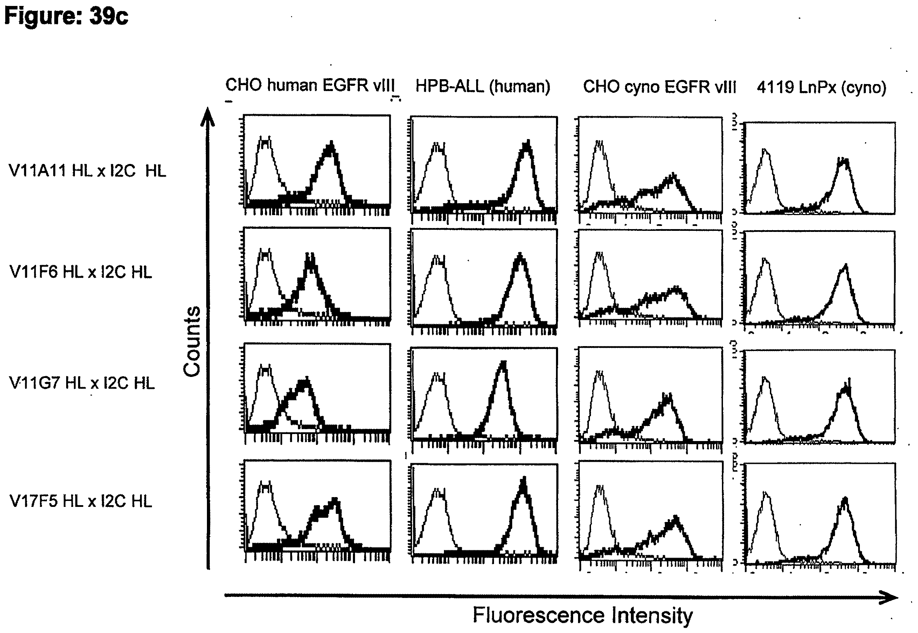

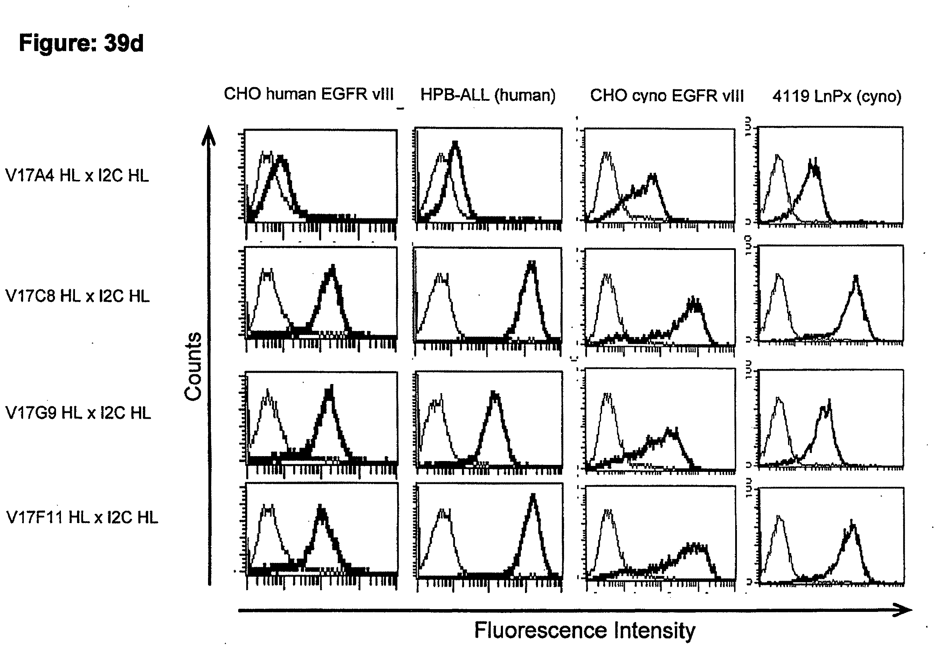

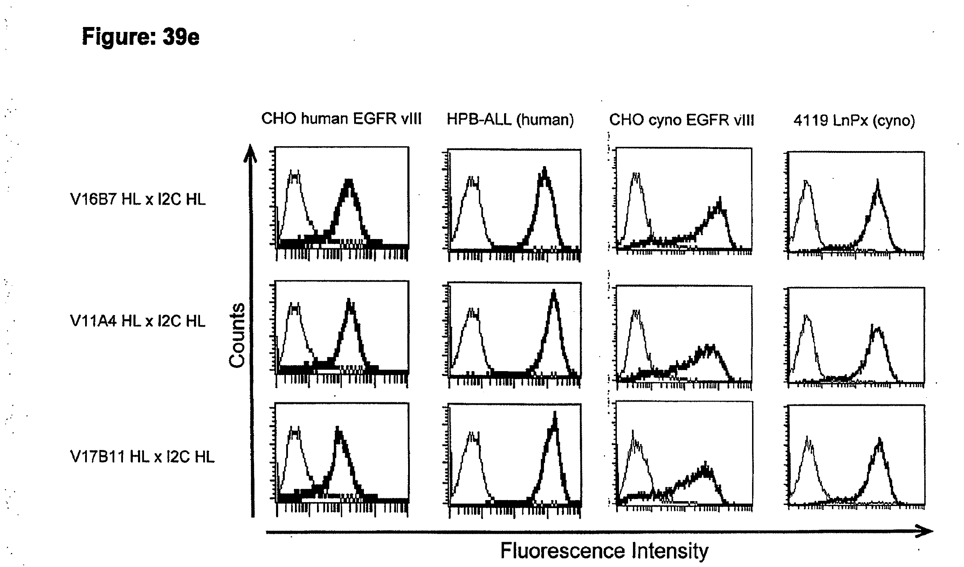

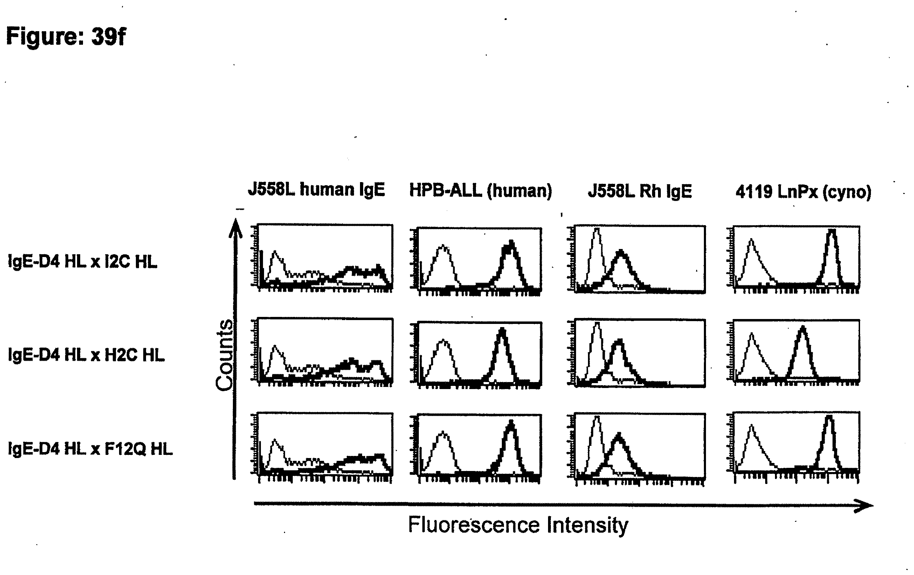

As depicted in Figures 39, the generated EGFRvIII and CD3 cross-species specific bispecific single chain antibodies demonstrated binding to both the human and cynomolgus antigens and proved to be fully cross-species specific.

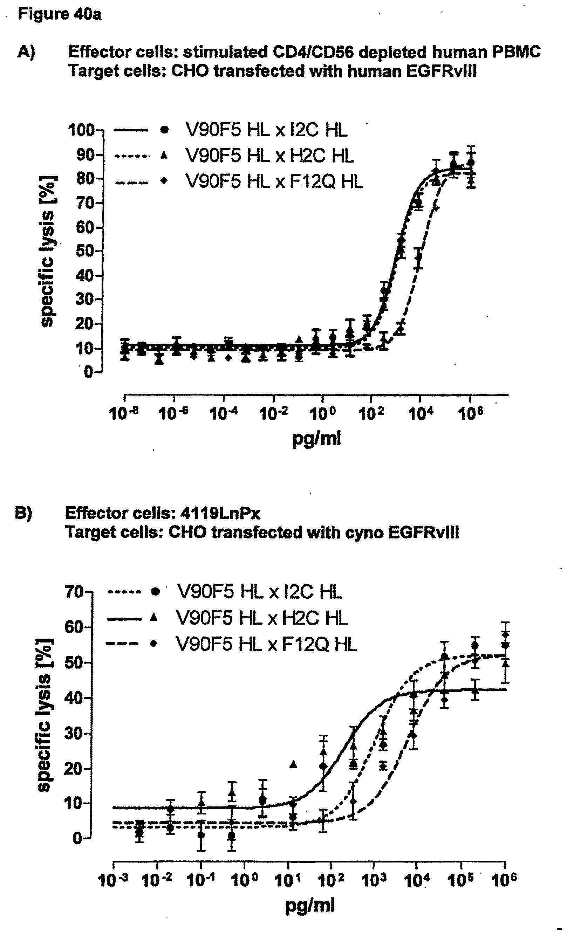

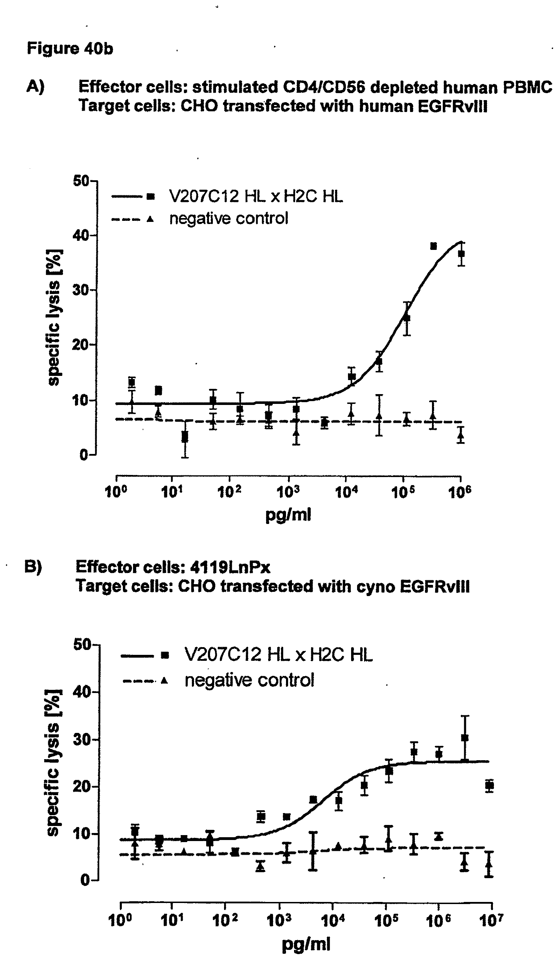

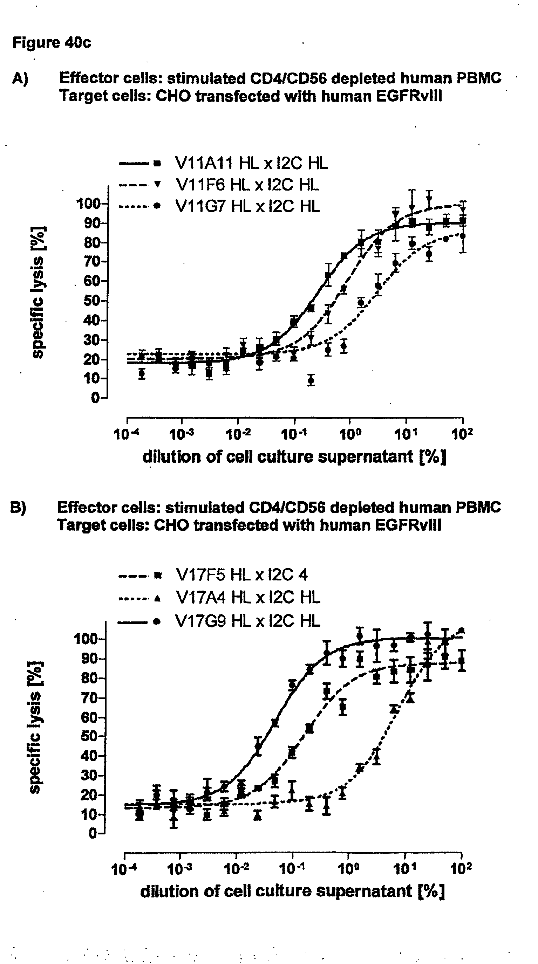

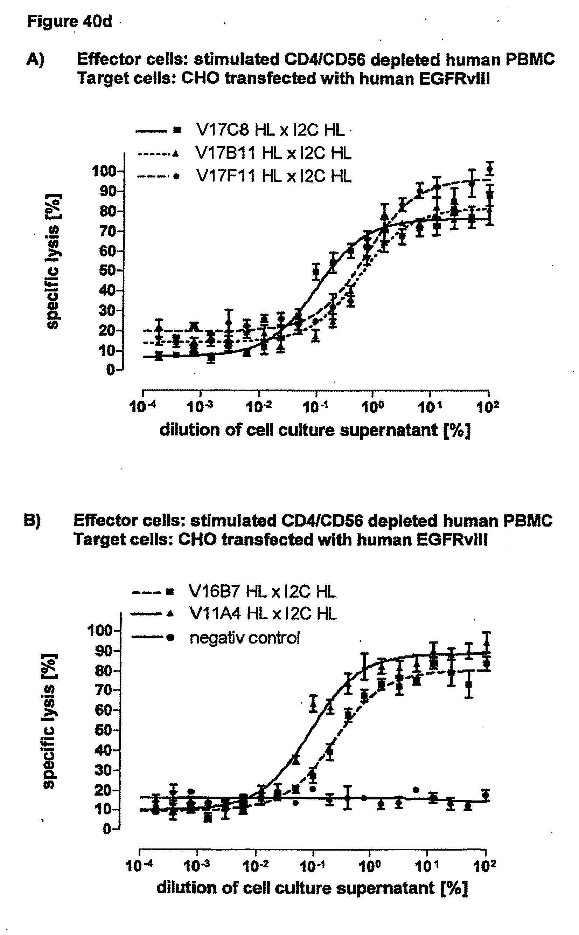

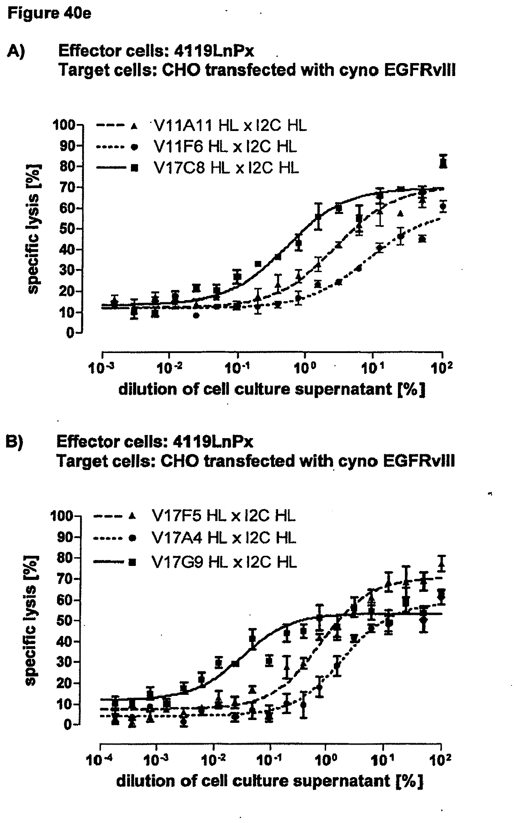

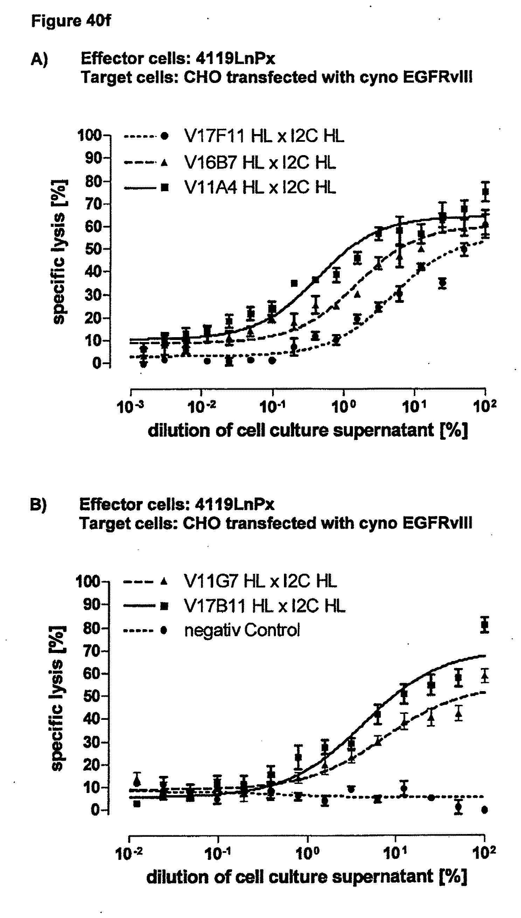

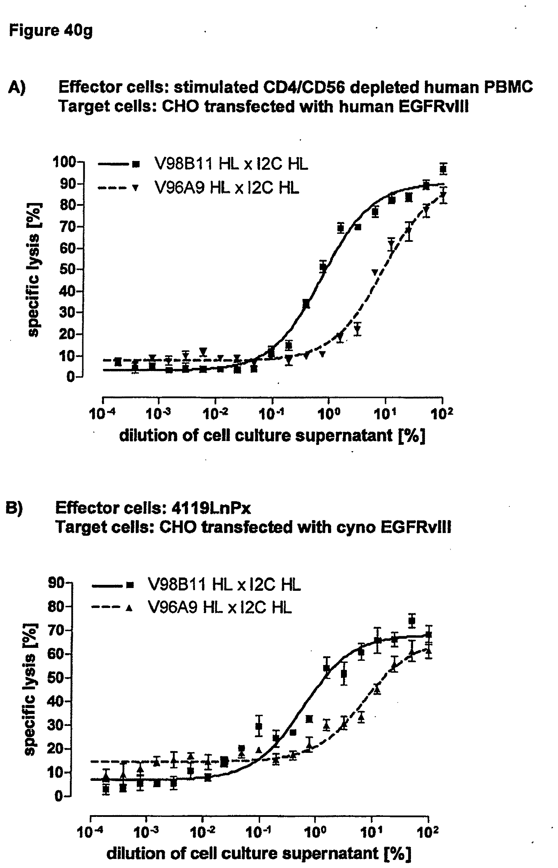

As shown in Figures 40, all of the generated cross-species specific bispecific single chain antibody constructs revealed cytotoxic activity against human EGFRvIII positive target cells elicited by human CD8+ cells and cynomolgus EGFRvIII positive target cells elicited by the macaque T cell line 4119LnPx. As a negative control, an irrelevant bispecific single chain antibody has been used.

**27. Generation and characterization of IgE and CD3 cross-species specific bispecific single chain molecules**

**27.1 Cloning and expression of the human and macaque membrane bound form of IgE**

The mouse cell line J558L (obtained from Interlab Project, Istituto Nazionale per la Ricerca sul Cancro, Genova, Italy, ECACC 88032902), a spontaneous heavy chain-loss-variant myeloma cell line that synthesizes and secretes a lambda light chain, was used to be complemented by a membrane bound heavy chain variant of the human and macaque IgE, respectively. In order to generate such constructs synthetic molecules were obtained by gene synthesis according to standard protocols (the nucleotide sequences of the constructs are listed under SEQ ID Nos 595 and 594). In these constructs the coding sequence for human and macaque CD3 epsilon chain was fused to the human transmembrane region of IgE, respectively. The built in specificity of the VH chain is directed against the hapten (4-hydroxy-3-nitro-phenyl)acetyl) (NP). The gene synthesis fragment was also designed as to contain a Kozak site for eukaryotic expression of the construct and a immunoglobulin leader and restriction sites at the beginning and the end of the DNA. The introduced restriction sites EcoRI at the 5' end and SalI at the 3' end were utilized during the cloning step into the expression plasmid designated pEF-DHFR. After sequence verification (macaque: XM_001116734 macaca mulatta Ig epsilon C region, mRNA; human: NC_000014 Homo sapiens chromosome 14, complete sequence, National Center for Biotechnology Information, http://www.ncbi.nlm.nih.gov/entrez ) the plasmids were used to transfect CHO/dhfr- cells as described above. Eukaryotic protein expression in DHFR deficient CHO cells is performed as described by Kaufmann R.J. (1990) Methods Enzymol. 185, 537-566. Gene amplification of the construct is induced by increasing concentrations of methothrexate (MTX) to a final concentration of up to 20 nM MTX.

**27.2 Generation of IgE and CD3 cross-species specific bispecific single chain molecules**

Generally, bispecific single chain antibody molecules, each comprising a domain with a binding specificity for the human and the non-chimpanzee primate CD3 antigen as well as a domain with a binding specificity for the human and non-chimpanzee primate IgE antigen, were designed as set out in the following Table 10:

Table 10: Formats of anti-CD3 and anti-IgE cross-species specific bispecific single chain antibody molecules

| SEQ ID (nucl/prot) | Formats of protein constructs (N → C) |
|---|---|
| 1549/1548 | IgE G9 HL × I2C HL |
| 1547/1546 | IgE G9 HL × F12Q HL |
| 1545/1544 | IgE G9 HL × H2C HL |
| 1555/1554 | IgE G9 LH × I2C HL |
| 1553/1552 | IgE G9 LH × F12Q HL |
| 1551/1550 | IgE G9 LH × H2C HL |
| 1529/1528 | IgE D4 LH × I2C HL, |
| 1527/1526 | IgE D4 LH × F12Q HL |
| 1525/1524 | IgE D4 LH × H2C HL |
| 1523/1522 | IgE D4 HL × I2C HL |
| 1521/1520 | IgE D4 HL × F12Q HL |
| 1519/1518 | IgE D4 HL × H2C HL |
| 1557/1556 | H2C HL × IgE-G9 HL |
| 1559/1558 | F12Q HL × IgE-G9 HL |
| 1567/1566 | IgE G9 LH × I2C LH |
| 1561/1560 | I2C HL × IgE G9 HL |
| 1563/1562 | I2C HL × IgE G9 LH |
| 1565/1564 | I2C LH × IgE G9 LH |

The aforementioned constructs containing the variable light-chain (L) and variable heavy-chain (H) domains cross-species specific for human and macaque IgE and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilized in the following cloning procedures. The gene synthesis fragment was cloned via these restriction sites into a plasmid designated pEF-DHFR following standard protocols. A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX. Alternatively the constructs were transfected into DHFR-deficient CHO-cells in a transient manner according to standard protocols.

The FACS binding experiments were performed with the human IgE transfected J558L cell line to assess the binding capability to the human IgE . The cross-species specificity to macaque IgE positive cells was tested by deploying the J558L cells transfected with the macaque IgE. The same changes in cell lines apply to the The J558L cell line was also used for the cytotoxicity assays performed with the IgE and CD3 cross-species specific bispecific single chain antibodies. The binding experiments as well as the cytotoxicity assays were performed as described above.

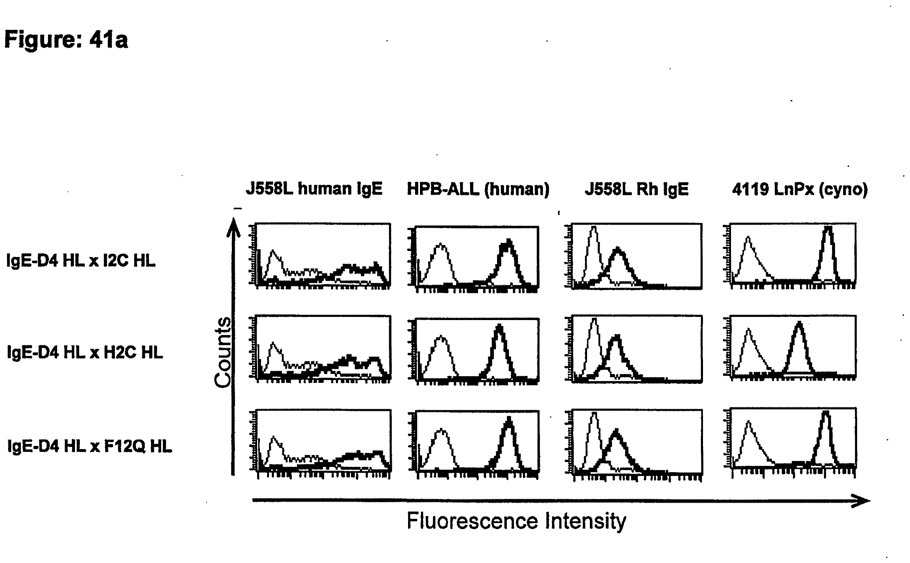

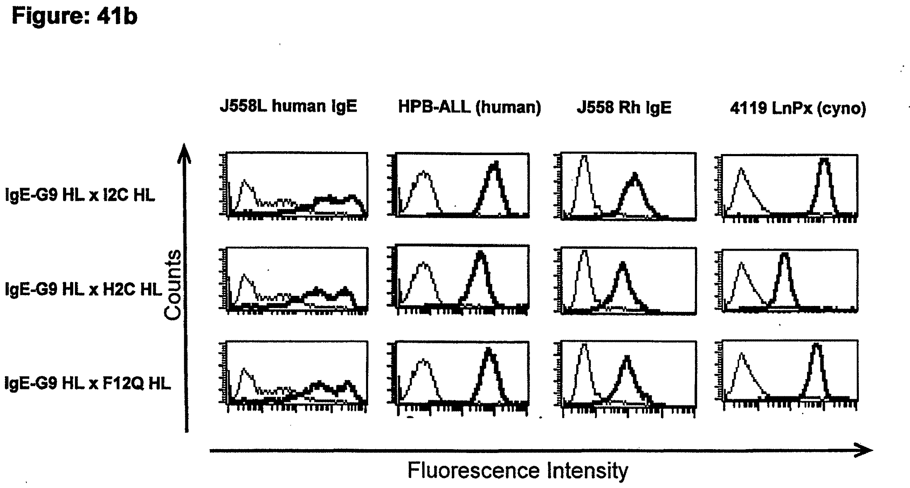

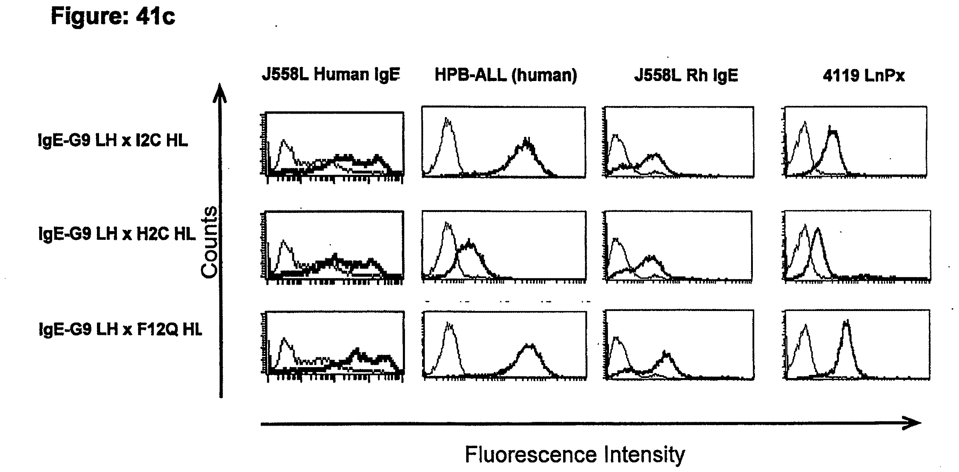

As depicted in Figures 41, the generated IgE and CD3 cross-species specific bispecific single chain antibodies demonstrated binding to both the human and cynomolgus antigens and proved to be fully cross-species specific.

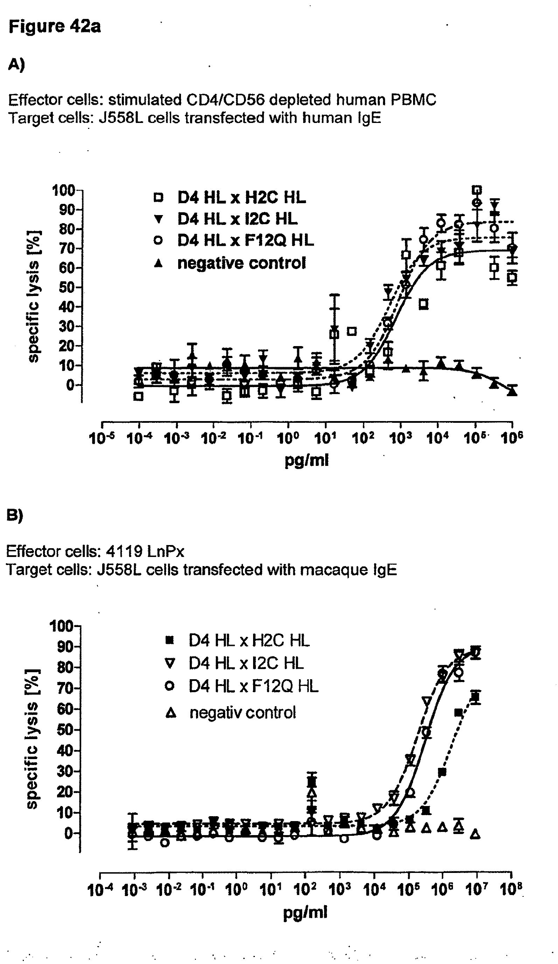

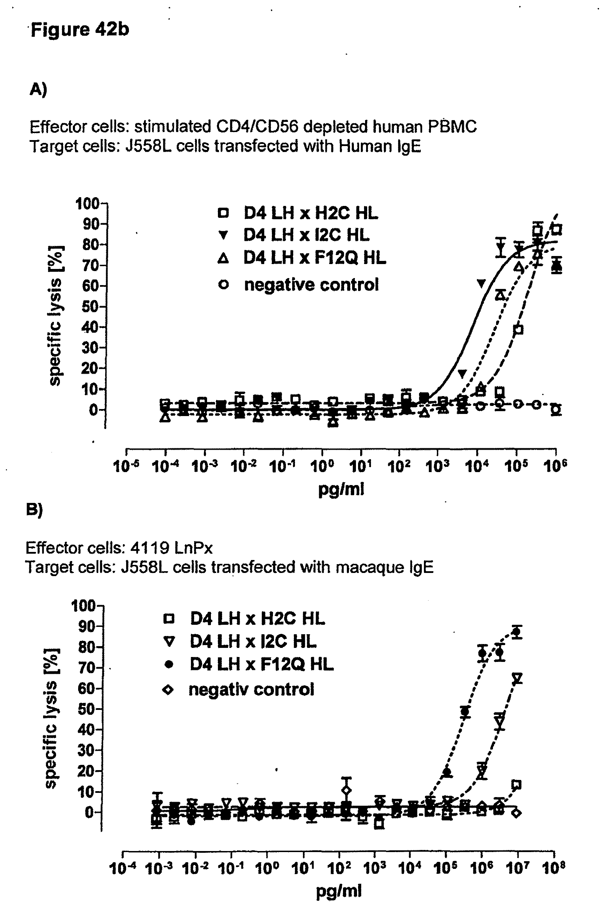

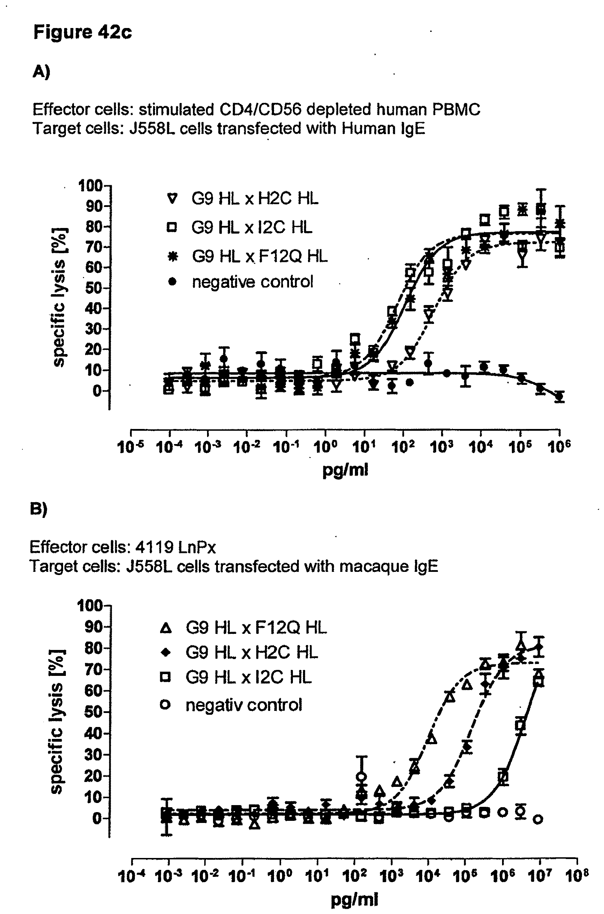

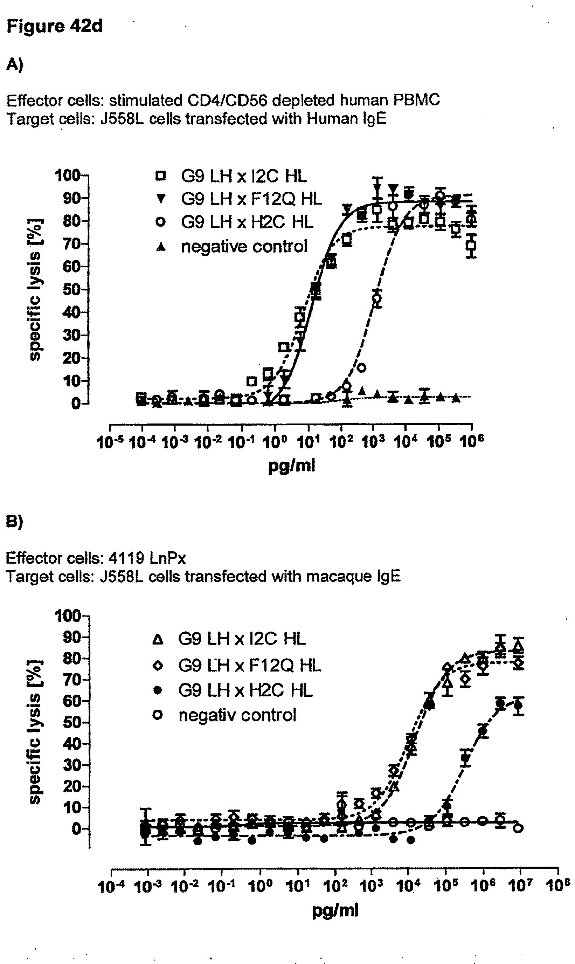

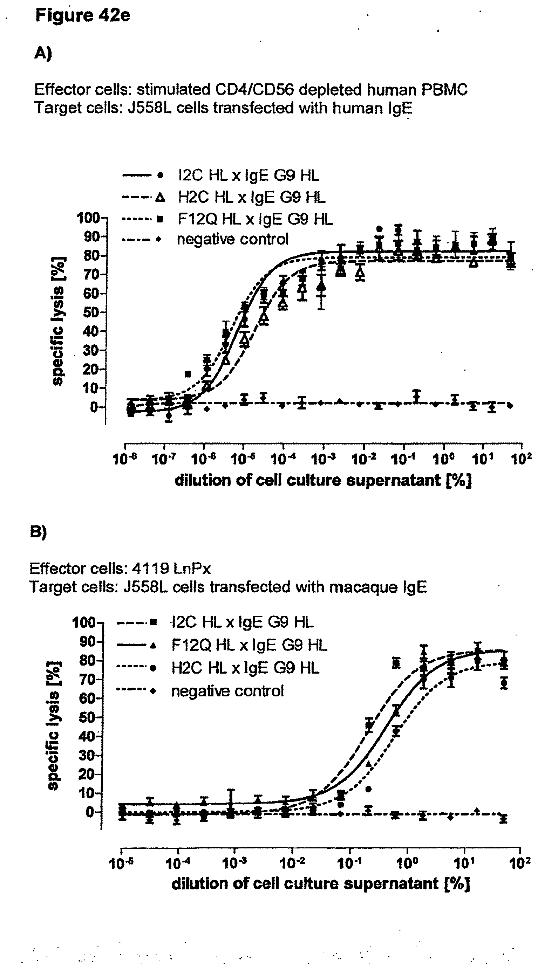

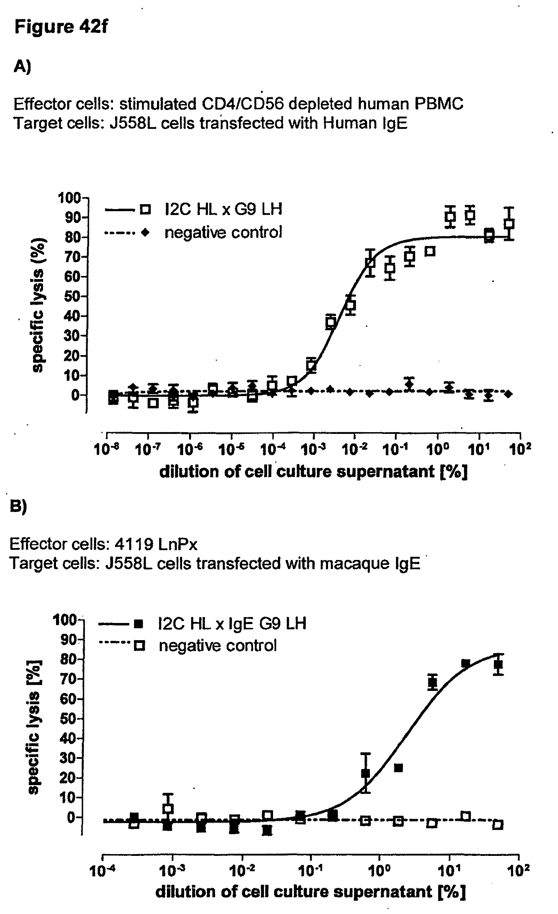

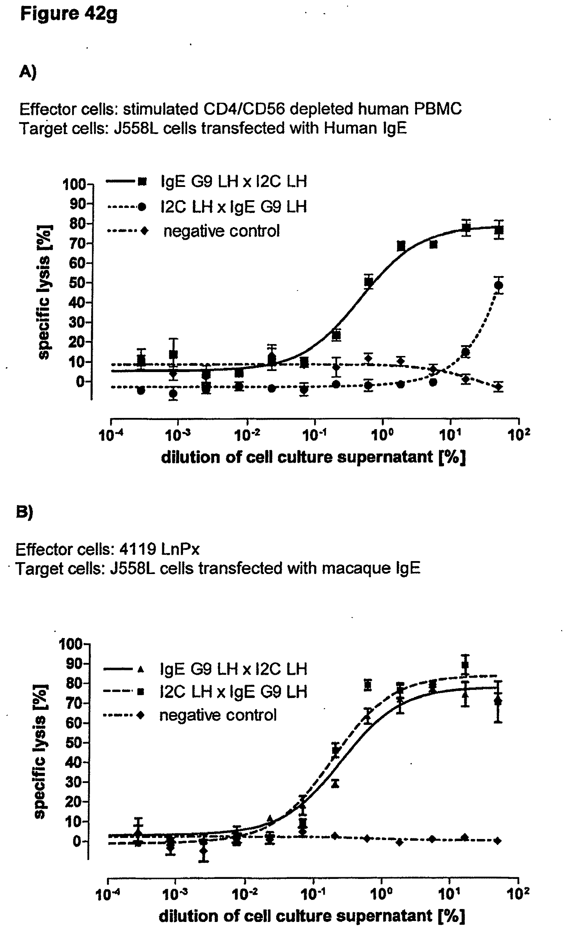

As shown in Figures 42, all of the generated cross-species specific bispecific single chain antibody constructs revealed cytotoxic activity against human IgE positive target cells elicited by human CD8+ cells and macaque IgE positive target cells elicited by the macaque T cell line 4119LnPx. As a negative control, an irrelevant bispecific single chain antibody has been used.

28. Generation and characterization of CD44 and CD3 cross-species specific bispecific single chain molecules

**28.1 Generation of CHO cells expressing human CD44**

The coding sequence of human CD44 as published in GenBank (Accession number AJ251595) and also containing the CD44v6 exon was obtained by gene synthesis according to standard protocols. The gene synthesis fragment was designed as to contain first a Kozak site for eukaryotic expression of the construct, followed by the coding sequence of the human CD44 protein and a stop codon (the cDNA and amino acid sequence of the construct is listed under SEQ ID Nos 608 and 609). The gene synthesis fragment was also designed as to introduce restriction sites at the beginning and at the end of the fragment. The introduced restriction sites, EcoRI at the 5' end and Sallat the 3' end, were utilized in the following cloning procedures. The gene synthesis fragment was cloned via EcoRI and Sall into a plasmid designated pEF-DHFR (pEF-DHFR was described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**28.2 Generation of CHO cells expressing human CD44 without the v6 exon**

The coding sequence of human CD44 as published in GenBank (Accession number AJ251595) deleted for the sequence coding the v6 exon was obtained by gene synthesis according to standard protocols. The gene synthesis fragment was designed as to contain first a Kozak site for eukaryotic expression of the construct, followed by the coding sequence of the human CD44 protein without the v6 exon and a stop codon (the cDNA and amino acid sequence of the construct is listed under SEQ ID Nos 610 and 611). The gene synthesis fragment was also designed as to introduce restriction sites at the beginning and at the end of the fragment. The introduced restriction sites, EcoRI at the 5' end and Sall at the 3' end, were utilized in the following cloning procedures. The gene synthesis fragment was cloned via EcoRI and Sall into a plasmid designated pEF-DHFR (pEF-DHFR is described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**28.3 Generation of CHO cells expressing macaque CD44**

The coding sequence of macaque CD44 as published in GenBank (Accession number XM_001115359) and also containing the CD44v6 exon was obtained by gene synthesis according to standard protocols (the cDNA and amino acid sequence of the construct is listed under SEQ ID Nos 612 and 613). In the utilized construct the coding sequence corresponds to macaque CD44 except for a point mutation at position 1 of the codon of the fifth amino acid of the signal peptide of the CD44 protein, which results in a mutation from arginine to tryptophan corresponding to the human sequence. The gene synthesis fragment was also designed as to contain a Kozak site for eukaryotic expression of the construct and restriction sites at the beginning and the end of the fragment. The introduced restriction sites EcoRI at the 5' end and Sall at the 3' end, were utilized in the following cloning procedures. The gene synthesis fragment was then cloned via EcoRI and Sail into a plasmid designated pEF-DHFR (pEF-DHFR is described in Raum et al. Cancer Immunol Immunother 50 (2001) 141-150). A sequence verified clone of this plasmid was used to transfect CHO/dhfr- cells as described above.

**28.4 Generation of CD44v6 and CD3 cross-species specific bispecific single chain molecules**

Generally, bispecific single chain antibody molecules, each comprising a domain with a binding specificity cross-species specific for human and non-chimpanzee primate CD3epsilon as well as a domain with a binding specificity cross-species specific for human and non-chimpanzee primate CD44v6, are designed as set out in the following Table 11:

Table 11: Formats of anti-CD3 and anti-CD44v6 cross-species specific bispecific single chain antibody molecules

| SEQ ID (nucl/prot) | Formats of protein constructs (N → C) |
|---|---|
| 1589/1588 | CD44-A8LHxI2C HL |
| 1587/1586 | CD44-A8HLxI2C HL |

The aforementioned constructs containing the variable light-chain (L) and variable heavy-chain (H) domains cross-species specific for human and macaque CD44 and the CD3 specific VH and VL combinations cross-species specific for human and macaque CD3 are obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a histidine$_6$-tag and a stop codon. The gene synthesis fragment was also designed as to introduce suitable restriction sites at the beginning and at the end of the fragment. The introduced restriction sites were utilized in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 28.5 Expression and purification of the bispecific single chain antibody molecules

The bispecific single chain antibody molecules were expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with ZnCl$_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight is determined with MultiMark protein standard (Invitrogen). The gel was stained with colloidal Coomassie (Invitrogen protocol). The purity of the isolated protein was >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. The antibodies used were directed against the histidine$_6$-tag (Penta His, Qiagen) and Goat-anti-mouse Ig labeled with alkaline phosphatase (AP) (Sigma), and BCIP/NBT (Sigma) as substrate. A single band was detected at 52 kD corresponding to the purified bispecific single chain antibody.

### 28.6 Flow cytometric binding analysis of the CD44 and CD3 cross-species specific bispecific antibodies

In order to test the functionality of the cross-species specific bispecific antibody constructs regarding the capability to bind to human and macaque CD44 and CD3, respectively, a FACS analysis was performed. For this purpose CHO cells transfected with human CD44 as described in Example 28.1 and the human CD3 positive T cell leukemia cell line HPB-ALL (DSMZ, Braunschweig, ACC483) were used to test the binding to human antigens. The binding reactivity to macaque antigens was tested by using the generated macaque CD44 transfectant described in Example 28.3 and macaque PBMC (preparation of macaque PBMC was performed by Ficoll gradient centrifugation of peripheral blood from macaque monkeys according to standard protocols). Specificity for the v6 exon of CD44 was tested by using the transfected CHO cells expressing human CD44 without the v6 exon generated as described in Example 28.2. 200.000 cells of the respective cell lines or macaque PBMC were incubated for 30 min. on ice with 50 μl of the purified protein of the cross-species specific bispecific antibody constructs (5 μg/ml) or 50 μl of a commercially available anti-CD44 antibody (Becton Dickinson biosciences, Heidelberg; also 5 μg/ml). The cells were washed twice in PBS with 2% FCS and binding of the construct was detected with a murine anti-His antibody (Penta His antibody; Qiagen; diluted 1:20 in 50 μl PBS with 2% FCS). The anti-His antibody was omitted for the samples incubated with the commercially available anti-CD44 antibody. After washing, bound anti-His antibody or anti-CD44 antibody was detected with an Fc gamma-specific antibody (Dianova) conjugated to phycoerythrin, diluted 1:100 in PBS with 2% FCS. PBS with 2% FCS was used as negative control.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

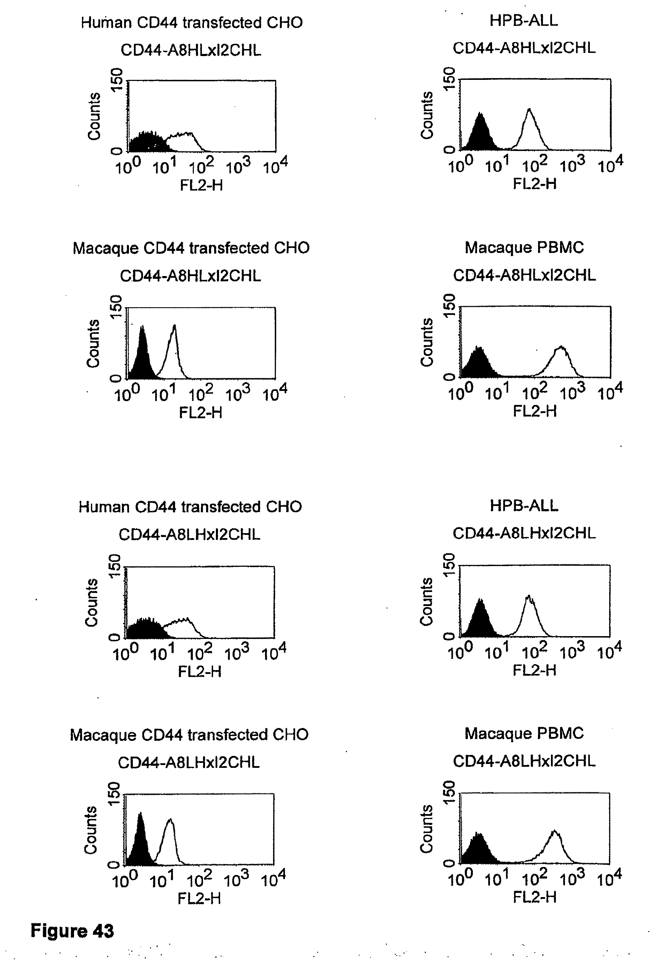

The bispecific binding of the single chain molecules listed above, which were cross-species specific for CD44 and cross-species specific for human and non-chimpanzee primate CD3 was clearly detectable as shown in Figure 43. In the FACS analysis all constructs showed binding to CD3 and CD44 as compared to the respective negative controls. Cross-species specificity of the bispecific antibodies to human and macaque CD3 and CD44 antigens was demonstrated.

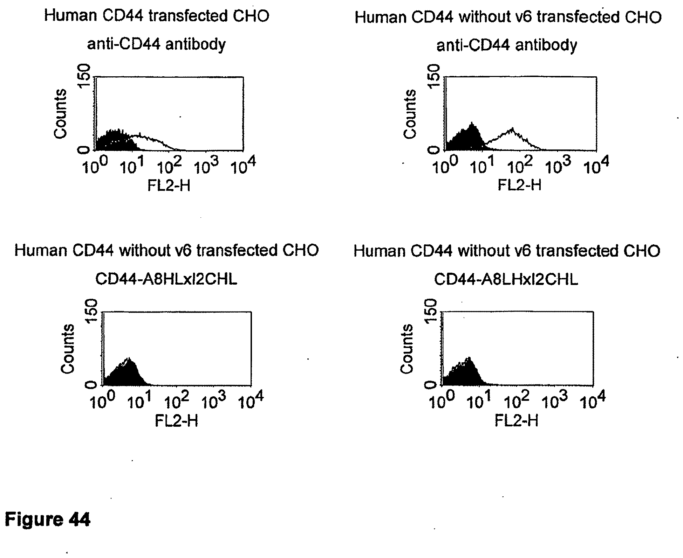

Specificity for the v6 exon of CD44 was demonstrated in Figure 44 by lack of binding of the constructs to CHO cells transfected with human CD44 lacking the v6 exon. Expression of CD44 by these cells was demonstrated by comparable binding of the commercially available anti-CD44 antibody to those cells and to the CHO cells transfected with the full length human CD44.

### 28.7 Bioactivity of CD44 and CD3 cross-species specific bispecific single chain antibodies

Bioactivity of the generated bispecific single chain antibodies was analyzed by chromium 51 ($^{51}$Cr) release in vitro cytotoxicity assays using the CD44 positive cell line described in Example 28.1. As effector cells stimulated human CD4/CD56 depleted PBMC were used.

Generation of stimulated human PBMC was performed as follows:

A Petri dish (145 mm diameter, Greiner) was coated with a commercially available anti-CD3 specific antibody (e.g. OKT3, Orthoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

By depletion of CD4+ T cells and CD56+ NK cells according to standard protocols CD8+ cytotoxic T lymphocytes (CTLs) were enriched.

Target cells were washed twice with PBS and labeled with 11.1 MBq $^{51}$Cr in a final volume of 100 μl RPMI with 50% FCS for 45 minutes at 37°C. Subsequently the labeled target cells were washed 3 times with 5 ml RPMI and then used in the cytotoxicity assay. The assay was performed in a 96 well plate in a total volume of 250 μl supplemented RPMI (as above) with an E:T ratio of 10:1. 1 μg/ml of the cross-species specific bispecific single chain antibody molecules and 20 threefold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

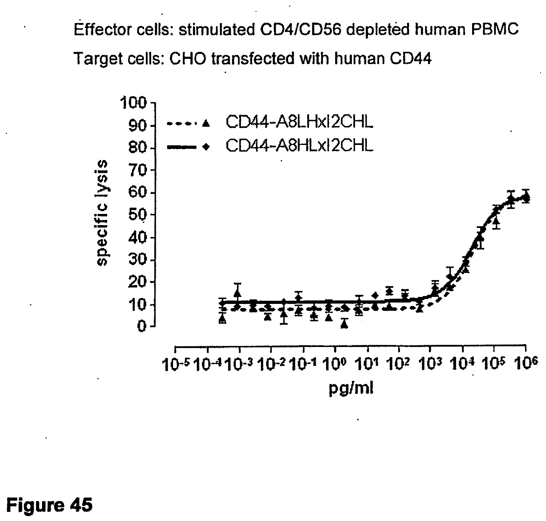

As shown in Figure 45 the generated cross-species specific bispecific single chain antibody constructs demonstrated cytotoxic activity against human CD44 positive target cells elicited by stimulated human CD4/CD56 depleted PBMC.

## 29. Generation and characterization of CD30 and CD3 cross-species specific bispecific single chain molecules

### 29.1 Generation of CHO cells expressing human CD30

The coding sequence of human CD30 as published in GenBank (Accession number M83554) was obtained by gene synthesis according to standard protocols. The gene synthesis fragment was designed as to contain first a Kozak site for eukaryotic expression of the construct, followed by a 19 amino acid immunoglobulin leader peptide, followed in frame by the coding sequence of the mature human CD30 protein and a stop codon (the cDNA and amino acid sequence of the construct is listed under SEQ ID Nos 1103 and 1104). The gene synthesis fragment was also designed as to introduce restriction sites at the beginning and at the end of the fragment. The introduced restriction sites, EcoRI at the 5' end and SalI at the 3' end, were utilised in the following cloning procedures. Internal restriction sites were removed by silent mutation of the coding sequence in the gene synthesis fragment (BsrGI: nucleotide 243 from T to C; SalI: nucleotide 327 from A to G; SalI: nucleotide 852 from A to G; EcoRI: nucleotide 1248 from T to C). The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 29.2 Generation of CHO cells expressing macaque CD30

The cDNA sequence of macaque CD30 was obtained by a set of 4 PCRs on cDNA from macaque monkey bone marrow prepared according to standard protocols. The following reaction conditions: 1 cycle at 94°C for 2 minutes followed by 35 cycles with 94°C for 1 minute, 55°C for 1 minute and 72°C for 1 minute followed by a terminal cycle of 72°C for 3 minutes and the following primers were used:

6. forward primer: 5'-cctcgccgcgctgggactgc-3'
reverse primer: 5'-ggtgccactggagggttccttgc-3'
7. forward primer: 5'-gcftcftccaftctgtctgcccagcagg-3'
reverse primer: 5'-ggtaggggacacagcgggcacaggagttgg-3'
8. forward primer: 5'-cctggcatgatctgtgccacatcagcc-3'
reverse primer: 5'-gcgttgagctcctcctgggtctgg-3'
9. forward primer: 5'-cctcccrgcccaagctagagcttgtgg-3'
reverse primer: 5'-cgactctagagcggccgctcactttccagaggcagctgtgggcaaggggtcttctttcccttcc-3'

The PCR reactions were performed under addition of PCR grade betain to a final concentration of 1M. Those PCRs generate four overlapping fragments, which were isolated and sequenced according to standard protocols using the PCR primers, and thereby provide a portion of the cDNA sequence coding macaque CD30 from codon 12 of the leader peptide to codon 562 of the mature protein. To generate a construct for expression of macaque CD30 a cDNA fragment was obtained by gene synthesis according to standard protocols (the cDNA and amino acid sequence of the construct is listed under SEQ ID Nos 1105 and 1106). In this construct the coding sequence for macaque CD30 from amino acid 12 of the leader peptide to amino acid 562 of the mature CD30 protein was fused into the coding sequence of human CD30 replacing the respective part of the human coding sequence. The gene synthesis fragment was also designed as to contain a Kozak site for eukaryotic expression of the construct and restriction sites at the beginning and the end of the fragment containing the cDNA coding for the whole extracellular domain of macaque CD30, the macaque CD30 transmembrane domain and a macaque-human chimeric intracellular CD30 domain. The introduced restriction sites Xbal at the 5' end and Sall at the 3' end, were utilized in the following cloning procedures. The gene synthesis fragment was then cloned via Xbal and Sall into a plasmid designated pEF-DHFR (pEF-DHFR is described in Raum et al. Cancer Immunol Immunother 50 (2001) 141-150). A sequence verified clone of this plasmid was used to transfect CHO/dhfr- cells as described above.

### 29.3 Generation of CD30 and CD3 cross-species specific bispecific single chain molecules

**Cloning of cross-species specific binding molecules**

Generally, bispecific single chain antibody molecules, each comprising a domain with a binding specificity cross-species specific for human and non-chimpanzee primate CD3epsilon as well as a domain with a binding specificity cross-species specific for human and non-chimpanzee primate CD30, were designed as set out in the following Table 12:

Table 12: Formats of anti-CD3 and anti-CD30 cross-species specific bispecific single chain antibody molecules

| SEQ ID (nucl/prot) | Formats of protein constructs (N → C) |
|---|---|
| 1126/1125 | M911HLxI2CHL |
| 1139/1138 | SR3HLxI2CHL |

The aforementioned constructs containing the variable light-chain (L) and variable heavy-chain (H) domains cross-species specific for human and macaque CD30 and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilised in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 29.4 Expression and purification of the bispecific single chain antibody molecules

The bispecific single chain antibody molecules were expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following procedure:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein was >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. The antibodies used were directed against the His Tag (Penta His, Qiagen) and Goat-anti-mouse Ig labeled with alkaline phosphatase (AP) (Sigma), and BCIP/NBT (Sigma) as substrate. A single band was detected at 52 kD corresponding to the purified bispecific single chain antibody.

**29.5 Flow cytometric binding analysis of the CD30 and CD3 cross-species specific bispecific antibodies**

In order to test the functionality of the cross-species specific bispecific antibody constructs regarding the capability to bind to human and macaque CD30 and CD3, respectively, a FACS analysis was performed. For this purpose CHO cells transfected with human CD30 as described in Example 29.1, the human CD30 positive B cell line MEC-1 (DSMZ, Braunschweig, ACC 497) and the human CD3 positive T cell leukemia cell line HPB-ALL (DSMZ, Braunschweig, ACC483) were used to test the binding to human antigens. The binding reactivity to macaque antigens was tested by using the generated macaque CD30 transfectant described in Example 29.2 and a macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of the purified protein of the cross-species specific bispecific antibody constructs (5 μg/ml). The cells were washed twice in PBS with 2% FCS and binding of the construct was detected with a murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in PBS with 2% FCS. PBS with 2% FCS was used as a negative control.

Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

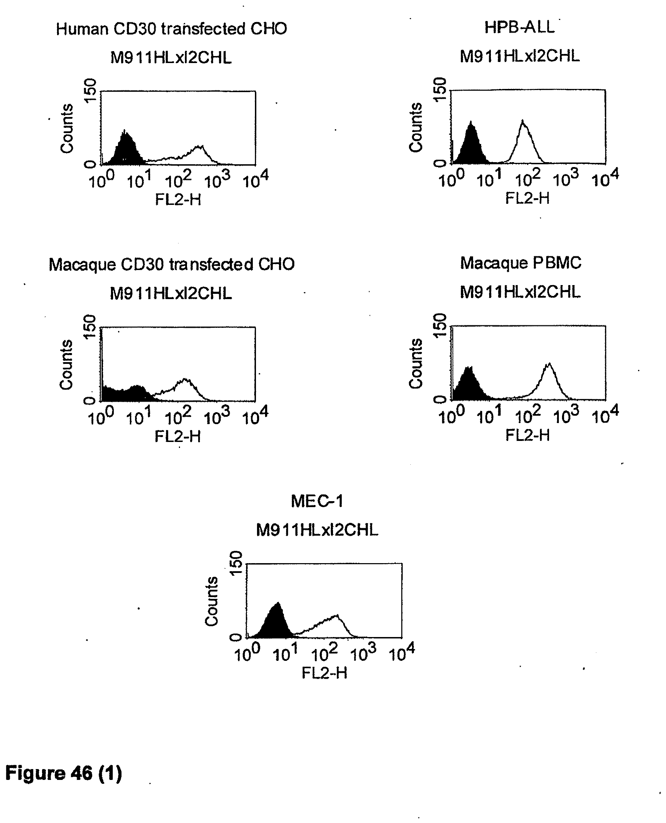

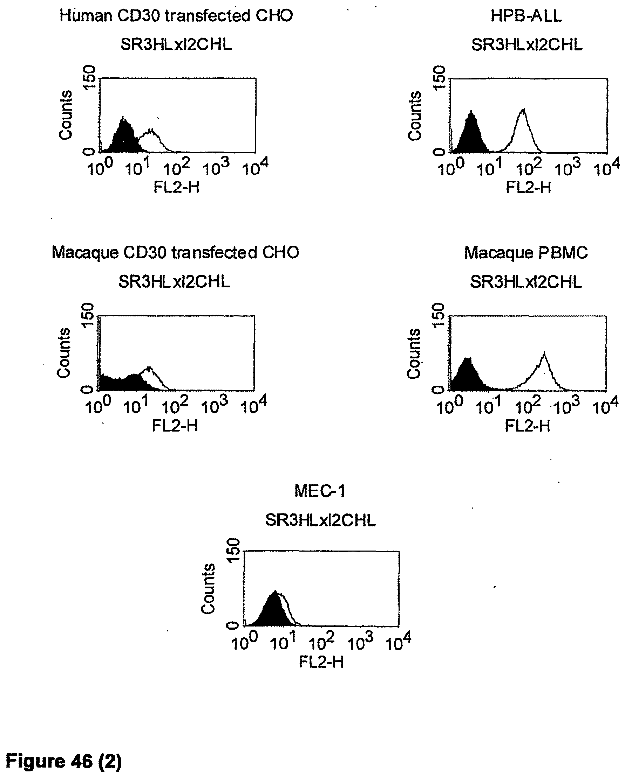

The bispecific binding of the single chain molecules listed above, which are cross-species specific for CD30 and cross-species specific for human and non-chimpanzee primate CD3 was clearly detectable as shown in Figure 46. In the FACS analysis all constructs showed binding to CD3 and CD30 compared to the negative control. Cross-species specificity of the bispecific antibodies to human and macaque CD3 and CD30 antigens was demonstrated.

**29.6 Bioactivity of CD30 and CD3 cross-species specific bispecific single chain antibodies**

Bioactivity of the generated bispecific single chain antibodies was analyzed by chromium 51 ($^{51}$Cr) release in vitro cytotoxicity assays using the human CD30 positive B cell line MEC-1 (DSMZ, Braunschweig, ACC 497) and the CHO cells transfected with macaque CD30 described in Example 29.2. As effector cells stimulated human CD4/CD56 depleted PBMC or the macaque T cell line 4119LnPx were used, respectively.

Generation of stimulated human PBMC was performed as follows:

A Petri dish (145 mm diameter, Greiner bio-one GmbH, Kremsmünster) was coated with a commercially available anti-CD3 specific antibody (e.g. OKT3, Orthoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. $3 - 5 \times 10^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}$Cr in a final volume of 100μl RPMI with 50% FCS for 60 minutes at 37°C. Subsequently the labelled target cells were washed 3 times with 5 ml RPMI and then used in the cytotoxicity assay. The assay was performed in a 96 well plate in a total volume of 250μl supplemented RPMI (as above) with an E:T ratio of 10:1. 1 μg/ml of the cross-species specific bispecific single chain antibody molecules and 20 threefold dilutions thereof were applied. The assay time was 4 hours for the assay using MEC-1 and human CD4/CD56 depleted PBMC and 18 hours for the assay using the macaque CD30 transfected CHO and the macaque T cell line 4119LnPx. Cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gammacounter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have R$^2$ values >0.90 as determined by the software. EC$_{50}$ values calculated by the analysis program were used for comparison of bioactivity.

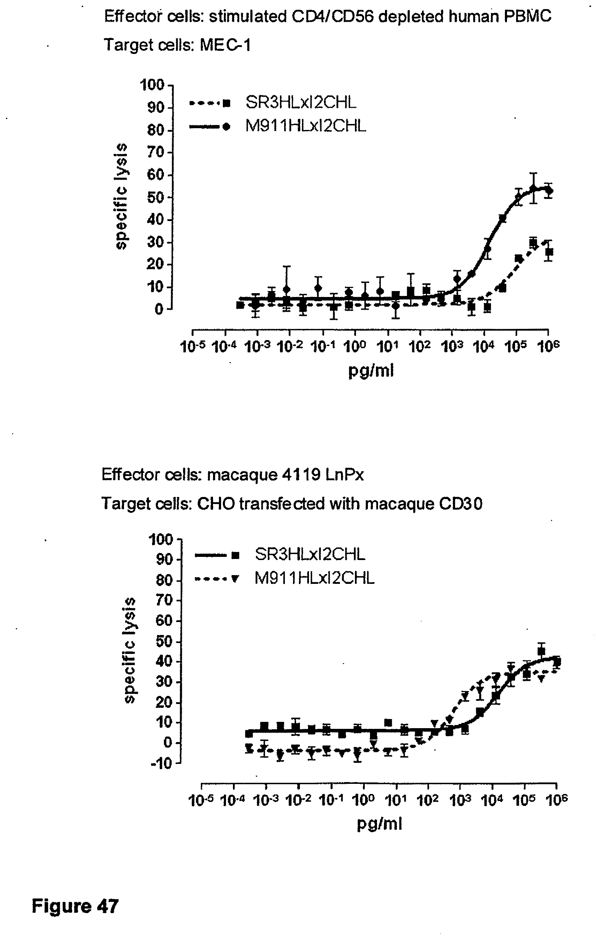

As shown in Figure 47 all of the generated cross-species specific bispecific single chain antibody constructs demonstrated cytotoxic activity against human CD30 positive target cells elicited by stimulated human CD4/CD56 depleted PBMC and macaque CD30 positive target cells elicited by the macaque T cell line 4119LnPx.

## 30. Generation and characterization of HER2 and CD3 cross-species specific bispecific single chain molecules

### 30.1 Generation of CHO cells expressing human HER2

The coding sequence of human HER2 as published in GenBank (Accession number X03363) was obtained by gene synthesis according to standard protocols. The gene synthesis fragment was designed as to contain the coding sequence of the human HER2 protein including its leader peptide (the cDNA and amino acid sequence of the construct is listed under SEQ ID Nos 1107 and 1108. The gene synthesis fragment was also designed as to introduce restriction sites at the beginning and at the end of the fragment. The introduced restriction sites, XbaI at the 5' end and SalI at the 3' end, were utilised in the following cloning procedures. The gene synthesis fragment was cloned via XbaI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 30.2 Generation of CHO cells expressing the extracellular domain of macaque HER2

The coding sequence of human HER2 as described above was modified to encode the amino acids 123 to 1038 of the macaque HER2 protein as published in GenBank (Accession number XP_001090430). The coding sequence for this chimeric protein was obtained by gene synthesis according to standard protocols (the cDNA and amino acid sequence of the construct is listed under SEQ ID Nos 1109 and 1110). The gene synthesis fragment was also designed as to contain a Kozak site for eukaryotic expression of the construct and restriction sites at the beginning and the end of the fragment. The introduced restriction sites XbaI at the 5' end and SalI at the 3' end, were utilized in the following cloning procedures. The gene synthesis fragment was then cloned via XbaI and SalI into a plasmid designated pEF-DHFR (pEF-DHFR is described in Raum et al. Cancer Immunol Immunother 50 (2001) 141-150). A sequence verified clone of this plasmid was used to transfect CHO/dhfr- cells as described above.

### 30.3 Generation of HER2 and CD3 cross-species specific bispecific single chain molecules

### Cloning of cross-species specific binding molecules

Generally, bispecific single chain antibody molecules, each comprising a domain with a binding specificity cross-species specific for human and non-chimpanzee primate CD3epsilon as well as a domain with a binding specificity cross-species specific for human and macaque HER2, are designed as set out in the following Table 13:

Table 13: Formats of anti-CD3 and anti-HER2 cross-species specific bispecific single chain antibody molecules

| SEQ ID (nucl/prot) | Formats of protein constructs (N → C) |
|---|---|
| 1153/1152 | 2D3 HL × I2C HL |
| 1167/1166 | 3E1 HL × I2C HL |

The aforementioned constructs containing the variable light-chain (L) and variable heavy-chain (H) domains cross-species specific for human and macaque HER2 and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable restriction sites at the beginning and at the end of the fragment. The introduced restriction sites were utilised in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

**30.4 Expression and purification of the bispecific single chain antibody molecules**

The bispecific single chain antibody molecules were expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells are grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -80°C. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein was >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

**30.5 Flow cytometric binding analysis of the HER2 and CD3 cross-species specific bispecific antibodies**

In order to test the functionality of the cross-species specific bispecific antibody constructs regarding the capability to bind to human and macaque HER2 and CD3, respectively, a FACS analysis was performed. For this purpose CHO cells transfected with human HER2 as described in Example 30.1 and the human CD3 positive T cell leukemia cell line HPB-ALL (DSMZ, Braunschweig, ACC483) are used to test the binding to human antigens. The binding reactivity to macaque antigens was tested by using the generated macaque HER2 transfectant described in Example 30.2 and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of the purified protein of the cross-species specific bispecific antibody constructs (2 μg/ml). The cells were washed twice in PBS with 2% FCS and binding of the construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. PBS with 2% FCS was used as negative control for binding to the T cell lines as well as to the HER2 transfected CHO cells.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

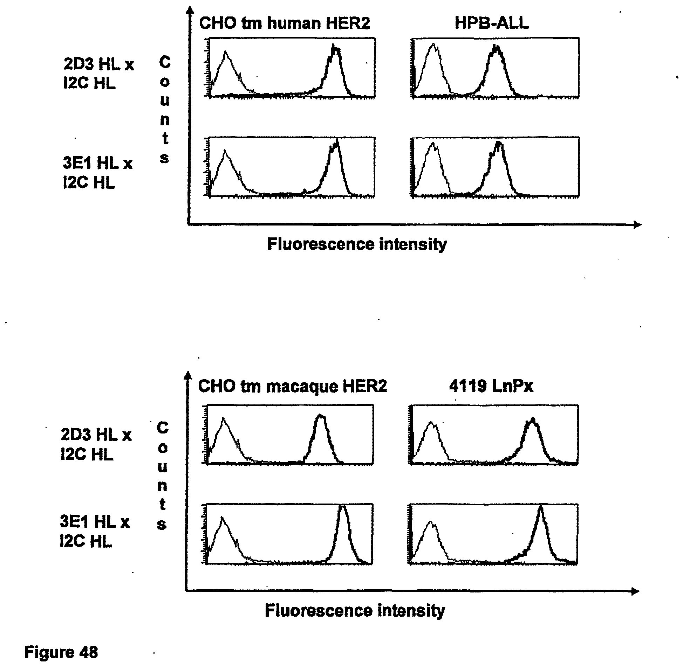

The bispecific binding of the single chain molecules listed above, which are cross-species specific for HER2 and cross-species specific for human and non-chimpanzee primate CD3 was clearly detectable as shown in Figure 48. In the FACS analysis all constructs showed binding to CD3 and HER2 as compared to the respective negative controls. Cross-species specificity of the bispecific antibodies to human and macaque CD3 and HER2 antigens was demonstrated.

**30.6 Bioactivity of HER2 and CD3 cross-species specific bispecific single chain antibodies**

Bioactivity of the generated bispecific single chain antibodies was analyzed by chromium 51 ($^{51}$Cr) release in vitro cytotoxicity assays using the HER2 positive cell lines described in Examples 30.1 and 30.2. As effector cells unstimulated human CD56 depleted PBMC or the macaque T cell line 4119LnPx were used as specified in the respective figures.

Generation of unstimulated human CD56 depleted PBMC was performed as follows: The fresh PBMC were isolated from peripheral blood (30 - 50 ml human blood) by Ficoll gradient centrifugation according to standard protocols. Depletion of CD56+ NK cells was carried out according to standard protocols.

Target cells were washed twice with PBS and labelled with 11.1 MBq $^{51}$Cr in a final volume of 100 μl RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250μl supplemented RPMI (as above) with an E:T ratio of 10:1. 1 μg/ml of the cross-species specific bispecific single chain antibody molecules and 15 - 21 fivefold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

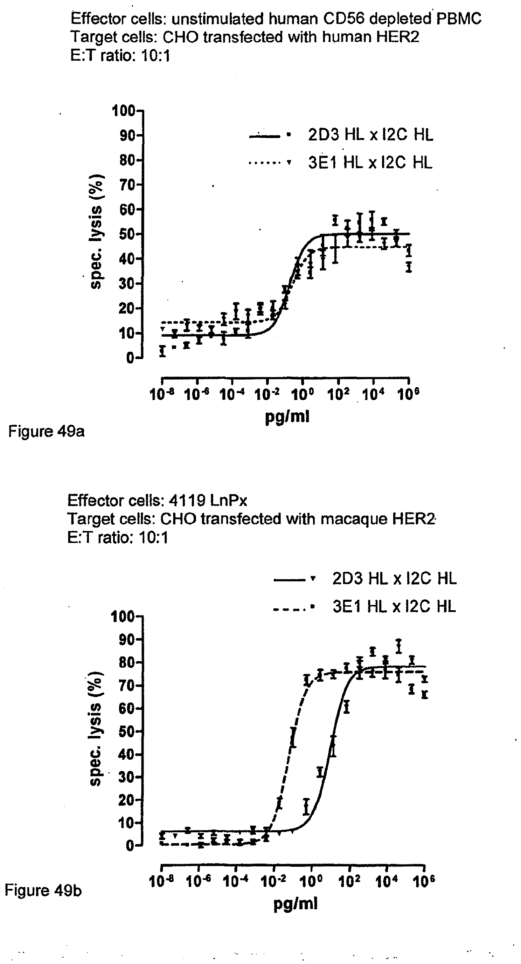

As shown in Figure 49, all of the generated cross-species specific bispecific single chain antibody constructs demonstrated cytotoxic activity against human HER2 positive target cells elicited by unstimulated CD56 depleted PBMC and against macaque HER2 positive target cells elicited by the macaque T cell line 4119LnPx.

**31. Purification of cross-species specific bispecific single chain molecules by an affinity procedure based on the context independent CD3 epsilon epitope corresponding to the N-terminal amino acids 1-27**

**31.1 Generation of an affinity column displaying** the **isolated context independent human CD3 epsilon epitope corresponding to the N-terminal amino acids 1-27**

The plasmid for expression of the construct 1-27 CD3-Fc consisting of the 1-27 N-terminal amino acids of the human CD3 epsilon chain fused to the hinge and Fc gamma region of human immunoglobulin IgG1 described above (Example 3; cDNA sequence and amino acid sequence of the recombinant fusion protein are listed under SEQ ID NOs 350 and 349) was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. For the isolation of the fusion protein a goat anti-human fc affinity column was prepared according to standard protocols using a commercially available affinity purified goat anti-human IgG fc fragment specific antibody with minimal cross-reaction to bovine, horse, and mouse serum proteins (Jackson ImmunoResearch Europe Ltd.). Using this affinity column the fusion protein was isolated out of cell culture supernatant on an Äkta Explorer System (GE Amersham) and eluted by citric acid. The eluate was neutralized and concentrated. After dialysis against amine free coupling buffer the purified fusion protein was coupled to an N-Hydroxy-Succinimide NHS activated 1 ml HiTrap column (GE Amersham).

After coupling remaining NHS groups were blocked and the column was washed and stored at 5°C in storage buffer containing 0.1% sodium azide.

**31.2 Purification of cross-species specific bispecific single chain molecules using a human CD3 peptide affinity column**

200 ml cell culture supernatant of cells expressing cross-species specific bispecific single chain molecules were 0.2 μm sterile filtered and applied to the CD3 peptide affinity column using an Äkta Explorer system (GE Amersham).

The column was then washed with phosphate buffered saline PBS pH 7.4 to wash out unbound sample. Elution was done with an acidic buffer pH 3.0 containing 20 mM Citric acid and 1 M sodium chloride. Eluted protein was neutralized immediately by 1 M Trishydroxymethylamine TRIS pH 8.3 contained in the collection tubes of the fraction collector.

Protein analysis was done by SDS PAGE and Western Blot.

For SDS PAGE BisTris Gels 4-12% are used (Invitrogen). The running buffer was 1 × MES-SDS-Puffer (Invitrogen). As protein standard 15 μl prestained Sharp Protein Standard (Invitrogen) was applied. Electrophoresis was performed for 60 minutes at 200 volts 120 mA max. Gels were washed in demineralised water and stained with Coomassie for one hour. Gels are destained in demineralised water for 3 hours. Pictures are taken with a Syngene Gel documentation system.

For Western Blot a double of the SDS PAGE gel was generated and proteins were electroblotted onto a nitrocellulose membrane. The membrane was blocked with 2% bovine serum albumin in PBS and incubated with a biotinylated murine Penta His antibody (Qiagen). As secondary reagent a streptavidin alkaline phosphatase conjugate (DAKO) was used. Blots were developed with BCIP/NBT substrate solution (Pierce).

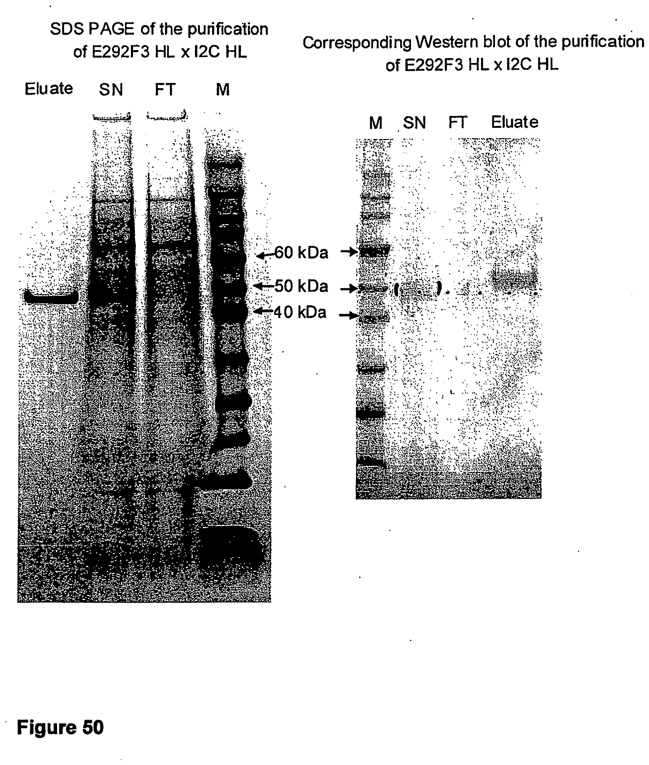

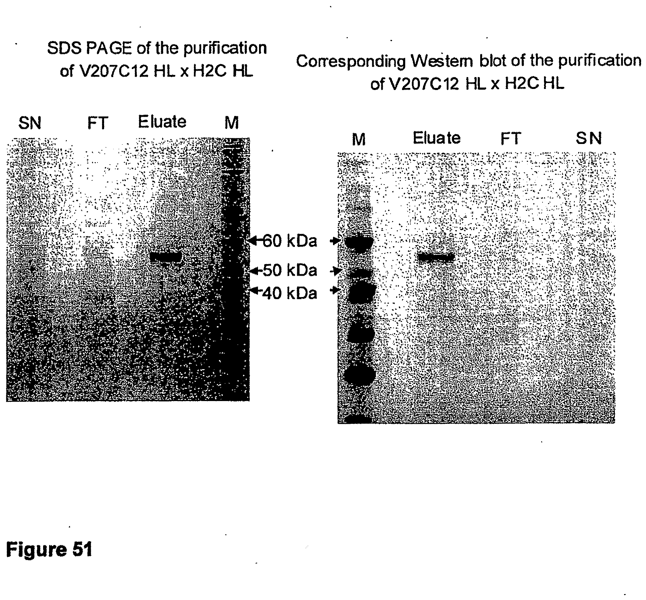

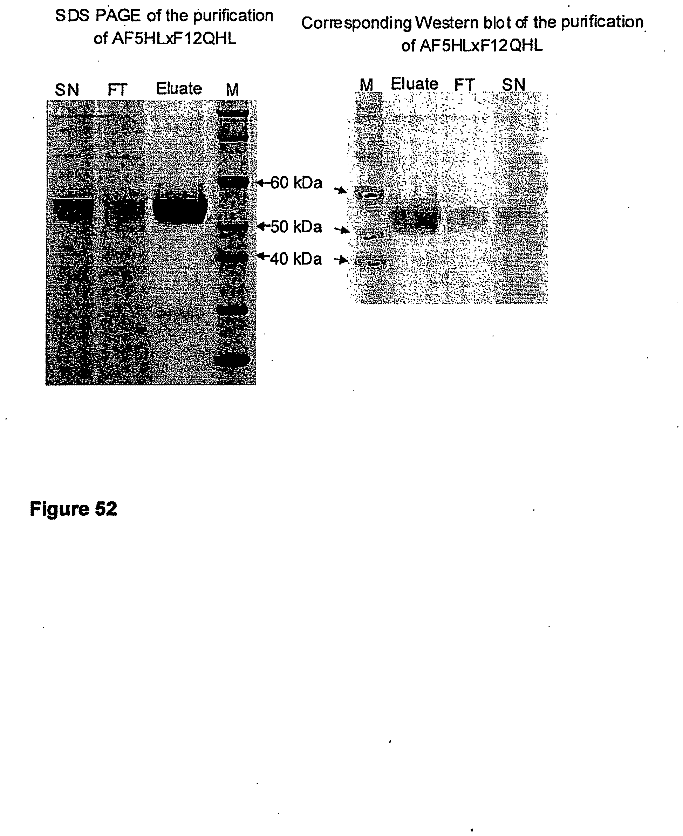

As demonstrated in Figures 50, 51 and 52 the use of a human CD3 peptide affinity column as described above allows the highly efficient purification of the bispecific single chain molecules from cell culture supernatant. The cross-species specific anti-CD3 single chain antibodies contained in the bispecific single chain molecules therefore enable via their specific binding properties an efficient generic one-step method of purification for the cross-species specific bispecific single chain molecules, without the need of any tags solely attached for purification purposes.

**32. Generic pharmacokinetic assay for cross-species specific bispecific single chain molecules**

**32.1 Production of 1-27 CD3-Fc for use in the pharmacokinetic assay**

The coding sequence of the 1-27 N-terminal amino acids of the human CD3 epsilon chain fused to the hinge and Fc gamma region of human immunoglobulin IgG1 was obtained by gene synthesis according to standard protocols (cDNA sequence and amino acid sequence of the recombinant fusion protein are listed under SEQ ID NOs 1111 and 1112). The gene synthesis fragment was designed as to contain first a Kozak site for eukaryotic expression of the construct, followed by a 19 amino acid immunoglobulin leader peptide, followed in frame by the coding sequence of the first 27 amino acids of the extracellular portion of the mature human CD3 epsilon chain, followed in frame by the coding sequence of the hinge region and Fc gamma portion of human IgG1 and a stop codon. The gene synthesis fragment was also designed as to introduce restriction sites at the beginning and at the end of the cDNA coding for the fusion protein. The introduced restriction sites, EcoRI at the 5' end and SalI at the 3' end, were utilised in the following cloning procedures. The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. For the isolation of the fusion protein a goat anti-human fc affinity column was prepared according to standard protocols using a commercially available affinity purified goat anti-human IgG fc fragment specific antibody with minimal cross-reaction to bovine, horse, and mouse serum proteins (Jackson ImmunoResearch Europe Ltd.). Using this affinity column the fusion protein was isolated out of cell culture supernatant on an Äkta Explorer System (GE Amersham) and eluted by citric acid. The eluate was neutralized and concentrated.

**32.2 Pharmacokinetic assay for cross-species specific bispecific single chain molecules**

The assay is based on the ECL-ELISA technology using ruthenium labelled detection on carbon plates measured on a Sektor Imager device (MSD). In a first step, carbon plates (MSD High Bind Plate 96 well Cat: L15xB-3) were coated with 5 μl/well at 50 ng/ml of the purified 1-27 CD3-Fc described in Example 32.1. The plate was then dried overnight at 25°C. Subsequently plates were blocked with 5% BSA (Paesel&Lorei #100568) in PBS at 150 μl/well for 1h at 25°C in an incubator while shaking (700 rpm). In the next step plates were washed three times with 0.05% Tween in PBS. A standard curve in 50% macaque serum in PBS was generated by serial 1:4 dilution starting at 100 ng/ml of the respective cross-species specific bispecific single chain molecule to be detected in the assay. Quality control (QC) samples were prepared in 50% macaque serum in PBS ranging from 1 ng/ml to 50 ng/ml of the respective cross-species specific bispecific single chain molecule dependent on the expected sample serum concentrations. Standard, QC or unknown samples were transferred to the carbon plates at 10 μl/well and incubated for 90 min at 25°C in the incubator while shaking (700 rpm). Subsequently plates were washed three times with 0.05% Tween in PBS. For detection 25 μl/well of penta-His-Biotin antibody (Qiagen, 200 μg/ml in 0.05% Tween in PBS) was added and incubated for 1 h at 25°C in an incubator while shaking (700 rpm). In a second detection step 25 μl/well Streptavidin-SulfoTag solution (MSD; Cat: R32AD-1; Lot: W0010903) was added and incubated for 1 h at 25°C in an incubator while shaking (700 rpm). Subsequently plates were washed three times with 0.05% Tween in PBS. Finally 150 μl/well MSD Reading Buffer (MSD, Cat: R9ZC-1) was added and plates were read in the Sektor Imager device.

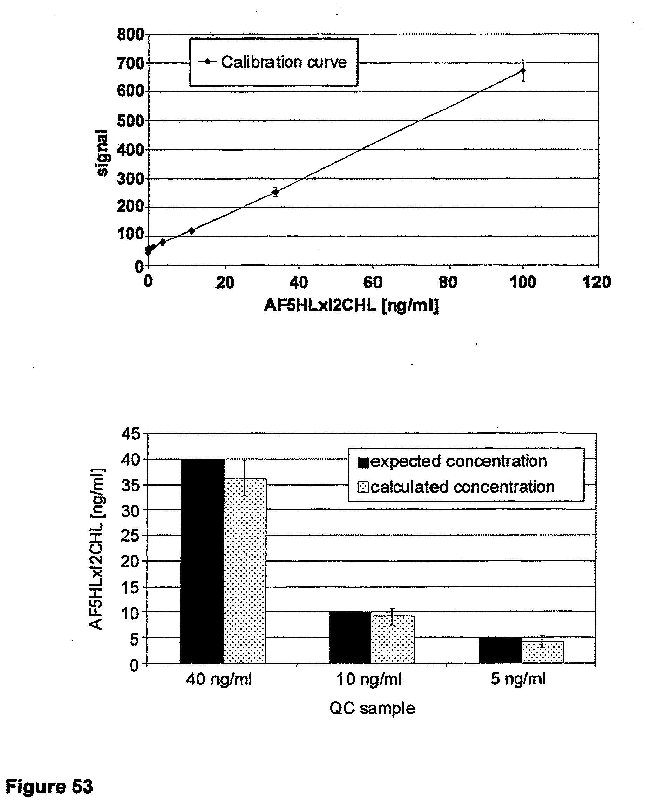

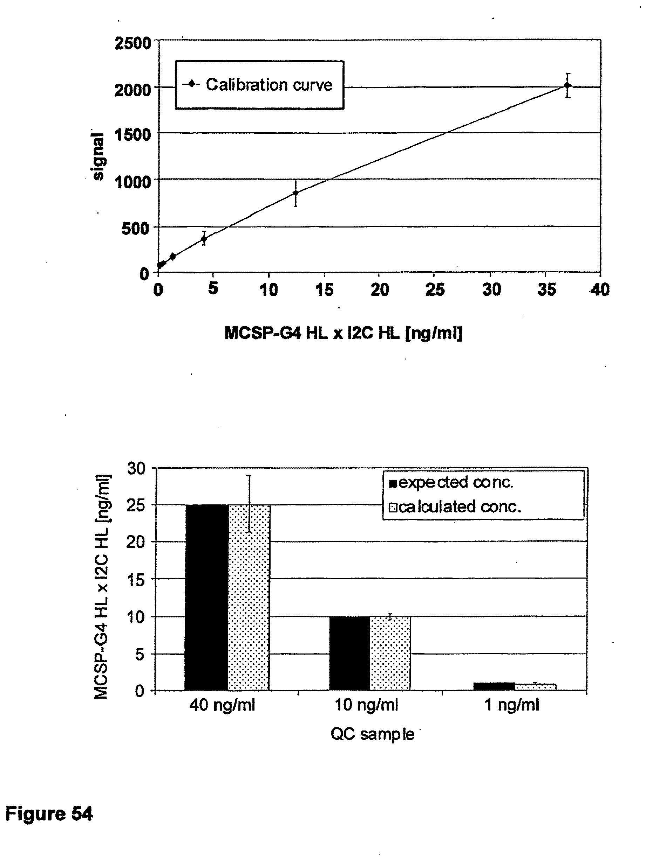

Figures 53 and 54 demonstrate the feasibility of detection of cross-species specific bispecific single chain molecules in serum samples of macaque monkeys for cross-species specific bispecific single chain molecules. The cross-species specific anti-CD3 single chain antibodies contained in the bispecific single chain molecules enable therefore via their specific binding properties a highly sensitive generic assay for detection of the cross-species specific bispecific single chain molecules. The assay set out above can be used in the context of formal toxicological studies that are needed for drug development and can be easily adapted for measurement of patient samples in connection with the clinical application of cross-species specific bispecific single chain molecules.

**33. Generation of recombinant transmembrane fusion proteins of the N-terminal amino acids 1-27 of CD3 epsilon from different non-chimpanzee primates fused to EpCAM from cynomolgus monkey (1-27 CD3-EpCAM).**

**33.1 Cloning and expression of 1-27 CD3-EpCAM**

CD3 epsilon was isolated from different non-chimpanzee primates (marmoset, tamarin, squirrel monkey) and swine. The coding sequences of the 1-27 N-terminal amino acids of CD3 epsilon chain of the mature human, common marmoset (Callithrix jacchus), cottontop tamarin (Saguinus oedipus), common squirrel monkey (Saimiri sciureus) and domestic swine (Sus scrofa; used as negative control) fused to the N-terminus of Flag tagged cynomolgus EpCAM were obtained by gene synthesis according to standard protocols (cDNA sequence and amino acid sequence of the recombinant fusion proteins are listed under SEQ ID NOs 351 to 360). The gene synthesis fragments were designed as to contain first a BsrGI site to allow for fusion in correct reading frame with the coding sequence of a 19 amino acid immunoglobulin leader peptide already present in the target expression vector, which was followed in frame by the coding sequence of the N-terminal 1-27 amino acids of the extracellular portion of the mature CD3 epsilon chains, which was followed in frame by the coding sequence of a Flag tag and followed in frame by the coding sequence of the mature cynomolgus EpCAM transmembrane protein. The gene synthesis fragments were also designed to introduce a restriction site at the end of the cDNA coding for the fusion protein. The introduced restriction sites BsrGI at the 5' end and SalI at the 3' end, were utilized in the following cloning procedures. The gene synthesis fragments were then cloned via BsrGI and SalI into a derivative of the plasmid designated pEF-DHFR (pEF-DHFR is described in Raum et al. Cancer Immunol Immunother 50 (2001) 141-150), which already contains the coding sequence of the 19 amino acid immunoglobulin leader peptide following standard protocols. Sequence verified plasmids were used to transfect DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

Transfectants were tested for cell surface expression of the recombinant transmembrane protein via an FACS assay according to standard protocols. For that purpose a number of $2.5 \times 10^5$ cells were incubated with 50 $\mu$l of the anti-Flag M2 antibody (Sigma-Aldrich Chemie GmbH, Taufkirchen, Germany) at 5 $\mu$g/ml in PBS with 2% FCS. Bound antibody was detected with an R-Phycoerythrin-conjugated affinity purified F(ab')2 fragment, goat anti-mouse IgG, Fc-gamma fragment specific 1:100 in PBS with 2% FCS (Jackson ImmunoResearch Europe Ltd., Newmarket, Suffolk, UK). Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

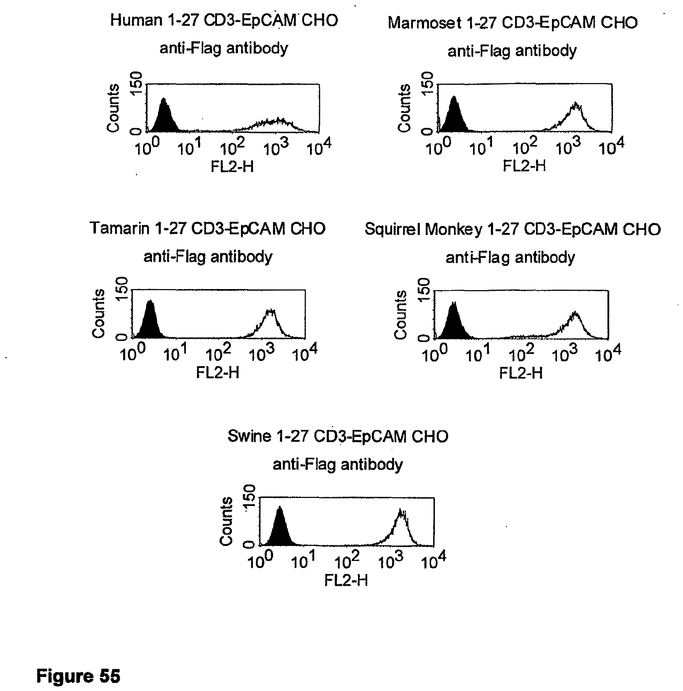

Expression of the Flag tagged recombinant transmembrane fusion proteins consisting of cynomolgus EpCAM and the 1-27 N-terminal amino acids of the human, marmoset, tamarin, squirrel monkey and swine CD3 epsilon chain respectively on transfected cells is clearly detectable (Figure 55).

### 33.2 Cloning and expression of the cross-species specific anti-CD3 single chain antibody I2C HL in form of an IgG1 antibody

In order to provide improved means of detection of binding of the cross-species specific single chain anti-CD3 antibody the I2C VHVL specificity is converted into an IgG1 antibody with murine IgG1 and murine kappa constant regions. cDNA sequences coding for the heavy chain of the IgG antibody were obtained by gene synthesis according to standard protocols. The gene synthesis fragments were designed as to contain first a Kozak site to allow for eukaryotic expression of the construct, which is followed by an 19 amino acid immunoglobulin leader peptide, which is followed in frame by the coding sequence of the heavy chain variable region or light chain variable region, followed in frame by the coding sequence of the heavy chain constant region of murine IgG1 as published in GenBank (Accession number AB097849) or the coding sequence of the murine kappa light chain constant region as published in GenBank (Accession number D14630), respectively.

Restriction sites were introduced at the beginning and the end of the cDNA coding for the fusion protein. Restriction sites EcoRI at the 5' end and SalI at the 3' end were used for the following cloning procedures. The gene synthesis fragments were cloned via EcoRI and SalI into a plasmid designated pEF-DHFR (pEF-DHFR is described in Raum et al. Cancer Immunol Immunother 50 (2001) 141-150) for the heavy chain construct and pEFADA (pEFADA is described in Raum et al. Cancer Immunol Immunother 50 (2001) 141-150) for the light chain construct according to standard protocols. Sequence verified plasmids were used for co-transfection of respective light and heavy chain constructs into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the constructs was induced by increasing concentrations of methotrexate (MTX) to a final concentration of up to 20 nM MTX and deoxycoformycin (dCF) to a final concentration of up to 300 nM dCF. After two passages of stationary culture cell culture supernatant was collected and used in the subsequent experiment.

### 33.3 Binding of the cross-species specific anti-CD3 single chain antibody I2C HL in form of an IgG1 antibody to 1-27 CD3-EpCAM

Binding of the generated I2C **IgG1** construct to the 1-27 N-terminal amino acids of the human, marmoset, tamarin and squirrel monkey CD3 epsilon chains respectively fused to cynomolgus Ep-CAM as described in Example 33.1 was tested in a FACS assay according to standard protocols. For that purpose a number of $2.5 \times 10^5$ cells were incubated with 50 $\mu$l of cell culture supernatant containing the I2C IgG1 construct as described in Example 33.2. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd.,

Newmarket, Suffolk, UK). Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

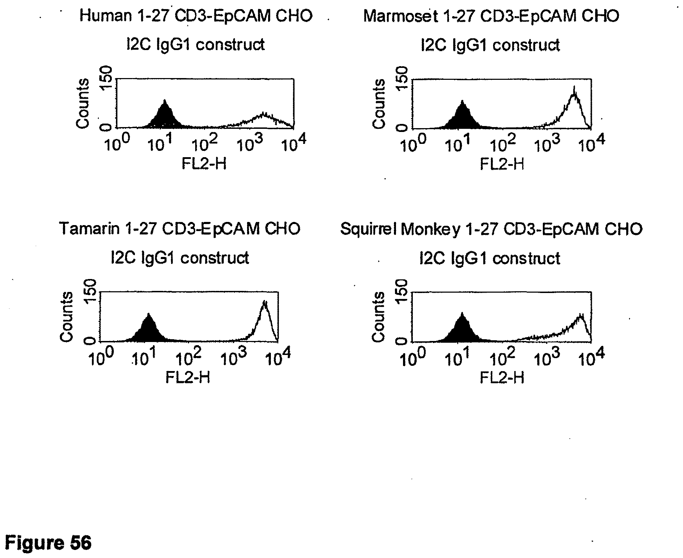

As shown in Figure 56 binding of the I2C IgG1 construct to the transfectants expressing the recombinant transmembrane fusion proteins consisting of the 1-27 N-terminal amino acids of CD3 epsilon of human, marmoset, tamarin or squirrel monkey fused to cynomolgus EpCAM as compared to the negative control consisting of the 1-27 N-terminal amino acids of CD3 epsilon of swine fused to cynomolgus EpCAM was observed. Thus multi-primate cross-species specificity of I2C was demonstrated. Signals obtained with the anti Flag M2 antibody and the I2C **IgG1** construct were comparable, indicating a strong binding activity of the cross-species specific specificity I2C to the N-terminal amino acids 1-27 of CD3 epsilon.

### 34. Binding of the cross-species specific anti-CD3 binding molecule I2C to the human CD3 epsilon chain with and without N-terminal His6 tag

A chimeric IgG1 antibody with the binding specificity I2C as described in Example 33.2 specific for CD3 epsilon was tested for binding to human CD3 epsilon with and without N-terminal His6 tag. Binding of the antibody to the EL4 cell lines transfected with His6-human CD3 epsilon as described in Example 6.1 and wild-type human CD3 epsilon as described in Example 5.1 respectively was tested by a FACS assay according to standard protocols. $2.5 \times 10^5$ cells of the transfectants were incubated with 50 $\mu$l of cell culture supernatant containing the I2C IgG1 construct or 50 $\mu$l of the respective control antibodies at 5$\mu$g/ml in PBS with 2% FCS. As negative control an appropriate isotype control and as positive control for expression of the constructs the CD3 specific antibody UCHT-1 were used respectively. Detection of the His6 tag was performed with the penta His antibody (Qiagen). The binding of the antibodies was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

Comparable binding of the anti-human CD3 antibody UCHT-1 to both transfectants demonstrates approximately equal levels of expression of the constructs. The binding of the penta His antibody confirmed the presence of the His6 tag on the His6-human GD3 construct but not on the wild-type construct.

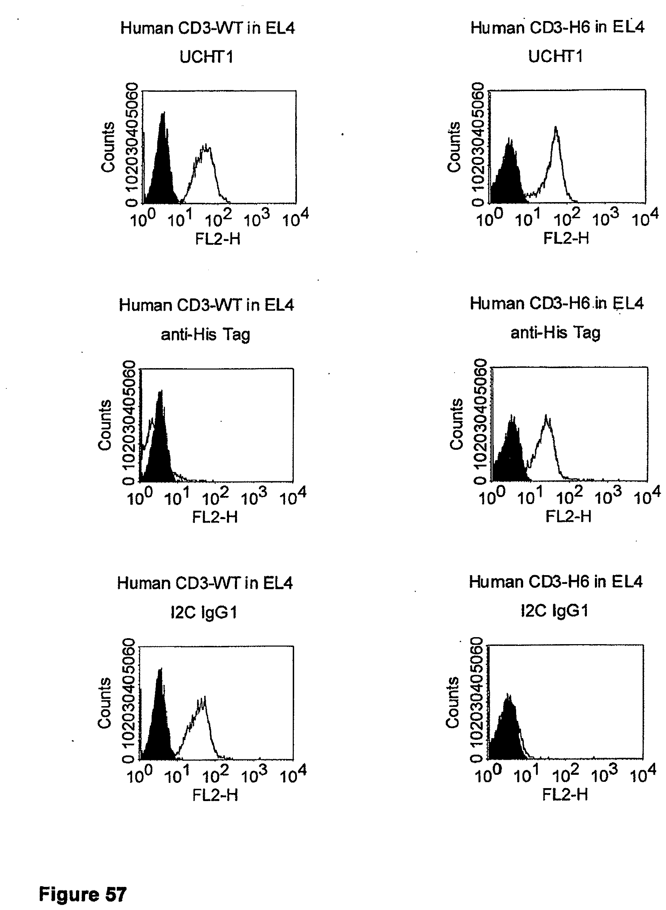

Compared to the EL4 cell line transfected with wild-type human CD3 epsilon a clear loss of binding of the I2C IgG1 construct to human-CD3 epsilon with an N-terminal His6 tag was detected. These results show that a free N-terminus of CD3 epsilon is essential for binding of the cross-species specific anti-CD3 binding specificity I2C to the human CD3 epsilon chain (Figure 57).

### 35. Bispecific single chain antibody constructs directed to human and non-chimpanzee primate CD3 and to tumor specific epitopes of Muc-1

The human antibody germline VH sequence VH3 3-72 (http://vbase.mrc-cpe.cam.ac.uk/) was chosen as framework context for CDRH1 (SEQ ID 1486), CDRH2 (SEQ ID 1487) and CDRH3 (SEQ ID 1488). Likewise human antibody germline VH sequence VH3 3-73 (http://vbase.mrc-cpe.cam.ac.uk/) was chosen as framework context for CDRH1 (SEQ ID 1492), CDRH2 (SEQ ID 1493) and CDRH3 (SEQ ID 1494). For each human VH several degenerated oligonucleotides had to be synthesized that overlap in a terminal stretch of approximately 15-20 nucleotides. To this end every second primer was an antisense primer. For VH3 3-72 the following oligonucleotides were used:

5' VH72-A-XhoI

GAAGTGAAGCTTCTCGAGTCTGGAGGAGGCTTGGTGCAACCTGGAGGATCCMT GARACTCTCTTGTGCTGCT

'3' VH72-B

CTGGCGGACCCAGTCCATCCAGGCATCACTAAAAGTGAATCCAGAAGCAGCAC
AAGAGAG

5' VH72-C

GACTGGGTCCGCCAGKCTCCAGRGAAGGGGCTTGAGTGGGTTGSTGAAATTAG
AAACAAAGCCAAT

3' VH72-D

TTTGGAATCATCTCTTGAGATGGTGAACCTCCCTTTCACAGACTCATCATAATAT
GTTGCATGATTATTGGCTTTGTTTCT

5' VH72-E

AGAGATGATTCCAAAARTAGMSTGTACCTGCAAATGAWMAGCTTAARARCTGAA
GACACTGSCSTTTATTACTGTACTGGG

3' VH72-F-BstEII

GACCGTGGTGACCAGAGTCCCCTGGCCCCAGTTAGCAAACTCCCCAGTACAGT
AATA

For VH3 3-73 the oligonucleotides were as follows:

5' VH73-A-XhoI
CAAGTTCAGCTGCTCGAGTCTGGAGGAGGCTTGGTGCAACCTGGAGGATCC

3'VH73-B

CCAGTTCATCCAGTAGTTACTGAAAGTGAATCCAGAGGCARCACAGGAGAGTTT
CAKGGATCCTCCAGGTTG

5'VH73-C

TACTGGATGAACTGGGTCCGCCAGKCTYCAGRGAAGGGGCTTGAGTGGGTTGS
TGAAATTAGATTGAAATCTAAT

3'VH73-D

TTTGGAATCATCTCTTGAGATGGTGAACCTCCCTTTCACAGACTCCGCATAATGT
GTTGCATAATTATTAGATTTCAATCT

5' VH73-E

AGAGATGATTCCAAAARTASTGYCTACCTGCAAATGAACARCTTAARARCTGAAG
ACACTGSCRTTTATTACTGTACGGGA

3' VH73-F-BstEII

GACCGTGGTGACCGTGGTCCCTTGGCCCCAGTAAGCAAATTGTCCCACTCCCG
TACAGTAATA

Each of these primer sets spans over the whole corresponding VH sequence.

Within each set primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

Each VH PCR product was then used as a template for a standard PCR reaction using primers that incorporate N-terminal and C-terminal suitable cloning restriction sites. The DNA fragment of the correct size (for a VH approximately 350 nucleotides) was isolated by agarose gel electrophoresis according to standard methods. In this way sufficient VH DNA fragment was amplified.

The human antibody germline VL sequence VkII A18 (http://vbase.mrc-cpe.cam.ac.uk/) was chosen as framework context for CDRL1 (SEQ ID 1489), CDRL2 (SEQ ID 1490) and CDRL3 (SEQ ID 1491). Likewise human antibody germline VL sequence VL7 7a (http://vbase.mrc-cpe.cam.ac.uk/) was chosen as framework context for CDRL1 (SEQ ID 1495), CDRL2 (SEQ ID 1496) and CDRL3 (SEQ ID 1497). For each human VL several degenerated oligonucleotides had to be synthesized that overlap in a terminal stretch of approximately 15-20 nucleotides. To this end every second primer was an antisense primer. Restriction sites needed for later cloning within the oligonucleotides were deleted. For VkII A18 the following oligonucleotides were used:

5'Vk2A18-A-SacI

CCTGATGAGCTCGTGATGACCCAAACTCCACTCTCCCTGYCTGTCASTCYTGGA
SAKCMAGCTTCCATCTCTTGC

3' Vk2A18-B

CCAATATAAATAGGTGTTTCCATTGTTGTGTACCAGGTTCTGACTAGATCTGCAA
GAGATGGAAGC

5' Vk2A18-C

ACCTATTTATATTGGTWCCTGCAGAAGYCAGGCCAGTCTCCAMAGCTCCTGATT
TATAGGGCTTCCATC

3' Vk2A18-D

CTTGAGTGTGAAATCTGTCYCTGATCCACTGCCACTGAACCTGTCTGGGACCCC
AGAAAATCGGATGGAAGCCCTATA

5' Vk2A18-E

ACAGATTTCACACTCAAGATCAGCAGAGTGGAGGCTGAGGATSTGGGAGTTTAT
TWCTGCTTTCAAGGTACACAT

3' Vk2A18-F-BsiWI/SpeI

CCCGATACTAGTCGTACGTTTGATTTCCAGCTTGGTGCCTTGACCGAACGTCCA
CGGAACATGTGTACCTTGAAAGCA

For VL7 7a the oligonucleotides were as follows:

5'Vlam7a-A-Sacl

CCTGCAGAGCTCGTTGTGACTCAGGAAYCTKCACTCACCRYATCACCTGGTGRA
ACAGTCACACTCACTTGT

3'Vlam7a-B

CCAGTTGGCATAGTTACTTGTTGTAACAGCCCCAGTACTTGAGCGACAAGTGAG
TGTGACTGT

5' Vlam7a-C

AACTATGCCAACTGGKTCCAASAAAAACCAGRTCAKKYAYYCMSTGSTCTAATAK
RTGGTACCAACAACCGA

3' Vlam7a-D

GGTGAGGGCAGCCTTGYCTCCAAKCAGGGAGCCTGAGAATCTGGCAGGARYM
CMTGGTGCTCGGTTGTTGGTACC

5' Vlam7a-E

AAGGCTGCCCTCACCMTCWCAGGGGYACAGMCTGAGGATGAGGCARWATATT
WCTGTGCTCTATGGTACAGC

3' Vlam7a-F-BsiWl/Spel

CCAGTAACTAGTCGTACGTAGGACAGTCAGTTTGGTTCCTCCACCGAACACCCA
ATGGTTGCTGTACCATAGAGCACA

Each of these primer sets spans over the whole corresponding VL sequence.

Within each set primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

Each VL PCR product was then used as a template for a standard PCR reaction using primers that incorporate N-terminal and C-terminal suitable cloning restriction sites. The DNA fragment of the correct size (for a VL approximately 330 nucleotides) was isolated by agarose gel electrophoresis according to standard methods. In this way sufficient VL DNA fragment was amplified.

The final VH3 3-72-based VH PCR product (i.e. the repertoire of human/humanized VH) was then combined with the final VkII A18 -based VL PCR product (i.e. the repertoire of human/humanized VL) and the final VH3 3-73-based VH PCR product (i.e. the repertoire of human/humanized VH) with the final VL7 7a-based VL PCR product (i.e. the repertoire of human/humanized VL) in the phage display vector pComb3H5Bhis to form two different libraries of functional scFvs from which - after display on filamentous phage - anti-Muc-1 binders were selected, screened, identified and confirmed as described as follows:

450 ng of the light chain fragments (Sacl-Spel digested) were ligated with 1400 ng of the phagemid pComb3H5Bhis (Sacl-Spel digested; large fragment). The resulting combinatorial antibody library was then transformed into 300 ul of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells were then harvested by centrifugation and plasmid preparation was carried out using a commercially available plasmid preparation kit (Qiagen).

2800 ng of this plasmid-DNA containing the VL-library (Xhol-BstEII digested; large fragment) were ligated with 900 ng of the heavy chain V-fragments (Xhol-BstEII digested) and again transformed into two 300 ul aliquots of electrocompetent E. coli XL1 Blue cells by electroporation (2.5 kV, 0.2 cm gap cuvette, 25 uFD, 200 Ohm) resulting in a total VH-VL scFv (single chain variable fragment) library size of more than $10^7$ independent clones.

After phenotype expression and slow adaptation to carbenicillin, the E. coli cells containing the antibody library were transferred into SB-Carbenicillin (50 ug/mL) selection medium. The E. coli cells containing the antibody library was then infected with an infectious dose of $10^{12}$ particles of helper phage VCSM13 resulting in the production and secretion of filamentous M13 phage, wherein phage particle contains single stranded pComb3H5BHis-DNA encoding a scFv-fragment and displayed the corresponding scFv-protein as a translational fusion to phage coat protein III. This pool of phages displaying the antibody library was used for the selection of antigen binding entities.

**Generation of CHO cells expressing human MUC-1**

The coding sequence of human MUC-1 as published in GenBank (Accession number J05581) was obtained by gene synthesis according to standard protocols. The gene synthesis fragment was designed as to contain first a Kozak site for eukaryotic expression of the construct, followed by the coding sequence of the human MUC-1 protein and a stop codon (the cDNA and amino acid sequence of the construct is listed under SEQ ID NOs 1498 and 1499). In the gene synthesis fragment the internal repeat sequence (nucleotides 382 to 441) of MUC-1, which is contained only once in the version deposited in GenBank is contained sixteen times reflecting the multiple repeats in physiologically expressed MUC-1. The gene synthesis fragment was also designed as to introduce restriction sites at the beginning and at the end of the fragment. The introduced restriction sites, EcoRI at the 5' end and Sall at the 3' end, were utilised in the following cloning procedures. Internal Xmal restriction sites were removed by silent mutation of the coding sequence in the gene synthesis fragment (nucleotide 441 from C to T; this is the last nucleotide of the first repeat and therefore mutated identically in the 15 subsequent repeat sequences; and nucleotide 2160 from G to C). The gene synthesis fragment was cloned via EcoRI and Sall into a plasmid designated pEF-DHFR (pEF-DHFR is described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

For the selection of antigen binding entities.the phage library carrying the cloned scFv-repertoire was harvested from the respective culture supernatant by PEG8000/NaCl precipitation and centrifugation. Approximately 1011 to 1012 scFv phage particles were resuspended in 0.4 ml of PBS/0.1% BSA and incubated with 105 to 107 Muc-1 transfected CHO cells (see example Muc-1 RoK) for 1 hour on ice under slow agitation. These Muc-1 transfected CHO cells were harvested beforehand by centrifugation, washed in PBS and resuspended in PBS/1 % FCS (containing Na Azide). scFv phage which do not specifically bind to the Muc-1 transfected CHO cells were eliminated by up to five washing steps with PBS/1 % FCS (containing Na Azide). After washing, binding entities were eluted from the cells by resuspending the cells in HCl-glycine pH 2.2 (10 min incubation with subsequent vortexing) and after neutralization with 2 M Tris pH 12, the eluate was used for infection of a fresh uninfected E. coli XL1 Blue culture (OD600 > 0.5). The E. coli culture containing E. coli cells successfully transduced with a phagemid copy, encoding a human/humanized scFv-fragment, were again selected for carbenicillin resistance and subsequently infected with VCMS 13 helper phage to start the second round of antibody display and in vitro selection. A total of 4 to 5 rounds of selections were carried out, normally.

In order to screen for Muc-1 specific binders plasmid DNA corresponding to 4 and 5 rounds of panning was isolated from E. coli cultures after selection. For the production of soluble scFv-protein, VH-VL-DNA fragments were excised from the plasmids (Xhol-Spel). These fragments were cloned via the same restriction sites into the plasmid pComb3H5BFiag/His differing from the original pComb3H5BHis in that the expression construct (e.g. scFv) includes a Flag-tag (DYKDDDDK) between the scFv and the His6-tag and the additional phage proteins were deleted. After ligation, each pool (different rounds of panning) of plasmid DNA was transformed into 100 μl heat shock competent E. coli TG1 or XLI blue and plated onto carbenicillin LB-agar. Single colonies were picked into 100 ul of LB carb (50 ug/ml).

E. coli transformed with pComb3H5BHis containing a VL-and VH-segment produce soluble scFv in sufficient amounts after excision of the gene III fragment and induction with 1 mM IPTG. Due to a suitable signal sequence, the scFv-chain was exported into the periplasma where it folds into a functional conformation.

Single E. coli TG1 bacterial colonies from the transformation plates were picked for periplasmic small scale preparations and grown in SB-medium (e.g. 10 ml) supplemented with 20 mM MgCl$_2$ and carbenicillin 50μg/ml (and re-dissolved in PBS (e.g. 1 ml) after harvesting. By four rounds of freezing at -70°C and thawing at 37°C, the outer membrane of the bacteria was destroyed by temperature shock and the soluble periplasmic proteins including the scFvs were released into the supernatant. After elimination of intact cells and cell-debris by centrifugation, the supernatant containing the anti-Muc-1 scFvs was collected and used for the identification of Muc-1 specific binders as follows:
Binding of scFvs to Muc-1 was tested by flow cytometry on Muc-1 transfected CHO cells; untransfected CHO cell were use as negative control.

For flow cytometry $2,5 \times 10^5$ cells were incubated with 50 ul of scFv periplasmic preparation or with 5 μg/ml of purified scFv in 50 μl PBS with 2% FCS. The binding of scFv was detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) is used. The samples were measured on a FACSscan (BD biosciences, Heidelberg, FRG).

Single clones were then analyzed for favourable properties and amino acid sequence. Muc-1 specific scFvs were converted into recombinant bispecific single chain antibodies by joining them via a Gly$_4$Ser$_1$-linker with the CD3 specific scFv I2C (SEQ ID 185) or any other CD3 specific scFv of the invention to result in constructs with the domain arrangement VH$_{MuC1}$ - (Gly$_4$Ser$_1$)$_3$ -VL$_{Muc1}$- Gly$_4$Ser$_1$-VH$_{CD3}$ - (Gly$_4$Ser$_1$)$_3$ - VL$_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal His$_6$-tag followed by a stop codon were both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the bispecific single chain antibodies into the culture supernatant and gene amplification with methotrexat for increasing expression levels were carried out as described (Mack et al. Proc. Natl. Acad. Sci. USA 92 (1995) 7021-7025). All other state of the art procedures were carried out according to standard protocols (Sambrook, Molecular Cloning; A Laboratory Manual, 3rd edition, Cold Spring Harbour Laboratory Press, Cold Spring Harbour, New York (2001)).

Identification of functional bispecific single-chain antibody constructs was carried out by flowcytometric analysis of culture supernatant from transfected CHO cells. For this purpose CD3 binding was tested on the human CD3 positive T cell leukemia cell line HPB-ALL (DSMZ, Braunschweig, ACC483) and on the macaque T cell line 4119LnPx. Binding to tumor specific Muc-1 epitopes was tested on Muc-1 transfected CHO cells. 200.000 cells of the respective cell line were incubated for 30 min. on ice with 50 μl of cell culture supernatant. The cells were washed twice in PBS with 2% FCS and bound bispecific single-chain antibody construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

Only those constructs showing bispecific binding to human and macaque CD3 as well as to tumor specific epitopes of Muc-1 were selected for further use.

For protein production CHO cells expressing fully functional bispecific single chain antibody and adapted to nucleoside-free HyQ PF CHO liquid soy medium (with 4.0 mM L-Glutamine with 0.1% Pluronic F - 68; HyClone) were grown in roller bottles with nucleoside-free HyQ PF CHO liquid soy medium (with 4.0 mM L-Glutamine with 0.1% Pluronic F - 68; HyClone) for 7 days. Culture supernatant was cleared from cells by centrifugation and stored at -20°C until purification. As chromatography equipment for purification of bispecific single chain antibody from culture supernatant Äkta® Explorer System (GE Health Systems) and Unicorn® Software were used. Immobilized metal affinity chromatography ("IMAC") was performed using a Fractogel EMD chelate® (Merck) which was loaded with ZnCl$_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

The potency in the human and the non-chimpanzee primate system of bispecific single chain antibodies interacting with human and macaque CD3 and with tumor specific epitopes of Muc-1 was determined by a cytotoxicity assay based on chromium 51 ($^{51}$Cr) release using Muc-1 transfected CHO cells as target cells and stimulated human CD4/CD56 depleted PBMC or the macaque T cell line 4119LnPx as effector cells.

Generation of stimulated human PBMC was performed as follows:

A Petri dish (145 mm diameter, Nunc) was coated with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

Target cells were washed twice with PBS and labelled with 11.1 MBq $^{51}$Cr in a final volume of 100 $\mu$l RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250$\mu$l supplemented RPMI (as above) with an E:T ratio of 10:1. 1 $\mu$g/ml of the cross-species specific bispecific single chain antibody molecules and 20 threefold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have R$^2$ values >0.90 as determined by the software. EC$_{50}$ values as measure of potency were calculated by the analysis program.

36. Generation of CD33 and CD3 cross-species specific bispecific single chain molecules

36.1 Generation of CD33 and CD3 cross-species specific bispecific single chain molecules

Generally, bispecific single chain antibody molecules, each comprising a domain with a binding specificity cross-species specific for human and macaque CD3epsilon as well as a domain with a binding specificity cross-species specific for human and macaque CD33, were designed as set out in the following Table 14:

Table 14: Formats of anti-CD3 and anti-CD33 cross-species specific bispecific single chain antibody molecules

| SEQ ID (nucl/prot) | Formats of protein constructs (N → C) |
|---|---|
| 1341/1340 | I2CHLxAF5HL |
| 1339/1338 | F12QHLxAF5HL |
| 1337/1336 | H2CHLxAF5HL |

The aforementioned constructs containing the variable light-chain (L) and variable heavy-chain (H) domains cross-species specific for human and macaque CD33 and the CD3 specific VH and VL combinations cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a histidine$_6$-tag and a stop codon. The gene synthesis fragment was also designed as to introduce suitable restriction sites at the beginning and at the end of the fragment. The introduced restriction sites were utilised in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX. After two passages of stationary culture cell culture supernatant was collected and used in the subsequent experiments.

**36.2 Flow cytometric binding analysis of the CD33 and CD3 cross-species specific bispecific antibodies**

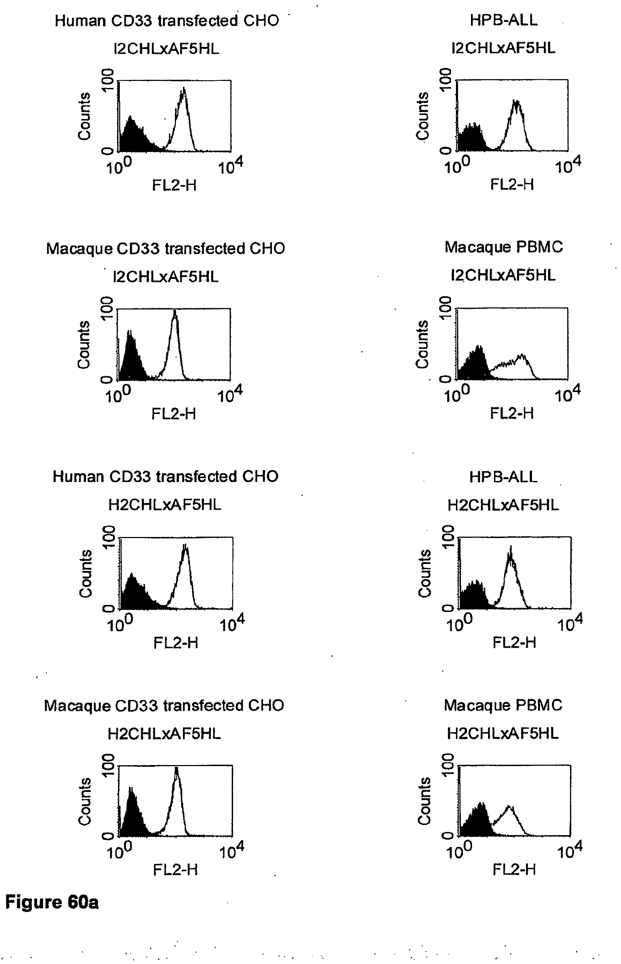

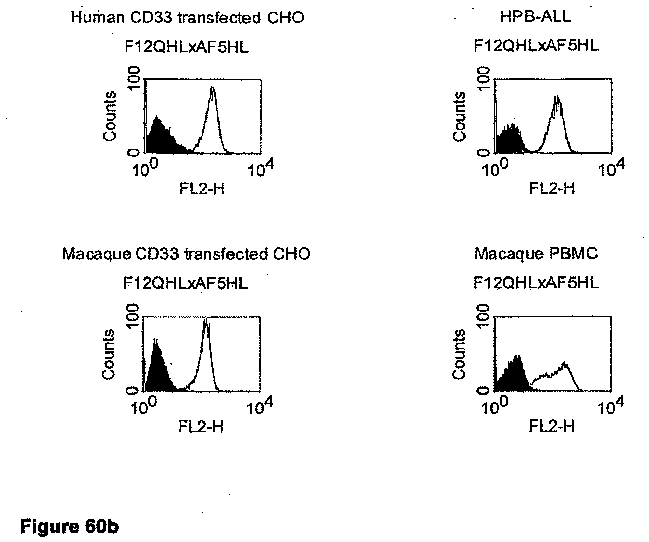

In order to test the functionality of the cross-species specific bispecific antibody constructs regarding the capability to bind to human and macaque CD33 and CD3, respectively, a FACS analysis is performed. For this purpose CHO cells transfected with human CD33 as described in Example 23.1 and the human CD3 positive T cell leukemia cell line HPB-ALL (DSMZ, Braunschweig, ACC483) were used to test the binding to human antigens. The binding reactivity to macaque antigens was tested by using the generated macaque CD33 transfectant described in Example 23.2 and macaque PBMC (preparation of macaque PBMC was performed by Ficoll gradient centrifugation of peripheral blood from macaque monkeys according to standard protocols). 200000 cells of the respective cell lines or PBMC were incubated for 30 min. on ice with 50 μl of cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control.
Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
The bispecific binding of the single chain molecules listed above, which were cross-species specific for CD33 and cross-species specific for human and non-chimpanzee primate CD3 was clearly detectable as shown in Figure 60. In the FACS analysis all constructs showed binding to CD3 and CD33 as compared to the respective negative controls. Cross-species specificity of the bispecific antibodies to human and macaque CD3 and CD33 antigens was demonstrated.

**36.3. Bioactivity of CD33 and CD3 cross-species specific bispecific single chain antibodies**

Bioactivity of the generated bispecific single chain antibodies was analyzed by chromium 51 ($^{51}$Cr) release in vitro cytotoxicity assays using the CD33 positive cell lines described in Examples 23.1 and 23.2. As effector cells stimulated human CD4/CD56 depleted PBMC or the macaque T cell line 4119LnPx were used as specified in the respective figures.
A Petri dish (145 mm diameter, Greiner bio-one GmbH, Kremsmünster) was coated with a commercially available anti-CD3 specific antibody (e.g. OKT3, Orthoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × $10^7$ PBMC were added to the precoated Petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 is added to a final concentration of 20 U/ml and the cells were cultivated again for one day in the same cell culture medium as above.

By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}$Cr in a final volume of 100 μl RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250μl supplemented RPMI (as above) with an E:T ratio of 10:1. Supernatant of cells expressing the cross-species specific bispecific single chain antibody molecules in a final concentration of 50% and 20 threefold dilutions thereof were applied. The assay time is 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

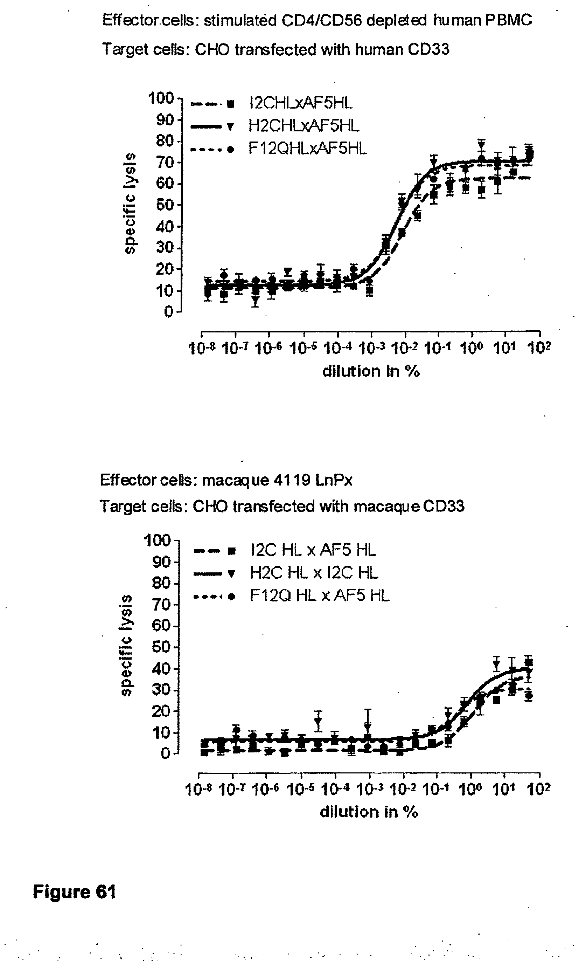

As shown in Figure 61, all of the generated cross-species specific bispecific single chain antibody constructs demonstrate cytotoxic activity against human CD33 positive target cells elicited by stimulated human CD4/CD56 depleted PBMC and against macaque CD33 positive target cells elicited by the macaque T cell line 4119LnPx.

### 37. Redistribution of circulating chimpanzee T cells upon exposure to a conventional bispecific CD3 binding molecule directed at a target molecule which is absent from circulating blood cells

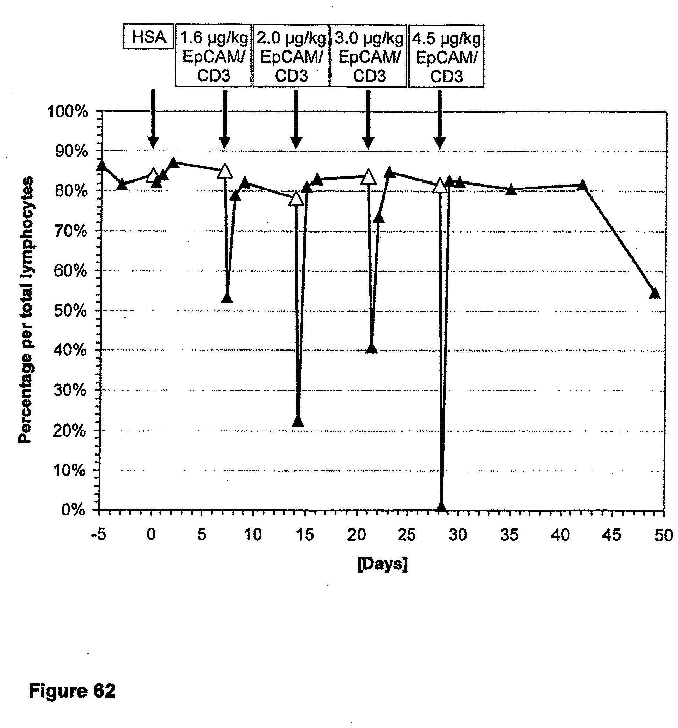

A single male chimpanzee was subjected to dose escalation with intravenous single-chain EpCAM/CD3-bispecific antibody construct (Schlereth (2005) Cancer Res 65: 2882). Like in the conventional single-chain CD19/CD3-bispecific antibody construct (Loffler (2000, Blood, Volume 95, Number 6) or WO 99/54440), the CD3 arm of said EpCAM/CD3-construct is also directed against a conventional context dependent epitope of human and chimpanzee CD3. At day 0, the animal received 50ml PBS/5% HSA without test material, followed by 50ml PBS/5% HSA plus single-chain EpCAM/CD3-bispecific antibody construct at 1.6, 2.0, 3.0 and 4.5 μg/kg on days 7, 14, 21 and 28, respectively. The infusion period was 2 hours per administration. For each weekly infusion the chimpanzee was sedated with 2-3 mg/kg Telazol intramuscularly, intubated and placed on isoflurane/$O_2$ anesthesia with stable mean blood pressures. A second intravenous catheter was placed in an opposite limb to collect (heparinized) whole blood samples at the time points indicated in Figure 62 for FACS analysis of circulating blood cells. After standard erythrocyte lysis, T cells were stained with a FITC-labeled antibody reacting with chimpanzee CD2 (Becton Dickinson) and the percentage of T cells per total lymphocytes determined by flowcytometry. As shown in Figure 62, every administration of single-chain EpCAM/CD3-bispecific antibody construct induced a rapid drop of circulating T cells as observed with single-chain CD19/CD3-bispecific antibody construct in B-NHL patients, who had essentially no circulating target B (lymphoma) cells. As there are no EpCAM-positive target cells in the circulating blood of humans and chimpanzees, the drop of circulating T cells upon exposure to the single-chain EpCAM/CD3-bispecific antibody construct can be attributed solely to a signal, which the T cells receive through pure interaction of the CD3 arm of the construct with a conventional context dependent CD3 epitope in the absence of any target cell mediated crosslinking. Like the redistribution of T cells induced through their exposure to single-chain CD19/CD3-bispecific antibody construct in B-NHL patients, who had essentially no circulating target B (lymphoma) cells, the T cell redistribution in the chimpanzee upon exposure to the single-chain EpCAM/CD3-bispecific antibody construct can be explained by a conformational change of CD3 following the binding event to a context dependent CD3 epitope further resulting in the transient increase of T cell adhesiveness to blood vessel endothelium (see Example 20). This finding confirms, that conventional CD3 binding molecules directed to context dependent CD3 epitopes - solely through this interaction - can lead to a redistribution pattern of peripheral blood T cells, which is associated with the risk of CNS adverse events in humans as describe in Example 20.

### 38. Specific binding of scFv clones to the N-terminus of human CD3 epsilon

### 38.1 Bacterial expression of scFv constructs in *E. coli* XL1 Blue

As previously mentioned, E. *coli* XL1 Blue transformed with pComb3H5Bhis/Flag containing a VL- and VH-segment produce soluble scFv in sufficient amounts after excision of the gene III fragment and induction with 1 mM IPTG. The scFv-chain is exported into the periplasma where it folds into a functional conformation.

The following scFv clones were chosen for this experiment:

    i) ScFvs 4-10, 3-106, 3-114, 3-148, 4-48, 3-190 and 3-271 as described in WO 2004/106380.
    ii) ScFvs from the human anti-CD3epsilon binding clones H2C, F12Q and I2C as described herein.

For periplasmic preparations, bacterial cells transformed with the respective scFv containing plasmids allowing for periplasmic expression were grown in SB-medium supplemented with 20 mM MgCl$_2$ and carbenicillin 50 μg/ml and redissolved in PBS after harvesting. By four rounds of freezing at -70°C and thawing at 37°C, the outer membrane of the bacteria was destroyed by osmotic shock and the soluble periplasmic proteins including the scFvs were released into the supernatant. After elimination of intact cells and cell-debris by centrifugation, the supernatant containing the human anti-human CD3-scFvs was collected and used for further examination. These crude supernatants containing scFv will be further termed periplasmic preparations (PPP).

### 38.2 Binding of scFvs to human CD3 epsilon (aa 1-27)- Fc fusion protein

ELISA experiments were carried out by coating the human CD3 epsilon (aa 1-27)- Fc fusion protein to the wells of 96 well plastic plates (Nunc, maxisorb) typically at 4° C over night. The antigen coating solution was then removed, wells washed once with PBS/0.05 % Tween 20 and subsequently blocked with PBS/3 % BSA for at least one hour. After removal of the blocking solution, PPPs and control solutions were added to the wells and incubated for typically one hour at room temperature. The wells were then washed three times with PBS/0.05 % Tween 20. Detection of scFvs bound to immobilized antigen was carried out using a Biotin-labeled anti FLAG-tag antibody (M2 anti Flag-Bio, Sigma, typically at a final concentration of 1μg/ml PBS) and detected with a peroxidase-labeled Streptavidine (Dianova, 1μg/ml PBS). The signal was developed by adding ABTS substrate solution and measured at a wavelength of 405 nm. Unspecific binding of the test-samples to the blocking agent and/or the human IgG1 portion of the human CD3 epsilon (aa 1-27)- Fc fusion protein was examined by carrying out the identical assay with the identical reagents and identical timing on ELISA plates which were coated with human IgG1 (Sigma). PBS was used as a negative control.

As shown in Figure 63, scFvs H2C, F12Q and I2C show strong binding signals on human CD3 epsilon (aa 1-27)- Fc fusion protein. The human scFvs 3-106, 3-114, 3-148, 3-190, 3-271, 4-10 and 4-48 (as described in WO 2004/106380) do not show any significant binding above negative control level.

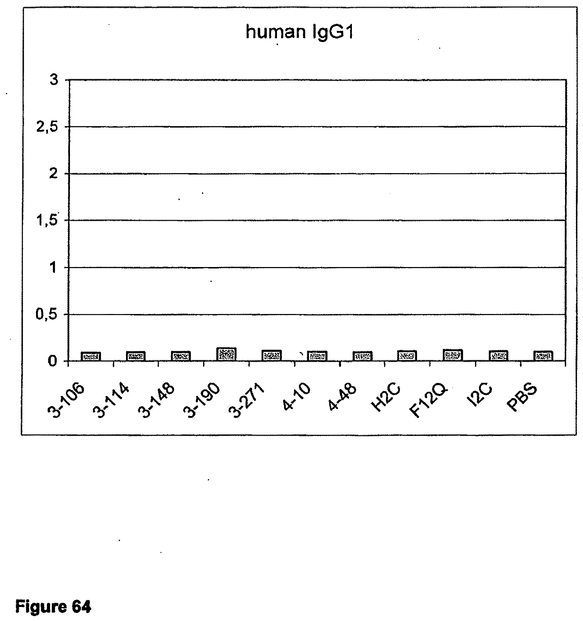

To exclude the possibility that the positive binding of scFvs H2C, F12Q and I2C to wells coated with human CD3 epsilon (aa 1-27)- Fc fusion protein might be due to binding to BSA (used as a blocking agent) and/or the human IgG1 Fc-gamma-portion of the human CD3 epsilon (aa 1-27)- Fc fusion protein, a second ELISA experiment was performed in parallel. In this second ELISA experiment, all parameters were identical to those in the first ELISA experiment, except that in the second ELISA experiment human IgG1 (Sigma) was coated instead of human CD3 epsilon (aa 1-27)- Fc fusion protein. As shown in Figure 64, none of the scFvs tested showed any significant binding to BSA and/or human IgG1 above background level.

Taken together, these results allow the conclusion that conventional CD3 binding molecules recognizing a context-dependent epitope of CD3 epsilon (e.g. as disclosed in WO 2004/106380) do not bind specifically to the human CD3 epsilon (aa 1-27)-region, whereas the scFvs H2C, F12Q and I2C binding a context-independent epitope of CD3 epsilon clearly show specific binding to the N-terminal 27 amino acids of human CD3 epsilon.

### 39. Generation of CD44 and CD3 cross-species specific bispecific single chain molecules

### 39.1 Cloning of cross-species specific binding molecules

Generally, bispecific single chain antibody molecules, each comprising a domain with a binding specificity cross-species specific for human and macaque CD3 epsilon as well as a domain with a binding specificity cross-species specific for human and macaque CD44, are designed as set out in the following table 15.

Table 15: Formats of anti-CD3 and anti-CD44 cross-species specific bispecific single chain antibody molecules

| SEQ ID (nucl/prot) | Formats of protein constructs (N → C) |
|---|---|
| 1589/1588 | CD44-A8 LH × I2C HL |
| 1603/1602 | CD44-C2 HL × I2C HL |
| 1631/1630 | CD44-H3 HL × I2C HL |

(continued)

| SEQ ID (nucl/prot) | Formats of protein constructs (N → C) |
|---|---|
| 1617/1616 | CD44-F6 HL × I2C HL |

The aforementioned constructs containing the variable heavy-chain (VH) and variable light-chain (VL) domains cross-species specific for human and macaque CD44 and the VH and VL domains cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a histidine$_6$-tag and a stop codon. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into dihydrofolate reductase (DHFR) deficient Chinese hamster ovary (CHO) cells for eukaryotic expression of the construct.

**39.2 Expression of the bispecific single chain antibody molecules**

The bispecific constructs were transiently expressed in DHFR deficient CHO cells. In brief, $4 \times 10^5$ cells per construct were cultivated in 3 ml RPMI 1640 all medium with stabilized glutamine supplemented with 10% fetal calf serum, 1% penicillin/streptomycin and nucleosides from a stock solution of cell culture grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37 °C, 95% humidity and 7% $CO_2$ one day before transfection. Transfection was performed with Fugene HD Transfection Reagent (Roche, # 04709691001) according to the manufacturer's protocol. 94 $\mu$l OptiMEM medium (Invitrogen) and 6 $\mu$l Fugene HD were mixed and incubated for 5 minutes at room temperature. Subsequently, 1.5 $\mu$g DNA per construct were added, mixed and incubated for 15 minutes at room temperature. Meanwhile, the DHFR deficient CHO cells were washed with 1x PBS and resuspended in 1.5 ml RPMI 1640 all medium. The transfection mix was diluted with 600 $\mu$l RPMI 1640 all medium, added to the cells and incubated overnight at 37 °C, 95% humidity and 7% $CO_2$. The day after transfection the incubation volume of each approach was extended to 5 ml RPMI 1640 all medium. Supernatant was harvested after 3 days of incubation.

**39.3 Flow cytometric binding analysis of the CD44 and CD3 cross-species specific bispecific antibodies**

In order to test the functionality of the cross-species specific bispecific antibody constructs regarding the capability to bind to human and macaque CD44 and CD3, respectively, a FACS analysis was performed. For this purpose CHO cells transfected with human CD44 (as described in Example 28.1) and the human CD3 positive T cell leukemia cell line HPB-ALL (DSMZ, Braunschweig, ACC483) were used to test the binding to human antigens. The binding reactivity to macaque antigens was tested by using the generated macaque CD44 transfectant (described in Example 28.3) and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). To demonstrate the specificity of the cross-species specific bispecific antibody constructs to CD44, the binding to human CD44delv6 (CD44 deleted for the v6 exon) was tested using CHO cells transfected with human CD44delv6 (generation described in Example 28.2). 200.000 cells of the respective cell lines were incubated for 30 min on ice with 50 $\mu$l of the cell supernatants of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 $\mu$l PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines. A single chain construct with irrelevant target specificity was used as negative control for binding to the CD44 and the CD44delv6 transfected CHO cells.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

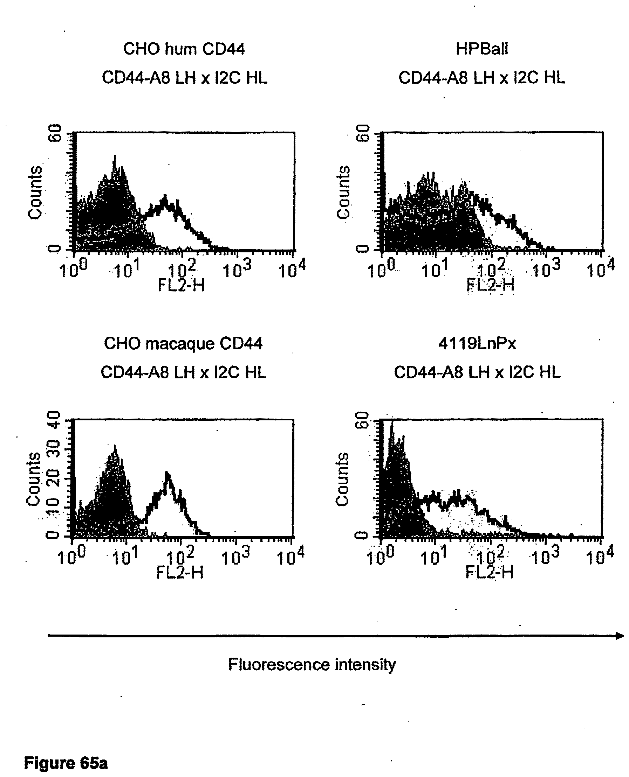

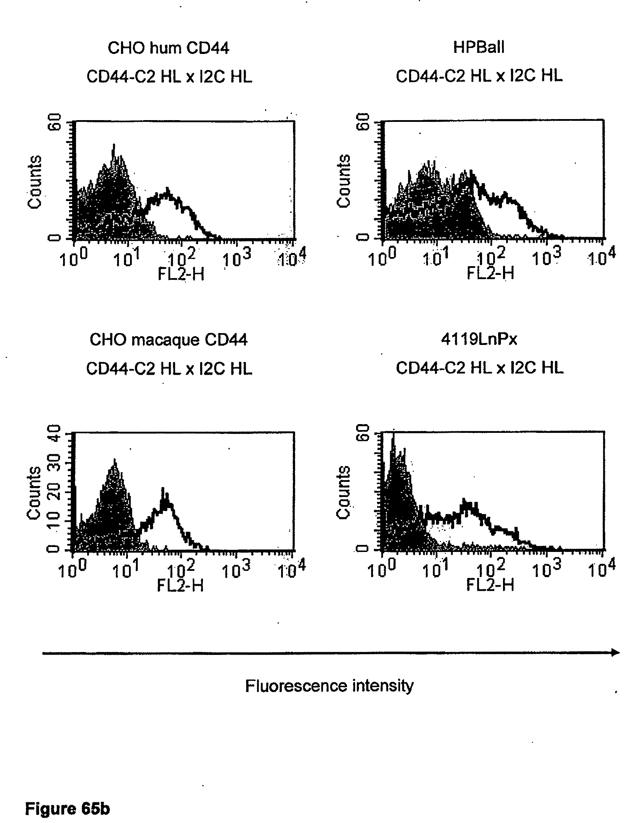

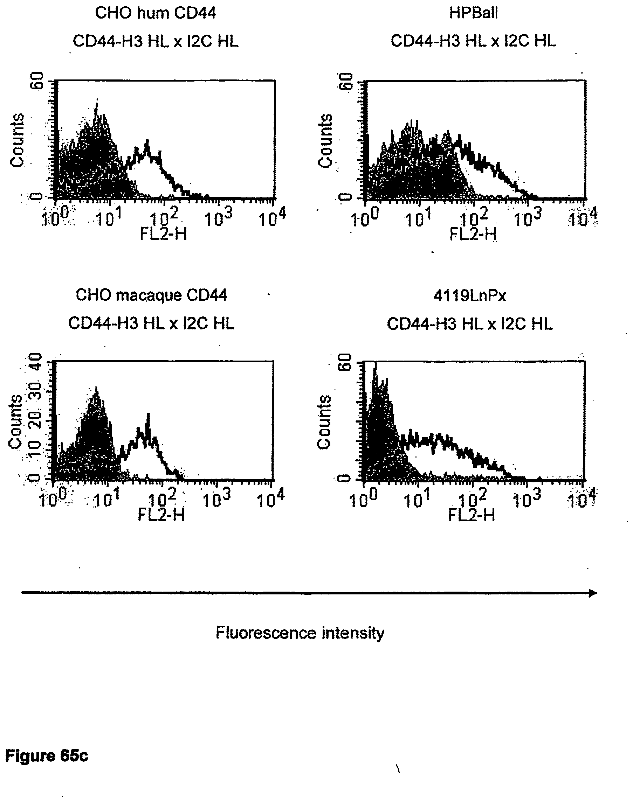

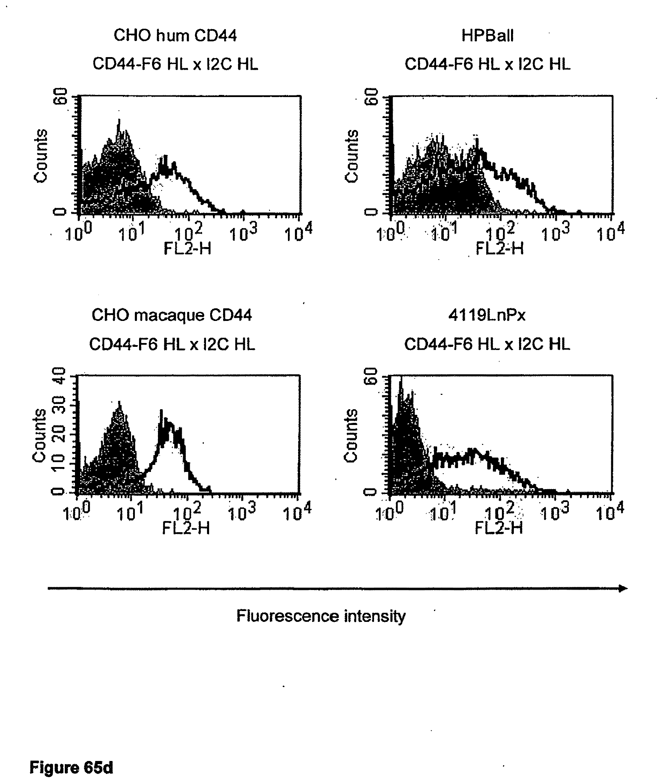

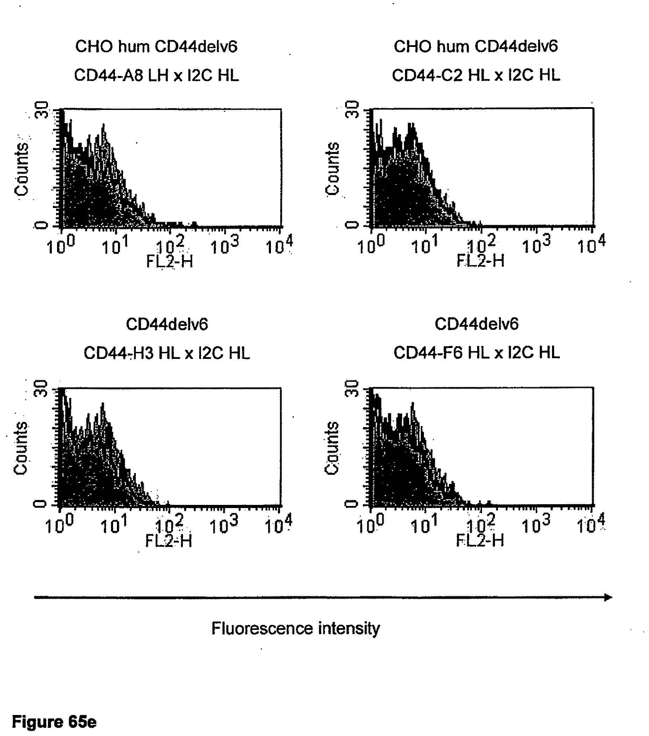

The bispecific binding of the single chain molecules listed above, which are cross-species specific for CD44 and cross-species specific for human and macaque CD3 was clearly detectable as shown in Figure 65 a-d. No binding of the molecules to CD44delv6 was seen in Figure 65 e. Cross-species specificity of the bispecific antibodies to human and macaque CD3 and CD44 antigens was therefore clearly demonstrated.

**39.4 Bioactivity of CD44 and CD3 cross-species specific bispecific single chain antibodies**

Bioactivity of the generated bispecific single chain antibodies was analyzed by chromium 51 ($^{51}$Cr) release in vitro cytotoxicity assays using the human CD44 positive cell line described in 28.1. As effector cells stimulated human CD4/CD56 depleted PBMC, stimulated human PBMC were used as specified in the respective figure.

Generation of the stimulated CD4/CD56 depleted PBMC was performed as follows:
Coating of a Petri dish (145 mm diameter, Greiner bio-one GmbH, Kremsmünster) was carried out with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 $\times$ 10$^7$ PBMC were added to the precoated petri dish in 120 ml of RPMI 1640 with stabilized glutamine /10% FCS / IL-2 20 U/ml (Proleukin, Chiron) and stimulated for 2 days. On the third day the cells were collected and washed once with RPMI 1640. IL-2 is added to a final concentration of 20 U/ml and the cells were cultivated again for one day in the same cell culture medium as above. By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}$Cr in a final volume of 100 $\mu$l RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250$\mu$l supplemented RPMI (as above) with an E:T ratio 10:1. Cell supernatant containing the cross-species specific bispecific single chain antibody molecules and 20 twofold dilutions thereof were applied for the human cytotoxicity assay. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

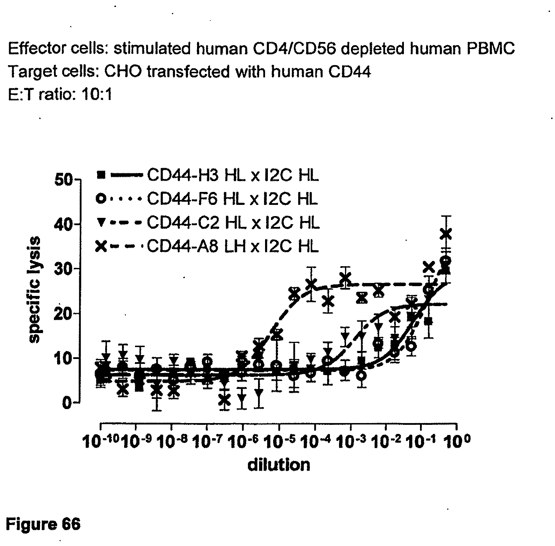

As shown in Figure 66, all of the generated cross-species specific bispecific single chain antibody constructs demonstrated cytotoxic activity against CD44 positive target cells elicited by stimulated human CD4/CD56 depleted PBMC.

**40. Generation of an HCV antigen and CD3 cross-species specific bispecific tandem single chain antibody fragment**

**40. 1. Generation of CHO cells expressing HCV envelope glycoprotein E2 (HCV Genotypes 1a and 1b)**

The coding sequences of hepatitis C virus (HCV) envelope glycoprotein E2 (HCV Genotype 1a and 1b, HCV isolates of the respective subtypes; sequences determined according to standard protocols) each as translational fusion proteins with the human CD4 transmembrane and intracellular domain were obtained by gene synthesis according to standard protocols. The gene synthesis fragments were designed as to contain first a Kozak site for eukaryotic expression of the construct, followed by the coding sequence of a 14 amino acid signal peptide, followed by the coding sequence of the first 278 (genotype 1a) or 279 (genotype 1b) amino acids of the HCV E2 envelope glycoprotein, a doubled myc epitope sequence and the coding sequence of the human CD4 transmembrane and intracellular domain corresponding to amino acids 372 to 433 of the mature protein (as published in GenBank accession number M12807) and a stop codon (the cDNA and amino acid sequences of the constructs were listed under SEQ ID NOs HCV-1 to HCV-4, genotypes 1a and 1b respectively). The gene synthesis fragments were also designed as to introduce restriction sites at the beginning and at the end of the fragment. The introduced restriction sites, EcoRI at the 5' end and Sall at the 3' end, were utilised in the following cloning procedures. The gene synthesis fragments were cloned via EcoRI and Sall into a plasmid designated pEF-DHFR (pEF-DHFR is described in Raum et al. Cancer Immunol Immunother 50 (2001) 141-150) following standard protocols. The afore-mentioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX. Finally two cell lines were generated: a CHO cell line with the HCV E2 domain genotype 1a expressed on the surface and another CHO cell line with the HCV E2 domain genotype 1b expressed on the surface.

**40.2 Cloning of a cross-species bispecific tandem single chain antibody fragment**

Generally, a bispecific tandem single chain antibody fragment, comprising a domain with a binding specificity cross-species specific for human and macaque CD3epsilon as well as a domain with a binding specificity for HCV, was designed as set out in the following Table 16:

Table 16: Format of an anti-CD3 cross-species specific and anti-HCV antigen specific bispecific tandem single chain antibody fragment

| SEQ ID (nucl/prot) | Format of protein construct (N → C) |
| --- | --- |
| 1664/1663 | HC1LH×I2CHL |

The aforementioned construct containing the variable light-chain (L) and variable heavy-chain (H) domains specific for HCV antigens and VH and VL combinations cross-species specific for human and macaque CD3 were obtained by gene synthesis. The gene synthesis fragment was designed as to contain first a Kozak site for eukaryotic expression of the construct, followed by a 19 amino acid immunoglobulin leader peptide, followed in frame by the coding sequence of the bispecific tandem single chain antibody fragment, followed in frame by the coding sequence of a histidine$_6$-tag and a stop codon. The gene synthesis fragment was also designed as to introduce suitable restriction sites at the beginning and at the end of the fragment. The introduced restriction sites were utilised in the following cloning procedures. The gene synthesis fragment was cloned via these restriction sites into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the bispecific tandem single chain antibody fragment. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**40.3 Expression and purification of the bispecific tandem single chain antibody fragment**

The bispecific tandem single chain antibody fragment was expressed in Chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by addition of increasing concentrations of MTX up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection is performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific tandem single chain antibody fragment was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein was >95% as determined by SDS-PAGE.

The bispecific tandem single chain antibody fragment has a molecular weight of about 52 kDa under native conditions as determined by gel filtration in PBS. Western Blot was performed using an Optitran® BA-S83 membrane and the Invitrogen Blot Module according to the protocol provided by the manufacturer. The antibodies used are directed against the His Tag (Penta His, Qiagen) and Goat-anti-mouse Ig labeled with alkaline phosphatase (AP) (Sigma), and BCIP/NBT (Sigma) as substrate. A single band was detected at 52 kD corresponding to the purified bispecific tandem single chain antibody fragment.

### 40.4 Flow cytometric binding analysis of the HCV antigen and CD3 cross-species specific bispecific tandem single chain antibody fragment

In order to test the functionality of the cross-species specific bispecific tandem single chain antibody fragment regarding the capability to bind to HCV antigen and to human and macaque CD3, respectively, a FACS analysis was performed. For this purpose CHO cells transfected with HCV antigen E2 from two different HCV genotypes (1a and 1b) as described in Example 40.1, the human CD3 positive T cell leukemia cell line HPB-ALL (DSMZ, Braunschweig, ACC483) and the macaque T cell line 4119LnPx were used to test the binding to the respective antigens. 200000 cells of the respective cell lines were incubated for 30 min. on ice with 50 µl of cell culture supernatant of transfected cells expressing the cross-species specific bispecific antibody construct. The cells were washed twice in PBS with 2% FCS and binding of the bispecific tandem single chain antibody fragment was detected with a murine anti-His antibody (Penta His antibody; Qiagen; diluted 1:20 in 50 µl PBS with 2% FCS). After washing, bound anti-His antibodies were detected with an Fc gamma-specific antibody (Dianova) conjugated to phycoerythrin, diluted 1:100 in PBS with 2% FCS. PBS with 2% FCS.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

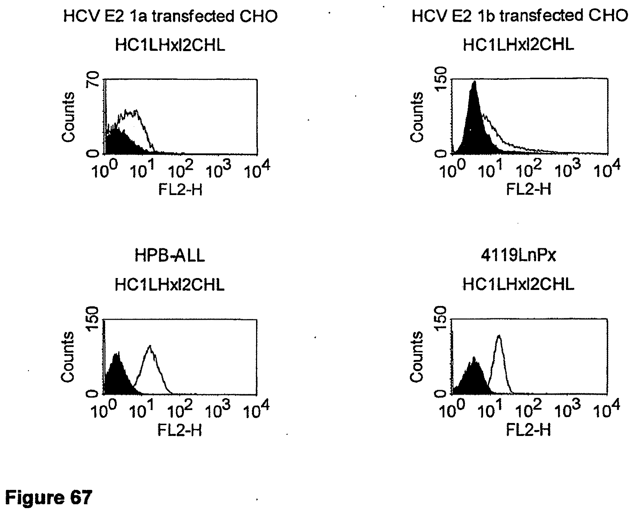

Bispecific binding of the bispecific tandem single chain antibody fragment to HCV antigen and to human and non-chimpanzee primate CD3 was clearly detectable as shown in Figure 67. In the FACS analysis the bispecific tandem single chain antibody fragment showed binding to CD3 and HCV antigens as compared to the respective negative controls. Cross-species specificity of the bispecific tandem single chain antibody fragment for human and macaque CD3 on the one hand and HCV-specificity on the other hand was demonstrated.

### 40.5 Bioactivity of the HCV-antigen and CD3 cross-species specific bispecific tandem single chain antibody fragment

Bioactivity of the bispecific tandem single chain antibody fragment was analyzed by . chromium 51 ($^{51}$Cr) release in vitro cytotoxicity assays using the HCV antigen positive cell line described in Example 40.1. As effector cells stimulated human CD4/CD56 depleted PBMC from two different donors were used.

A Petri dish (145 mm diameter, Greiner bio-one GmbH, Kremsmünster) was coated with a commercially available anti-CD3 specific antibody (e.g. OKT3, Orthoclone) in a final concentration of 1 µg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 $\times$ 10$^7$ PBMC were added to the precoated Petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultured again for one day in the same cell culture medium as above.

By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}$Cr in a final volume of 100 µl RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250 µl supplemented RPMI (as above) with an E:T ratio of 10:1. 76.4 µg/ml of the cross-species specific bispecific tandem single chain antibody fragment and 20 twofold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA).

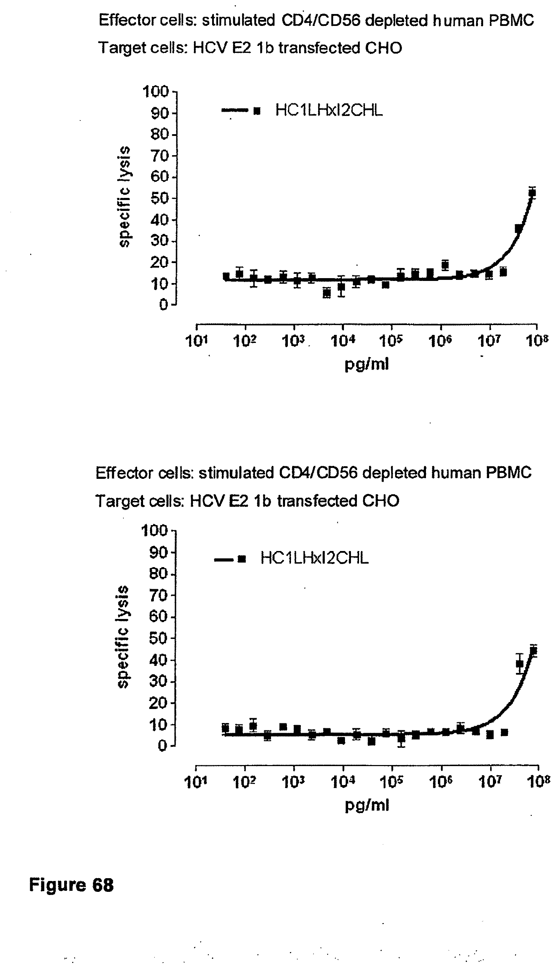

As shown in Figure 68, the cross-species specific bispecific tandem single chain antibody fragment demonstrated cytotoxic activity against human HCV antigen positive target cells.

**41. Bispecific tandem single chain antibody fragments directed at human and non-chimpanzee primate CD3 and at hepatitis virus antigens displayed on the surface of infected cells**

**41.1 Hepatitis B virus (HBV)**

The following degenerated oligonucleotides, that mutually overlap in a terminal stretch of approximately 15-20 nucleotides and wherein every second primer is an antisense primer, were used to provide a human VH sequence context for CDRH1 (SEQ ID 1634), CDRH2 (SEQ ID 1635) and CDRH3 (SEQ ID 1636):

5'C8-VH-A-XhoI

CCACTTCTCGAGTCTGGCGSCGRASTGMWGMAGCCTGGCGSCTCCSTGMRGS
TGTCCTGCRMGGCCTCCGGC

3'C8-VH-B

CCCTGGAGCCTGCCGCACCCAGGACATGGCGTAGCCGGWGAAGGTGWAGCC
GGAGGCCKYGCAGGA

5'C8-VH-C

CGGCAGGCTCCAGGGMAGGGACTGGAATGGRTGRGCTCCATCTCCGGCTCCG
GCGGCTCCACCTACTACGCCGACTCCGTGAAGGGCCGG

3'C8-VH-D

CTTGGCGCAGTAGTACASGGCGGTGTCCTCGGMCCGCAGGGAGYTCAKCTSCA
KGTACASGGTGYTCKTGGAGKTGTCCCGGSTSATTGTGAMCCGGCCCTTCACG
GA

3'C8-VH-E-BstEII

GACCGTGGTGACCAGGGTGCCCTGGCCCCAGTTGCCCAGAGGGAAGTAGTAG
ATGGAGGAGCCGTAGTATTCCTGCCGGCCAGGAGGCTTGGCGCAGTAGTA

This primer sets spans over the whole VH sequence. Within this set, primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

The resulting VH PCR product was then used as a template for a standard PCR reaction using primers that incorporate N-terminal and C-terminal suitable cloning restriction sites (primer 5'C8-VH-A-XhoI and primer 3'C8-VH-E-BstEII). The DNA fragment of the correct size (for a VH approximately 350 nucleotides) was isolated by agarose gel electrophoresis according to standard methods. In this way sufficient VH DNA fragment was amplified.

The following degenerated oligonucleotides, that mutually overlap in a terminal stretch of approximately 15-20 nucleotides and wherein every second primer is an antisense primer, were used to provide a human VL sequence context for CDRL1 (SEQ ID 1639), CDRL2 (SEQ ID 1640) and CDRL3 (SEQ ID 1641), which were appendant to CDRH1 (SEQ ID 1634), CDRH2 (SEQ ID 1635) and CDRH3 (SEQ ID 1636):

5'C8-VI-A-SacI

CCTCAAGAGCTCGCCCTGAYGCAGCCTSCCTCCGTGTCCGTGKCCCYTGGCMA
GACCGCCMGCATCACCTGCGGCGGCAAC

3'C8-VI-B

CAGCACAGGGGMCTGTCCTGGCTTCTGCTGATACCAGTGCACGGACTTGGAGC
CGATGTTGTTGCCGCCGCAGGTGAT

5'C8-VI-C

CAGAAGCCAGGACAGKCCCCTGTGCTGGTCRTATACGACGACTCCGACCGCCC
TTCCGGCATCCCTGAGCGGTTCTCCGGCTCC

3'C8-VI-D

GACCTGGCAGTAGTAGTCGGCCTCGTCCMYGGCCTSCRCCCSGGAGATGGTCA
GGGTGRCGGTGKTGCCGGAGYTGGAGCCGGAGAACCG

3'C8-VI-E-BsiWI/SpeI

CCCGATACTAGTCGTACGCAGCACGGTCAGCTTTGTTCCGCCGCCAAACACCA
CCAGGTCGGAGGAGGAGTCCCAGACCTGGCAGTAGTAGTCGGC

This primer set spans over the whole corresponding VL sequence. Within this set, primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.

The resulting VL PCR product was then used as a template for a standard PCR reaction using primers that incorporate N-terminal and C-terminal suitable cloning restriction sites (primer 5'C8-VI-A-SacI and primer 3'C8-VI-E-BsiWI/SpeI). The DNA fragment of the correct size (for a VL approximately 330 nucleotides) was isolated by agarose gel electrophoresis according to standard methods. In this way sufficient VL DNA fragment was amplified.

The final VH PCR product (i.e. the repertoire of human/humanized VH) was then combined with its respective final VL PCR product (i.e. the repertoire of human/humanized VL) in the phage display vector pComb3H5Bhis to form a library of functional scFv antibody fragments from which - after display on filamentous phage - anti-HBS binders were selected, screened, identified and confirmed as described as follows:

450 ng of the light chain fragments (Sacl-Spel digested) were ligated with 1400 ng of the phagemid pComb3H5Bhis (Sacl-Spel digested; large fragment). The resulting combinatorial antibody library was then transformed into 300 µl of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells were then harvested by centrifugation and plasmid preparation was carried out using a commercially available plasmid preparation kit (Qiagen).

2800 ng of this plasmid-DNA containing the VL-library (Xhol-BstEll digested; large fragment) were ligated with 900 ng of the heavy chain V-fragments (Xhol-BstEll digested) and again transformed into two 300 µl aliquots of electrocompetent E. coli XL1 Blue cells by electroporation (2.5 kV, 0.2 cm gap cuvette, 25 uFD, 200 Ohm) resulting in a total library size of VH-VL scFv antibody fragments of more than $10^7$ independent clones.

After phenotype expression and slow adaptation to carbenicillin, the E. coli cells containing the antibody library were transferred into SB-Carbenicillin (50 µg/mL) selection medium. The E. coli cells containing the antibody library was then infected with an infectious dose of $10^{12}$ particles of helper phage VCSM13 resulting in the production and secretion of filamentous M13 phage, wherein phage particle contains single stranded pComb3H5BHis-DNA encoding a scFv antibody fragment and displayed the corresponding scFv antibody fragment as a translational fusion to phage coat protein III. This pool of phages displaying the antibody library was used for the selection of antigen binding entities.

Likewise the foregoing procedure was applied for another target binder. For this purpose the following degenerated oligonucleotides, that mutually overlap in a terminal stretch of approximately 15-20 nucleotides and wherein every second primer is an antisense primer, were used to provide a human VH sequence context for CDRH1 (SEQ ID 1669), CDRH2 (SEQ ID 1670) and CDRH3 (SEQ ID 1671):

5'C9-VH-A-Xhol

CCACTTCTCGAGTCTGGGGGSAGRGGTGRWGMAGCCTGGGRSGTCCSTGARAS

TCTCCTGTGCAGCCTCTGGA

3'C9-VH-B

CCACTCCAGCCCCTKGCCTGGAGCCTGGCGGACCCAGTGTATGCCATAATTAC

TGAAGGTGWATCCAGAGGCTGCACAGGA

5'C9-VH-C

GCTCCAGGCMAGGGGCTGGAGTGGRTGGSACTTATATCATATGATGGAAATAA

GAAATTCTATGCAGACTCCGTGAAGGGCCGA

3'C9-VH-D

GTAATATACAGCCGTGTCCTCAGRTCTCAGGCTGCTCAKTTSCAKATMCACCGT

GYTCKTAGAAGTGTCTCTGGTSATGGYGAMTCGGCCCTTCACGGAGTC

3'C9-VH-E-BstEll

GACCGTGGTGACCAGGGGTTCCCTGGCCCCAGTAGTCAAATTCCCCCCAGTACA

AGGCAATACCCCCAGATTTCGCACAGTAATATACAGCCGTGTC

As primers for the final standard PCR reaction to incorporate N-terminal and C-terminal suitable cloning restriction sites into VH the oligonucleotides 5'C9-VH-A-Xhol and 3'C9-VH-E-BstEll were used.

Analogous to VH, the following degenerated oligonucleotides, that mutually overlap in a terminal stretch of approximately 15-20 nucleotides and wherein every second primer is an antisense primer, were used to provide a human VL sequence context for CDRL1 (SEQ ID 1672), CDRL2 (SEQ ID 1673) and CDRL3 (SEQ ID 1674), which are appendant to CDRH1 (SEQ ID 1669), CDRH2 (SEQ ID 1670) and CDRH3 (SEQ ID 1671):

5'C9-VI-A-Sacl

CCTCAAGAGCTCGWACTGACTCAGCCACCCTCGGTGTCAGYGGCCCCAGGACA
GAMGGYCAYGATTTCCTGTGGGGGAAAC

3'C9-VI-B

GCCTGGCWKCTGCTGATACCAGTGCACGGTTGTACTTCCAATGTTGTTTCCCCC
ACAGGAAAT

5'C9-VI-C

TGGTATCAGCAGMWGCCAGGCMMGGCCCCTRWGCTGSTCRTCTATGATGATA
ACGAGCGACCCTCAGGGATCCCTGASCGATTCTCTGGCTCC

3'C9-VI-D

CACTTGACAATAATAGTCGGCCTCATCCCCGGYTTSGASCCYGKTGATGSYCAG
GGTGGCCGAGGTCCCAGASTTGGAGCCAGAGAATCG

3'C9-VI-E-BsiWI/Spel

CCCGATACTAGTCGTACGTAGGACGGTCAGCTTCGTCCCTCCGCCGAATACCA
CATGATCACTACCACTATCCCACACTTGACAATAATAGTC

As primers for the final standard PCR reaction to incorporate N-terminal and C-terminal suitable cloning restriction sites into VL the oligonucleotides 5'C9-VI-A-Sacl and 3'C9-VI-E-BsiWI/Spel were used.

For the selection of antigen binding entities the phage library carrying the cloned scFv-repertoire was harvested from the respective culture supernatant by PEG (polyethyleneglycole) 8000/NaCl precipitation and centrifugation. Approximately $10^{11}$ to $10^{12}$ scFv phage particles are resuspended in 0.4 ml of PBS/0.1 % BSA and incubated with immobilized HBS antigen (e.g. EngerixR -B, GlaxoSmithKline, Germany; HBvaxProTM, Aventis Pasteur MSD SNC, France) for 2 h under gentle agitation at 37°C. The immobilized HBS antigen (e.g. 5 μg/ml PBS) was coated to the wells and the wells subsequently blocked according to standard protocols.

Phage particles displaying scFv antibody fragments that do not specifically bind to the target antigen were eliminated by washing steps with PBS/0.1 % BSA (Tween 20 can be added to the washing solution to increase washing stringency). After washing, binding entities were eluted by using HCl-glycine pH 2.2 and after neutralization with 2 M Tris pH 12, the eluate was used for infection of a fresh uninfected E. coli XL1 Blue culture.

To elute remaining strong binding entities in the eluted wells, 200 μl of a fresh E. coli XL1 blue culture (OD600 > 0.5) were added to the eluted wells and incubated for further 10 minutes under gentle agitation. Both E. coli cultures were then mixed and cells successfully transduced with a phagemid copy, encoding a human scFv antibody fragment, were again selected for carbenicillin resistance and subsequently infected with VCMS 13 helper phage to start the second round of antibody display and in vitro selection. A total of 4 to 5 rounds of selections was carried out, normally.

In order to screen for HBS specific binders plasmid DNA corresponding to 4 and 5 rounds of panning was isolated from E. coli cultures after selection. For the production of soluble scFv antibody fragment, VH-VL-DNA fragments were excised from the plasmids (Xhol-Spel). These fragments were cloned via the same restriction sites into the plasmid pComb3H5BFlag/His differing from the original pComb3H5BHis in that the expression construct (e.g. scFv antibody fragment) includes a Flag-tag (DYKDDDDK) between the scFv antibody fragment and the $His_6$-tag and the additional phage proteins were deleted. After ligation, each pool (different rounds of panning) of plasmid DNA was transformed into 100 $\mu$l heat shock competent E. coli TG1 or XLI blue and plated onto carbenicillin LB-agar. Single colonies were picked into 100 $\mu$l of LB carb (50 $\mu$g/ml).

E. coli transformed with pComb3H5BHis containing a VL-and VH-segment produce soluble scFv antibody fragment in sufficient amounts after excision of the gene III fragment and induction with 1 mM IPTG. Due to a suitable signal sequence, the scFv antibody fragment was exported into the periplasma where it folds into a functional conformation.

Single E. coli TG1 bacterial colonies from the transformation plates were picked for periplasmic small scale preparations and grown in SB-medium (e.g. 10 ml) supplemented with 20 mM $MgCl_2$ and carbenicillin 50$\mu$g/ml (and re-dissolved in PBS (e.g. 1 ml) after harvesting. By four rounds of freezing at -70°C and thawing at 37°C, the outer membrane of the bacteria was destroyed by temperature shock and the soluble periplasmic proteins including the scFv antibody fragments were released into the supernatant. After elimination of intact cells and cell-debris by centrifugation, the supernatant containing the anti-HBS scFv antibody fragments was collected and used for the identification of HBS specific binders as follows:

HBS antigen was coated on wells of 96 well plastic plates (Nunc, maxisorb) typically at 4°C over night. Wells were washed once with PBS/0.05 % Tween 20 and subsequently blocked with PBS/3 % BSA for at least one hour. After removal of the blocking solution, periplasma preparations and control solutions were added to the wells and incubated for typically one hour at room temperature. The wells were then washed three times with PBS/0.05% Tween 20. Detection of scFv antibody fragments and control antibodies bound to immobilized antigen was carried out using a monoclonal murine anti-$HiS_6$ or anti FLAG-tag antibody (Qiagen anti-PentaHis and M2 anti Flag Sigma, typically each at a final concentration of 1 $\mu$g/ml PBS) detected with a peroxidase-labeled polyclonal goat anti-mouse Fab-fragment antibody (Dianova,1ug/ml PBS). The signal was developed by adding ABTS substrate solution and measured at a wavelength of 405 nm. Unspecific binding of the test-samples to the blocking agent was examined by carrying out the identical assays with the identical reagents and identical timing on ELISA plates which were not antigen-coated but blocked with BSA, only.

To exclude the possibility that the positive binding might be due to binding to BSA (to be used as a blocking agent in the first ELISA experiment), a second ELISA experiment was performed in parallel. In this second ELISA experiment, all parameters were identical to those in the first ELISA experiment, except that in the second ELISA experiment no coating with antigen, but only blocking with BSA takes place.

In order to identify those HBS-specific scFv antibody fragments identified by ELISA, which also bind to HBS-antigen as displayed on the surface of HBV-infected cells, indirect immunofluorescence with FLAG-tagged scFv antibody fragment followed by FITC-conjugated anti-FLAG antibody on HepG2.2.15 cells (Bohne F, Chmielewski M, Ebert G, et al. T cells redirected against hepatitis B virus surface proteins eliminate infected hepatocytes. Gastroenterology 2008; 134:239-47) was carried out according to standard protocols (e.g. Current Protocols in Immunology, Coligan, Kruisbeek, Margulies, Shevach and Strober, Wiley-Interscience, 2002, unit 21.3). Uninfected HepG2 cells served as negative control. Culture conditions of HepG2.2.15 cells for optimal display of HBS-antigen on the cell surface were described by Bohne et al. (Bohne F, Chmielewski M, Ebert G, et al. T cells redirected against hepatitis B virus surface proteins eliminate infected hepatocytes. Gastroenterology 2008; 134:239-47).

Clones confirmed to specifically bind to the HBS antigen on the surface of HBV infected cells were then analyzed for favourable protein properties and amino acid sequence. HBS specific scFv antibody fragments were converted into recombinant bispecific tandem single chain antibody fragments by joining them via a $Gly_4Ser_1$-linker with the CD3 specific scFv antibody fragment I2C (SEQ ID 185) or any other CD3 specific scFv antibody fragment of the invention to result in constructs with the domain arrangement $VH_{Muc1}$ - $(Gly_4Ser_1)_3$ -$VL_{Muc1}$- $Gly_4Ser_1$-$VH_{CD3}$ - $(Gly_4Ser_1)_3$-$VL_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal $His_6$-tag followed by a stop codon were both attached in frame to the nucleotide sequence encoding the bispecific tandem single chain antibody fragments prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the bispecific single chain antibodies into the culture supernatant and gene amplification with methotrexat for increasing expression levels were carried out as described (Mack et al. Proc. Natl. Acad. Sci. USA 92 (1995) 7021-7025). All other state of the art procedures were carried out according to standard protocols (Sambrook, Molecular Cloning; A Laboratory Manual, 3rd edition, Cold Spring Harbour Laboratory Press, Cold Spring Harbour, New York (2001)).

Functional bispecific tandem single chain antibody fragments were identified by ELISA and flowcytometry using culture supernatant from transfected CHO cells. For this purpose CD3 binding was tested on the human CD3 positive T cell leukemia cell line HPB-ALL (DSMZ, Braunschweig, ACC483) and on the macaque T cell line 4119LnPx. 200.000 cells of the respective cell line were incubated for 30 min. on ice with 50 μl of cell culture supernatant. The cells were washed twice in PBS with 2% FCS and bound bispecific tandem single chain antibody fragment was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control.

Flow cytometry was performed on a FACS-Calibur apparatus; the CellQuest software is used to acquire and analyze the data (Becton Dickinson biosciences, Heidelberg). FACS staining and measuring of the fluorescence intensity are performed as described in Current Protocols in Immunology (Coligan, Kruisbeek, Margulies, Shevach and Strober, Wiley-Interscience, 2002). Binding to HBS antigen was tested by ELISA as described above. Detection of bound bispecific tandem single chain antibody fragment was carried out using a monoclonal murine anti-His$_6$ antibody (Qiagen anti-PentaHis typically at a final concentration of 1 μg/ml PBS) followed by a peroxidase-labeled polyclonal goat anti-mouse Fab-fragment antibody (Dianova, 1ug/ml PBS).

Only those constructs showing bispecific binding to human and macaque CD3 as well as to HBS antigen were selected for further use.

For protein production, CHO cells expressing fully functional bispecific tandem single chain antibody fragments and adapted to nucleoside-free HyQ PF CHO liquid soy medium (with 4.0 mM L-Glutamine with 0.1% Pluronic F - 68; HyClone) were grown in roller bottles with nucleoside-free HyQ PF CHO liquid soy medium (with 4.0 mM L-Glutamine with 0.1% Pluronic F - 68; HyClone) for 7 days. Culture supernatant was cleared from cells by centrifugation and stored at -20°C until purification. As chromatography equipment for purification of bispecific tandem single chain antibody fragment from culture supernatant Äkta® Explorer System (GE Health Systems) and Unicorn® Software were used. Immobilized metal affinity chromatography ("IMAC") was performed using a Fractogel EMD chelate® (Merck) which was loaded with ZnCl$_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations are determined using OD280 nm.

Purified bispecific tandem single chain antibody fragment was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific tandem single chain antibody fragment has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific tandem single chain antibody fragments, an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific tandem single chain antibody fragment.

The potency in the human and the non-chimpanzee primate system of bispecific tandem single chain antibody fragments interacting with human and macaque CD3 and with HBS antigen was determined by a non-radioactive cytotoxicity assay using HepG2.2.15 cells as target cells as described by Bohne et al. (Bohne F, Chmielewski M, Ebert G, et al. T cells redirected against hepatitis B virus surface proteins eliminated infected hepatocytes. Gastroenterology 2008; 134:239-47) and stimulated human CD4/CD56 depleted PBMC or the macaque T cell line 4119LnPx instead of T cells transduced with a chimeric T cell receptor as effector cells. In order to obtain dose-response-curves, the concentration of each bispecific tandem single chain antibody fragment (instead of the E:T-ratio as described by Bohne et al.) was titrated in the cytotoxicity assay at a fixed E:T-ratio (e.g. 10:1 or 5:1).

Generation of stimulated human PBMC was performed as follows:

A Petri dish (85 mm diameter, Nunc) was coated with a commercially available anti-CD3 specific antibody (e.g. OKT3, Othoclone) in a final concentration of 1 $\mu$g/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × $10^7$ PBMC were added to the precoated petri dish in 50 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

Analysis of the experimental data from the cytotoxicity assay was performed with Prism 4 for Windows (version 4.02, GraphPad Software Inc., San Diego, California, USA). Sigmoidal dose response curves typically have $R^2$ values >0.90 as determined by the software. $EC_{50}$ values as measure of potency were calculated by the analysis program.

### 41.2 Hepatitis C virus (HCV)

The following degenerated oligonucleotides, that mutually overlap in a terminal stretch of approximately 15-20 nucleotides and wherein every second primer is an antisense primer, are used to provide a human VH sequence context for CDRH1 (SEQ ID 1652), CDRH2 (SEQ ID 1653) and CDRH3 (SEQ ID 1654):

5'HC1-VH-A-Xhol

CAGCTACTCGAGTSGGGCGSAGGACTGKTGMAGCCTKSGGRGTCCCTGAGWC TCTCCTGCGCTG

3'HC1-VH-B

CCACTCCAGCCCCTTCCCAGGGGMCTGGCGGAYCCAGGTCCAGAAGTAACCAC TWAAGGTAMMACCAKAGRCAGCGCAGGAGAGWCTCAGGGAC

5'HC1-VH-C

CTGGGAAGGGGCTGGAGTGGRTTRGTGAAAGCAATTATAGTGGAAGTACCAGG TACAACCCGTCCCTCAAGAGTCGAKTCACCATATCA

3'HC1-VH-D

ATACAGCCGTGTCCKCGGCTSTCASAGAAYTCAKCTKCAGGKAGARCKKGTTCT KGGAGKTGTCTMYTGATATGGTGAMTCGACTCTTG

5'HC1-VH-E

AGCCGMGGACACGGCTGTATATTACTGTGCGAGAGGTTGGGCGGTGGACGGT ATGGACGTCTGGGGCCGAGGGACCTTGGTCACCGTC

3'HC1-VH-F-BstEll
GAGACGGTGACCAAGGTCCCTCGGCCCCAGACGTCCATACC

This primer sets spans over the whole VH sequence. Within this set, primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

The resulting VH PCR product was then used as a template for a standard PCR reaction using primers that incorporate N-terminal and C-terminal suitable cloning restriction sites (primer 5'HC1-VH-A-XhoI and primer 3'HC1-VH-F-BstEII). The DNA fragment of the correct size (for a VH approximately 350 nucleotides) was isolated by agarose gel electrophoresis according to standard methods. In this way sufficient VH DNA fragment was amplified.

The following degenerated oligonucleotides, that mutually overlap in a terminal stretch of approximately 15-20 nucleotides and wherein every second primer was an antisense primer, were used to provide a human VL sequence context for CDRL1 (SEQ ID 1657), CDRL2 (SEQ ID 1658) and CDRL3 (SEQ ID 1659), which are appendant to CDRH1 (SEQ ID 1652), CDRH2 (SEQ ID 1653) and CDRH3 (SEQ ID HC1 1654):

5'HC1-VI-A-SacI
ACTAGCGAGCTCGWGCTGACACAGCCACCCTCG

5'HC1-VI-B

GCTGACACAGCCACCCTCGGYGTCAGKGACCCCAGGACAGASGGYCASGATCT
CCTGCTCTGGAGATGCATTGCCAAAGCAATATGC

3'HC1-VI-C

CCCTGAGGGCCTCTCATTATCTTTATATATCASCAACWYAGGGGGCCKKGCCTGG
CWKCTGCTGATACCAGTAAGCATATTGCTTTGGCAATGC

5'HC1-VI-D

GATAATGAGAGGCCCTCAGGGGRTCCCTGASCGATTCTCTGGCTCCARGTCAGG
GACATCAGYCTCGTTGRCCATCAGTGGASTCMRGKCAGAAGACGAGGCTGACT
A

3'HC1-VI-E-BsiWI/SpeI

GCTACTACTAGTCGTACGTAGGACGGTCAGCTGGGTCCCTCCGCCGAACACCC
AGGAAGAACCACTGCTGTCTGCTGATTGACAGTAATAGTCAGCCTCGTCTTCTG

This primer set spans over the whole corresponding VL sequence. Within this set, primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

The resulting VL PCR product was then used as a template for a standard PCR reaction using primers that incorporate N-terminal and C-terminal suitable cloning restriction sites (primer 5'HC1-VI-A-SacI. and primer 3'HC1-VI-E-BsiWI/SpeI). The DNA fragment of the correct size (for a VL approximately 330 nucleotides) was isolated by agarose gel electrophoresis according to standard methods. In this way sufficient VL DNA fragment was amplified.

The final VH PCR product (i.e. the repertoire of human/humanized VH) was then combined with its respective final VL PCR product (i.e. the repertoire of human/humanized VL) in the phage display vector pComb3H5Bhis to form a library of functional scFv antibody fragments from which - after displayon filamentous phage - anti-HBS binders were selected, screened, identified and confirmed as described as follows:

450 ng of the light chain fragments (Sacl-Spel digested) are ligated with 1400 ng of the phagemid pComb3H5Bhis (Sacl-Spel digested; large fragment). The resulting combinatorial antibody library was then transformed into 300 $\mu$l of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells are then harvested by centrifugation and plasmid preparation was carried out using a commercially available plasmid preparation kit (Qiagen).

2800 ng of this plasmid-DNA containing the VL-library (Xhol-BstEll digested; large fragment) were ligated with 900 ng of the heavy chain V-fragments (Xhol-BstEII digested) and again transformed into two 300 $\mu$l aliquots of electrocompetent E. coli XL1 Blue cells by electroporation (2.5 kV, 0.2 cm gap cuvette, 25 uFD, 200 Ohm) resulting in a total VH-VL scFv (single chain variable fragment) library size of more than $10^7$ independent clones.

After phenotype expression and slow adaptation to carbenicillin, the E. coli cells containing the antibody library were transferred into SB-Carbenicillin (50 $\mu$g/mL) selection medium. The E. coli cells containing the antibody library was then infected with an infectious dose of $10^{12}$ particles of helper phage VCSM13 resulting in the production and secretion of filamentous M13 phage, wherein phage particle contains single stranded pComb3H5BHis-DNA encoding a scFv antibody fragment and displayed the corresponding scFv antibody fragment as a translational fusion to phage coat protein III. This pool of phages displaying the antibody library was used for the selection of antigen binding entities.

For this purpose the phage library carrying the cloned repertoire of scFv antibody fragments was harvested from the respective culture supernatant by PEG8000/NaCl precipitation and centrifugation. Approximately $10^{11}$ to $10^{12}$ phage particles displaying scFv antibody fragment are resuspended in 0.4 ml of PBS/0.1% BSA and incubated with $10^5$ to $10^7$ CHO cells transfected with HCV antigen E2 from HCV genotypes 1a and/or 1b as described in Example 40.1 for 1 hour on ice under slow agitation. These HCV-antigen transfected CHO cells were harvested beforehand by centrifugation, washed in PBS and resuspended in PBS/1 % FCS (containing Na Azide). Phage particles displaying scFv antibody fragments, which do not specifically bind to the HCV-antigen transfected CHO cells, were eliminated by up to five washing steps with PBS/1 % FCS (containing Na Azide). After washing, binding entities were eluted from the cells by resuspending the cells in HCl-glycine pH 2.2 (10 min incubation with subsequent vortexing) and after neutralization with 2 M Tris pH 12, the eluate was used for infection of a fresh uninfected E. coli XL1 Blue culture (OD600 > 0.5). The E. coli culture containing E. coli cells successfully transduced with a phagemid copy, encoding a human/humanized scFv antibody fragment, were again selected for carbenicillin resistance and subsequently infected with VCMS 13 helper phage to start the second round of antibody display and in vitro selection. A total of 4 to 5 rounds of selections are carried out, normally.

In order to screen for HCV-antigen specific binders plasmid DNA corresponding to 4 and 5 rounds of panning was isolated from E. coli cultures after selection. For the production of soluble scFv antibody fragment, VH-VL-DNA fragments were excised from the plasmids (Xhol-Spel). These fragments were cloned via the same restriction sites into the plasmid pComb3H5BFlag/His differing from the original pComb3H5BHis in that the expression construct (e.g. scFv antibody fragment) includes a Flag-tag (DYKDDDDK) between the scFv antibody fragment and the His$_6$-tag and the additional phage proteins were deleted. After ligation, each pool (different rounds of panning) of plasmid DNA was transformed into 100 $\mu$l heat shock competent E. coli TG1 or XLI blue and plated onto carbenicillin LB-agar. Single colonies were picked into 100 $\mu$l of LB carb (50 $\mu$g/ml).

E. coli transformed with pComb3H5BHis containing a VL-and VH-segment produce soluble scFv antibody fragment in sufficient amounts after excision of the gene III fragment and induction with 1 mM IPTG. Due to a suitable signal sequence, the scFv antibody fragment was exported into the periplasma where it folds into a functional conformation.

Single E. coli TG1 bacterial colonies from the transformation plates were picked for periplasmic small scale preparations and grown in SB-medium (e.g. 10 ml) supplemented with 20 mM MgCl$_2$ and carbenicillin 50$\mu$g/ml (and redissolved in PBS (e.g. 1 ml) after harvesting. By four rounds of freezing at -70°C and thawing at 37°C, the outer membrane of the bacteria was destroyed by temperature shock and the soluble periplasmic proteins including the scFv antibody fragments are released into the supernatant. After elimination of intact cells and cell-debris by centrifugation, the supernatant containing the anti-HCV scFv antibody fragments was collected and used for the identification of HCV-anigen specific binders as follows:

Binding of scFv antibody fragments to HCV is tested by flow cytometry on HCV-antigen transfected CHO cells (see Example 40.1); untransfected CHO cell were used as negative control.

For flow cytometry 2,5$\times$10$^5$ cells were incubated with 50 ul periplasmic preparation scFv antibody fragment or with 5 $\mu$g/ml of purified scFv antibody fragments in 50 $\mu$l PBS with 2% FCS. The binding of scFv antibody fragment was detected with an anti-His antibody (Penta-His Antibody, BSA free, Qiagen GmbH, Hilden, FRG) at 2 $\mu$g/ml in 50 $\mu$l PBS with 2% FCS. As a second step reagent a R-Phycoerythrin-conjugated affinity purified F(ab')2 fragment, goat anti-mouse IgG (Fc-gamma fragment specific), diluted 1:100 in 50 $\mu$l PBS with 2% FCS (Dianova, Hamburg, FRG) was used. The samples were measured on a FACSscan (BD biosciences, Heidelberg, FRG). Single clones were then analyzed for favourable properties and amino acid sequence. HCV specific scFv antibody fragments were converted into recombinant bispecific tandem single chain antibody fragments by joining them via a $Gly_4Ser_1$-linker with the CD3 specific scFv antibody fragment I2C (SEQ ID 185) or any other CD3 specific scFv antibody fragment of the invention to result in constructs with the domain arrangement $VH_{Muc1}$ - $(Gly_4Ser_1)_3$ -$VL_{Muc1}$- $Gly_4Ser_1$-$VH_{CD3}$ - $(Gly_4Ser_1)_3$-$VL_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal $His_6$-tag followed by a stop codon were both attached in frame to the nucleotide sequence encoding the bispecific tandem single chain antibody fragments prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the bispecific tandem single chain antibody fragments into the culture supernatant and gene amplification with methotrexat for increasing expression levels were carried out as described (Mack et al. Proc. Natl. Acad. Sci. USA 92 (1995) 7021-7025). All other state of the art procedures were carried out according to standard protocols (Sambrook, Molecular Cloning; A Laboratory Manual, 3rd edition, Cold Spring Harbour Laboratory Press, Cold Spring Harbour, New York (2001)).

Identification of functional bispecific tandem single chain antibody fragments was carried out by flowcytometric analysis of culture supernatant from transfected CHO cells. For this purpose CD3 binding was tested on the human CD3 positive T cell leukemia cell line HPB-ALL (DSMZ, Braunschweig, ACC483) and on the macaque T cell line 4119LnPx. Binding to HCV was tested on HCV-antigen transfected CHO cells (see Example 40.1). 200.000 cells of the respective cell line were incubated for 30 min. on ice with 50 $\mu$l of cell culture supernatant. The cells were washed twice in PBS with 2% FCS and bound bispecific tandem single chain antibody fragment was detected with a murine anti-His antibody (Penta His antibody; Qiagen; diluted 1:20 in 50 $\mu$l PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

Only those constructs showing bispecific binding to human and macaque CD3 as well as to HCV-antigen were selected for further use.

For protein production CHO cells expressing fully functional bispecific tandem single chain antibody fragment and adapted to nucleoside-free HyQ PF CHO liquid soy medium (with 4.0 mM L-Glutamine with 0.1% Pluronic F - 68; HyClone) were grown in roller bottles with nucleoside-free HyQ PF CHO liquid soy medium (with 4.0 mM L-Glutamine with 0.1% Pluronic F - 68; HyClone) for 7 days. Culture supernatant was cleared from cells by centrifugation and stored at -20°C until purification. As chromatography equipment for purification of bispecific tandem single chain antibody fragment from culture supernatant Äkta® Explorer System (GE Health Systems) and Unicorn® Software were used. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample.

Bound protein was eluted using a two step gradient of buffer B (20 mM sodium phosphate buffer pH 7.2, 0.1 M NaCl, 0.5 M Imidazole) according to the following:

    Step 1: 20% buffer B in 6 column volumes
    Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific tandem single chain antibody fragment was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific tandem single chain antibody fragment has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. For detection of the bispecific tandem single chain antibody fragments, an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific tandem single chain antibody fragment.

The potency in the human and the non-chimpanzee primate system of bispecific tandem single chain antibody fragments interacting with human and macaque CD3 and with HCV-antigen was determined by a cytotoxicity assay based on chromium 51 ($^{51}$Cr) release using HCV-antigen transfected CHO cells as target cells (see Example 40.1) and stimulated human CD4/CD56 depleted PBMC or the macaque T cell line 4119LnPx as effector cells.

Generation of stimulated human PBMC was performed as follows:

A Petri dish (85 mm diameter, Nunc) was coated with a commercially available anti-CD3 specific antibody (e.g. OKT3, Othoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 × 10$^7$ PBMC were added to the precoated petri dish in 50 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above.

Target cells were washed twice with PBS and labelled with 11.1 MBq $^{51}$Cr in a final volume of 100 μl RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250μl supplemented RPMI (as above) with an E:T ratio of 10:1. 1 μg/ml of the cross-species specific bispecific tandem single chain antibody fragments and 20 threefold dilutions thereof were applied. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values as measure of potency were calculated by the analysis program.

The present invention can also be characterized by the following items:

(1) A polypeptide comprising a binding domain capable of binding to an epitope of human and non-chimpanzee primate CD3ε (epsilon) chain, wherein the epitope is part of an amino acid sequence comprised in the group consisting of SEQ ID NOs:2, 4, 6, or 8.

(2) The polypeptide of item **1,** wherein said binding domain capable of binding to an epitope of the human and non-chimpanzee primate CD3 ε (epsilon) chain is of human origin.

(3) The polypeptide according to any one of items **1** to **2,** wherein the polypeptide comprises a first binding domain capable of binding to an epitope of human and non-chimpanzee primate CD3 ε chain and a second binding domain capable of binding to a cell surface antigen.

(4) The polypeptide according to item **3,** wherein the second binding domain binds to a cell surface antigen of a human and a non-chimpanzee primate.

(5) The polypeptide according to any one of items **1** to **4,** wherein the first binding domain capable of binding to an epitope of human and non-chimpanzee primate CD3 ε chain comprises a VL region comprising CDR-L1, CDR-L2 and CDR-L3 selected from:

(a) CDR-L1 as depicted in SEQ ID NO:27, CDR-L2 as depicted in SEQ ID NO:28 and CDR-L3 as depicted in SEQ ID NO:29;
(b) CDR-L1 as depicted in SEQ ID NO:117, CDR-L2 as depicted in SEQ ID NO:118 and CDR-L3 as depicted in SEQ ID NO:119; and
(c) CDR-L1 as depicted in SEQ ID NO:153, CDR-L2 as depicted in SEQ ID NO:154 and CDR-L3 as depicted in SEQ ID NO:155.

(6) The polypeptide according to any one of items **1** to **4,** wherein the first binding domain capable of binding to an epitope of human and non-chimpanzee primate CD3[epsilon] chain comprises a VH region comprising CDR-H1, CDR-H2 and CDR-H3 selected from:

(a) CDR-H1 as depicted in SEQ ID NO:12, CDR-H2 as depicted in SEQ ID NO:13 and CDR-H3 as depicted in SEQ ID NO:14;
(b) CDR-H1 as depicted in SEQ ID NO:30, CDR-H2 as depicted in SEQ ID NO:31 and CDR-H3 as depicted in SEQ ID NO:32;
(c) CDR-H1 as depicted in SEQ ID NO:48, CDR-H2 as depicted in SEQ ID NO:49 and CDR-H3 as depicted in SEQ ID NO:50;
(d) CDR-H1 as depicted in SEQ ID NO:66, CDR-H2 as depicted in SEQ ID NO:67 and CDR-H3 as depicted in SEQ ID NO:68;
(e) CDR-H1 as depicted in SEQ ID NO:84, CDR-H2 as depicted in SEQ ID NO:85 and CDR-H3 as depicted in SEQ ID NO:86;
(f) CDR-H 1 as depicted in SEQ ID NO:102, CDR-H2 as depicted in SEQ ID NO:103 and CDR-H3 as depicted in SEQ ID NO:104;
(g) CDR-H1 as depicted in SEQ ID NO:120, CDR-H2 as depicted in SEQ ID NO:121 and CDR-H3 as depicted in SEQ ID NO:122;
(h) CDR-H1 as depicted in SEQ ID NO:138, CDR-H2 as depicted in SEQ ID NO:139 and CDR-H3 as depicted in SEQ ID NO:140;
(i) CDR-H 1 as depicted in SEQ ID NO:156, CDR-H2 as depicted in SEQ ID NO:157 and CDR-H3 as depicted in SEQ ID NO:158; and
(j) CDR-H 1 as depicted in SEQ ID NO:174, CDR-H2 as depicted in SEQ ID NO:175 and CDR-H3 as depicted in SEQ ID NO:176.

(7) The polypeptide according to any one of items **1** to **5,** wherein the first binding domain capable of binding to an epitope of human and non-chimpanzee primate CD3[epsilon] chain comprises a VL region selected from the group consisting of a VL region as depicted in SEQ ID NO:35, 39, 125, 129, 161 or 165.

(8) The polypeptide according to any one of items 1 to 4 and 6, wherein the binding domain capable of binding to an epitope of human and non-chimpanzee primate CD3[epsilon] chain comprises a VH region selected from the group consisting of a VH region as depicted in SEQ ID NO:15, 19, 33, 37, 51, 55, 69, 73, 87, 91, 105, 109, 123, 127, 141, 145, 159, 163, 177 or 181.

(9) The polypeptide according to any one of items **1** to **8,** wherein the first binding domain capable of binding to an epitope of human and non-chimpanzee primate CD3[epsilon] chain comprises a VL region and a VH region selected from the group consisting of:

(a) a VL region as depicted in SEQ ID NO:17 or 21 and a VH region as depicted in SEQ ID NO:15 or 19;
(b) a VL region as depicted in SEQ ID NO:35 or 39 and a VH region as depicted in SEQ ID NO:33 or 37;
(c) a VL region as depicted in SEQ ID NO:53 or 57 and a VH region as depicted in SEQ ID NO:51 or 55;
(d) a VL region as depicted in SEQ ID NO:71 or 75 and a VH region as depicted in SEQ ID NO:69 or 73;
(e) a VL region as depicted in SEQ ID NO:89 or 93 and a VH region as depicted in SEQ ID NO:87 or 91;
(f) a VL region as depicted in SEQ ID NO:107 or 111 and a VH region as depicted in SEQ ID NO:105 or 109;
(g) a VL region as depicted in SEQ ID NO:125 or 129 and a VH region as depicted in SEQ ID NO:123 or 127;
(h) a VL region as depicted in SEQ ID NO:143 or 147 and a VH region as depicted in SEQ ID NO:141 or 145;
(i) a VL region as depicted in SEQ ID NO:161 or 165 and a VH region as depicted in SEQ ID NO:159 or 163; and
(j) a VL region as depicted in SEQ ID NO:179 or 183 and a VH region as depicted in SEQ ID NO:177 or 181.

(10) The polypeptide according to item **9,** wherein the first binding domain capable of binding to an epitope of human and non-chimpanzee primate CD3[epsilon] chain comprises an amino acid sequence selected from the group consisting of SEQ ID NOs:23, 25, 41, 43, 59, 61, 77, 79, 95, 97, 113, 115, 131, 133, 149, 151, 167, 169, 185 or 187.

(11) The polypeptide according to any one of items **3** to **10,** wherein the cell surface antigen is a tumor antigen.

(12) The polypeptide according to any one of items **3** to **10,** wherein the cell surface antigen is selected from EGFR, EGFRvIII, MCSP, Carbonic anhydrase IX, CD30, CD33, CD44v6, EpCAM, Her2/neu, MUC1, IgE, HBV and HCV.

(13) The polypeptide of any one of items **3** to **12,** wherein said polypeptide is a bispecific single chain antibody molecule.

(14) The polypeptide according to item **13,** wherein the bispecific single chain antibody molecule comprises a group of the following sequences as CDR H1, CDR H2, CDR H3, CDR L1, CDR L2 and CDR L3 in the second binding domain selected from the group consisting of:

SEQ ID NOs:189-194, SEQ ID NOs:201-206, SEQ ID NOs:219-224, SEQ ID NOs:237-242, SEQ ID NOs:255-260, SEQ ID NOs:273-279, SEQ ID NOs:382-384 and 387-389, SEQ ID NOs:400-402 and 405-407, SEQ ID NOs:418-420 and 423-425, SEQ ID NOs:436-438 and 441-443, SEQ ID NOs:454-456 and 459-461, SEQ ID NOs:472-474 and 477-479, SEQ ID NOs:490-492 and 495-497, SEQ ID NOs:508-510 and 513-515, SEQ ID NOs:530-532 and 1568 to 1570, SEQ ID NOs:545-547 and 550-552, SEQ ID NOs:559-561 and 564-566, SEQ ID NOs:577-579 and 582-584,
SEQ ID NOs:615-617 and 620-622, SEQ ID NOs:633-635, 638, 639 and 1571 , SEQ ID NOs:650-652 and 655-657, SEQ ID NOs:668-670 and 673-675, SEQ ID NOs:682-684 and 687-689, SEQ ID NOs:696-698 and 701-703, SEQ ID NOs:710-712 and 715-717, SEQ ID NOs:724-726 and 729-731, SEQ ID NOs:738-740 and 743-745, SEQ ID NOs:752-754 and 757-759, SEQ ID NOs:766- 768 and 771-773, SEQ ID NOs:780-782 and 785-787, SEQ ID NOs:794-796 and 799-801, SEQ ID NOs:808-810 and 813-815, SEQ ID NOs:822-824 and 827-829, SEQ ID NOs:836-838 and 841-843, SEQ ID NOs:850-852 and 855- 857,
SEQ ID NOs:866-868 and 871-873, SEQ ID NOs:884-886 and 889-891, SEQ ID NOs:902-904 and 907-909, SEQ ID NOs:920-922 and 925-927, SEQ ID NOs:938-940 and 943-945, SEQ ID NOs:956-958 and 961-963, SEQ ID NOs:974-976 and 979-981 , SEQ ID NOs:992-994 and 997-999, SEQ ID NOs:1006- 1008 and 1011 -1013, SEQ ID NOs:1020-1022 and 1025-1027, SEQ ID NOs:1034-1036 and 1039-1041 , SEQ ID NOs:1048-1050 and 1053-1055, SEQ ID NOs:1062-1064 and 1067-1069, SEQ ID NOs:1076-1078 and 1081-1083, SEQ ID NOs:1090-1092 and 1095-1097,
SEQ ID NOs:1114-1116 and 1119-1121, SEQ ID NOs:1128-1130 and 1133-1135, SEQ ID NOs:1141-1143 and 1146-1148, SEQ ID NOs:1155-1157 and 1160-1162, SEQ ID NOs:1169-1171 and 1174-1176, SEQ ID NOs:1183-1185 and 1188-1190, SEQ ID NOs:1197-1199 and 1202-1204, SEQ ID NOs:1211 -1213 and 1216-1218, SEQ ID NOs:1125-1227 and 1230-1232, SEQ ID NOs:1239-1241 and 1244-1246, SEQ ID NOs:, 1253-1255 and 1258-1260, SEQ ID NOs:1267-1269 and 1272-1274, SEQ ID NOs:1281-1283 and 1286-1288, SEQ ID NOs:1295-1297 and 1300-1302, SEQ ID NOs:1309-1311 and 1314-1316, SEQ ID NOs:1323-1325 and 1328-1330
SEQ ID NOs:1343-1345 and 1348-1350, SEQ ID NOs:1361-1363 and 1366-1368, SEQ ID NOs:1375-1377 and 1380-1382, SEQ ID NOs:1389- 1391 and 1394-1396, SEQ ID NOs:1403-1405 and 1408-1410, SEQ ID NOs:1417-1419 and 1422-1424, SEQ ID NOs:1431-1433 and 1436-1438, SEQ ID NOs:1445-1447 and 1450-1452, SEQ ID NOs:1459-1461 and 1464-1466, SEQ ID NOs:1473-1475 and 1478-1480,
SEQ ID NOs:1486-1491, SEQ ID NOs:1492-1497,
SEQ ID NOs:1505-1507 and 1510-1512, SEQ ID NOs:1531-1533 and 1536-1538,
SEQ ID NOs:1573-1575 and 1578-1580, SEQ ID NOs:1591-1593 and 1596-1598, SEQ ID NOs:1605-1607 and 1610-1612, SEQ ID NOs:1619-1621 and 1624-1626, SEQ ID NOs:1634-1636 and 1639-1641 and SEQ ID NOs:1652- 1654, SEQ ID NOs:1657-1659 and SEQ ID NOs:1669-1674.

(15) The polypeptide according to item **13,** wherein the bispecific single chain antibody molecule comprises a sequence selected from:

(a) an amino acid sequence as depicted in any of SEQ ID NOs:291, 293, 295, 297, 299, 301, 303, 305, 307, 309, 311, 313, 315, 317, 319, 321, 323, 325, 327, 329, 331, 333, 335, 337, 339, 341, 343, 345, 347, 393, 395, 397, 411, 413, 415, 429, 431, 433, 447, 449, 451, 465, 467, 469, 483, 485, 487, 501, 503, 505, 519, 521, 523, 1336, 1338, 1340, 538, 540, 542, 556, 570, 572, 574, 588, 590, 592, 626, 628, 630, 643, 645, 647, 661, 663, 665, 679, 693, 707, 721, 735, 749, 763, 777, 791, 805, 819, 833, 847, 861, 863, 877, 879, 881, 895, 897, 899, 913, 915, 917, 931, 933, 935, 949, 951, 953, 967, 969, 971, 985, 987, 989, 1003, 1017, 1031, 1045, 1059, 1073, 1087, 1101, 1125, 1138, 1152, 1166, 1180, 1194, 1208, 1222, 1236, 1250, 1264, 1278, 1292, 1306, 1320, 1334, 1354, 1356, 1358, 1372, 1386, 1400, 1414, 1428, 1442, 1456, 1470, 1484, 1500, 1502, 1518, 1520, 1522, 1524, 1526, 1528, 1544, 1546, 1548, 1550, 1552, 1554, 1556, 1558, 1560, 1562, 1564, 1566, 1586, 1588, 1602, 1616, 1630,1647, 1649 or 1663; and
(b) an amino acid sequence encoded by a nucleic acid sequence as depicted in any of SEQ ID NOs:292, 294, 296, 298, 300, 302, 304, 306, 308, 310, 312, 314, 316, 318, 320, 322, 324, 326, 328, 330, 332, 334, 336, 338, 340, 342, 344, 346, 348, 394, 396, 398, 412, 414, 416, 430, 432, 434, 448, 450, 452, 466, 468, 470, 484, 486, 488, 502, 504, 506, 520, 522, 524, 1337, 1339, 1341, 539, 541, 543, 557, 571, 573, 575, 589, 591, 593, 627, 629, 631, 644, 646, 648, 662, 664, 666, 680, 694, 708, 722, 736, 750, 764, 778, 792, 806, 820, 834, 848, 862, 864, 878, 880, 882, 896, 898, 900, 914, 916, 918, 932, 934, 936, 950, 952, 954, 968, 970, 972, 986, 988, 990, 1004, 1018, 1032, 1046, 1060, 1074, 1088, 1102, 1126, 1139, 1153, 1167, 1181, 1195, 1209, 1223, 1237, 1251, 1265, 1279, 1293, 1307, 1321, 1335, 1355, 1357, 1359, 1373, 1387, 1401, 1415, 1429, 1443, 1457, 1471, 1485, 1501, 1503, 1519, 1521, 1523, 1525, 1527, 1529, 1545, 1547, 1549, 1551, 1553, 1555, 1557, 1559, 1561, 1563, 1565, 1567, 1587, 1589, 1603, 1617, 1631, 1648, 1650 or 1664.

(16) A nucleic acid sequence encoding a polypeptide as defined in any of items **1** to **15.**

(17) A vector, which comprises a nucleic acid sequence as defined in item **16.**

(18) The vector of item **17,** wherein said vector further comprises a regulatory sequence, which is operably linked to said nucleic acid sequence defined in item **16.**

(19) The vector of item **18,** wherein said vector is an expression vector.

(20) A host transformed or transfected with a vector defined in any one of items **17** to **19.**

(21) A process for the production of a polypeptide according to any of items **1** to **15,** said process comprising culturing a host defined in item **20** under conditions allowing the expression of the polypeptide as defined in any of items **1** to **15** and recovering the produced polypeptide from the culture.

(22) A pharmaceutical composition comprising a polypeptide according to any one of items 1 to 15, or produced according to the process of item **21.**

(23) The pharmaceutical composition of item **22,** which is further comprising suitable formulations of carriers, stabilizers and/or excipients.

(24) A pharmaceutical composition comprising a polypeptide according to any one of items **1** to **15,** or produced according to the process of item **21** for the prevention, treatment or amelioration of a disease selected from a proliferative disease, a tumorous disease, or an immunological disorder.

(25) The pharmaceutical composition of item **24,** which is further comprising suitable formulations of carriers, stabilizers and/or excipients.

(26) Use of a polypeptide as defined in any of items **1** to **15,** or produced according to item **21,** for the preparation of a pharmaceutical composition for the prevention, treatment or amelioration of a disease selected from a proliferative disease, a tumorous disease, or an immunological disorder.

(27) The use according to item **26,** wherein said tumorous disease is a malignant disease.

(28) The use according to item **26** or **27,** wherein said pharmaceutical composition is suitable to be administered in combination with an additional drug.

(29) The use of item **28,** wherein said drug is a non-proteinaceous compound or a proteinaceous compound.

(30) The use of item **29,** wherein said proteinaceous compound or non- proteinaceous compound is administered simultaneously or non-simultaneously with a pharmaceutical composition according to item **22** or **23.**

(31) A method for the prevention, treatment or amelioration of a disease in a subject in the need thereof, said method comprising the step of administration of an effective amount of a pharmaceutical composition of item **22** or **23.**

(32) The method of item **31,** wherein said disease is a proliferative disease, a tumorous disease, or an immunological disorder.

(33) The method of item **32,** wherein said tumorous disease is a malignant disease.

(34) The method of any of items **31** to **33,** wherein said pharmaceutical composition is administered in combination with an additional drug.

(35) The method of item **34,** wherein said drug is a non-proteinaceous compound or a proteinaceous compound.

(36) The method of item **35,** wherein said proteinaceous compound or non-proteinaceous compound is administered simultaneously or non-simultaneously with a pharmaceutical composition according to item **22** or **23.**

(37) The method of any one of items **31** to **36,** wherein said subject is a human.

(38) A kit comprising a polypeptide as defined in any of items **1** to **15,** a nucleic acid molecule as defined in item 16, a vector as defined in any one of items **17** to **19,** or a host as defined in item **20.**

(39) A method for the identification of (a) polypeptide(s) comprising a cross-species specific binding domain capable of binding to an epitope of human and non- chimpanzee primate CD3 ε (CD3 epsilon), the method comprising the steps of:

(a) contacting the polypeptide(s) with an N-terminal fragment of the extracellular domain of CD3[epsilon] of maximal 27 amino acids comprising the amino acid sequence Gln-Asp-Gly-Asn-Glu-Glu-Met-Gly (SEQ ID NO:381) or Gln-Asp- Gly-Asn-Glu-Glu-Ile-Gly (SEQ ID NO:382), fixed via its C-terminus to a solid phase;
(b) eluting the bound polypeptide(s) from said fragment; and
(c) isolating the polypeptide(s) from the eluate of (b).

(40) The method of item **1,** wherein the polypeptide(s) comprise(s) the identified binding domain as a first binding domain and a second binding domain binding to a cell surface antigen.

## Claims

1. A polypeptide comprising a binding domain which is an antibody capable of binding to an epitope of human and Callithrix jacchus, Saguinus oedipus or Saimiri sciureus CD3ε chain, wherein the epitope is part of an amino acid sequence comprised in the group consisting of SEQ ID NOs:2, 4, 6, or 8 and comprises at least the amino acid sequence Gln-Asp-Gly-Asn-Glu,

   wherein the antibody is a scFv,
   wherein said scFv does not comprise the CDR-L1 amino acid sequence RSSTGAVTTSNYAN, the CDR-L2 amino acid sequence GTNKRAP, the CDR-L3 amino acid sequence ALWYSNLWV, the CDR-H1 amino acid sequence TYAMN, the CDR-H2 amino acid sequence RIRSKYNNYATYYADSVKD and the CDR-H3 amino acid sequence HGNFGNSYVSWFAY or CDR-H3 comprising the amino acid sequence VSWFAY, wherein said CDRs are defined according to the Kabat numbering scheme, and
   wherein the polypeptide further comprises a second binding domain capable of binding to a cell surface antigen which is a tumor antigen.

2. A polypeptide comprising a binding domain which is an antibody capable of binding to an epitope of human and Callithrix jacchus, Saguinus oedipus or Saimiri sciureus CD3ε chain, wherein the epitope is part of an amino acid sequence comprised in the group consisting of SEQ ID NOs:2, 4, 6, or 8 and comprises at least the amino acid sequence Gln-Asp-Gly-Asn-Glu, wherein the antibody is a scFv or a single domain antibody, wherein the first binding domain comprises a VL region comprising CDR-L1, CDR-L2 and CDR-L3 selected from:

   (a) CDR-L1 as depicted in SEQ ID NO:27, CDR-L2 as depicted in SEQ ID NO:28 and CDR-L3 as depicted in SEQ ID NO:29;
   (b) CDR-L1 as depicted in SEQ ID NO:117, CDR-L2 as depicted in SEQ ID NO:118 and CDR-L3 as depicted in SEQ ID NO:119; and
   (c) CDR-L1 as depicted in SEQ ID NO:153, CDR-L2 as depicted in SEQ ID NO:154 and CDR-L3 as depicted in SEQ ID NO:155,

and wherein the polypeptide further comprises a second binding domain capable of binding to a cell surface antigen which is a tumor antigen.

3. A polypeptide comprising a binding domain which is an antibody capable of binding to an epitope of human and Callithrix jacchus, Saguinus oedipus or Saimiri sciureus CD3ε chain, wherein the epitope is part of an amino acid sequence comprised in the group consisting of SEQ ID NOs:2, 4, 6, or 8 and comprises at least the amino acid sequence Gln-Asp-Gly-Asn-Glu, wherein the antibody is a scFv or a single domain antibody,, wherein the first binding domain comprises a VH region comprising CDR-H1, CDR-H2 and CDR-H3 selected from:

> (a) CDR-H1 as depicted in SEQ ID NO:12, CDR-H2 as depicted in SEQ ID NO:13 and CDR-H3 as depicted in SEQ ID NO:14;
> (b) CDR-H1 as depicted in SEQ ID NO:30, CDR-H2 as depicted in SEQ ID NO:31 and CDR-H3 as depicted in SEQ ID NO:32;
> (c) CDR-H1 as depicted in SEQ ID NO:48, CDR-H2 as depicted in SEQ ID NO:49 and CDR-H3 as depicted in SEQ ID NO:50;
> (d) CDR-H1 as depicted in SEQ ID NO:66, CDR-H2 as depicted in SEQ ID NO:67 and CDR-H3 as depicted in SEQ ID NO:68;
> (e) CDR-H1 as depicted in SEQ ID NO:84, CDR-H2 as depicted in SEQ ID NO:85 and CDR-H3 as depicted in SEQ ID NO:86;
> (f) CDR-H 1 as depicted in SEQ ID NO:102, CDR-H2 as depicted in SEQ ID NO:103 and CDR-H3 as depicted in SEQ ID NO:104;
> (g) CDR-H1 as depicted in SEQ ID NO:120, CDR-H2 as depicted in SEQ ID NO:121 and CDR-H3 as depicted in SEQ ID NO:122;
> (h) CDR-H1 as depicted in SEQ ID NO:138, CDR-H2 as depicted in SEQ ID NO:139 and CDR-H3 as depicted in SEQ ID NO:140;
> (i) CDR-H 1 as depicted in SEQ ID NO:156, CDR-H2 as depicted in SEQ ID NO:157 and CDR-H3 as depicted in SEQ ID NO:158; and
> (j) CDR-H 1 as depicted in SEQ ID NO:174, CDR-H2 as depicted in SEQ ID NO:175 and CDR-H3 as depicted in SEQ ID NO:176,

and wherein the polypeptide further comprises a second binding domain capable of binding to a cell surface antigen which is a tumor antigen.

4. The polypeptide according to claim 2, wherein the first binding domain comprises a VL region selected from the group consisting of a VL region as depicted in SEQ ID NO:35, 39, 125, 129, 161 or 165.

5. The polypeptide according to claim 3, wherein the first binding domain comprises a VH region selected from the group consisting of a VH region as depicted in SEQ ID NO:15, 19, 33, 37, 51, 55, 69, 73, 87, 91, 105, 109, 123, 127, 141, 145, 159, 163, 177 or 181.

6. The polypeptide according to any one of claims 2 to 5, wherein the first binding comprises a VL region and a VH region selected from the group consisting of:

> (a) a VL region as depicted in SEQ ID NO:17 or 21 and a VH region as depicted in SEQ ID NO:15 or 19;
> (b) a VL region as depicted in SEQ ID NO:35 or 39 and a VH region as depicted in SEQ ID NO:33 or 37;
> (c) a VL region as depicted in SEQ ID NO:53 or 57 and a VH region as depicted in SEQ ID NO:51 or 55;
> (d) a VL region as depicted in SEQ ID NO:71 or 75 and a VH region as depicted in SEQ ID NO:69 or 73;
> (e) a VL region as depicted in SEQ ID NO:89 or 93 and a VH region as depicted in SEQ ID NO:87 or 91;
> (f) a VL region as depicted in SEQ ID NO:107 or 111 and a VH region as depicted in SEQ ID NO:105 or 109;
> (g) a VL region as depicted in SEQ ID NO:125 or 129 and a VH region as depicted in SEQ ID NO:123 or 127;
> (h) a VL region as depicted in SEQ ID NO:143 or 147 and a VH region as depicted in SEQ ID NO:141 or 145;
> (i) a VL region as depicted in SEQ ID NO:161 or 165 and a VH region as depicted in SEQ ID NO:159 or 163; and
> (j) a VL region as depicted in SEQ ID NO:179 or 183 and a VH region as depicted in SEQ ID NO:177 or 181.

7. The polypeptide according to claim 6, wherein the first binding domain comprises an amino acid sequence selected from the group consisting of SEQ ID NOs:23, 25, 41, 43, 59, 61, 77, 79, 95, 97, 113, 115, 131, 133, 149, 151, 167, 169, 185 or 187.

8. The polypeptide of any one of claims 1 to 7, wherein said second binding domain is an antibody.

9. The polypeptide of any one of claims 1 to 8, wherein said antibody is humanized.

10. The polypeptide of any one of claims 1 to 9, wherein said polypeptide is a bispecific single chain antibody molecule.

11. A nucleic acid sequence encoding a polypeptide as defined in any of claims 1 to 10.

12. A vector, which comprises a nucleic acid sequence as defined in claim 11.

13. A host cell transformed or transfected with a vector defined in claim 12.

14. A process for the production of a polypeptide according to any of claims 1 to 10, said process comprising culturing a host cell defined in claim 13 under conditions allowing the expression of the polypeptide as defined in any of claims 1 to 10 and recovering the produced polypeptide from the culture.

15. A pharmaceutical composition comprising a polypeptide according to any one of claims 1 to 10, or produced according to the process of claim 14.

16. The polypeptide according to any one of claims 1 to 10, or produced according to the process of claim 14 for use in the prevention, treatment or amelioration of a disease selected from a proliferative disease, a tumorous disease, or an immunological disorder.

17. A kit comprising a polypeptide as defined in any of claims 1 to 10, a nucleic acid molecule as defined in claim 11, a vector as defined in any one of claims 12, or a host cell as defined in claim 13.

# Figure 1

N-terminal amino acids 1-27 of the primate CD3 epsilon (e.g. human)

Hinge region

Human IgG 1 Fc region

Hexa Histidin tag

Figure 2

Figure 3

EP 4 059 964 A1

# Figure 4

N-terminal amino acids 1-27 of the
primate CD3 epsilon (e.g. human)

Flag tagged full-length cynomolgus
EpCAM (membrane bound)

Cell membrane

Cellular cytoplasm

Figure 5

Figure 6A

Figure 6B

EP 4 059 964 A1

Marmoset 27 mer – H2C HLP

Marmoset 27 mer – F12Q HLP

Marmoset 27 mer – E2M HLP

Marmoset 27 mer – G4H HLP

Figure 6C

Tamarin 27 mer – H2C HLP

Tamarin 27 mer – E2M HLP

Tamarin 27 mer – F12Q HLP

Tamarin 27 mer – G4H HLP

Figure 6D

Squirrel monkey 27 mer – H2C HLP

Squirrel monkey 27 mer – F12Q HLP

Squirrel monkey 27 mer – E2M HLP

Squirrel monkey 27 mer – G4H HLP

EP 4 059 964 A1

Figure 6E

Swine 27 mer – H2C HLP

Swine 27 mer – F12Q HLP

Swine 27 mer – E2M HLP

Swine 27 mer – G4H HLP

Figure 7

human CD3 WT in EL4

EP 4 059 964 A1

**Figure 8A**

A2J HLP

Figure 8B

Figure 8C

F12Q HLP

EP 4 059 964 A1

Figure 9

**Figure 10**

Figure 11a

EP 4 059 964 A1

**Figure: 11b**

**Figure: 11c**

Figure: 11d

**Figure: 11e**

# Figure: 11f

EP 4 059 964 A1

**Figure: 11g**

Figure: 11h

Figure: 11i

146

**Figure 12**

**A)**

Effector cells: stimulated CD4/CD56 depleted human PBMC
Target cells: CHO transfected with human EGFR

**B)**

Effector cells: stimulated CD4/CD56 depleted human PBMC
Target cells: CHO transfected with human EGFR

Figure 13 a

**A)**

Effector cells: macaque T cell line 4119 LnPx
Target cells: CHO transfected with cynomolgus EGFR

**B)**

Effector cells: macaque T cell line 4119 LnPx
Target cells: CHO transfected with cynomolgus EGFR

Figure 13b

A)   Effector cells: stimulated CD4/CD56 depleted human PBMC
     Target cells: CHO transfected with human EGFR

B)   Effector cells: 4119LnPx
     Target cells: CHO transfected with cyno EGFR

Figure 13c

A)    Effector cells: stimulated CD4/CD56 depleted human PBMC
      Target cells: CHO transfected with human EGFR

B)    Effector cells: 4119LnPx
      Target cells: CHO transfected with cyno EGFR

Figure 13d

A)    Effector cells: stimulated CD4/CD56 depleted human PBMC
      Target cells: CHO transfected with human EGFR

B)    Effector cells: 4119LnPx
      Target cells: CHO transfected with cyno EGFR

Figure 13e

A)  Effector cells: stimulated CD4/CD56 depleted human PBMC
    Target cells: CHO transfected with human EGFR

B)  Effector cells: 4119LnPx
    Target cells: CHO transfected with cyno EGFR

**Figure 13f**

**A)** **Effector cells: stimulated CD4/CD56 depleted human PBMC**
**Target cells: CHO transfected with human EGFR**

- – – • E98C3 HL x H2C HL
- ·····△ E98C3 HL x F12Q HL
- ——■ E98C3 HL x I2C HL

**B)** **Effector cells: 4119LnPx**
**Target cells: CHO transfected with cyno EGFR**

- ·····△ E98C3 HL x H2C HL
- ——■ E98C3 HL x F12Q HL
- – – • E98C3 HL x I2C HL

**Figure 13g**

**A)** Effector cells: stimulated CD4/CD56 depleted human PBMC
Target cells: CHO transfected with human EGFR

**B)** Effector cells: 4119LnPx
Target cells: CHO transfected with cyno EGFR

. **Figure 13h**

**A)** **Effector cells: stimulated CD4/CD56 depleted human PBMC**
**Target cells: CHO transfected with human EGFR**

**B)** **Effector cells: 4119LnPx**
**Target cells: CHO transfected with cyno EGFR**

Figure 13i

A) Effector cells: stimulated CD4/CD56 depleted human PBMC
Target cells: CHO transfected with human EGFR

B) Effector cells: 4119LnPx
Target cells: CHO transfected with cyno EGFR

Figure 13j

A)  Effector cells: stimulated CD4/CD56 depleted human PBMC
Target cells: CHO transfected with human EGFR

B)  Effector cells: 4119LnPx
Target cells: CHO transfected with cyno EGFR

Figure 14

Figure 15

EP 4 059 964 A1

Figure 16

**Figure 17**

**A)**

Effector cells: stimulated CD4/CD56 depleted human PBMC
Target cells: CHO transfected with human MCSP D3

**B)**

Effector cells: macaque T cell line 4119 LnPx
Target cells: CHO transfected with cynomolgus MCSP D3

Figure 18

**A)**

Effector cells: macaque T cell line 4119 LnPx
Target cells: CHO transfected with cynomolgus MCSP D3

**B)**

Effector cells: macaque T cell line 4119 LnPx
Target cells: CHO transfected with cynomolgus MCSP D3

**Figure 19**

A)

Effector cells: stimulated CD4/CD56 depleted human PBMC
Target cells: CHO transfected with human MCSP D3

B)

Effector cells: stimulated CD4/CD56 depleted human PBMC
Target cells: CHO transfected with human MCSP D3

Figure 20

A)

Effector cells: stimulated CD4/CD56 depleted human PBMC
Target cells: CHO transfected with human MCSP D3

B)

Effector cells: macaque T cell line 4119 LnPx
Target cells: CHO transfected with cynomolgus MCSP D

Figure 21

A)
Effector cells: stimulated CD4/CD56 depleted human PBMC
Target cells: CHO transfected with human MCSP D3

B)

Effector cells: macaque T cell line 4119 LnPx
Target cells: CHO transfected with cynomolgus MCSP D3

# Figure 22 (1)

y-axis: specific lysis (100, 90, 80, 70, 60, 50, 40, 30, 20, 10, 0)

x-axis: pg/ml (1E-1, 1E+1, 1E+3, 1E+5, 1E+7)

■ —— G4 VH-VL x I2C VH-VL
▼ ..... G4 VH-VL x I2C VH-VL plasma
● – – – G4 VH-VL x I2C VH-VL plasma 37°C
       G4 VH-VL x I2C VH-VL plasma 4°C
▲ - - - Negative control
◆ ——

# Figure 22 (2)

| | |
|---|---|
| ■—— | G4 VH-VL x H2C VH-VL |
| ▼····· | G4 VH-VL x H2C VH-VL plasma |
| ●--- | G4 VH-VL x H2C VH-VL plasma 37°C |
| | G4 VH-VL x H2C VH-VL plasma 4°C |
| ▲····· | Negative control |
| ◆—— | |

# Figure 22 (3)

- ■—— G4 VH-VL x F12Q VH-VL
- ▾...... G4 VH-VL x F12Q VH-VL plasma
- •――― G4 VH-VL x F12Q VH-VL plasma 37°C
- G4 VH-VL x F12Q VH-VL plasma 4°C
- ▲·····'Negative control
- ◆——

**Pat. 1 (0.5 µg/m²/24h CD19xCD3)**

**Pat. 7 (1.5 µg/m²/24h CD19xCD3)**

**Figure 23a**

**Pat. 23 (15 µg/m$^2$/24h CD19xCD3)**

**Figure 23b**

**Pat. 30 (30 µg/m$^2$/24h CD19xCD3)**

**Figure 23c**

**Pat. 31 (30 µg/m$^2$/24h CD19xCD3)**

**Figure 23d**

Pat. 33 (60 μg/m$^2$/24h CD19xCD3)

**Figure 23e**

**Figure 23f**

A)

Pat. 19 (continuous infusion: 5 µg/m²/24h for 1 day followed by 15 µg/m²/24h CD19xCD3 maintenance)

B)

Pat. 003002 (bolus infusion trial)

**Figure 24**

Pat. 20 (15 µg/m$^2$/24h CD19xCD3)
Ramp initiation

**Figure 25**

**Pat. 13 (15 µg/m²/24h CD19xCD3)**

**Figure 26**

**Figure 27**

Pat. 24 (15 µg/m²/24h CD19xCD3 without HSA)
1st Treatment start

Pat. 24 (15 µg/m²/24h CD19xCD3 with HSA)
2nd Treatment start

Model of T cell adhesion to endothelial cells induced by monovalent binding to context dependent CD3 epitopes

Figure 28

**Figure 29**

**Cytotoxic activity of cynomolgus test material
CD33-AF5 VH-VL x I2C VH-VL**

Target cells: cyno CD33 CHO; effector cells: 4119 LnPx
E:T-ratio 10:1; assay duration: 18 h
EC50: 2.7 ng/ml

## Dose- and time-dependent depletion of blood CD33-monocytes through CD33-AF5 VH-VL x I2C VH-VL

**Figure 30 A**

**Animal 9 (120 µg/m²/24h CD33xCD3)**

**Animal 10 (120 µg/m²/24h CD33xCD3)**

**Figure 30 B (1)**

**Figure 30 B (2)**

**Figure 31**

**Cytotoxic activity of cynomolgus test material
MCSP-G4 VH-VL x I2C VH-VL**

Target cells: cyno MCSP CHO; effector cells: 4119 LnPx
E:T-ratio 10:1; assay duration: 18 h
EC50: 1.9 ng/ml

Figure 32 (1)
### Animal 1 (60 µg/m$^2$/24h MCSP-G4 VH-VL x I2C VH-VL )

### Animal 2 (240 µg/m$^2$/24h MCSP-G4 VH-VL x I2C VH-VL)

**Figure 32 (2)**

## Animal 3 (240 µg/m²/24h MCSP-G4 VH-VL x I2C VH-VL)

## Animal 4 (1000 µg/m²/24h MCSP-G4 VH-VL x I2C VH-VL)

Human CD33 transfected CHO
AH11HLxF12QHL

HPB-ALL
AH11HLxF12QHL

Macaque CD33 transfected CHO
AH11HLxF12QHL

Macaque PBMC
AH11HLxF12QHL

Human CD33 transfected CHO
AH11HLxH2CHL

HPB-ALL
AH11HLxH2CHL

Macaque CD33 transfected CHO
AH11HLxH2CHL

Macaque PBMC
AH11HLxH2CHL

**Figure 33(1)**

Human CD33 transfected CHO
AH11HLxI2CHL

HPB-ALL
AH11HLxI2CHL

Macaque CD33 transfected CHO
AH11HLxI2CHL

Macaque PBMC
AH11HLxI2CHL

Human CD33 transfected CHO
AH3HLxF12QHL

HPB-ALL
AH3HLxF12QHL

Macaque CD33 transfected CHO
AH3HLxF12QHL

Macaque PBMC
AH3HLxF12QHL

Figure 33(2)

Human CD33 transfected CHO
AH3HLxH2CHL

HPB-ALL
AH3HLxH2CHL

Macaque CD33 transfected CHO
AH3HLxH2CHL

Macaque PBMC
AH3HLxH2CHL

Human CD33 transfected CHO
AH3HLxI2CHL

HPB-ALL
AH3HLxI2CHL

Macaque CD33 transfected CHO
AH3HLxI2CHL

Macaque PBMC
AH3HLxI2CHL

**Figure 33(3)**

Figure 33(4)

**Figure 33(5)**

Figure 33(6)

Human CD33 transfected CHO
B10HLxF12QHL

HPB-ALL
B10HLxF12QHL

Macaque CD33 transfected CHO
B10HLxF12QHL

Macaque PBMC
B10HLxF12QHL

Human CD33 transfected CHO
B10HLxH2CHL

HPB-ALL
B10HLxH2CHL

Macaque CD33 transfected CHO
B10HLxH2CHL

Macaque PBMC
B10HLxH2CHL

**Figure 33(7)**

Human CD33 transfected CHO
B10HLxl2CHL

HPB-ALL
B10HLxl2CHL

Macaque CD33 transfected CHO
B10HLxl2CHL

Macaque PBMC
B10HLxl2CHL

Human CD33 transfected CHO
B3HLxF12QHL

HPB-ALL
B3HLxF12QHL

Macaque CD33 transfected CHO
B3HLxF12QHL

Macaque PBMC
B3HLxF12QHL

**Figure 33(8)**

Human CD33 transfected CHO

B3HLxH2CHL

HPB-ALL

B3HLxH2CHL

Macaque CD33 transfected CHO

B3HLxH2CHL

Macaque PBMC

B3HLxH2CHL

Human CD33 transfected CHO

B3HLxI2CHL

HPB-ALL

B3HLxI2CHL

Macaque CD33 transfected CHO

B3HLxI2CHL

Macaque PBMC

B3HLxI2CHL

**Figure 33(9)**

**Figure 33(10)**

**Figure 33(11)**

Figure 33(12)

Effector cells: stimulated CD4/CD56 depleted human PBMC

Target cells: CHO transfected with human CD33

Effector cells: macaque 4119 LnPx

Target cells: CHO transfected with macaque CD33

**Figure 34(1)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(2)**

200

Effector cells: stimulated CD4/CD56 depleted human PBMC

Target cells: CHO transfected with human CD33

Effector cells: macaque 4119 LnPx

Target cells: CHO transfected with macaque CD33

**Figure 34(3)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(4)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(5)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(6)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(7)**

Effector cells: stimulated CD4/CD56 depleted human PBMC

Target cells: CHO transfected with human CD33

Effector cells: macaque 4119 LnPx

Target cells: CHO transfected with macaque CD33

**Figure 34(8)**

Human CAIX transfected CHO

CA9-E8 HL x I2C HL

HPB-ALL

CA9-E8 HL x I2C HL

Macaque CAIX transfected CHO

CA9-E8 HL x I2C HL

4119 LnPx

CA9-E8 HL x I2C HL

**Figure 35a**

Figure 35b

Figure 35c

Human CAIX transfected CHO

CA9-A8 HL x F12Q HL

HPB-ALL

CA9-A8 HL x F12Q HL

Macaque CAIX transfected CHO

CA9-A8 HL x F12Q HL

4119 LnPx

CA9-A8 HL x F12Q HL

**Figure 35d**

Figure 35e

Figure 35f

**Figure 35g**

Effector cells: stimulated human CD4/CD56 depleted human PBMC

Target cells: CHO transfected with human CAIX

E:T ratio: 10:1

Effector cells: 4119 LnPx

Target cells: CHO transfected with macaque CAIX

E:T ratio: 50:1

**Figure 36a**

Effector cells: stimulated human CD4/CD56 depleted human PBMC

Target cells: CHO transfected with human CAIX

E:T ratio: 10:1

Effector cells: 4119 LnPx

Target cells: CHO transfected with macaque CAIX

E:T ratio: 50:1

**Figure 36b**

Effector cells: stimulated human CD4/CD56 depleted human PBMC

Target cells: CHO transfected with human CAIX

E:T ratio: 5:1

Effector cells: 4119 LnPx

Target cells: CHO transfected with macaque CAIX

E:T ratio: 50:1

**Figure 36c**

Figure 37

**Figure 38a: CHO-EpCAM (human)**

Figure 38b: CHO (untransfected)

Counts

Positivity

**Figure 38c: human PBMCs**

Figure 38d: macaque 4119LnPx (CD3+)

# Figure: 39a

EP 4 059 964 A1

Figure: 39b

**Figure: 39c**

Figure: 39d

EP 4 059 964 A1

Figure: 39e

# Figure: 39f

**Figure 40a**

A)   Effector cells: stimulated CD4/CD56 depleted human PBMC
     Target cells: CHO transfected with human EGFRvIII

B)   Effector cells: 4119LnPx
     Target cells: CHO transfected with cyno EGFRvIII

Figure 40b

A)   Effector cells: stimulated CD4/CD56 depleted human PBMC
     Target cells: CHO transfected with human EGFRvIII

B)   Effector cells: 4119LnPx
     Target cells: CHO transfected with cyno EGFRvIII

Figure 40c

A) Effector cells: stimulated CD4/CD56 depleted human PBMC
Target cells: CHO transfected with human EGFRvIII

B) Effector cells: stimulated CD4/CD56 depleted human PBMC
Target cells: CHO transfected with human EGFRvIII

**Figure 40d**

**A)** **Effector cells: stimulated CD4/CD56 depleted human PBMC**
**Target cells: CHO transfected with human EGFRvIII**

— ■ V17C8 HL x I2C HL
------▲ V17B11 HL x I2C HL
---- • V17F11 HL x I2C HL

**B)** **Effector cells: stimulated CD4/CD56 depleted human PBMC**
**Target cells: CHO transfected with human EGFRvIII**

---- ■ V16B7 HL x I2C HL
— ▲ V11A4 HL x I2C HL
— • negativ control

**Figure 40e**

A) **Effector cells: 4119LnPx**
   **Target cells: CHO transfected with cyno EGFRvIII**

B) **Effector cells: 4119LnPx**
   **Target cells: CHO transfected with cyno EGFRvIII**

Figure 40f

A)  Effector cells: 4119LnPx
    Target cells: CHO transfected with cyno EGFRvIII

- ●  V17F11 HL x I2C HL
- ▲  V16B7 HL x I2C HL
- ■  V11A4 HL x I2C HL

B)  Effector cells: 4119LnPx
    Target cells: CHO transfected with cyno EGFRvIII

- ▲  V11G7 HL x I2C HL
- ■  V17B11 HL x I2C HL
- ●  negativ Control

**Figure 40g**

**A)** **Effector cells: stimulated CD4/CD56 depleted human PBMC
Target cells: CHO transfected with human EGFRvIII**

**B)** **Effector cells: 4119LnPx
Target cells: CHO transfected with cyno EGFRvIII**

# Figure: 41a

|  | J558L human IgE | HPB-ALL (human) | J558L Rh IgE | 4119 LnPx (cyno) |
| --- | --- | --- | --- | --- |
| IgE-D4 HL x I2C HL | | | | |
| IgE-D4 HL x H2C HL | | | | |
| IgE-D4 HL x F12Q HL | | | | |

Counts

Fluorescence Intensity

Figure: 41b

**Figure: 41c**

## Figure 42a

**A)**

Effector cells: stimulated CD4/CD56 depleted human PBMC
Target cells: J558L cells transfected with human IgE

□ D4 HL x H2C HL
▼ D4 HL x I2C HL
○ D4 HL x F12Q HL
▲ negative control

**B)**

Effector cells: 4119 LnPx
Target cells: J558L cells transfected with macaque IgE

■ D4 HL x H2C HL
▽ D4 HL x I2C HL
○ D4 HL x F12Q HL
△ negativ control

238

## Figure 42b

A)

Effector cells: stimulated CD4/CD56 depleted human PBMC
Target cells: J558L cells transfected with Human IgE

B)

Effector cells: 4119 LnPx
Target cells: J558L cells transfected with macaque IgE

## Figure 42c

**A)**

Effector cells: stimulated CD4/CD56 depleted human PBMC
Target cells: J558L cells transfected with Human IgE

**B)**

Effector cells: 4119 LnPx
Target cells: J558L cells transfected with macaque IgE

## Figure 42d

**A)**

Effector cells: stimulated CD4/CD56 depleted human PBMC
Target cells: J558L cells transfected with Human IgE

□ G9 LH x I2C HL
▼ G9 LH x F12Q HL
○ G9 LH x H2C HL
▲ negative control

**B)**

Effector cells: 4119 LnPx
Target cells: J558L cells transfected with macaque IgE

△ G9 LH x I2C HL
◇ G9 LH x F12Q HL
● G9 LH x H2C HL
○ negativ control

241

**Figure 42e**

**A)**

Effector cells: stimulated CD4/CD56 depleted human PBMC
Target cells: J558L cells transfected with human IgE

I2C HL x IgE G9 HL
H2C HL x IgE G9 HL
F12Q HL x IgE G9 HL
negative control

specific lysis [%]

dilution of cell culture supernatant [%]

**B)**

Effector cells: 4119 LnPx
Target cells: J558L cells transfected with macaque IgE

I2C HL x IgE G9 HL
F12Q HL x IgE G9 HL
H2C HL x IgE G9 HL
negative control

specific lysis [%]

dilution of cell culture supernatant [%]

## Figure 42f

**A)**

Effector cells: stimulated CD4/CD56 depleted human PBMC
Target cells: J558L cells transfected with Human IgE

**B)**

Effector cells: 4119 LnPx
Target cells: J558L cells transfected with macaque IgE

## Figure 42g

**A)**

Effector cells: stimulated CD4/CD56 depleted human PBMC
Target cells: J558L cells transfected with Human IgE

**B)**

Effector cells: 4119 LnPx
Target cells: J558L cells transfected with macaque IgE

Figure 43

Human CD44 transfected CHO
anti-CD44 antibody

Human CD44 without v6 transfected CHO
anti-CD44 antibody

Human CD44 without v6 transfected CHO
CD44-A8HLxl2CHL

Human CD44 without v6 transfected CHO
CD44-A8LHxl2CHL

**Figure 44**

Effector cells: stimulated CD4/CD56 depleted human PBMC
Target cells: CHO transfected with human CD44

**Figure 45**

Human CD30 transfected CHO
M911HLxI2CHL

HPB-ALL
M911HLxI2CHL

Macaque CD30 transfected CHO
M911HLxI2CHL

Macaque PBMC
M911HLxI2CHL

MEC-1
M911HLxI2CHL

**Figure 46 (1)**

Human CD30 transfected CHO
SR3HLxl2CHL

HPB-ALL
SR3HLxl2CHL

Macaque CD30 transfected CHO
SR3HLxl2CHL

Macaque PBMC
SR3HLxl2CHL

MEC-1
SR3HLxl2CHL

**Figure 46 (2)**

Effector cells: stimulated CD4/CD56 depleted human PBMC

Target cells: MEC-1

Effector cells: macaque 4119 LnPx

Target cells: CHO transfected with macaque CD30

**Figure 47**

Figure 48

Effector cells: unstimulated human CD56 depleted PBMC
Target cells: CHO transfected with human HER2
E:T ratio: 10:1

Figure 49a

Effector cells: 4119 LnPx
Target cells: CHO transfected with macaque HER2
E:T ratio: 10:1

Figure 49b

SDS PAGE of the purification
of E292F3 HL x I2C HL

Corresponding Western blot of the purification
of E292F3 HL x I2C HL

Eluate    SN    FT    M

M    SN    FT    Eluate

◄60 kDa →

◄50 kDa →

◄40 kDa →

**Figure 50**

SDS PAGE of the purification
of V207C12 HL x H2C HL

Corresponding Western blot of the purification
of V207C12 HL x H2C HL

SN    FT    Eluate    M

M    Eluate    FT    SN

◄60 kDa
◄50 kDa
◄40 kDa

**Figure 51**

SDS PAGE of the purification
of AF5HLxF12QHL

Corresponding Western blot of the purification
of AF5HLxF12QHL

SN  FT  Eluate  M

M  Eluate  FT  SN

←60 kDa

←50 kDa

←40 kDa

**Figure 52**

Figure 53

**Figure 54**

Figure 55

Human 1-27 CD3-EpCAM CHO
I2C IgG1 construct

Marmoset 1-27 CD3-EpCAM CHO
I2C IgG1 construct

Tamarin 1-27 CD3-EpCAM CHO
I2C IgG1 construct

Squirrel Monkey 1-27 CD3-EpCAM CHO
I2C IgG1 construct

**Figure 56**

Human CD3-WT in EL4
UCHT1

Human CD3-H6 in EL4
UCHT1

Human CD3-WT in EL4
anti-His Tag

Human CD3-H6 in EL4
anti-His Tag

Human CD3-WT in EL4
I2C IgG1

Human CD3-H6 in EL4
I2C IgG1

**Figure 57**

CHO hum MCSP D3
MCSP-A9 HL x H2C HL

HPBall
MCSP-A9 HL x H2C HL

CHO macaque MCSP D3
MCSP-A9 HL x H2C HL

4119LnPx
MCSP-A9 HL x H2C HL

Fluorescence intensity

**Figure 58a**

CHO hum MCSP D3
MCSP-A9 HL x F12Q HL

HPBall
MCSP-A9 HL x F12Q HL

CHO macaque MCSP D3
MCSP-A9 HL x F12Q HL

4119LnPx
MCSP-A9 HL x F12Q HL

Fluorescence intensity

**Figure 58b**

Figure 58c

CHO hum MCSP D3
MCSP-C8 HL x I2C HL

HPBall
MCSP-C8 HL x I2C HL

CHO macaque MCSP D3
MCSP-C8 HL x I2C HL

4119LnPx
MCSP-C8 HL x I2C HL

Fluorescence intensity

**Figure 58d**

CHO hum MCSP D3

MSCP-B8 HL x I2C HL

HPBall

MSCP-B8 HL x I2C HL

CHO macaque MCSP D3

MSCP-B8 HL x I2C HL

4119 LnPx

MSCP-B8 HL x I2C HL

Fluorescence intensity

**Figure 58e**

Figure 58f

266

Figure 58g

CHO hum MCSP D3

MSCP-F8 HL x I2C HL

HPBall

MSCP-F8 HL x I2C HL

CHO macaque MCSP D3

MSCP-F8 HL x I2C HL

4119 LnPx

MSCP-F8 HL x I2C HL

Fluorescence intensity

**Figure 58h**

CHO hum MCSP D3

MSCP-G5 HL x I2C HL

HPBall

MSCP-G5 HL x I2C HL

CHO macaque MCSP D3

MSCP-G5 HL x I2C HL

4119 LnPx

MSCP-G5 HL x I2C HL

Fluorescence intensity

**Figure 58i**

CHO hum MCSP D3
MSCP-G8 HL x I2C HL

HPBall
MSCP-G8 HL x I2C HL

CHO macaque MCSP D3
MSCP-G8 HL x I2C HL

4119 LnPx
MSCP-G8 HL x I2C HL

Fluorescence intensity

**Figure 58j**

CHO hum MCSP D3
MSCP-B7 HL x I2C HL

HPBall
MSCP-B7 HL x I2C HL

CHO macaque MCSP D3
MSCP-B7 HL x I2C HL

4119 LnPx
MSCP-B7 HL x I2C HL

Fluorescence intensity

**Figure 58k**

CHO hum MCSP D3

MSCP-G10 HL x I2C HL

HPBall

MSCP-G10 HL x I2C HL

CHO hum MCSP D3

MSCP-G10 HL x I2C HL

4119 LnPx

MSCP-G10 HL x I2C HL

Fluorescence intensity

**Figure 58l**

Effector cells: stimulated human CD4/CD56 depleted human PBMC
Target cells: CHO transfected with human MCSP D3
E:T ratio: 10:1

Effector cells: 4119 LnPx
Target cells: CHO transfected with macaque MCSP D3
E:T ratio: 10:1

**Figure 59a**

Effector cells: stimulated human CD4/CD56 depleted human PBMC
Target cells: CHO transfected with human MCSP D3
E:T ratio: 10:1

Effector cells: 4119 LnPx
Target cells: CHO transfected with macaque MCSP D3
E:T ratio: 10:1

**Figure 59b**

Effector cells: stimulated human CD4/CD56 depleted human PBMC
Target cells: CHO transfected with human MCSP D3
E:T ratio: 10:1

Effector cells: 4119 LnPx
Target cells: CHO transfected with macaque MCSP D3
E:T ratio: 10:1

**Figure 59c**

Effector cells: stimulated human CD4/CD56 depleted human PBMC
Target cells: CHO transfected with human MCSP D3
E:T ratio: 10:1

Effector cells: 4119 LnPx
Target cells: CHO transfected with macaque MCSP D3
E:T ratio: 10:1

**Figure 59d**

Human CD33 transfected CHO
I2CHLxAF5HL

HPB-ALL
I2CHLxAF5HL

Macaque CD33 transfected CHO
I2CHLxAF5HL

Macaque PBMC
I2CHLxAF5HL

Human CD33 transfected CHO
H2CHLxAF5HL

HPB-ALL
H2CHLxAF5HL

Macaque CD33 transfected CHO
H2CHLxAF5HL

Macaque PBMC
H2CHLxAF5HL

**Figure 60a**

Human CD33 transfected CHO

F12QHLxAF5HL

HPB-ALL

F12QHLxAF5HL

Macaque CD33 transfected CHO

F12QHLxAF5HL

Macaque PBMC

F12QHLxAF5HL

**Figure 60b**

Effector cells: stimulated CD4/CD56 depleted human PBMC

Target cells: CHO transfected with human CD33

Effector cells: macaque 4119 LnPx

Target cells: CHO transfected with macaque CD33

**Figure 61**

Figure 62

**human CD3 epsilon (aa 1-27) -Fc fusion protein**

**Figure 63**

**human IgG1**

**Figure 64**

Figure 65a

CHO hum CD44
CD44-C2 HL x I2C HL

HPBall
CD44-C2 HL x I2C HL

CHO macaque CD44
CD44-C2 HL x I2C HL

4119LnPx
CD44-C2 HL x I2C HL

Fluorescence intensity

**Figure 65b**

Figure 65c

CHO hum CD44

CD44-F6 HL x I2C HL

HPBall

CD44-F6 HL x I2C HL

CHO macaque CD44

CD44-F6 HL x I2C HL

4119LnPx

CD44-F6 HL x I2C HL

Fluorescence intensity

**Figure 65d**

CHO hum CD44delv6

CD44-A8 LH x I2C HL

CHO hum CD44delv6

CD44-C2 HL x I2C HL

CD44delv6

CD44-H3 HL x I2C HL

CD44delv6

CD44-F6 HL x I2C HL

Fluorescence intensity

**Figure 65e**

287

Effector cells: stimulated human CD4/CD56 depleted human PBMC
Target cells: CHO transfected with human CD44
E:T ratio: 10:1

**Figure 66**

HCV E2 1a transfected CHO
HC1LHxl2CHL

HCV E2 1b transfected CHO
HC1LHxl2CHL

HPB-ALL
HC1LHxl2CHL

4119LnPx
HC1LHxl2CHL

**Figure 67**

Effector cells: stimulated CD4/CD56 depleted human PBMC
Target cells: HCV E2 1b transfected CHO

Effector cells: stimulated CD4/CD56 depleted human PBMC
Target cells: HCV E2 1b transfected CHO

**Figure 68**

| | Europäisches Patentamt European Patent Office Office européen des brevets | **EUROPEAN SEARCH REPORT** | **Application Number** EP 22 15 6910 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2007/042261 A (MICROMET AG [DE]; KISCHEL ROMAN [DE]; RAUM TOBIAS [DE]; SCHLERETH BERN) 19 April 2007 (2007-04-19) * figures 18,19,24, 11 * * examples 17-19, 25 * * figure 26 * * sequences 2, 10, 110, 194 * ----- | 1,8-17 | INV. C07K16/28 C07K16/30 C07K16/32 C07K16/42 C07K16/46 C07K14/725 A61K39/395 A61P35/00 |
| X | BLUMBERG R S ET AL: "Structure of the T-cell antigen receptor: evidence for two CD3 epsilon subunits in the T-cell receptor-CD3 complex.", PROCEEDINGS OF THE NATIONAL ACADEMY OF SCIENCES, vol. 87, no. 18, 1 September 1990 (1990-09-01), pages 7220-7224, XP055948585, ISSN: 0027-8424, DOI: 10.1073/pnas.87.18.7220 * the whole document * ----- | 1,8-17 | |
| X | BROK ET AL: "an extensive monoclonal antibody panal for the phenotyping of leukocyte subsets in the common marmoest and the cotton-top tamarin", CYTOMETRY, vol. 45, 1 January 2001 (2001-01-01), pages 294-303, XP055416225, * page 295, right-hand column * ----- -/-- | 1,8-17 | TECHNICAL FIELDS SEARCHED (IPC) C07K G01N A61K |

The present search report has been drawn up for all claims

| Place of search | Date of completion of the search | Examiner |
|---|---|---|
| The Hague | 5 August 2022 | Bumb, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

# EUROPEAN SEARCH REPORT

**Application Number**

EP 22 15 6910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILSON A D ET AL: "Selection of monoclonal antibodies for the identification of lymphocyte surface antigens in the New World primate Saguinus oedipus oedipus (cotton top tamarin)", JOURNAL OF IMMUNOLOGICAL METHODS, ELSEVIER SCIENCE PUBLISHERS B.V.,AMSTERDAM, NL, vol. 178, no. 2, 27 January 1995 (1995-01-27), pages 195-200, XP004021112, ISSN: 0022-1759, DOI: 10.1016/0022-1759(94)00256-V * table 1 * * page 196, left-hand column * ----- | 1,8-17 | |
| X | CONTAMIN H ET AL: "Flow cytometry identification and characterization of mononuclear cell subsets in the neotropical primate Saimiri sciureus (squirrel monkey)", JOURNAL OF IMMUNOLOGICAL METHODS, ELSEVIER SCIENCE PUBLISHERS B.V.,AMSTERDAM, NL, vol. 297, no. 1-2, 1 February 2005 (2005-02-01), pages 61-71, XP027659114, ISSN: 0022-1759 [retrieved on 2005-02-01] * table 1 * ----- -/-- | 1,8-17 | **TECHNICAL FIELDS SEARCHED (IPC)** |

The present search report has been drawn up for all claims

| Place of search | Date of completion of the search | Examiner |
|---|---|---|
| The Hague | 5 August 2022 | Bumb, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt

European
Patent Office

Office européen
des brevets

# EUROPEAN SEARCH REPORT

**Application Number**

EP 22 15 6910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LYDIE CASSARD ET AL: "Fc gamma receptors and cancer", SPRINGER SEMINARS IN IMMUNOPATHOLOGY, SPRINGER, BERLIN, DE, vol. 28, no. 4, 10 November 2006 (2006-11-10), pages 321-328, XP019459258, ISSN: 1432-2196, DOI: 10.1007/S00281-006-0058-8 * the whole document * | 1-17 | |
| T | CLAUDIA BLUEMEL ET AL: "Epitope distance to the target cell membrane and antigen size determine the potency of T cell-mediated lysis by BiTE antibodies specific for a large melanoma surface antigen", CANCER IMMUNOLOGY, IMMUNOTHERAPY, SPRINGER, BERLIN, DE, vol. 59, no. 8, 23 March 2010 (2010-03-23), pages 1197-1209, XP019842190, ISSN: 1432-0851 * the whole document * | 1-17 | |
| T | STEFFEN DICKOPF ET AL: "Format and geometries matter: Structure-based design defines the functionality of bispecific antibodies", COMPUTATIONAL AND STRUCTURAL BIOTECHNOLOGY JOURNAL, vol. 18, 14 May 2020 (2020-05-14), pages 1221-1227, XP055740966, Sweden ISSN: 2001-0370, DOI: 10.1016/j.csbj.2020.05.006 * the whole document * | 1-17 | **TECHNICAL FIELDS SEARCHED (IPC)** |

-/--

The present search report has been drawn up for all claims

| Place of search | Date of completion of the search | Examiner |
|---|---|---|
| The Hague | 5 August 2022 | Bumb, Peter |

EPO FORM 1503 03.82 (P04C01)

Europäisches
Patentamt
European
Patent Office
Office européen
des brevets

# EUROPEAN SEARCH REPORT

Application Number

EP 22 15 6910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | RODA-NAVARRO PEDRO ET AL: "Understanding the Spatial Topology of Artificial Immunological Synapses Assembled in T Cell-Redirecting Strategies: A Major Issue in Cancer Immunotherapy", FRONTIERS IN CELL AND DEVELOPMENTAL BIOLOGY, vol. 7, 10 January 2020 (2020-01-10), XP55830746, DOI: 10.3389/fcell.2019.00370 * the whole document * | 1-17 | |
| T | MOLEMA G ET AL: "CD3 DIRECTED BISPECIFIC ANTIBODIES INDUCE INCREASED LYMPHOCYTE-ENDOTHELIAL CELL INTERACTIONS IN VITRO", BRITISH JOURNAL OF CANCER, NATURE PUBLISHING GROUP UK, LONDON, vol. 82, no. 2, 1 January 2000 (2000-01-01), pages 472-479, XP000944237, ISSN: 0007-0920, DOI: 10.1054/BJOC.1999.0945 * the whole document * | 1-17 | TECHNICAL FIELDS SEARCHED (IPC) |

The present search report has been drawn up for all claims

| Place of search | Date of completion of the search | Examiner |
|---|---|---|
| The Hague | 5 August 2022 | Bumb, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 6910

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2022

| Patent document cited in search report | Publication date | Patent family member(s) | Publication date |
|---|---|---|---|
| WO 2007042261 A | 19-04-2007 | ------------------------------------- | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9954440 A
- WO 04106380 A
- US 4946778 A
- WO 2004106380 A

- EP 623679 B1
- WO 9420627 A
- WO 9429469 A
- WO 9700957 A
- US 5580859 A
- US 5589466 A

**Non-patent literature cited in the description**

- **DAVIS ; BJORKMAN.** *Nature,* 1988, vol. 334, 395-402
- **CALL.** *Cell,* 2002, vol. 111, 967-979
- **ALARCON.** *Immunol. Rev.,* 2003, vol. 191, 38-46
- **MALISSEN.** *Immunol. Rev.,* 2003, vol. 191, 7-27
- **DAVIS.** *Cell,* 2002, vol. 110, 285-287
- **DAVIS ; VAN DER MERWE.** *Curr. Biol.,* 2001, vol. 11, R289-R291
- **DAVIS.** *Nat. Immunol.,* 2003, vol. 4, 217-224
- **GIL.** *J. Biol. Chem.,* 2001, vol. 276, 11174-11179
- **GIL.** *Cell,* 2002, vol. 109, 901-912
- **BERKHOUT.** *J. Biol. Chem.,* 1988, vol. 263, 8528-8536
- **WANG.** *J. Exp. Med.,* 1998, vol. 188, 1375-1380
- **KAPPES.** *Curr. Opin. Immunol.,* 1995, vol. 7, 441-447
- **SUN.** *Cell,* 2001, vol. 105, 913-923
- **BORROTO.** *J. Biol. Chem.,* 1998, vol. 273, 12807-12816
- **MANOLIOS.** *Eur. J. Immunol.,* 1994, vol. 24, 84-92
- **MANOLIOS ; LI.** *Immunol. Cell Biol.,* 1995, vol. 73, 532-536
- **KJER-NIELSEN.** *PNAS,* 2004, vol. 101, 7675-7680
- **SGRO.** *Toxicology,* 1995, vol. 105, 23-29
- **CHATENOUD.** *Clin. Transplant,* 1993, vol. 7, 422-430
- **CHATENOUD.** *Nat. Rev. Immunol.,* 2003, vol. 3, 123-132
- **KUMAR.** *Transplant. Proc.,* 1998, vol. 30, 1351-1352
- **UTSET.** *J. Rheumatol.,* 2002, vol. 29, 1907-1913

- **SMITH.** *J. Exp. Med.,* 1997, vol. 185, 1413-1422
- **VAN WAUVE.** *J. Immunol.,* 1980, vol. 124, 2708-18
- **WONG.** *Transplantation,* 1990, vol. 50, 683-9
- **TUNNACLIFFE.** *Int. Immunol.,* 1989, vol. 1, 546-50
- **SALMERON.** *J. Immunol.,* 1991, vol. 147, 3047-52
- **VON WUSSOW.** *J. Immunol.,* 1981, vol. 127, 1197
- **PALACIOUS.** *J. Immunol.,* 1982, vol. 128, 337
- **KUNICKA.** *Lymphocyte Typing II,* 1986, vol. 1, 223
- **LEEWENBERG.** *J. Immunol.,* 1985, vol. 134, 3770
- **PHILLIPS.** *J. Immunol.,* 1986, vol. 136, 1579
- **PLATSOUCAS.** *Proc. Natl. Acad. Sci. USA,* 1981, vol. 78, 4500
- **ITOH.** *Cell. Immunol.,* 1987, vol. 108, 283-96
- **MENTZER.** *J. Immunol.,* 1985, vol. 135, 34
- **LANDEGREN.** *J. Exp. Med.,* 1982, vol. 155, 1579
- **CHOI.** *Eur. J. Immunol.,* 2001, vol. 31, 94-106
- **XU.** *Cell Immunol.,* 2000, vol. 200, 16-26
- **KIMBALL.** *Transpl. Immunol.,* 1995, vol. 3, 212-221
- **SELA.** *Science,* 1969, vol. 166, 1365
- **LAVER.** *Cell,* 1990, vol. 61, 553-6
- **LAW.** *Int. Immunol.,* 2002, vol. 14, 389-400
- **COULIE.** *Eur. J. Immunol.,* 1991, vol. 21, 1703-9
- **RISUENO.** *Blood,* 2005, vol. 106, 601-8
- **SANDUSKY et al.** *J. Med. Primatol.,* 1986, vol. 15, 441-451
- **UDA et al.** *J. Med. Primatol.,* 2001, vol. 30, 141-147

- **UDA et al.** *J Med Primatol.,* 2003, vol. 32, 105-10
- **UDA et al.** *J Med Primatol.,* 2004, vol. 33, 34-7
- *Lancet,* 2006, vol. 368, 2206-7
- **WILDMAN et al.** *PNAS,* 2003, vol. 100, 7181
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KOHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **HARLOW ; LANE.** Antibodies: A Laboratory Manual. Cold Spring Harbor Laboratory Press, 1988
- Using Antibodies: A Laboratory Manual. Cold Spring Harbor Laboratory Press, 1999
- **SAMBROOK et al.** Molecular Cloning: A Laboratory Manual. Cold Spring Harbor Laboratory Press, 2001
- **ROONEY, C.M. et al.** Use of gene-modified virus-specific T lymphocytes to control Epstein-Barr-virus-related lymphoproliferation. *Lancet,* 1995, vol. 345 (8941), 9-13
- **WALTER, E.A. et al.** Reconstitution of cellular immunity against cytomegalovirus in recipients of allogeneic bone marrow by transfer of T-cell clones from the donor. *N Engl J Med,* 1995, vol. 333 (16), 1038-44
- **GORELICK, F.S. ; C. SHUGRUE.** Exiting the endoplasmic reticulum. *Mol Cell Endocrinol,* 2001, vol. 177 (1-2), 13-8
- **CHU, C.M. ; Y.F. LIAW.** Membrane staining for hepatitis B surface antigen on hepatocytes: a sensitive and specific marker of active viral replication in hepatitis B. *J Clin Pathol,* 1995, vol. 48 (5), 470-3
- **MASIERO, S. et al.** T-cell engineering by a chimeric T-cell receptor with antibody-type specificity for the HIV-1 gp120. *Gene Ther,* 2005, vol. 12 (4), 299-310
- **MILSTEIN ; KÖHLER.** *Nature,* 1975, vol. 256, 495-7
- **LIU.** *Br. J. Cancer,* 2000, vol. 82 (12), 1991-1999
- **BONNER.** *Semin. Radiat. Oncol.,* 2002, vol. 12, 11-20
- **KIYOTA.** *Oncology,* 2002, vol. 63 (1), 92-98
- **KUAN.** *Brain Tumor Pathol.,* 2000, vol. 17 (2), 71-78
- **UEMURA.** *Br. J. Cancer,* 1999, vol. 81 (4), 741-746
- **LONGCASTER.** *Cancer Res.,* 2001, vol. 61 (17), 6394-6399
- **CHIA.** *J. Clin. Oncol.,* 2001, vol. 19 (16), 3660-3668
- **BEASLEY.** *Cancer Res.,* 2001, vol. 61 (13), 5262-5267
- **ABUTALIB.** *Curr Pharm Biotechnol.,* 2006, vol. 7, 343-69
- **CAMPOLI.** *Crit Rev Immunol.,* 2004, vol. 24, 267-96
- **INFUHR.** *Allergy,* 2005, vol. 60, 977-85
- **OLAYIOYE.** *EMBO J.,* 2000, vol. 19, 3159-67
- **MENDELSOHN.** *J. Clin. Oncol.,* 2003, vol. 21, 2787-99
- **MENDELSOHN.** *J. Clin. Oncol.,* 2002, vol. 20 (18), 1S-13S
- **PREWETT.** *Clin. Cancer Res.,* 2002, vol. 8, 994-1003
- **CIARDIELLO.** *Clin. Cancer Res.,* 2001, vol. 7, 2958-70
- **PEREZ-SOLER.** *Oncologist,* 2004, vol. 9, 58-67
- **LASKIN.** *Cancer Treat. Review,* 2004, vol. 30, 1-17
- **PLUSCHKE.** *PNAS,* 1996, vol. 93, 9710
- **NISHIYAMA.** *J. Cell Biol.,* 1991, vol. 114, 359
- **BURG.** *Cancer Res.,* 1999, vol. 59, 2869
- **LIDA.** *J. Biol. Chem.,* 2001, vol. 276, 18786
- **EISENMANN.** *Nat. Cell Biol.,* 1999, vol. 1, 507
- **LIDA.** *Cancer Res.,* 1995, vol. 55, 2177
- **NATALI.** *J. Natl. Cancer Inst.,* 1984, vol. 72, 13
- **NATALI.** *Cancer Res.,* 1985, vol. 45, 2883
- **REZA ZIAI.** *Cancer Res.,* 1987, vol. 47, 2474
- **KAGESHITA.** *Cancer Res.,* 1991, vol. 51, 1726
- **AUSUBEL.** Current Protocols in Molecular Biology. Green Publishing Associates and Wiley Interscience, 1989
- **MACK et al.** *PNAS,* 1995, vol. 92, 7021-7025
- **RAUM et al.** *Cancer Immunol Immunother,* 2001, vol. 50 (3), 141-150
- **SMITH.** *J. Virol.,* 1983, vol. 46, 584
- **ENGELHARD.** *Proc. Nat. Acad. Sci. USA,* 1994, vol. 91, 3224-3227
- **REISS.** *Plant Physiol. (Life Sci. Adv.),* 1994, vol. 13, 143-149
- **HERRERA-ESTRELLA.** *EMBO J.,* 1983, vol. 2, 987-995
- **MARSH.** *Gene,* 1984, vol. 32, 481-485
- **HARTMAN.** *Proc. Natl. Acad. Sci. USA,* 1988, vol. 85, 8047
- **MCCONLOGUE.** Current Communications in Molecular Biology. Cold Spring Harbor Laboratory, 1987
- **TAMURA.** *Biosci. Biotechnol. Biochem.,* 1995, vol. 59, 2336-2338
- **GIACOMIN.** *Pl. Sci.,* 1996, vol. 116, 59-72

- **SCIKANTHA.** *J. Bact.,* 1996, vol. 178, 121
- **GERDES.** *FEBS Lett.,* 1996, vol. 389, 44-47
- **JEFFERSON.** *EMBO J.,* 1987, vol. 6, 3901-3907
- **GIORDANO.** *Nature Medicine,* 1996, vol. 2, 534-539
- **SCHAPER.** *Circ. Res.,* 1996, vol. 79, 911-919
- **ANDERSON.** *Science,* 1992, vol. 256, 808-813
- **VERMA.** *Nature,* 1994, vol. 389, 239
- **ISNER.** *Lancet,* 1996, vol. 348, 370-374
- **MUHLHAUSER.** *Circ. Res.,* 1995, vol. 77, 1077-1086
- **ONODERA.** *Blood,* 1998, vol. 91, 30-36
- **VERMA.** *Gene Ther.,* 1998, vol. 5, 692-699
- **NABEL.** *Ann. N.Y. Acad. Sci.,* 1997, vol. 811, 289-292
- **VERZELETTI.** *Hum. Gene Ther.,* 1998, vol. 9, 2243-51
- **WANG.** *Nature Medicine,* 1996, vol. 2, 714-716
- **SCHAPER.** *Current Opinion in Biotechnology,* 1996, vol. 7, 635-640
- **NAGY.** *Proc. Natl. Acad. Sci. USA,* 1993, vol. 90, 8424-8428
- **KROOS.** *Biotechnol. Prog.,* 2003, vol. 19, 163-168
- **SCOPES.** Protein Purification. Springer-Verlag, 1982
- **SCHLERETH et al.** *Cancer Immunol. Immunother.,* 2005, vol. 20, 1-12
- **CHESON BD ; HORNING SJ ; COIFFIER B ; SHIPP MA ; FISHER RI ; CONNORS JM ; LISTER TA ; VOSE J ; GRILLO-LOPEZ A ; HAGENBEEK A.** Report of an international workshop to standardize response criteria for non-Hodgkin's lymphomas. NCI Sponsored International Working Group. *J Clin Oncol.,* April 1999, vol. 17 (4), 1244
- **HERMANSON.** Bioconjugate Techniques. Academic Press, Inc, 1996
- Phage Display: A Laboratory Manual. Cold Spring Harbor Laboratory Press, 2001
- **KNAPPIK et al.** *J Mol. Biol.,* 2000, vol. 296, 57
- **HOLT et al.** *Trends Biotechnol.,* 2003, vol. 21, 484
- **MUYLDERMANS.** *J Biotechnol,* vol. 74, 277
- **DE GENST et al.** *Dev Como Immunol.,* 2006, vol. 30, 187
- **GROVES ; OSBOURN.** *Expert Opin Biol Ther.,* 2005, vol. 5, 125
- **LIPOVSEK ; PLUCKTHUN.** *J Immunol Methods,* 2004, vol. 290, 52
- **LEFKOVITS.** Immunology Methods Manual; The Comprehensive Sourcebook of Techniques. Academic Press, 1997
- **GOLEMIS.** Protein-Protein Interactions: A Molecular Cloning Manual. Cold Spring Laboratory Press, 2002

- **GIL et al.** *Cell,* 2002, vol. 109, 901
- **LEGATE et al.** *Nat Rev Mol Cell Biol,* 2006, vol. 7, 20
- **MACK et al.** *Proc. Natl. Acad. Sci. USA,* 1995, vol. 92, 7021-7025
- **RAUM et al.** *Cancer Immunol Immunother.,* 2001, vol. 50 (3), 141-50
- **COLIGAN ; KRUISBEEK ; MARGULIES ; SHEVACH ; STROBER.** Current Protocols in Immunology. Wiley-Interscience, 2002
- **RAUM et al.** *Cancer Immunol. Immunother.,* 2001, vol. 50, 141-150
- **SAMBROOK.** Molecular Cloning; A Laboratory Manual. Cold Spring Harbour Laboratory Press, 2001
- **KAUFMANN R.J.** *Methods Enzymol.,* 1990, vol. 185, 537-566
- **RAUM et al.** *Cancer Immunol Immunother,* 2001, vol. 50, 141-150
- **KNAPPE A ; FICKENSCHER H. et al.** *Blood,* 2000, vol. 95, 3256-61
- **COLIGAN ; KRUISBEEK ; MARGULIES ; SHEVACH ; STROBER.** Current Protocols in Immunology. Wiley-Interscience, 2002
- **PROF. FICKENSCHER.** Virology. Hygiene Institute
- **LOFFLER.** *Blood,* 2000, vol. 95 (6
- **MONACO et al.** *J Leukoc Biol,* 2002, vol. 71, 659-668
- **KAUFMANN R.J.** *Methods Enzymol.,* 1990, vol. 185, 537-566
- **RAUM et al.** *Cancer Immunol Immunother,* 2001, vol. 50, 141
- **KUAN CT ; WIKSTRAND CJ ; BIGNER DD.** EGF mutant receptor vIII as a molecular target in cancer therapy. *Endocr Relat Cancer,* June 2001, vol. 8 (2), 83-96
- **PROF FICKENSCHER.** Virology. Hygiene Institute
- **SCHLERETH.** *Cancer Res,* 2005, vol. 65, 2882

- **BOHNE F ; CHMIELEWSKI M ; EBERT G et al.** T cells redirected against hepatitis B virus surface proteins eliminate infected hepatocytes. *Gastroenterology,* 2008, vol. 134, 239-47
- **BOHNE F ; CHMIELEWSKI M ; EBERT G et al.** T cells redirected against hepatitis B virus surface proteins eliminate infected hepatocytes. *Gastroenterology,* 2008, vol. 134, 239-47
- **BOHNE F ; CHMIELEWSKI M ; EBERT G et al.** T cells redirected against hepatitis B virus surface proteins eliminated infected hepatocytes. *Gastroenterology,* 2008, vol. 134, 239-47